(19) Europäisches Patentamt
European Patent Office
Office européen des brevets

(11) EP 4 694 635 A1

(12) **EUROPEAN PATENT APPLICATION**
published in accordance with Art. 153(4) EPC

(43) Date of publication:
**11.02.2026 Bulletin 2026/07**

(21) Application number: **24780531.0**

(22) Date of filing: **27.03.2024**

(51) International Patent Classification (IPC):
***H10K 50/12*** *(2023.01)* ***C07D 403/14*** *(2006.01)*
***C07D 491/048*** *(2006.01)* ***C09K 11/06*** *(2006.01)*
***H10K 85/60*** *(2023.01)* ***H10K 101/20*** *(2023.01)*
***H10K 101/40*** *(2023.01)*

(52) Cooperative Patent Classification (CPC):
**C07D 403/14; C07D 491/048; C09K 11/06; H10K 50/12; H10K 85/60;** H10K 2101/20; H10K 2101/40

(86) International application number:
**PCT/JP2024/012428**

(87) International publication number:
**WO 2024/204430 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023050649**

(71) Applicant: **Kyulux, Inc.**
**Fukuoka-shi, Fukuoka 819-0388 (JP)**

(72) Inventors:
- **CHO, Yong Joo**
  **Fukuoka-shi, Fukuoka 819-0388 (JP)**
- **MORIO, Momoko**
  **Fukuoka-shi, Fukuoka 819-0388 (JP)**
- **SUZUKI, Yoshitake**
  **Fukuoka-shi, Fukuoka 819-0388 (JP)**
- **HWANG, Songhye**
  **Fukuoka-shi, Fukuoka 819-0388 (JP)**
- **OZAWA, Hiroaki**
  **Fukuoka-shi, Fukuoka 819-0388 (JP)**
- **KANAHARA, Kousei**
  **Fukuoka-shi, Fukuoka 819-0388 (JP)**
- **SUZUKI, Iori**
  **Fukuoka-shi, Fukuoka 819-0388 (JP)**
- **HAMASAKI, Taro**
  **Fukuoka-shi, Fukuoka 819-0388 (JP)**
- **SHIMOI, Yuko**
  **Fukuoka-shi, Fukuoka 819-0388 (JP)**

(74) Representative: **Zimmermann & Partner Patentanwälte mbB**
**P.O. Box 330 920**
**80069 München (DE)**

# (54) ORGANIC LIGHT EMITTING ELEMENT

(57) An organic light-emitting device using a compound of the following general formula has excellent light emission characteristics. $R^1$ to $R^5$ are H, D, an alkyl group, an aryl group, a donor group, a triazinyl group substituted with $Ar^3$ and $Ar^4$, or the like, $R^2$ or $R^3$ is the triazinyl group or the like, two or more of $R^1$ to $R^5$ are donor groups, and $Ar^1$ to $Ar^4$ are an aryl group or a heteroaryl group. At least one of $R^1$ to $R^3$ is a donor group having a fused ring structure of four or more rings, or at least one of $Ar^1$ to $Ar^4$ is a heteroaryl group.

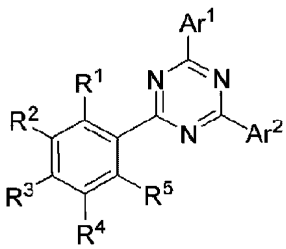

EP 4 694 635 A1

## Description

Technical Field

The present invention relates to an organic light-emitting device such as an organic electroluminescent device.

Background Art

An organic light-emitting device is a light-emitting device using an organic material, which can be produced by coating and which does not use a rare element, and therefore, attention has recently been paid to the organic light-emitting device. Above all, an organic electroluminescent device (organic EL device) emits self-luminous light and does not require a backlight, and is therefore advantageous in that it can be a lightweight and flexible device. In addition, it has features of high responsiveness and high visibility, and is expected as a next generation light source. Consequently, studies relating to development of materials useful for organic light-emitting devices such as organic electroluminescent devices have been promoted actively. In particular, studies relating to light-emitting materials have been carried out actively. Fluorescent materials, phosphorescent materials, and fluorescent materials have been known as light-emitting materials for a long time. However, fluorescent materials have a problem of low light emission efficiency, and phosphorescent materials have a problem of being expensive because they contain rare metals and being difficult to emit deep blue light. In recent years, a delayed fluorescent material has been developed as a light-emitting material that addresses these problems.

A delayed fluorescent material is a material which, in an excited state, after having undergone reverse intersystem crossing from an excited triplet state to an excited singlet state, emits fluorescence when returning back from the excited singlet state to a ground state thereof. Fluorescence through the route is observed later than fluorescence from the excited singlet state directly occurring from the ground state (ordinary fluorescence), and is therefore referred to as delayed fluorescence. Here, for example, in the case where a light-emitting compound is excited through carrier injection thereinto, the occurring probability of the excited singlet state to the excited triplet state is statistically 25%/75%, and therefore improvement of light emission efficiency by the fluorescence alone from the directly occurring excited singlet state is limited. In a delayed fluorescent material, not only the excited singlet state thereof but also the excited triplet state can be utilized for fluorescent emission through the route via the above-mentioned reverse intersystem crossing, and therefore as compared with an ordinary fluorescent material, a delayed fluorescent material can realize a higher light emission efficiency.

Since such a principle has been clarified, various studies have led to the discovery of various delayed fluorescent materials. Among these, many compounds in which a benzene ring is substituted with a donor group and an acceptor group are included. For example, it has been proposed to use a compound having a skeleton, in which the benzene ring is substituted with a carbazol-9-yl group of a donor group and with a cyano group and a substituted triazinyl group of acceptor groups, in an organic light-emitting device (see PTL 1).

Citation List

Patent Literature

PTL 1:WO2019/191665A1

Summary of Invention

Technical Problem

Even when a material emits delayed fluorescence, one having extremely good characteristics and having no problem in practical use has not yet been provided. Therefore, it is more useful if a delayed fluorescent material having more excellent characteristics can be provided. However, the improvement of delayed fluorescent materials is in the stage of trial and error, and it is not easy to generalize the chemical structure of useful light-emitting materials.

Under such circumstances, the present inventors have conducted research for the purpose of providing a compound more useful as a delayed fluorescent material for a light-emitting device. Then, the present inventors have conducted intensive studies for the purpose of deriving and generalizing a general formula of a compound more useful as a delayed fluorescent material and using such a compound in an organic light-emitting device.

Solution to Problem

As a result of intensive studies to achieve the above-mentioned object, the present inventors have found that a

compound having a structure satisfying specific conditions is useful as a light-emitting material and can provide an excellent organic light-emitting device. The present invention has been proposed based on these findings, and specifically has the following configuration.

[1] An organic light-emitting device containing a compound represented by the following general formula (1) and a host material or a dopant material in the same layer:

General Formula (1)

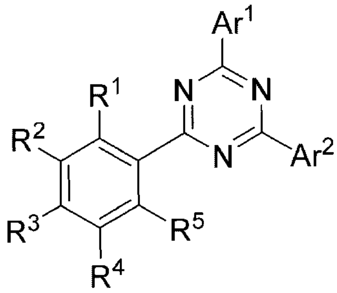

in which $R^1$ to $R^5$ each independently represent a hydrogen atom, a deuterium atom, a substituted or unsubstituted alkyl group, a substituted or unsubstituted aryl group, a donor group, or a group represented by the following formula (2); $R^2$ or $R^3$ is a group represented by the following general formula (2), and two or more of $R^1$ to $R^5$ are donor groups; $Ar^1$ and $Ar^2$ each independently represent a substituted or unsubstituted aryl group, or a substituted or unsubstituted heteroaryl group;

General Formula (2)

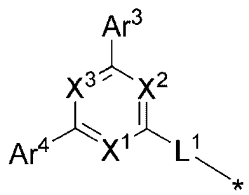

in which $X^1$ to $X^3$ each independently represent N or C(R), and at least one of $X^1$ to $X^3$ is N; R represents a hydrogen atom, a deuterium atom, or a substituent; $Ar^3$ and $Ar^4$ each independently represent a substituted or unsubstituted aryl group, or a substituted or unsubstituted heteroaryl group; $L^1$ represents a single bond or a divalent linking group; * represents a bonding position; and

provided that, in the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions.

[2] The organic light-emitting device according to [1], in which at least one of $R^1$ to $R^5$ in the general formula (1) is a donor group having a fused ring structure of four or more rings.

[3] The organic light-emitting device according to [2], in which at least one of $R^1$ to $R^5$ in the general formula (1) is a donor group represented by the following general formula (3):

General Formula (3)

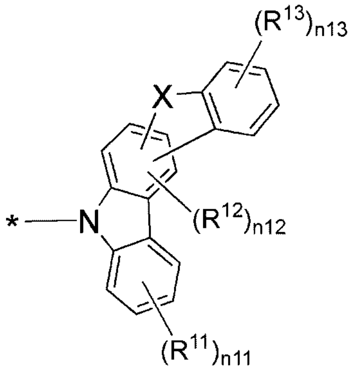

in which X represents O, S or N-$R^{14}$; $R^{11}$ to $R^{13}$ each independently represent a deuterium atom, or a substituent; $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group; $R^{11}$'s, $R^{12}$'s, and $R^{13}$'s each may be bonded to each other to form a cyclic structure; n11 and n13 each independently represent an integer of 0 to 4, and n12 represents an integer of 0 to 2.

[4] The organic light-emitting device according to [2] or [3], in which $Ar^1$ to $Ar^4$ in the general formulae (1) and (2) are each independently a substituted or unsubstituted aryl group.

[5] The organic light-emitting device according to any one of [1] to [3], in which at least one of $Ar^1$ to $Ar^4$ in the general formulae (1) and (2) is a substituted or unsubstituted heteroaryl group (excluding a nitrogen-containing 6-membered ring group).

[6] The organic light-emitting device according to [5], in which at least one of $Ar^1$ to $Ar^4$ in the general formulae (1) and (2) is a heteroaryl group having a 5-membered ring bonded via a nitrogen atom.

[7] The organic light-emitting device according to any one of [1] to [6], in which $R^2$ in the general formula (1) is a group represented by the general formula (2).

[8] The organic light-emitting device according to any one of [1] to [6], in which $R^3$ in the general formula (1) is a group represented by the general formula (2).

[9] The organic light-emitting device according to any one of [1] to [8], in which the donor group is a substituted or unsubstituted carbazol-9-yl group.

[10] The organic light-emitting device according to any one of [1] to [9], in which three of $R^1$ to $R^5$ in the general formula (1) are donor groups.

[11] The organic light-emitting device according to any one of [1] to [10], in which $X^1$ to $X^3$ in the general formula (2) are N.

[12] The organic light-emitting device according to any one of [1] to [11], in which $L^1$ in the general formula (2) is a single bond.

[13] The organic light-emitting device according to any one of [1] to [12], in which $R^1$ in the general formula (1) is a hydrogen atom.

[14] The organic light-emitting device according to any one of [1] to [13], in which the compound has at least one deuterium atom.

[15] The organic light-emitting device according to any one of [1] to [14], in which the layer contains the dopant material.

[16] The organic light-emitting device according to [15], in which an amount of light emitted from the dopant material is larger than an amount of light emitted from the compound.

[17] The organic light-emitting device according to any one of [1] to [16], in which the dopant material is a compound represented by the following general formula (4):

General Formula (4)

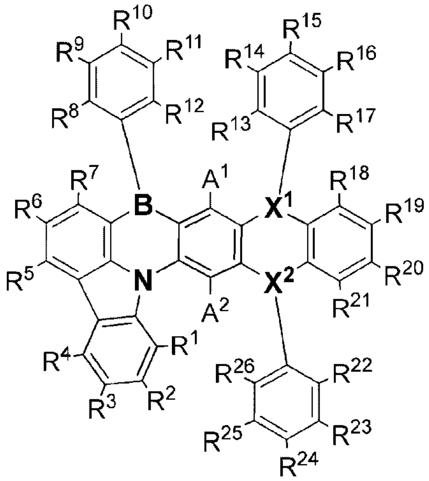

in which one of $X^1$ and $X^2$ is a nitrogen atom, and the other is a boron atom; $R^1$ to $R^{26}$, $A^1$ and $A^2$ each independently represent a hydrogen atom, a deuterium atom, or a substituent; $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ each may be bonded to each other to form a cyclic structure; provided that when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring; provided that when $X^1$ is a nitrogen atom, $R^7$ and $R^8$, and $R^{21}$ and $R^{22}$ are each bonded via a nitrogen atom to form a 6-membered ring, and $R^{17}$ and $R^{18}$ are bonded to each other to form a single bond, at least one of $R^1$ to $R^6$ is a substituted or unsubstituted aryl group, or any one of $R^1$ and $R^2$, $R^2$ and $R^3$, $R^3$ and $R^4$, $R^4$ and $R^5$, or $R^5$ and $R^6$ is bonded to each other to form an aromatic ring or a heteroaromatic ring; when $X^1$ is a boron atom, $X^2$ is a nitrogen atom, and $R^7$ and $R^8$, and $R^{17}$ and $R^{18}$ are each bonded to each other to form a cyclic structure containing a boron atom, the cyclic structure is a 5- to 7-membered ring, and in the case of a 6-membered ring, $R^7$ and $R^8$, and $R^{17}$ and $R^{18}$ are each bonded to each other to form -B($R^{32}$)-, -CO-, -CS-, or -N($R^{27}$)-, and $R^{27}$ represents a hydrogen atom, a deuterium atom, or a substituent.

[18] The organic light-emitting device according to any one of [1] to [17], in which the layer contains the host material.

[19] The organic light-emitting device according to [18], in which an amount of light emitted from the compound is largest among the materials contained in the layer.

[20] The organic light-emitting device according to any one of [1] to [19], in which the host material is a compound represented by the following general formula (5):

General Formula (5)

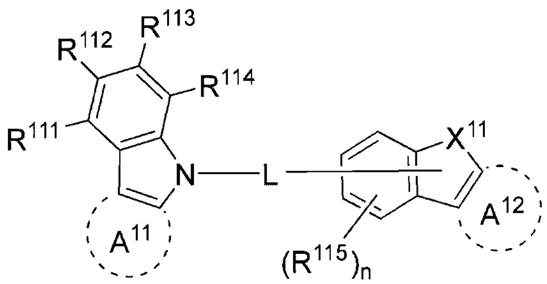

in which $X^{11}$ represents O, S, N($R^A$), or C($R^B$)($R^C$); $A^{11}$ and $A^{12}$ each independently represent a benzene ring, a furan ring, a thiol ring, a pyrrole ring, or a cyclopentadiene ring, which may be further fused with another ring or substituted; $R^{111}$ to $R^{114}$, $R^B$, and $R^C$ each independently represent a hydrogen atom, a deuterium atom, a substituted or unsubstituted aryl group, a substituted or unsubstituted heteroaryl group, a substituted or unsubstituted alkyl group, or a cyano group; each of $R^{115}$'s independently represents a hydrogen atom, a deuterium atom, a substituted or unsubstituted aryl group, a substituted or unsubstituted heteroaryl group, a substituted or unsubstituted alkyl group, a cyano group, or a bond with L; $R^A$ represents a substituted or unsubstituted aryl group, a substituted or unsubstituted heteroaryl group, a substituted or unsubstituted alkyl group, or a bond with L; $R^{111}$ and $R^{112}$, $R^{112}$ and $R^{113}$, $R^{113}$ and

$R^{114}$, two adjacent $R^{115}$, and $R^B$ and $R^C$ may be bonded to each other to form a cyclic structure; n represents an integer of 3 or 4; and L represents a single bond, a substituted or unsubstituted arylene group, a substituted or unsubstituted heteroarylene group, or a linking group in which two or more of these groups are bonded.
[21] The organic light-emitting device according to any one of [1] to [20], which emits delayed fluorescence.

Advantageous Effects of Invention

The organic light-emitting device of the present invention exhibits excellent light emission characteristics.

Description of Embodiments

The contents of the present invention will be described in detail below. The constitutional elements may be described below with reference to representative embodiments and specific examples of the present invention, but the present invention is not limited to the embodiments and the specific examples. In the description herein, a numerical range expressed using "to" means a range that includes the numerical values described before and after "to" as the lower limit and the upper limit. A part or all of hydrogen atoms existing in the molecule of the compound for use in the present invention can be substituted with deuterium atoms ($^2H$, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the description herein, the term "substituent" means an atom or an atomic group except a hydrogen atom and a deuterium atom. Also, the term "substituted or unsubstituted" means that a hydrogen atom may be substituted with a deuterium atom or a substituent.

[Compound Represented by General Formula (1)]

The compound represented by the following general formula (1) is described.

General Formula (1)

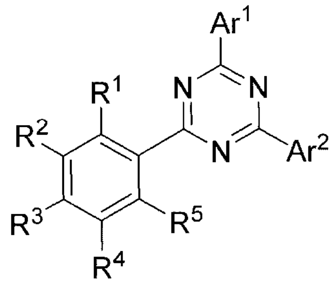

In the general formula (1), $R^1$ to $R^5$ each independently represent a hydrogen atom, a deuterium atom, a substituted or unsubstituted alkyl group, a substituted or unsubstituted aryl group, a donor group, or a group represented by the following formula (2). $R^2$ or $R^3$ is a group represented by the following general formula (2), and two or more of $R^1$ to $R^5$ are donor groups. $Ar^1$ and $Ar^2$ each independently represent a substituted or unsubstituted aryl group, or a substituted or unsubstituted heteroaryl group. In the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions. Here, $Ar^3$ and $Ar^4$ are groups present in the general formula (2).
The alkyl group that $R^1$ to $R^5$ can adopt may be linear, branched or cyclic. Two or more of a linear moiety, a cyclic moiety, and a branched moiety may be in the group as mixed. The carbon number of the alkyl group can be, for example, 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group which is the substituent may be further substituted with, for example, a deuterium atom, an aryl group, an alkoxy group, an aryloxy group, and a halogen atom. In one aspect of the present invention, the substituent for the alkyl group is one or more selected from the group consisting of an aryl group and a deuterium atom. In one preferred aspect of the present invention, the alkyl group is unsubstituted, and, for example, can be selected from the group consisting of a methyl group, an ethyl group, an isopropyl group, and a tert-butyl group.

The aryl group that $R^1$ to $R^5$ and $Ar^1$ and $Ar^2$ can adopt each may be a monocyclic ring or may be a fused ring in which two or more rings are fused. In the case of a fused ring, the number of fused rings is preferably 2 to 6, and can be selected from, for example, 2 to 4. Specific examples of the ring include a benzene ring, a naphthalene ring, an anthracene ring, a phenanthrene ring, and a triphenylene ring. In one aspect of the present invention, the aryl group is a substituted or unsubstituted phenyl group, a substituted or unsubstituted naphthalen-1-yl group, or a substituted or unsubstituted naphthalen-2-yl group, and is preferably a substituted or unsubstituted phenyl group. For example, the substituent for the aryl group may be selected from Substituent Group A, may be selected from Substituent Group B, may be selected from Substituent Group C, may be selected from Substituent Group D, or may be selected from Substituent Group E. In one aspect of the present invention, the substituent for the aryl group is one or more selected from the group consisting of an alkyl group, an aryl group and a deuterium atom. In one preferred aspect of the present invention, the aryl group is substituted with at least one deuterium atom. In one aspect of the present invention, the aryl group is unsubstituted.

Specific examples of the substituted or unsubstituted aryl group that $R^1$ to $R^5$ and $Ar^1$ and $Ar^2$ can adopt are shown below. Here, the aryl group which can be employed in the present invention should not be limitatively interpreted by the following specific examples. In the following specific examples, * indicates a bonding position. The expression of a methyl group is omitted. Consequently, Ar2 to Ar7 represent structures substituted with a methyl group.

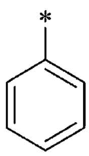
Ar1

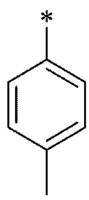
Ar2

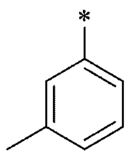
Ar3

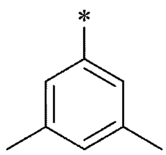
Ar4

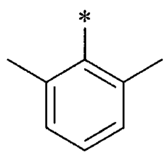
Ar5

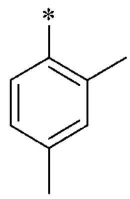
Ar6

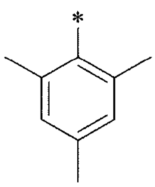
Ar7

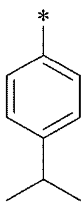
Ar8

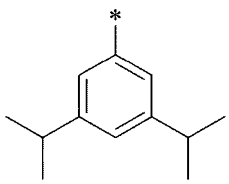
Ar9

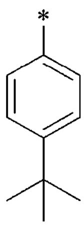
Ar10

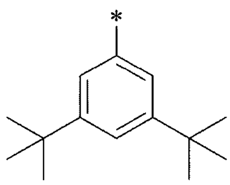
Ar11

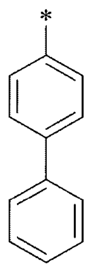
Ar12

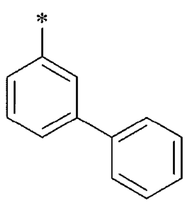
Ar13

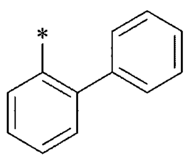
Ar14

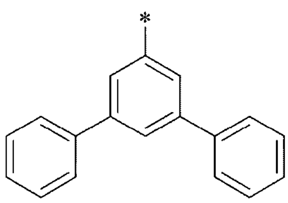
Ar15

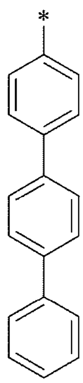
Ar16

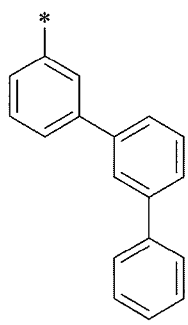
Ar17

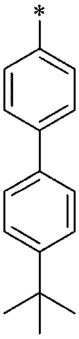
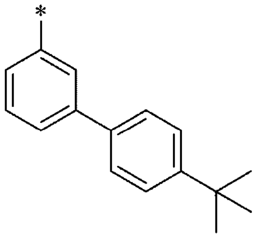
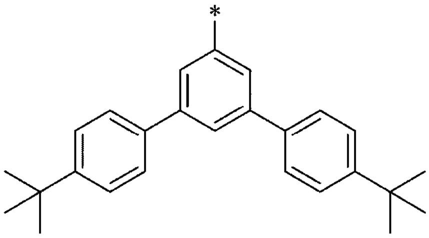
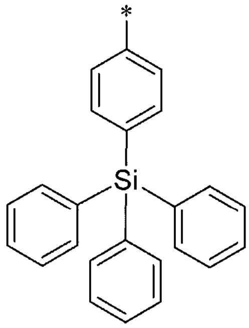

Ar18 Ar19 Ar20 Ar21

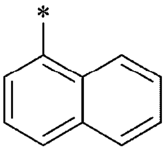
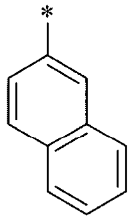
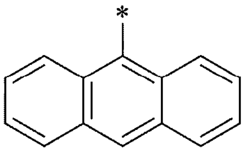
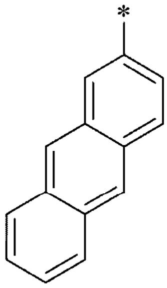
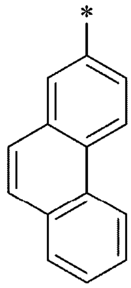

Ar22 Ar23 Ar24 Ar25 Ar26

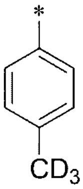
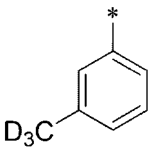
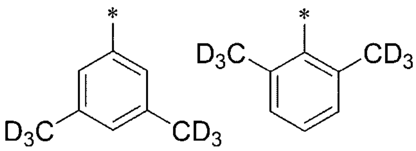
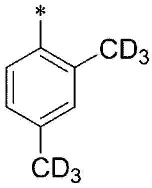
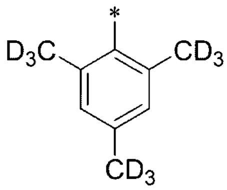

Ar27 Ar28 Ar29 Ar30 Ar31 Ar32

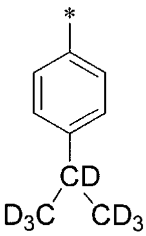
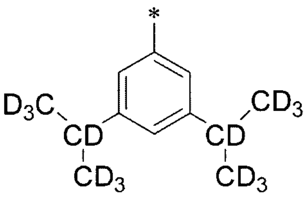
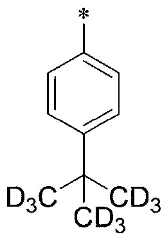
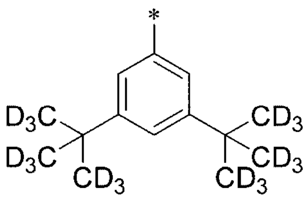

Ar33 Ar34 Ar35 Ar36

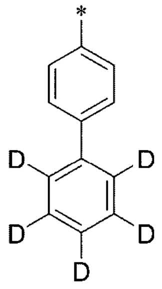
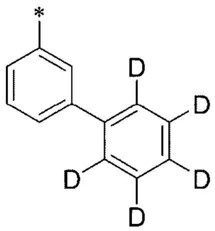
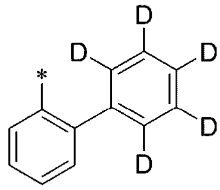
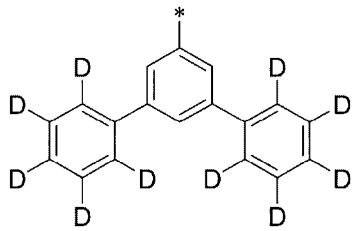

Ar37 Ar38 Ar39 Ar40

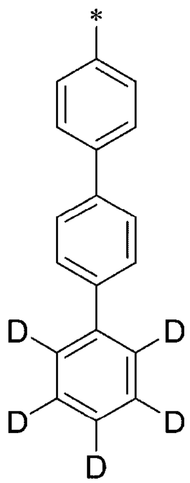
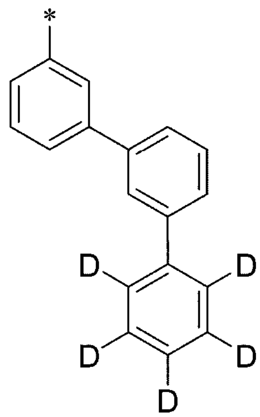
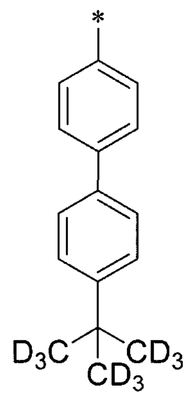
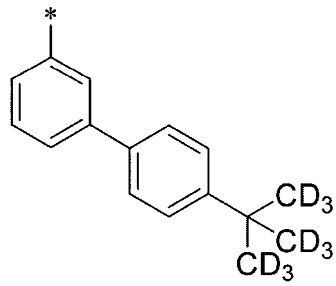

Ar41 Ar42 Ar43 Ar44

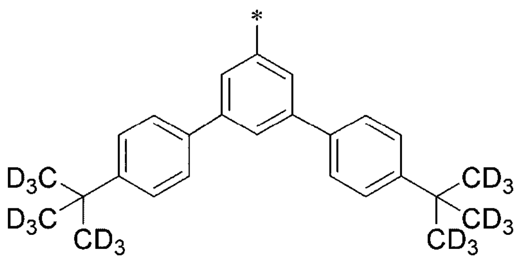
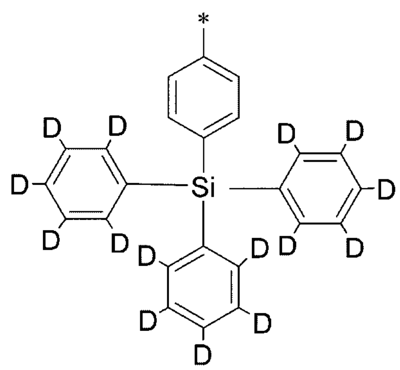

Ar45 Ar46

In addition to the above-mentioned specific examples, groups obtained by substituting all hydrogen atoms present in Ar1 to Ar26 with deuterium atoms are exemplified as Ar47 to Ar72, respectively.

In one aspect of the present invention, the aryl group that $R^1$ to $R^5$ can adopt is selected from the group consisting of Ar1 to Ar72. In one aspect of the present invention, the aryl group that $R^1$ to $R^5$ can adopt is Ar1 or Ar47. In one aspect of the present invention, the aryl group that $R^1$ to $R^5$ can adopt is selected from the group consisting of Ar2 to Ar11, Ar27 to Ar36, and Ar48 to Ar57. In one aspect of the present invention, the aryl group that $R^1$ to $R^5$ can adopt is selected from the group consisting of Ar12 to Ar20, Ar37 to Ar45, and Ar58 to Ar66. In one aspect of the present invention, the aryl group that $R^1$ to $R^5$ can adopt is selected from the group consisting of Ar22 to Ar26 and Ar68 to Ar72. In one preferred aspect of the present invention, the aryl group that $R^1$ to $R^5$ can adopt is selected from the group consisting of Ar1, Ar12 to Ar15, Ar24, Ar37 to Ar40, Ar47, Ar58 to Ar61, and Ar70.

In one aspect of the present invention, the aryl group that $Ar^1$ and $Ar^2$ can adopt is selected from the group

consisting of Ar1 to Ar72. In one aspect of the present invention, the aryl group that $Ar^1$ and $Ar^2$ can adopt is Ar1 or Ar47. In one aspect of the present invention, the aryl group that $Ar^1$ or $Ar^2$ can adopt is selected from the group consisting of Ar2 to Ar11, Ar27 to Ar36, and Ar48 to Ar57. In one aspect of the present invention, the aryl group that $Ar^1$ or $Ar^2$ can adopt is selected from the group consisting of Ar12 to Ar20, Ar37 to Ar45, and Ar58 to Ar66. In one preferred aspect of the present invention, the aryl group that $R^1$ to $R^5$ can adopt is selected from the group consisting of Ar1, Ar12 to Ar14, Ar37 to Ar40, Ar47, and Ar58 to Ar61.

At least two of $R^1$ to $R^5$ in the general formula (1) are donor groups. The donor group that $R^1$ to $R^5$ can adopt does not include a substituted or unsubstituted aryl group.

The "donor group" can be selected from groups having a negative Hammett's $\sigma p$ value. The "acceptor group" can be selected from groups having a positive Hammett's $\sigma p$ value. The Hammett's $\sigma p$ value is proposed by L. P. Hammett and quantifies the influence of a substituent on the reaction rate or equilibrium of a para-substituted benzene derivative. Specifically, the value is a constant ($\sigma p$) specific to the substituent in the following equations that is established between substituents and reaction rate constants or equilibrium constants in para-substituted benzene derivatives:

$$\log(k/k_0) = \rho\sigma p$$

or

$\log(K/K_0) = \rho\sigma p$. In the above equations, $k_0$ represents a rate constant of a benzene derivative not having a substituent; k represents a rate constant of a benzene derivative substituted with a substituent; $K_0$ represents an equilibrium constant of a benzene derivative not having a substituent; K represents an equilibrium constant of a benzene derivative substituted with a substituent; and $\rho$ represents a reaction constant to be determined by the type and the condition of reaction. Regarding the description relating to the "Hammett's $\sigma p$ value" and the numerical value of each substituent in the present invention, reference can be made to the description relating to $\sigma p$ value in Hansch, C. et. al., Chem. Rev., 91, 165-195 (1991).

The donor group that $R^1$ to $R^5$ can adopt preferably has $\sigma p$ of -0.3 or less, more preferably -0.5 or less, and still more preferably -0.7 or less. For example, the value may be selected from a range of -0.9 or less, or from a range of -1.1 or less.

The donor group in the present invention is preferably a group containing a substituted amino group. The donor group may be a substituted amino group, or may be a substituted amino group-bonded aryl group, especially a substituted amino group-bonded phenyl group. In one preferred aspect of the present invention, the donor group is a substituted amino group.

The substituent bonding to the nitrogen atom of a substituted amino group is preferably a substituted or unsubstituted alkyl group, a substituted or unsubstituted alkenyl group, a substituted or unsubstituted aryl group, or a substituted or unsubstituted heteroaryl group, more preferably a substituted or unsubstituted aryl group or a substituted or unsubstituted heteroaryl group. Especially, the substituted amino group is preferably a substituted or unsubstituted diarylamino group, or a substituted or unsubstituted diheteroarylamino group. The two aryl groups constituting the diarylamino group referred to herein may be bonded to each other, and the two heteroaryl groups constituting the diheteroarylamino group may be bonded to each other.

The donor group that $R^1$ to $R^5$ can adopt is preferably a group represented by the following general formula (a).

General Formula (a)

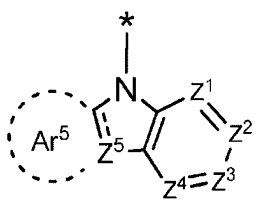

In the general formula (a), $Z^1$ represents C-$R^{14}$ or N, $Z^2$ represents C-$R^{15}$ or N, $Z^3$ represents C-$R^{16}$ or N, and $Z^4$ represents C-$R^{17}$ or N. $Z^5$ represents C or N, $Ar^5$ represents a substituted or unsubstituted aromatic ring or a substituted or unsubstituted heteroaromatic ring. $R^{14}$ and $R^{15}$, $R^{15}$ and $R^{16}$, and $R^{16}$ and $R^{17}$ each may be bonded to each other to form a cyclic structure.

Among $Z^1$ to $Z^4$, the number of groups represented by N is preferably 0 to 3, and preferably 0 to 2. In one aspect of the present invention, among $Z^1$ to $Z^4$, the number of groups represented by N is 1. In one aspect of the present invention, among $Z^1$ to $Z^4$, the number of groups represented by N is 0.

$R^{14}$ to $R^{17}$ each independently represent a hydrogen atom, a deuterium atom, or a substituent.

For example, the substituent may be selected from Substituent Group A, may be selected from Substituent

Group B, may be selected from Substituent Group C, may be selected from Substituent Group D, or may be selected from Substituent Group E. When two or more of $R^{14}$ to $R^{17}$ represent substituents, the two or more substituents may be the same or different. Zero to two of $R^{14}$ to $R^{17}$ are preferably substituents, and for example, one may be a substituent, or zero may be a substituent ($R^{14}$ to $R^{17}$ are a hydrogen atom or a deuterium atom).

$R^{14}$ and $R^{15}$, $R^{15}$ and $R^{16}$, and $R^{16}$ and $R^{17}$ each may be bonded to each other to form a cyclic structure. The cyclic structure may be any of an aromatic ring, an heteroaromatic ring, an aliphatic hydrocarbon ring, and an aliphatic heterocyclic ring, and may be a ring obtained by fusing these rings. The structure is preferably an aromatic ring or a heteroaromatic ring. Examples of the aromatic ring include a substituted or unsubstituted benzene ring. Another benzene ring may be further fused to the benzene ring, and a heterocyclic ring such as a pyridine ring may be fused to the benzene ring. The heteroaromatic ring means a ring containing a hetero atom as a ring skeleton-constituting atom and exhibiting aromaticity, and is preferably a 5- to 7-membered ring, and for example, a 5-membered ring or a 6-membered ring can be employed. In one aspect of the present invention, a furan ring, a thiophene ring, or a pyrrole ring can be employed as the heteroaromatic ring. In one preferred aspect of the present invention, the cyclic structure is a furan ring of a substituted or unsubstituted benzofuran, a thiophene ring of a substituted or unsubstituted benzothiophene, or a pyrrole ring of a substituted or unsubstituted indole. The benzofuran, benzothiophene, and indole referred to herein may be unsubstituted, may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. It is preferable that a substituted or unsubstituted aryl group bonds to the nitrogen atom constituting the pyrrole ring of indole, and examples of the substituent include a substituent selected from any of Substituent Group A to Substituent Group E. The cyclic structure may be a substituted or unsubstituted cyclopentadiene ring. In one aspect of the present invention, a pair selected from (i) $R^{14}$ and $R^{15}$, (ii) $R^{15}$ and $R^{16}$, and (iii) $R^{16}$ and $R^{17}$ are bonded to each other to form a cyclic structure. In one aspect of the present invention, none of $R^{14}$ and $R^{15}$, $R^{15}$ and $R^{16}$, and $R^{16}$ and $R^{17}$ are bonded to each other to form a cyclic structure.

In the general formula (a), $Z^5$ represents C or N, $Ar^5$ represents a substituted or unsubstituted aromatic ring or a substituted or unsubstituted heteroaromatic ring. In one aspect of the present invention, $Z^5$ represents C, and $Ar^5$ represents a substituted or unsubstituted aromatic ring or a substituted or unsubstituted heteroaromatic ring. In one aspect of the present invention, $Z^5$ represents N, and $Ar^5$ represents a substituted or unsubstituted heteroaromatic ring.

Examples of the aromatic ring that $Ar^5$ can adopt include a benzene ring. Another benzene ring may be further fused to the benzene ring, and a heterocyclic ring such as a pyridine ring may be fused to the benzene ring. The heteroaromatic ring that $Ar^5$ can adopt is preferably a 5- to 7-membered ring, and for example, a 5-membered ring or a 6-membered ring can be adopted. In one aspect of the present invention, as the heteroaromatic ring, a furan ring, a thiophene ring, a pyrrole ring, an imidazole ring, a pyridine ring, a pyridazine ring, a pyrimidine ring, or a pyrazine ring can be employed. In one aspect of the present invention, $Z^5$ represents C, and the heteroaromatic ring is a furan ring of a substituted or unsubstituted benzofuran, a thiophene ring of a substituted or unsubstituted benzothiophene, a pyridine ring of a substituted or unsubstituted quinoline, or a pyridine ring of a substituted or unsubstituted isoquinoline. In one aspect of the present invention, $Z^5$ represents N, and the heteroaromatic ring is a pyrrole ring of a substituted or unsubstituted indole, or an imidazole ring of a substituted or unsubstituted benzimidazole. The benzofuran, benzothiophene, quinoline, isoquinoline, indole and benzimidazole referred to herein may be unsubstituted, or may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E.

When $Z^5$ in the general formula (a) represents C, a group represented by the following general formula (b) is preferred.

General Formula (b)

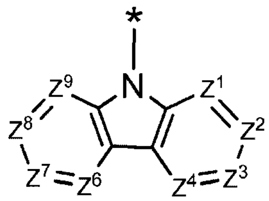

In the general formula (b), $Z^1$ represents C-$R^{14}$ or N, $Z^2$ represents C-$R^{15}$ or N, $Z^3$ represents C-$R^{16}$ or N, $Z^4$ represents C-$R^{17}$ or N, $Z^6$ represents C-$R^{18}$ or N, $Z^7$ represents C-$R^{19}$ or N, $Z^8$ represents C-$R^{20}$ or N, and $Z^9$ represents C-$R^{21}$ or N. $R^{14}$ and $R^{15}$, $R^{15}$ and $R^{16}$, $R^{16}$ and $R^{17}$, $R^{18}$ and $R^{19}$, $R^{19}$ and $R^{20}$, and $R^{20}$ and $R^{21}$ each may be bonded to each other to form a cyclic structure.

Regarding $Z^1$ to $Z^4$ and $R^{14}$ to $R^{17}$ in the general formula (b), reference can be made to the corresponding description of the general formula (a). $Z^6$ to $Z^9$ and $R^{18}$ to $R^{21}$ in the general formula (b) correspond to $Z^1$ to $Z^4$ and $R^{14}$ to $R^{17}$ in the general formula (a), respectively, and regarding the contents thereof, reference can be made to the descriptions of $Z^1$ to $Z^4$ and $R^{14}$ to $R^{17}$ in the general formula (a).

In one aspect of the present invention, among $Z^1$ to $Z^4$ and $Z^6$ to $Z^9$, the number of groups represented by N is preferably 0 to 2, and more preferably 0 or 1. In one aspect of the present invention, among $Z^1$ to $Z^4$ and $Z^6$ to $Z^9$, the number of groups represented by N is 1. In one preferred aspect of the present invention, among $Z^1$ to $Z^4$ and $Z^6$ to $Z^9$, the number of groups represented by N is 0. When the number is 0, the formula represents a substituted or unsubstituted carbazol-9-yl group.

The donor group that $R^1$ to $R^5$ can adopt is preferably a substituted or unsubstituted carbazol-9-yl group. The carbazol-9-yl group referred to herein may be unsubstituted, or may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. One or more rings may be fused to the two benzene rings constituting the carbazol-9-yl group. In one preferred aspect of the present invention, the donor group that $R^1$ to $R^5$ can adopt is a carbazol-9-yl group optionally substituted with a group selected from Substituent Group E, and optionally fused with one or more rings. In the case where the carbazol-9-yl group not fused with a ring is substituted, the substitution site is not specifically limited, but is preferably at least one of 2 to 7-positions, more preferably at least one of 3 to 6-positions, and further preferably a 3-position and a 6-position.

In one aspect of the present invention, the donor group that $R^1$ to $R^5$ can adopt is a carbazol-9-yl group fused with one or more rings, and hereinafter, this will be referred to as a "ring-fused carbazol-9-yl group". The ring-fused carbazol-9-yl group that $R^1$ to $R^5$ can adopt may be unsubstituted, or may be substituted with a substituent selected from Substituent Group A, may be substituted with a substituent selected from Substituent Group B, may be substituted with a substituent selected from Substituent Group C, may be substituted with a substituent selected from Substituent Group D, and may be substituted with a substituent selected from Substituent Group E. Preferably, the group is unsubstituted, or substituted with a substituent selected from Substituent Group E. In one aspect of the present invention, the ring-fused carbazol-9-yl group is unsubstituted. In one preferred aspect of the present invention, the ring-fused carbazol-9-yl group is substituted with an aryl group optionally substituted with one atom or group selected from the group consisting of a deuterium atom, an alkyl group, and an aryl group or with a group formed by combining two or more thereof.

The total number of rings constituting the fused ring in the ring-fused carbazol-9-yl group is 4 or more, preferably 5 or more, more preferably 5 to 9, and still more preferably 5 to 7. In one preferred aspect of the present invention, the number of rings constituting the fused ring is 5. Here, the number of rings includes the number of rings of carbazole to be fused (i.e. 3).

The ring-fused carbazol-9-yl group is a group that bonds via the nitrogen atom constituting the ring skeleton of carbazole, and has a structure in which a ring is fused to at least one of the two benzene rings constituting carbazole. The fused ring may be any of an aromatic hydrocarbon ring, an aromatic heterocyclic ring, an aliphatic hydrocarbon ring, and an aliphatic heterocyclic ring, and may be a ring obtained by further fusing these rings. An aromatic hydrocarbon ring and an aromatic heterocyclic ring are preferable. Examples of the aromatic hydrocarbon ring include a substituted or unsubstituted benzene ring. Another benzene ring may be further fused to the benzene ring, and a heterocyclic ring such as a pyridine ring may be fused to the benzene ring. The aromatic heterocyclic ring means a ring containing a hetero atom as a ring skeleton-constituting atom and exhibiting aromaticity, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. In one aspect of the present invention, the fused ring is a furan ring of a substituted or unsubstituted benzofuran, a thiophene ring of a substituted or unsubstituted benzothiophene, or a pyrrole ring of a substituted or unsubstituted indole. It is preferable that a substituent selected from Substituent Group E (but except for a deuterium atom alone) bonds to the nitrogen atom of the pyrrole ring, and it is more preferable that an aryl group which may be substituted with an alkyl group or an aryl group is bonded. In the present invention, it is preferable to employ a carbazol-9-yl group in which a ring having one or more atoms selected from the group consisting of an oxygen atom, a sulfur atom, and a nitrogen atom as a ring skeleton-constituting atom is fused. Above all, preferably employed are a benzofuro structure-fused carbazol-9-yl group, a benzothieno structure-fused carbazol-9-yl group, and an indolo structure-fused carbazol-9-yl group. In one aspect of the present invention, the compound has at least one benzofuro structure-fused carbazol-9-yl group, and for example, has two or more such groups. In one aspect of the present invention, the compound has at least one benzothieno structure-fused carbazol-9-yl group, and for example, has two or more such groups.

The ring-fused carbazol-9-yl group employable herein includes a substituted or unsubstituted benzofuro[2,3-a]carbazol-12-yl group, a substituted or unsubstituted benzofuro[3,2-a]carbazol-12-yl group, a substituted or unsubstituted benzofuro[2,3-b]carbazol-7-yl group, a substituted or unsubstituted benzofuro[3,2-b]carbazol-11-yl group, a substituted or unsubstituted benzofuro[2,3-c]carbazol-8-yl group, and a substituted or unsubstituted benzofuro[3,2-c]carbazol-5-yl

group. Further, the ring-fused carbazol-9-yl group employable herein includes a substituted or unsubstituted benzothieno [2,3-a]carbazol-12-yl group, a substituted or unsubstituted benzothieno[3,2-a]carbazol-12-yl group, a substituted or unsubstituted benzothieno[2,3-b]carbazol-7-yl group, a substituted or unsubstituted benzothieno[3,2-b]carbazol-11-yl group, a substituted or unsubstituted benzothieno[2,3-c]carbazol-8-yl group, and a substituted or unsubstituted benzothieno[3,2-c]carbazol-5-yl group. Furthermore, the ring-fused carbazol-9-yl group employable herein includes a substituted or unsubstituted 11-phenylindolo[2,3-a]carbazol-12-yl group, a substituted or unsubstituted 5-phenylindolo [3,2-a]carbazol-12-yl group, a substituted or unsubstituted 5-phenylindolo[2,3-b]carbazol-7-yl group, a substituted or unsubstituted 5-phenylindolo[3,2-b]carbazol-11-yl group, a substituted or unsubstituted 5-phenylindolo[2,3-c]carbazol-8-yl group, and a substituted or unsubstituted 12-phenylindolo[3,2-a]carbazol-5-yl group.

The number of the substituents substituted on the ring-fused carbazol-9-yl group is preferably 1 to 10, more preferably 1 to 6, further preferably 1 to 4, and may be, for example 1, or may be, for example 2. In one preferred aspect of the present invention, any of the 3-position or the 6-position of the ring-fused carbazol-9-yl group is substituted. In one preferred aspect of the present invention, the compound has at least one substituent on the para-position of the benzene ring viewed from the hetero atom present in the ring-fused carbazol-9-yl group. In one preferred aspect of the present invention, the compound has at least one substituent only on the para-position of the benzene ring viewed from the hetero atom present in the ring-fused carbazol-9-yl group. In one preferred aspect of the present invention, the compound has substituents on all the substitutable para-positions of the benzene ring viewed from the hetero atom present in the ring-fused carbazol-9-yl group.

Specific examples of the donor group that $R^1$ to $R^5$ in the general formula (1) can adopt are shown below. Here, the donor group which can be employed in the present invention shall not be construed as being limited by the following specific examples.

First, specific examples of the substituted or unsubstituted carbazol-9-yl group in which the donor group has a fused ring structure of three rings are shown below. In the following specific examples, Ph represents a phenyl group ($C_6H_5$), and * indicates a bonding position. Since the expression of a methyl group is omitted, for example, D2 has one methyl group. Here, a deuterated methyl group is expressed as $CD_3$. $C_6D_5$ represents a phenyl group in which all hydrogen atoms are deuterated. D represents a deuterium atom.

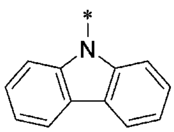
D1

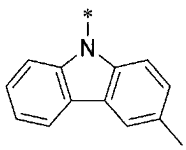
D2

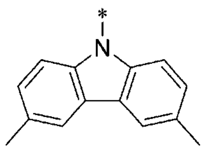
D3

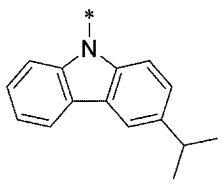
D4

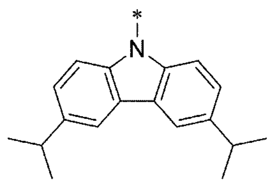
D5

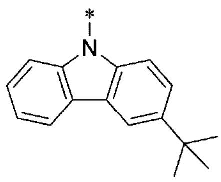
D6

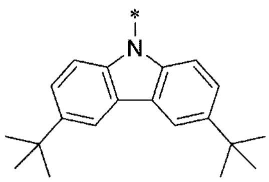
D7

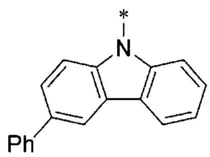
D8

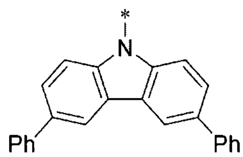
D9

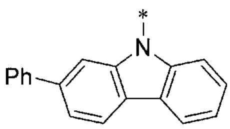
D10

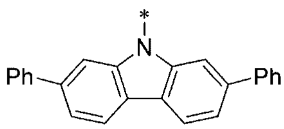
D11

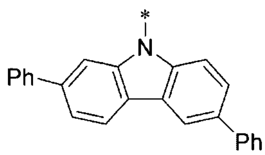
D12

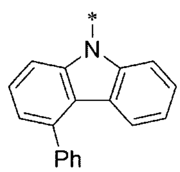
D13

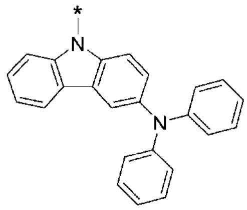

D14

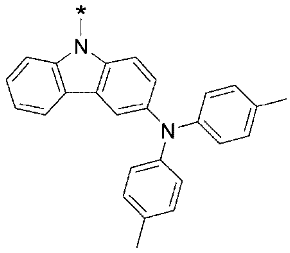

D15

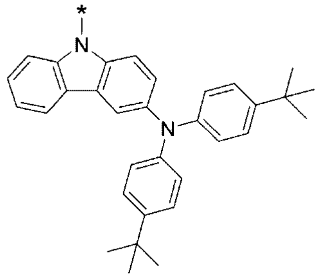

D16

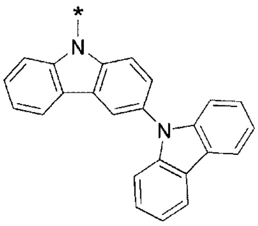

D17

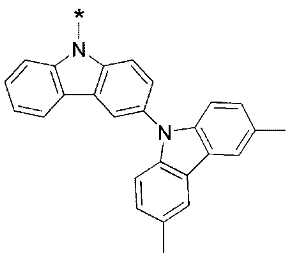

D18

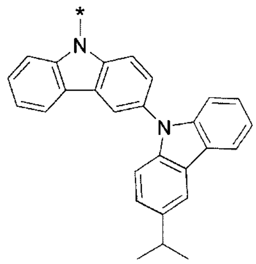

D19

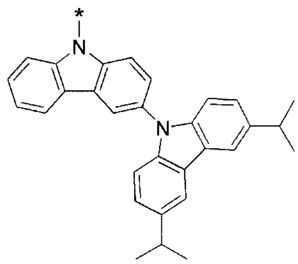

D20

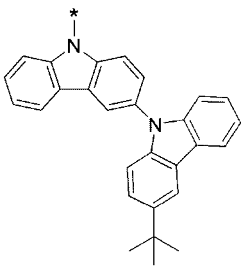

D21

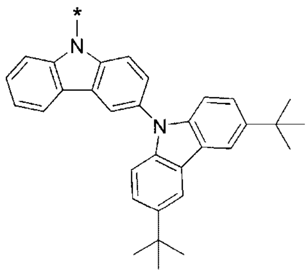

D22

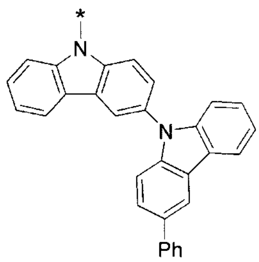

D23

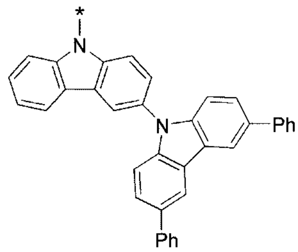

D24

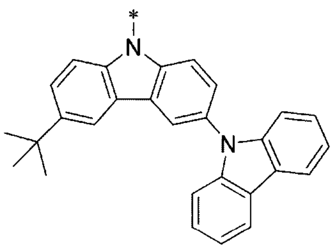

D25

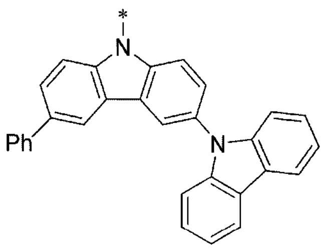

D26

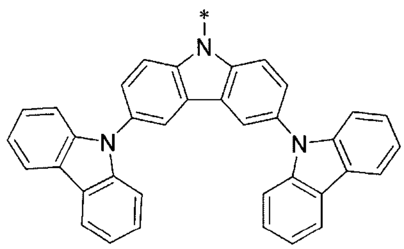

D27

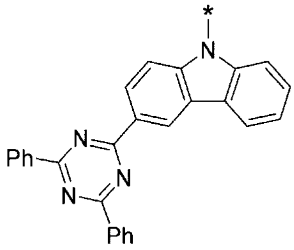

D28

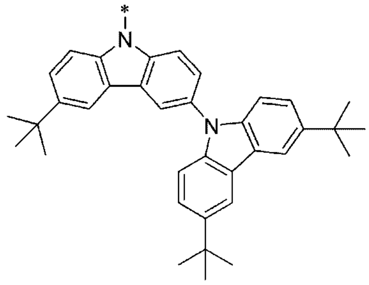

D29

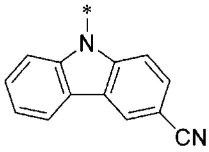

D30

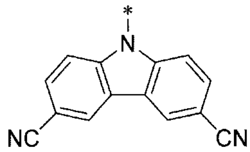

D31

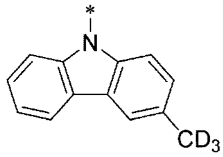

D32

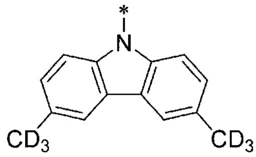

D33

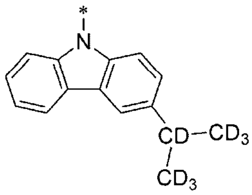

D34

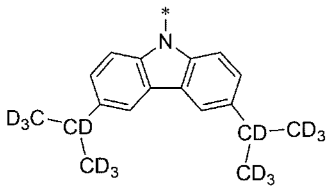

D35

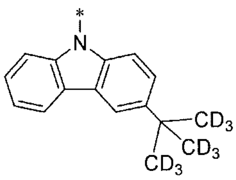

D36

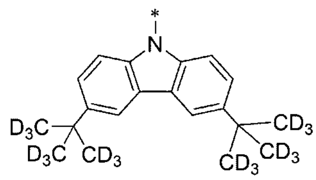

D37

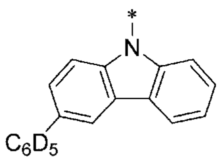

D38

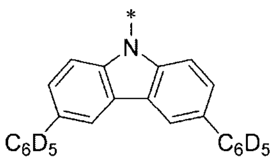

D39

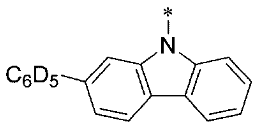

D40

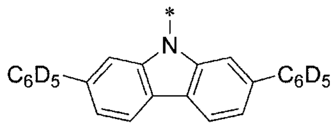

D41

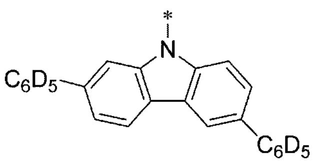

D42

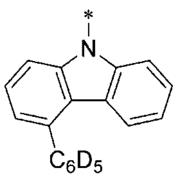

D43

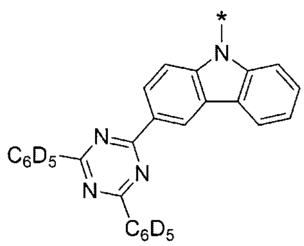

D44

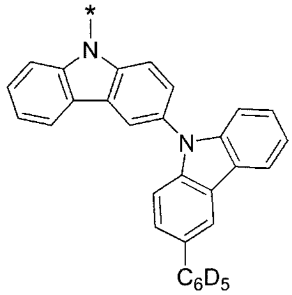

D45

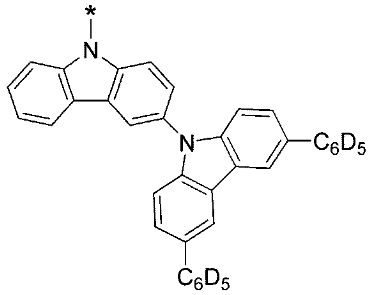

D46

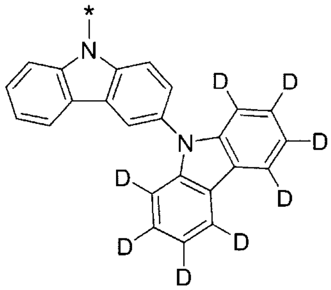

D47

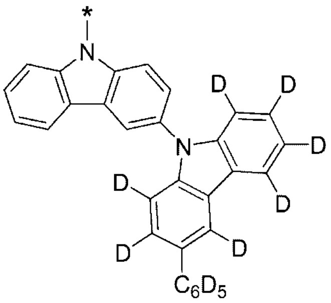

D48

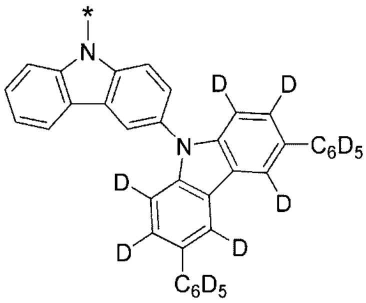

D49

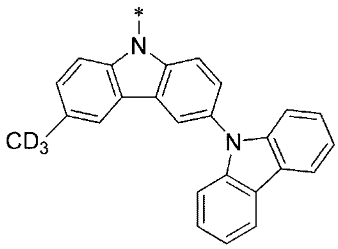

D50

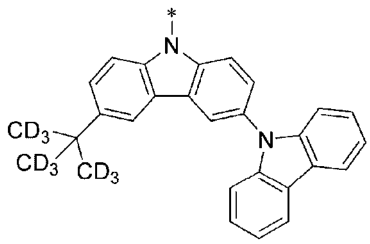

D51

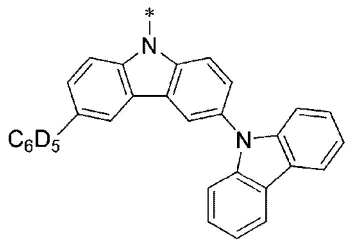

D52

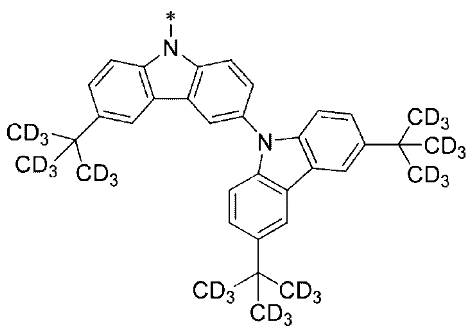

D53

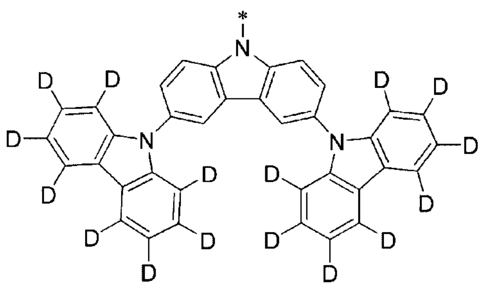

D54

Groups obtained by substituting all hydrogen atoms present in the above D1 to D31 with deuterium atoms are disclosed as D55 to D85.

Next, specific examples of the donor group having a fused ring structure of four or more rings are shown below.

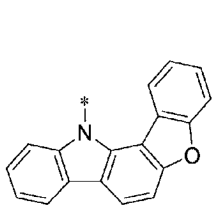

D86

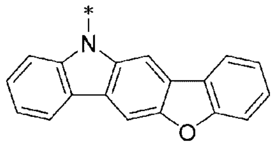

D87

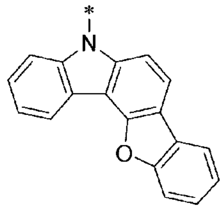

D88

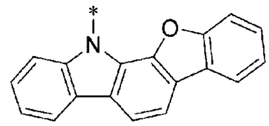

D89

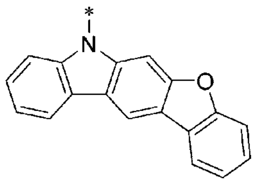

D90

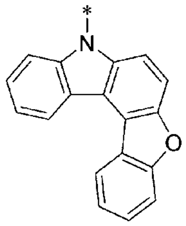

D91

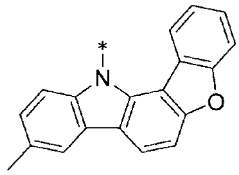

D92

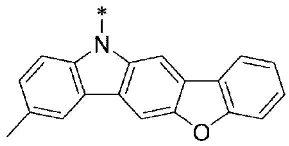

D93

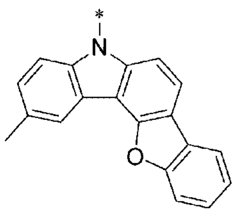

D94

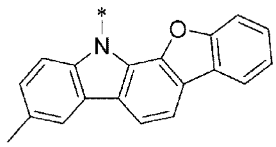

D95

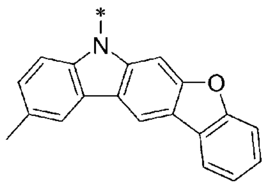

D96

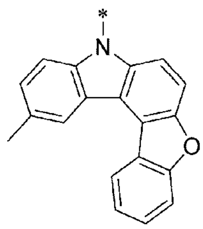

D97

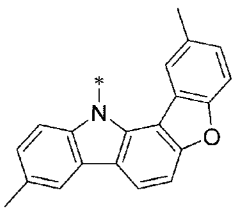

D98

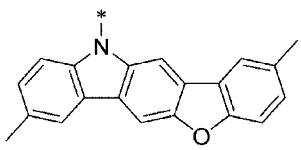

D99

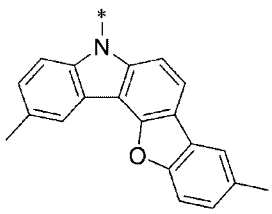

D100

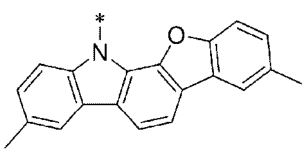

D101

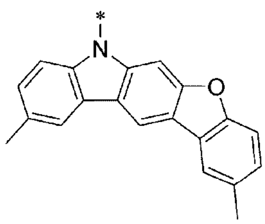

D102

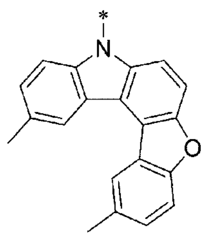

D103

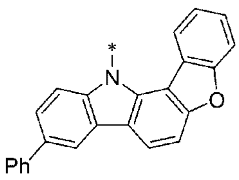 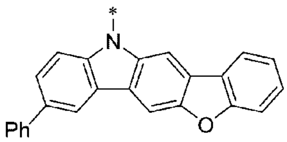 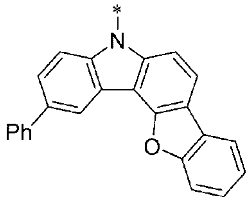 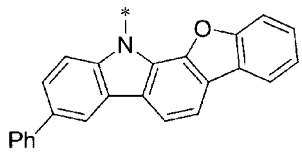

D104 D105 D106 D107

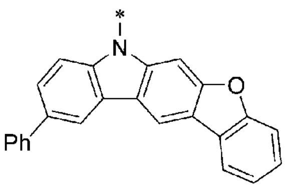 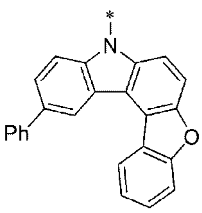 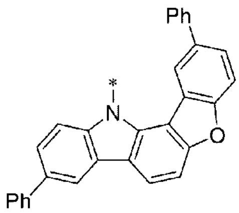 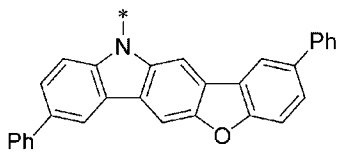

D108 D109 D110 D111

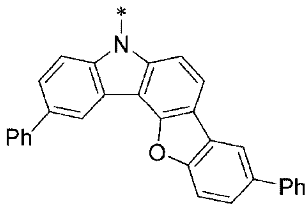 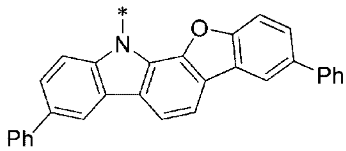 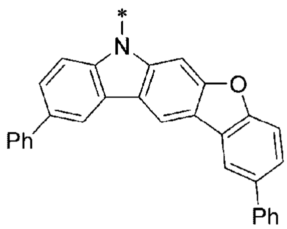

D112 D113 D114

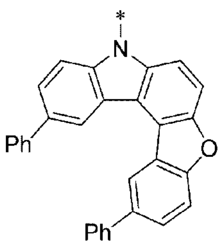 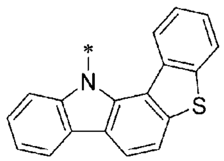 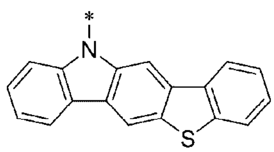 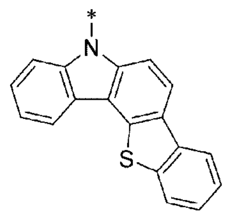

D115 D116 D117 D118

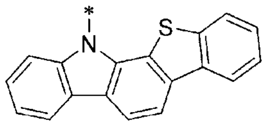 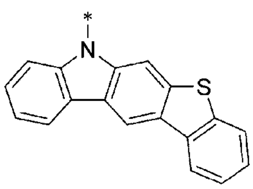 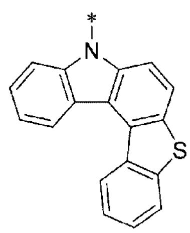 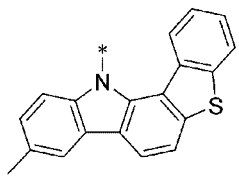

D119 D120 D121 D122

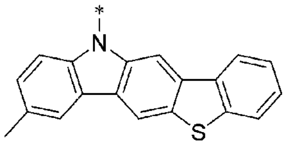
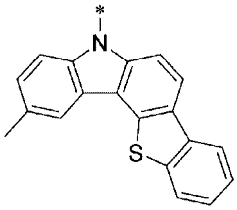
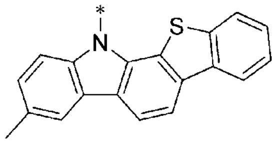
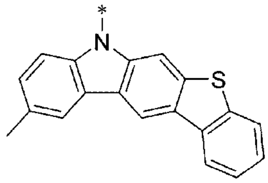

D123 D124 D125 D126

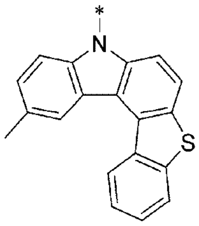
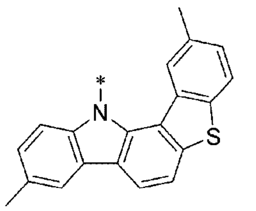
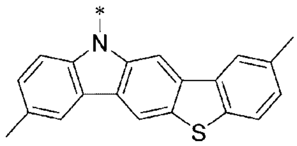
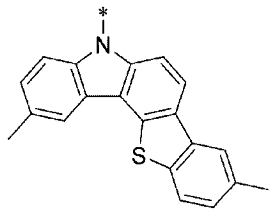

D127 D128 D129 D130

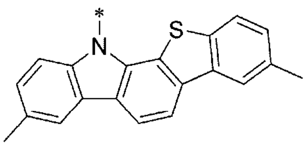
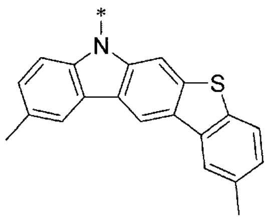
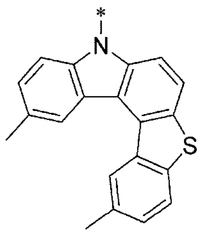
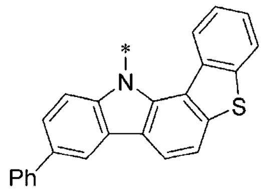

D131 D132 D133 D134

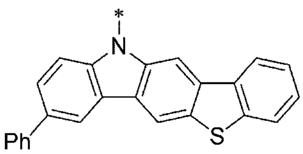
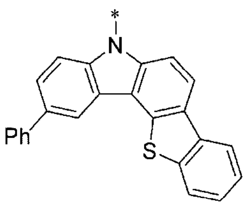
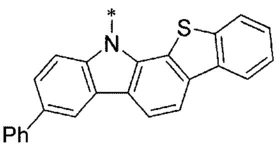
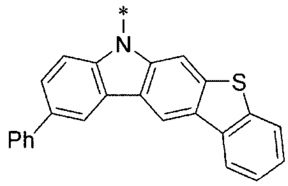

D135 D136 D137 D138

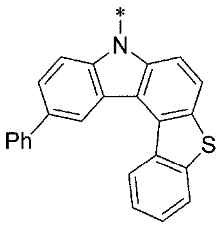
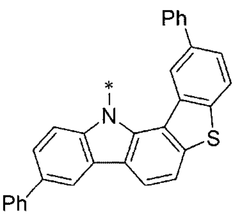
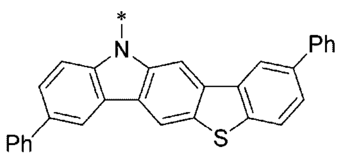
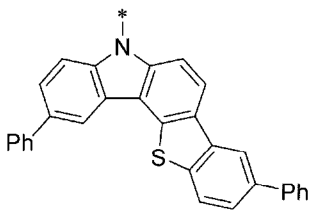

D139 D140 D141 D142

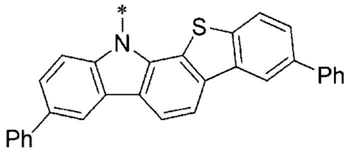

D143

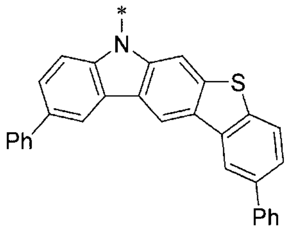

D144

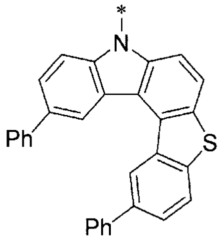

D145

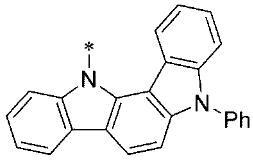

D146

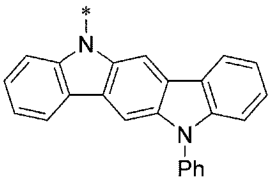

D147

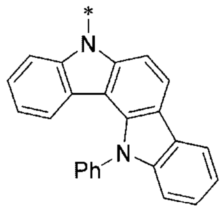

D148

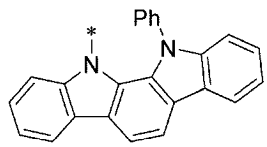

D149

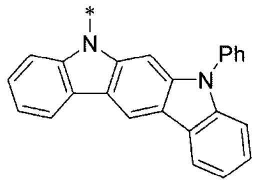

D150

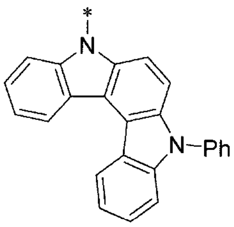

D151

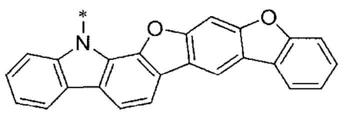

D152

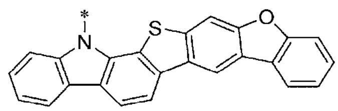

D153

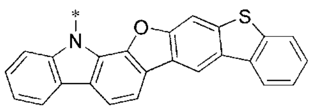

D154

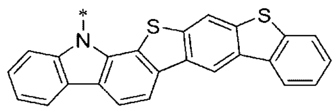

D155

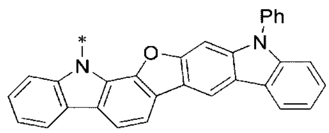

D156

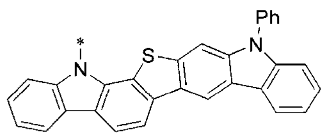

D157

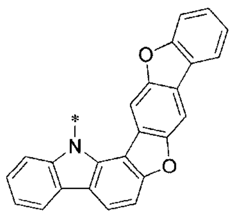

D158

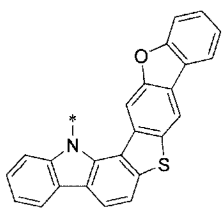

D159

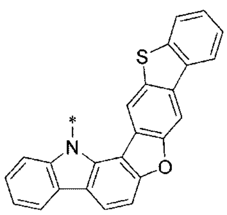

D160

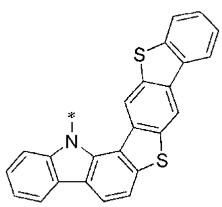 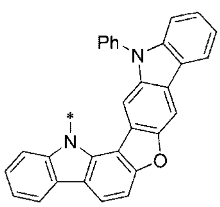 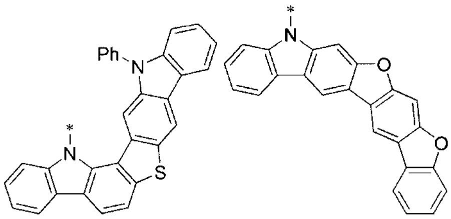 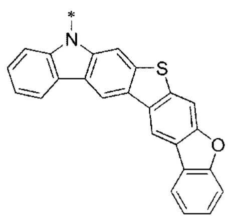

D161 D162 D163 D164 D165

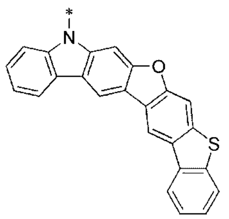 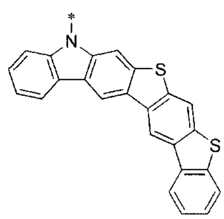 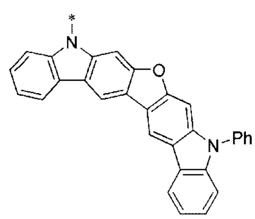 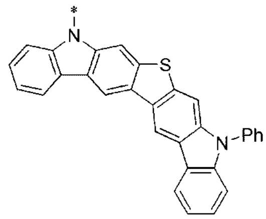

D166 D167 D168 D169

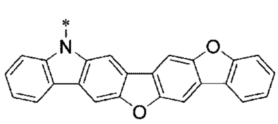 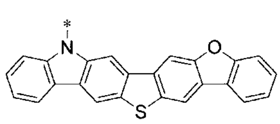 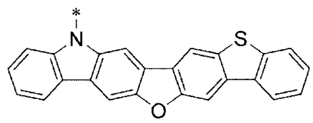

D170 D171 D172

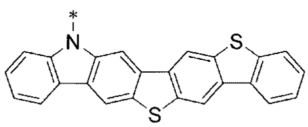 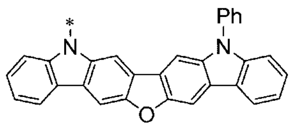 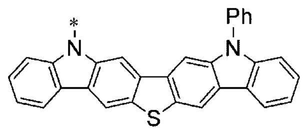

D173 D174 D175

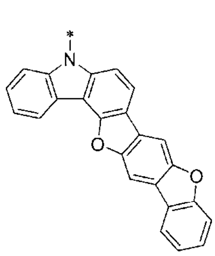 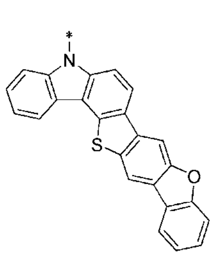 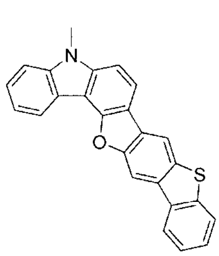 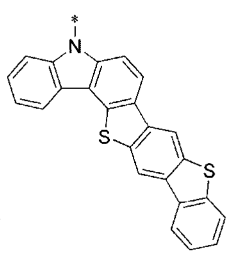 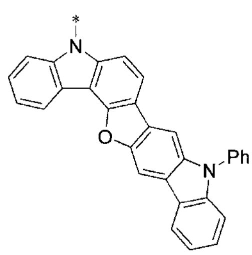

D176 D177 D178 D179 D180

*

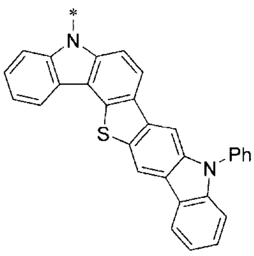
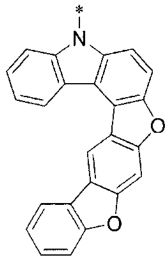
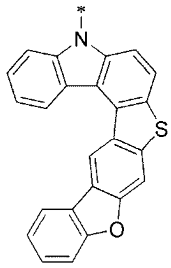
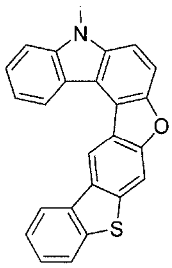

D181 D182 D183 D184

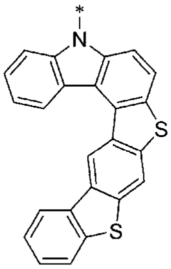
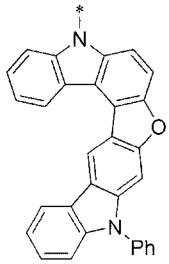
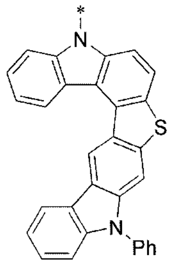
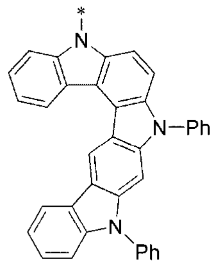

D185 D186 D187 D188

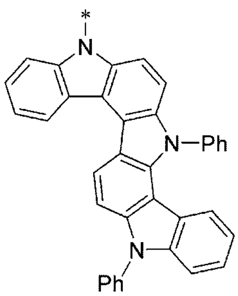
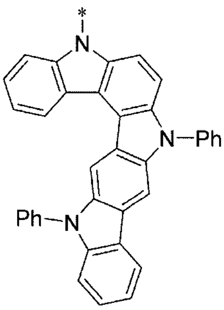
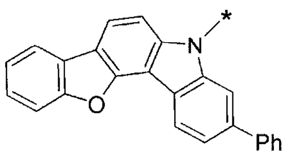
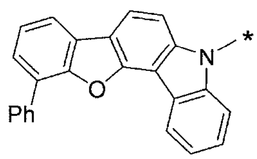

D189 D190 D191 D192

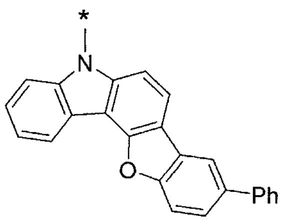
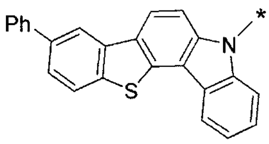
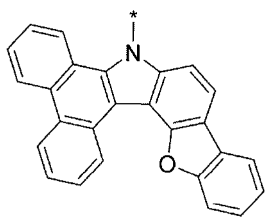
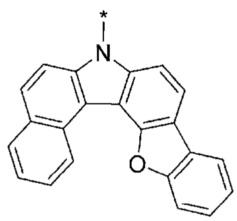

D193 D194 D195 D196

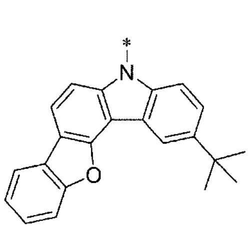
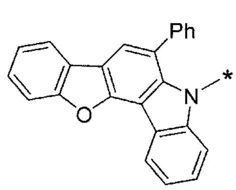
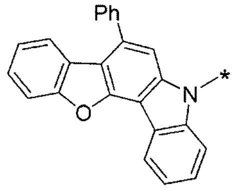
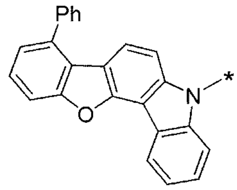

D197 D198 D199 D200

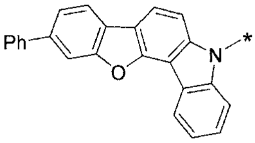

D201

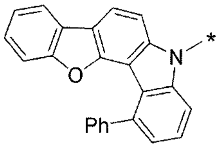

D202

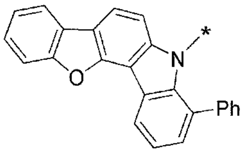

D203

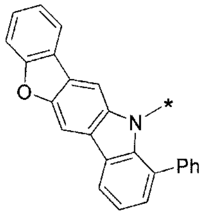

D204

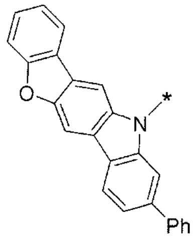

D205

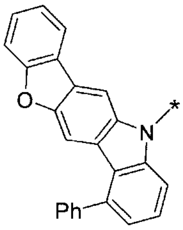

D206

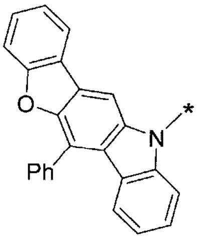

D207

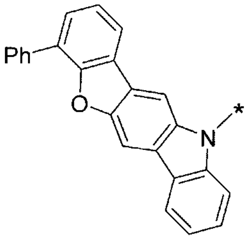

D208

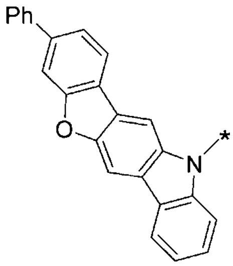

D209

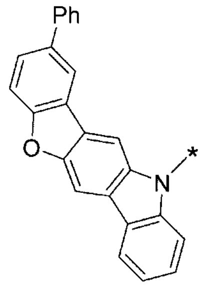

D210

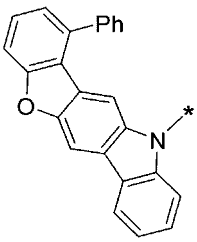

D211

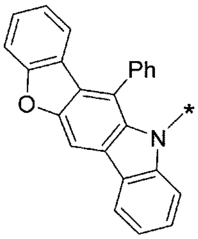

D212

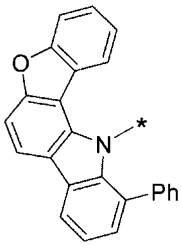

D213

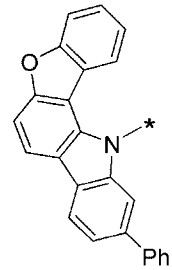

D214

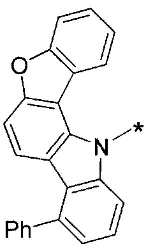

D215

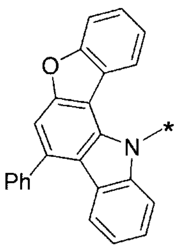

D216

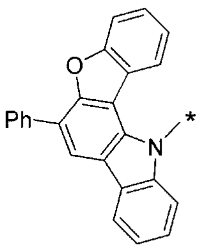

D217

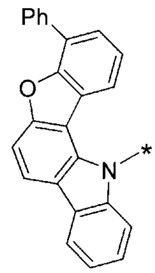

D218

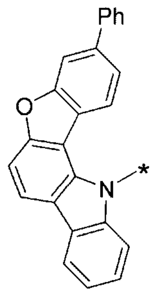
D219

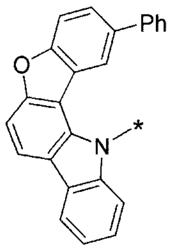
D220

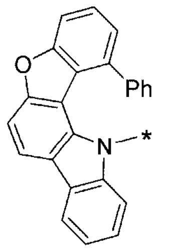
D221

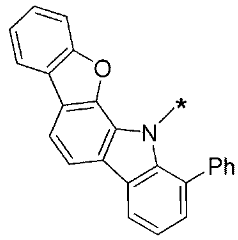
D222

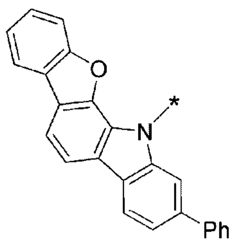
D223

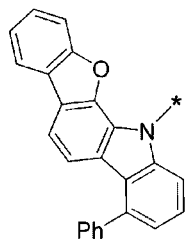
D224

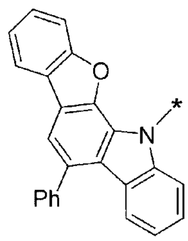
D225

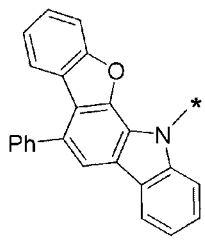
D226

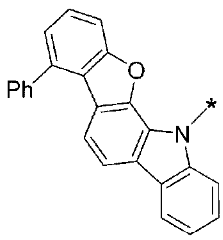
D227

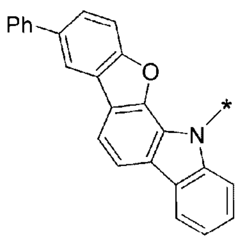
D228

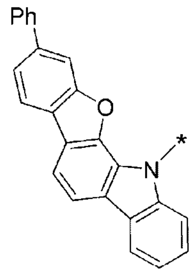
D229

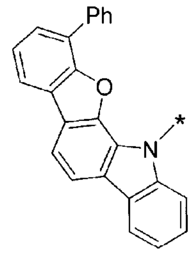
D230

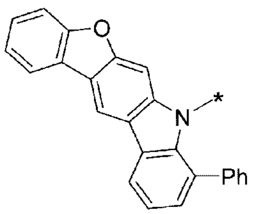
D231

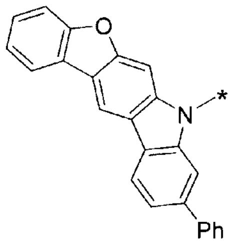
D232

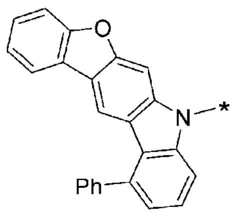
D233

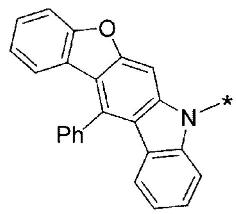
D234

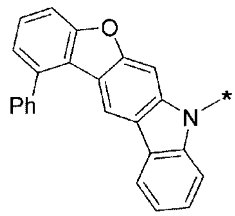
D235

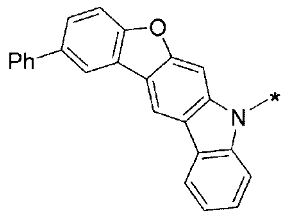
D236

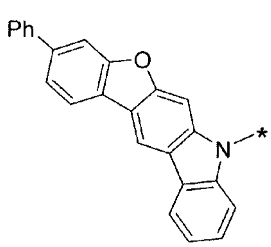
D237

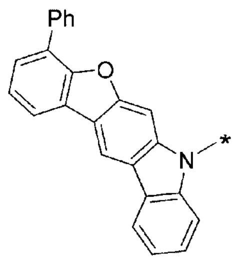
D238

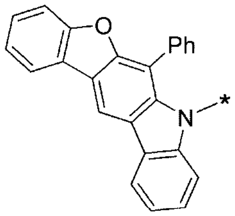
D239

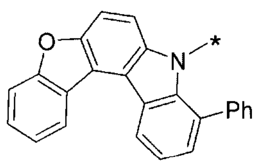
D240

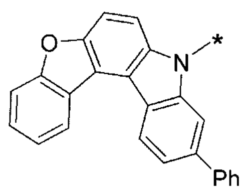
D241

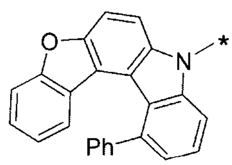
D242

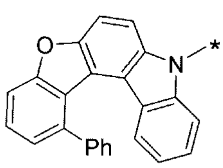
D243

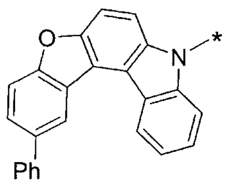
D244

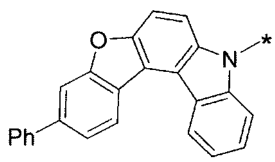
D245

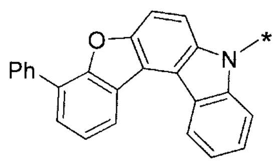
D246

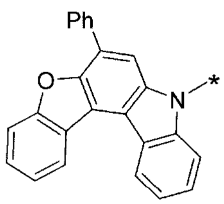
D247

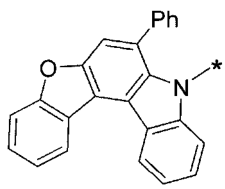
D248

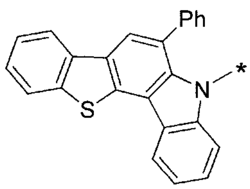
D249

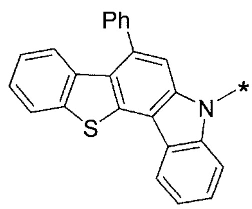
D250

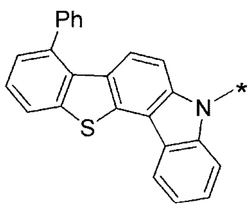
D251

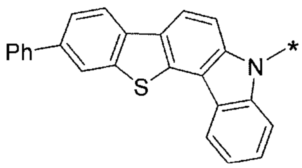
D252

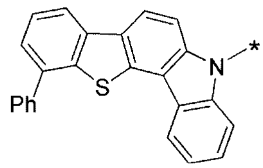
D253

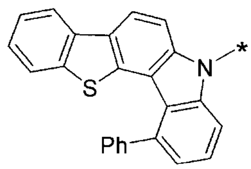
D254

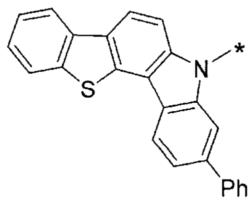
D255

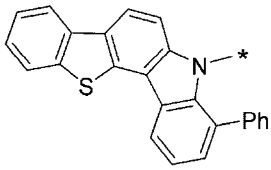
D256

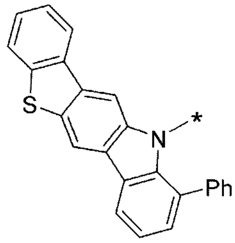
D257

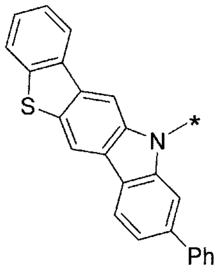
D258

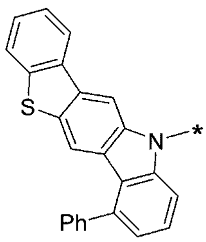
D259

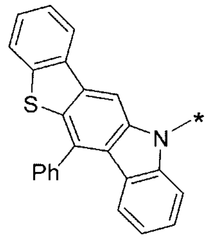
D260

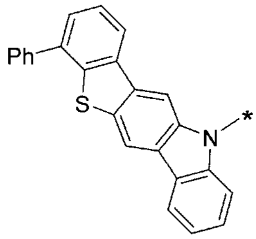
D261

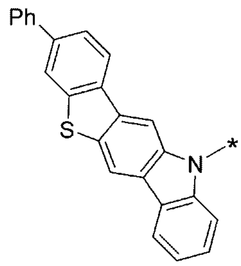
D262

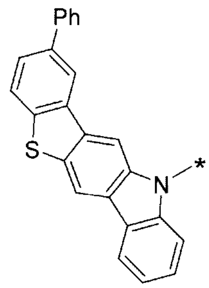
D263

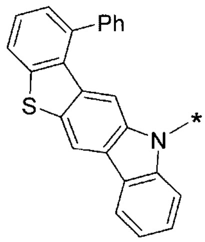
D264

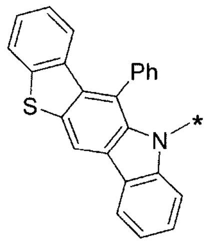
D265

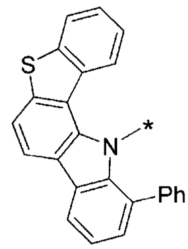
D266

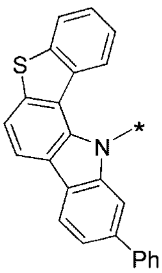
D267

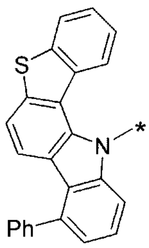
D268

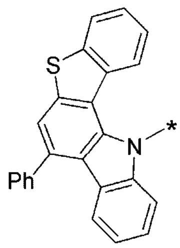
D269

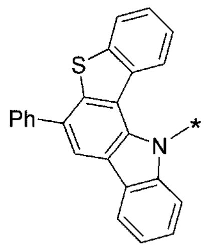
D270

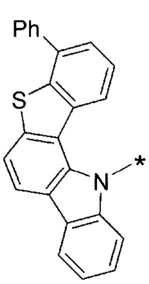

D271

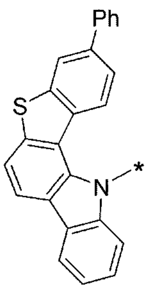

D272

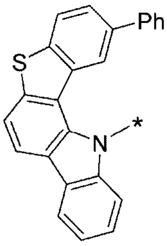

D273

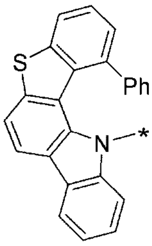

D274

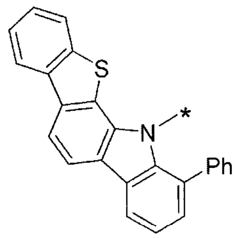

D275

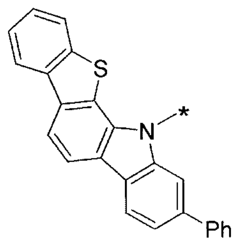

D276

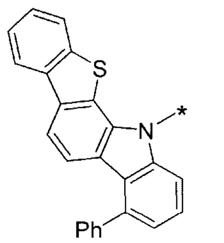

D277

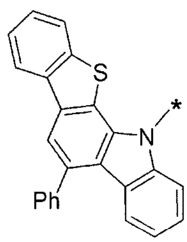

D278

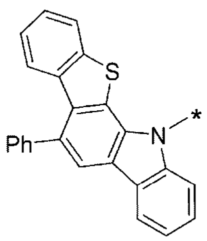

D279

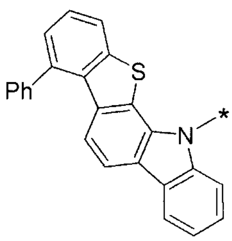

D280

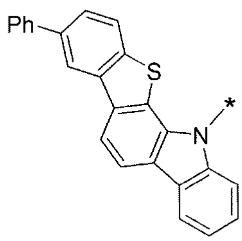

D281

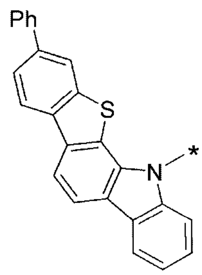

D282

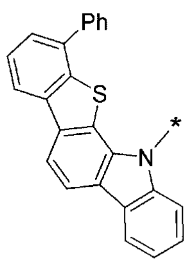

D283

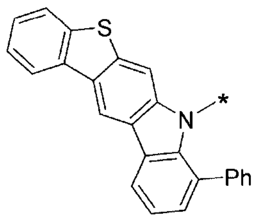

D284

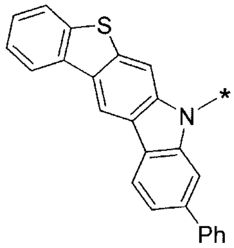

D285

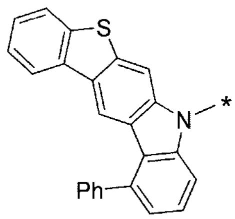

D286

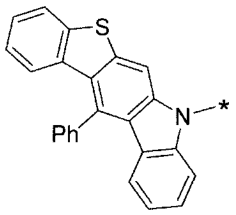

D287

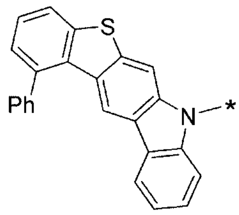

D288

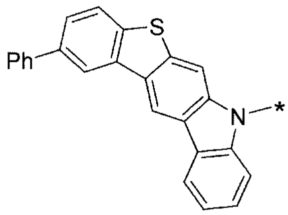

D289

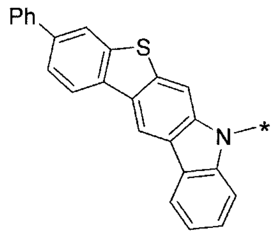

D290

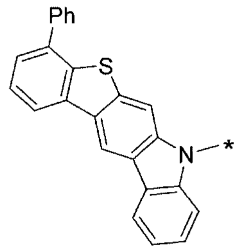

D291

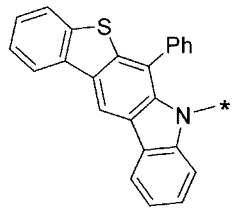

D292

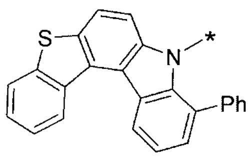

D293

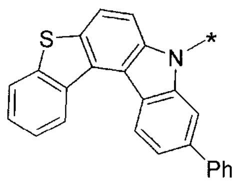

D294

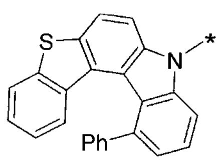

D295

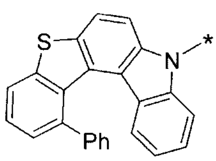

D296

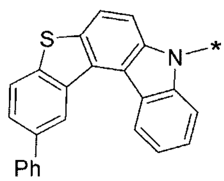

D297

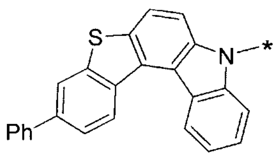

D298

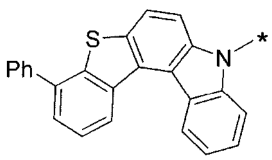

D299

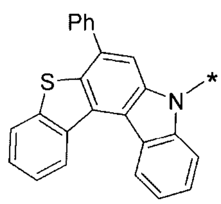

D300

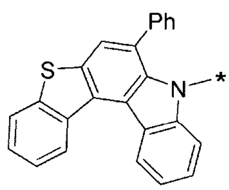

D301

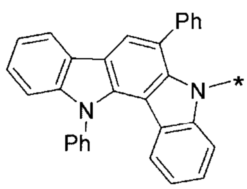

D302

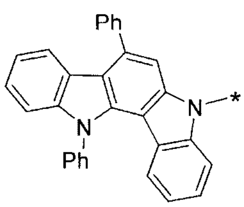

D303

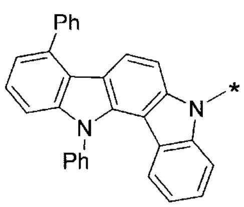

D304

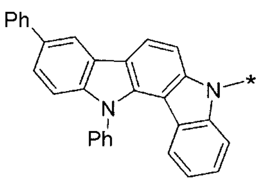

D305

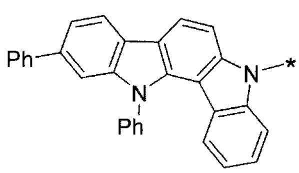

D306

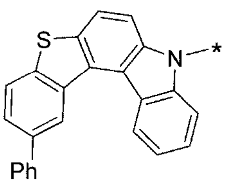
D307

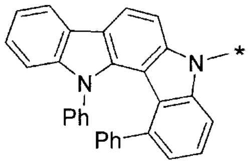
D308

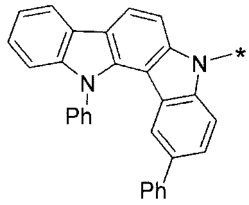
D309

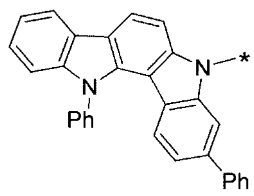
D310

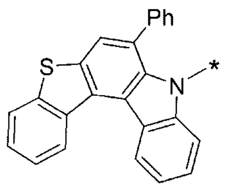
D311

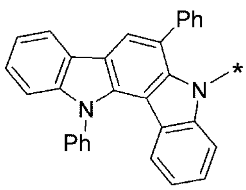
D312

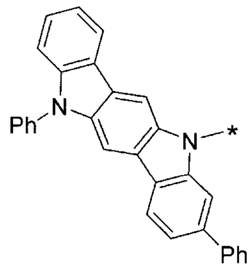
D313

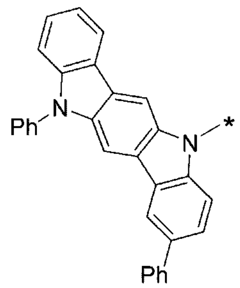
D314

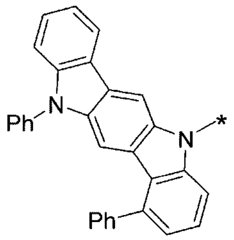
D315

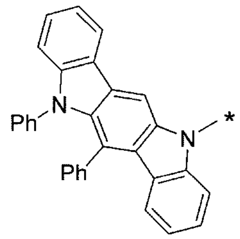
D316

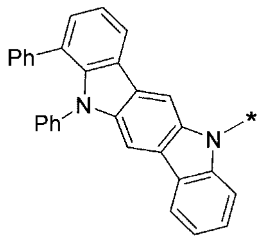
D317

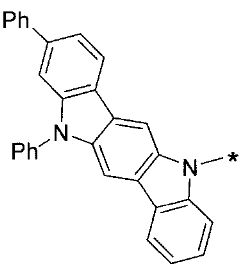
D318

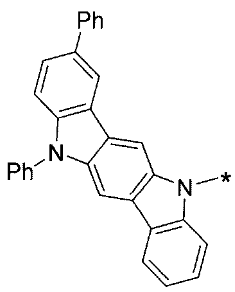
D319

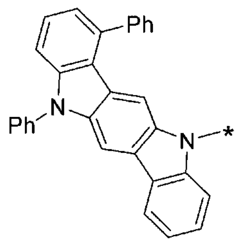
D320

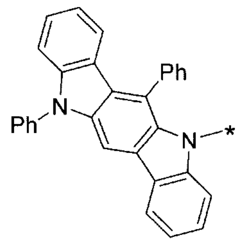
D321

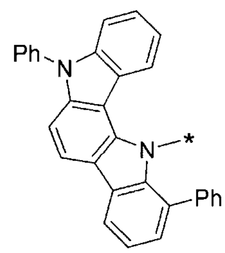
D322

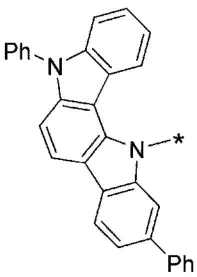
D323

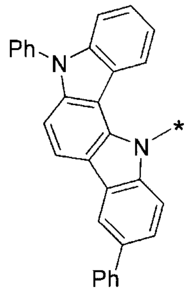
D324

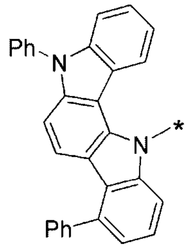
D325

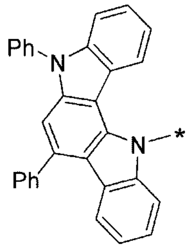
D326

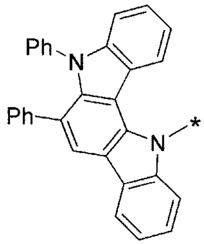
D327

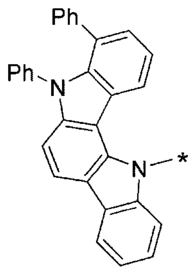
D328

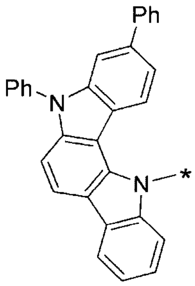
D329

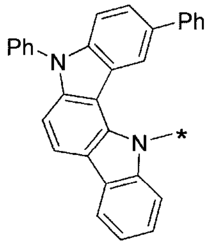
D330

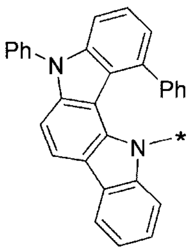
D331

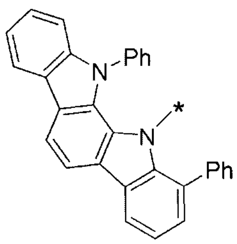
D332

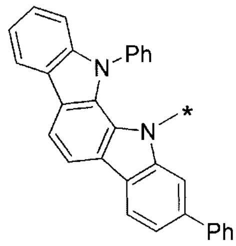
D333

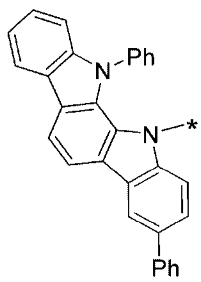
D334

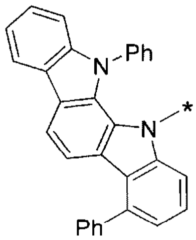
D335

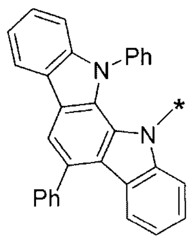
D336

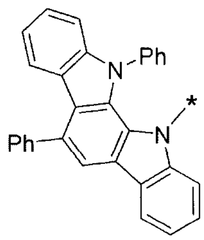
D337

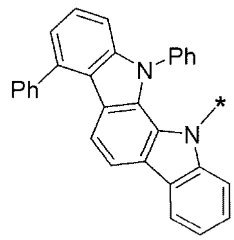
D338

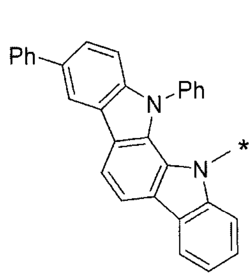

D339

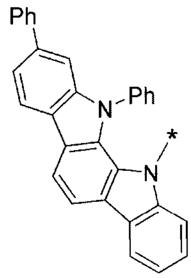

D340

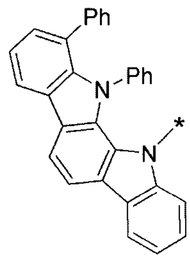

D341

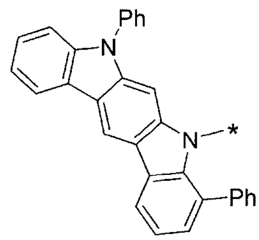

D342

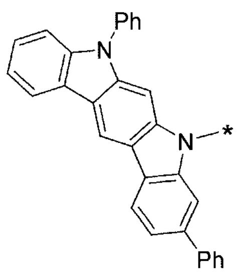

D343

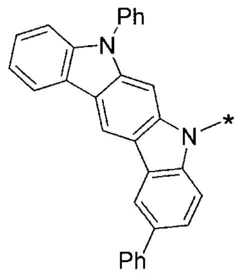

D344

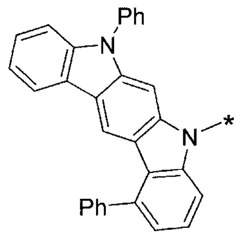

D345

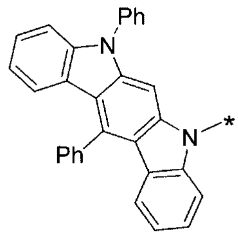

D346

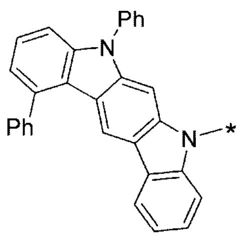

D347

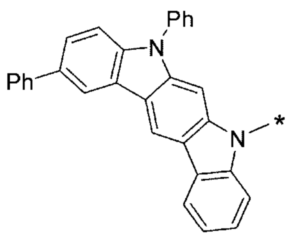

D348

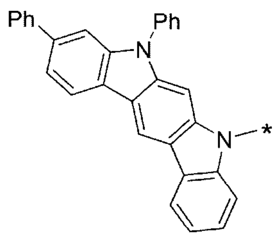

D349

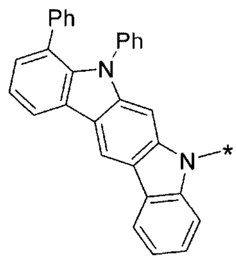

D350

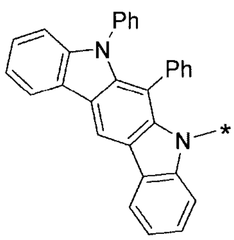

D351

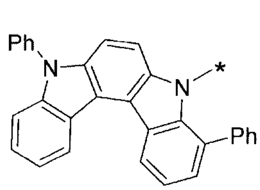

D352

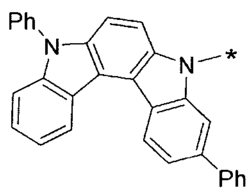

D353

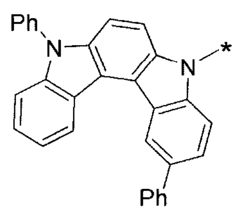

D354

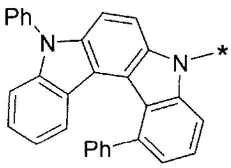

D355

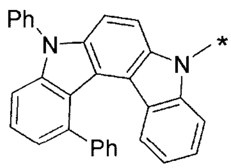

D356

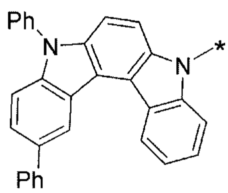

D357

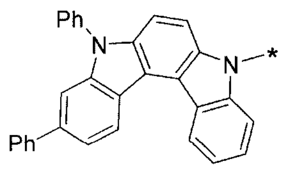

D358

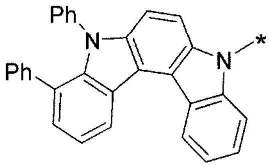
D359

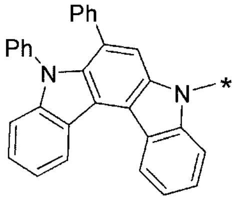
D360

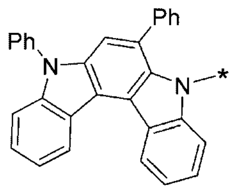
D361

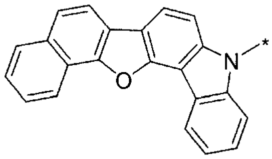
D362

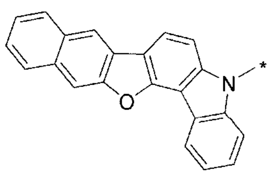
D363

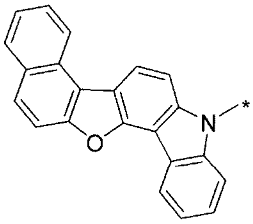
D364

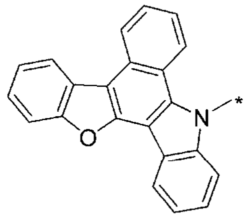
D365

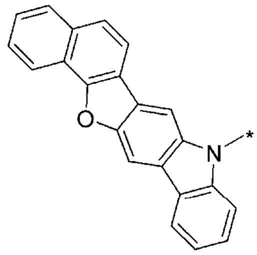
D366

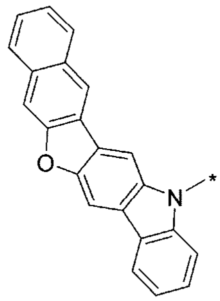
D367

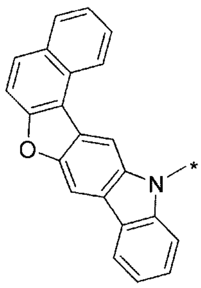
D368

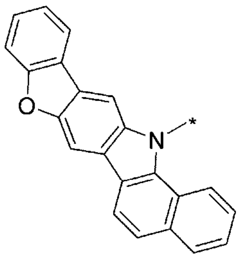
D369

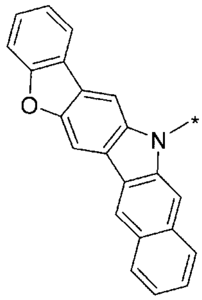
D370

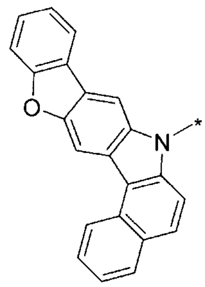
D371

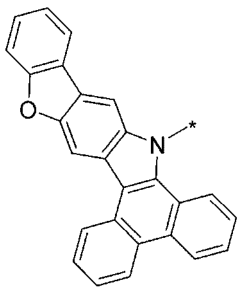
D372

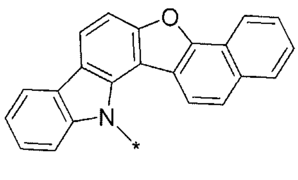
D373

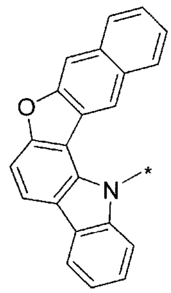
D374

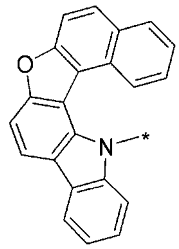
D375

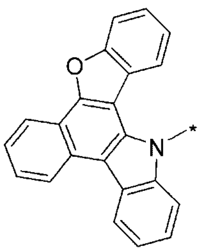
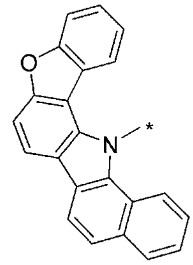
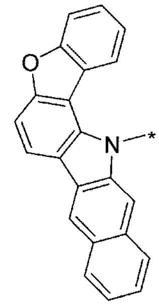
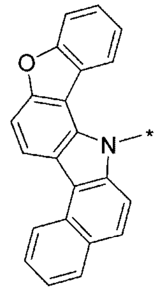

D376 D377 D378 D379

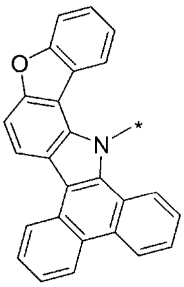
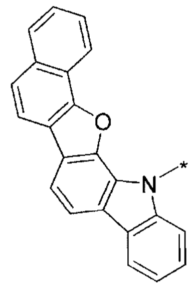
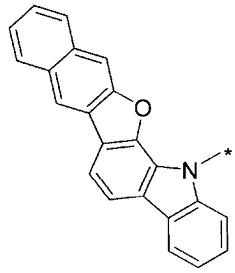
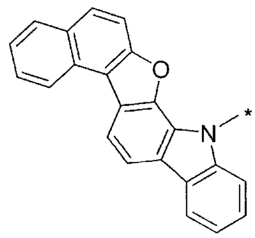

D380 D381 D382 D383

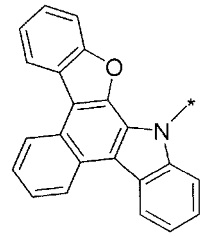
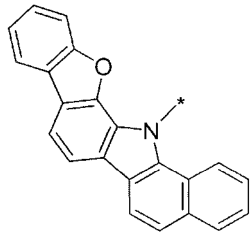
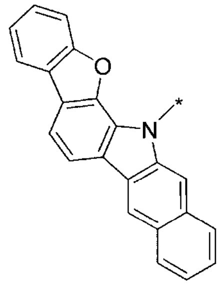
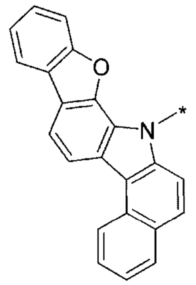

D384 D385 D386 D387

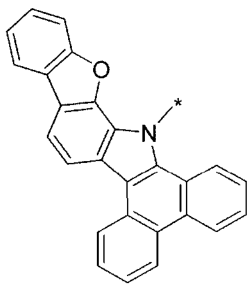
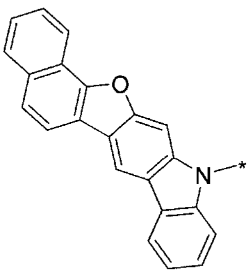
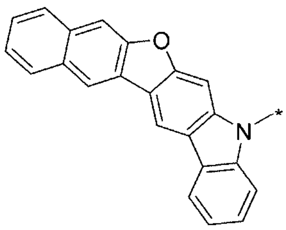
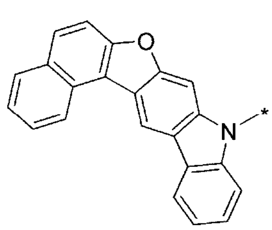

D388 D389 D390 D391

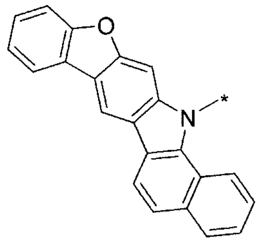

D392

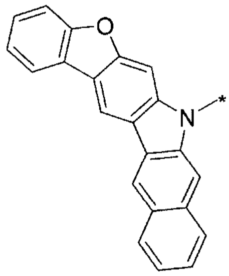

D393

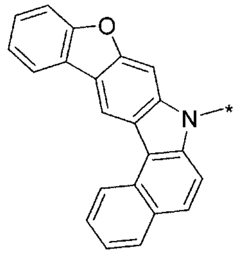

D394

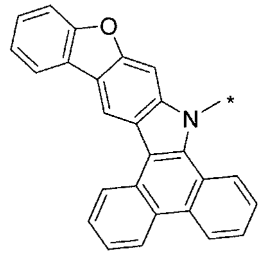

D395

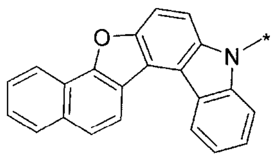

D396

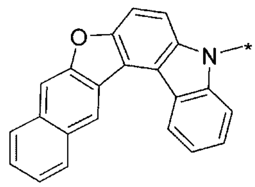

D397

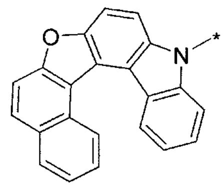

D398

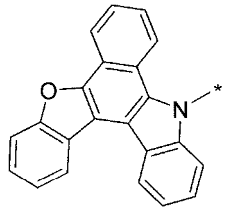

D399

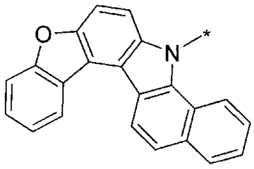

D400

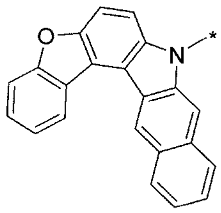

D401

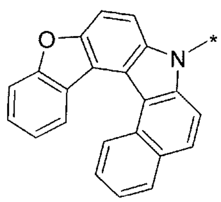

D402

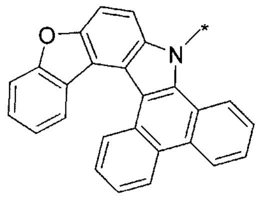

D403

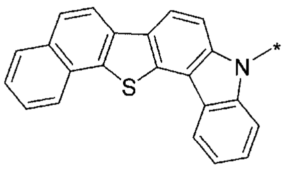

D404

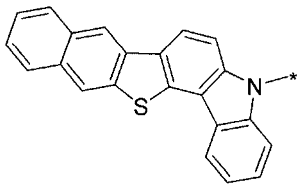

D405

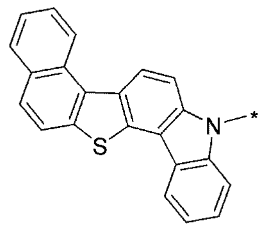

D406

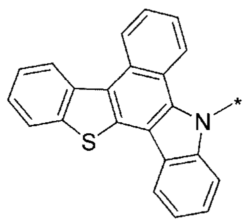

D407

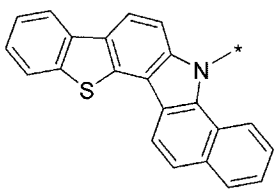

D408

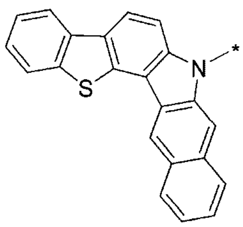

D409

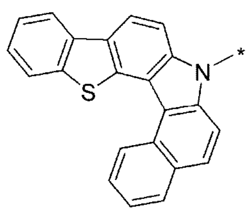

D410

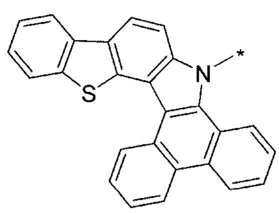

D411

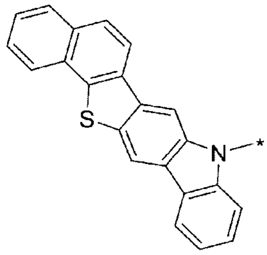

D412

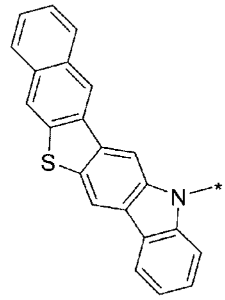

D413

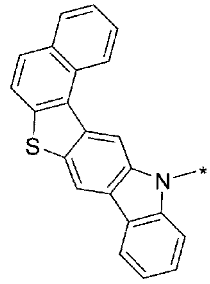

D414

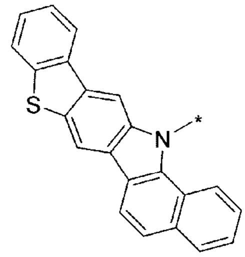

D415

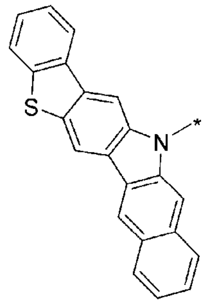

D416

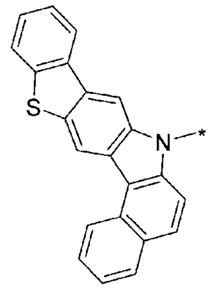

D417

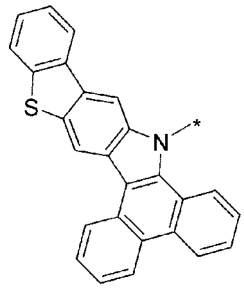

D418

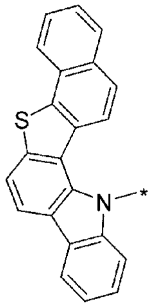

D419

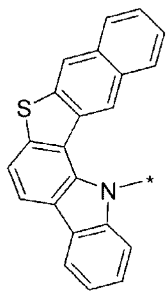

D420

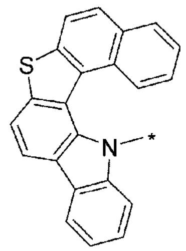

D421

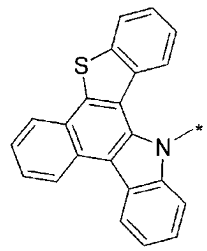

D422

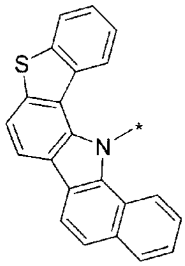

D423

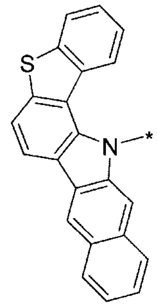

D424

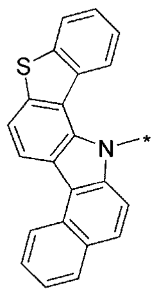

D425

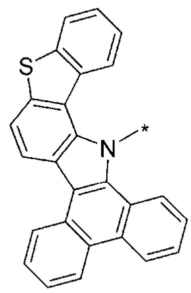

D426

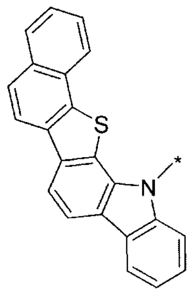

D427

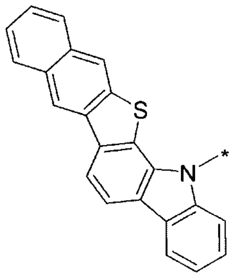

D428

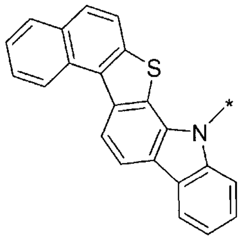

D429

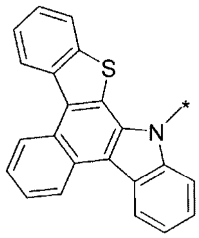

D430

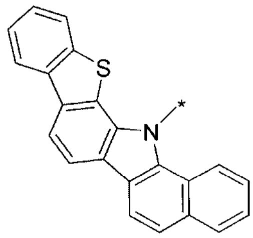

D431

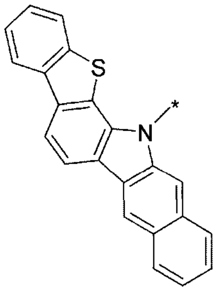

D432

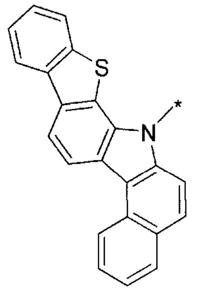

D433

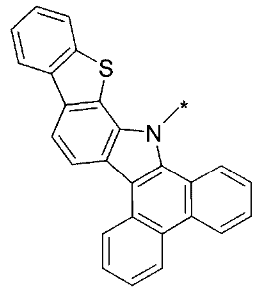

D434

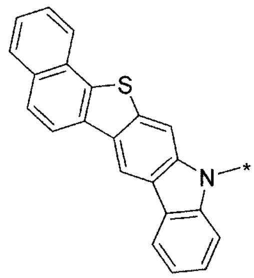

D435

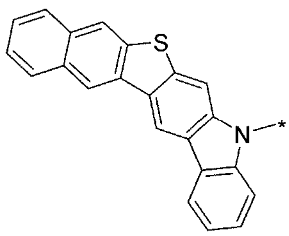

D436

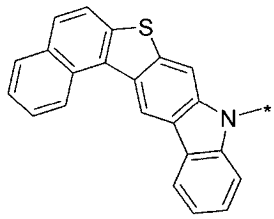

D437

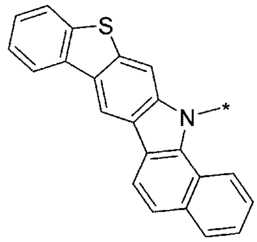

D438

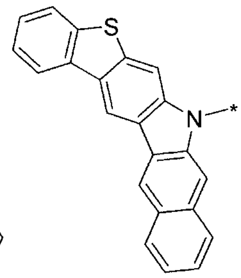

D439

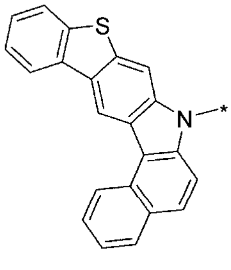

D440

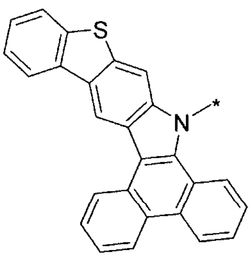

D441

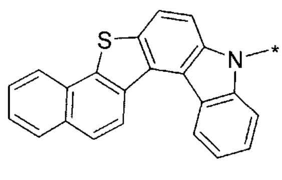

D442

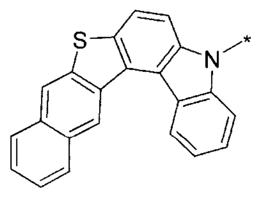

D443

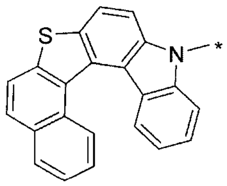
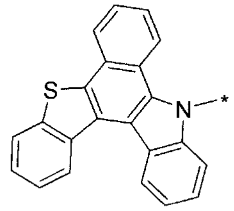
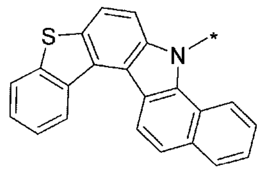
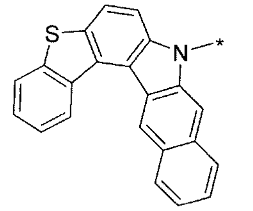

D444 D445 D446 D447

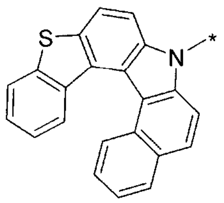
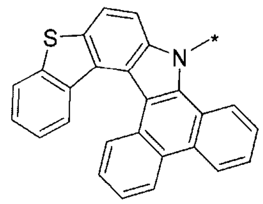
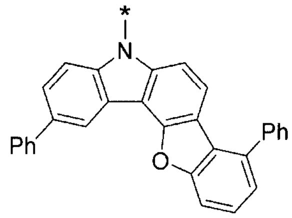
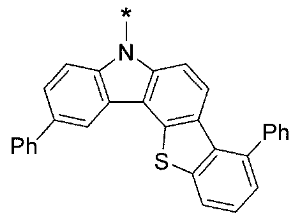

D448 D449 D450 D451

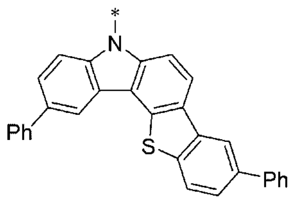
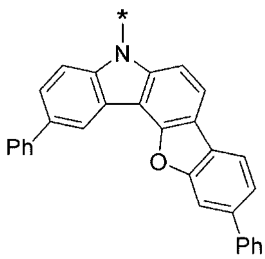
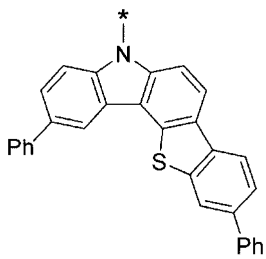
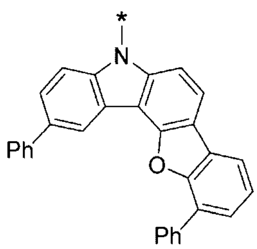

D452 D453 D454 D455

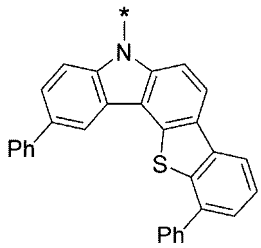
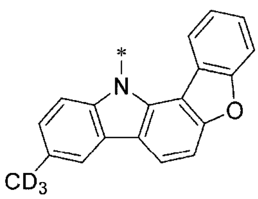
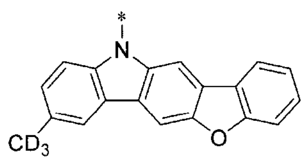

D456 D457 D458

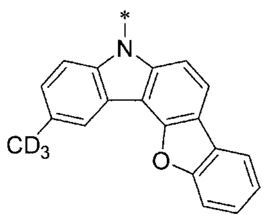
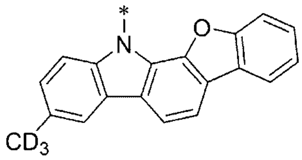
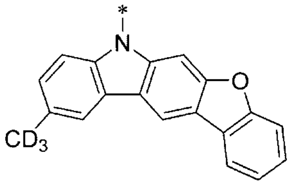
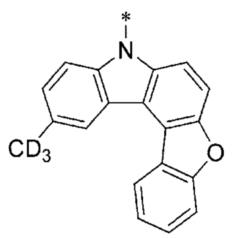

D459 D460 D461 D462

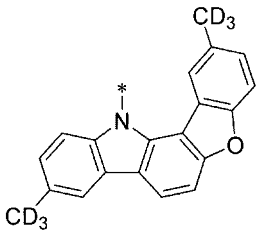
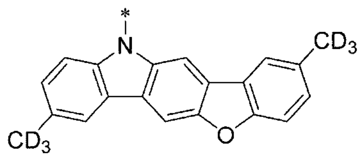
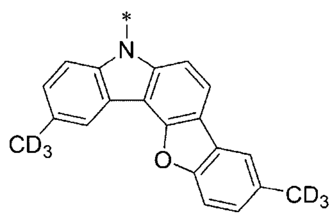

D463 D464 D465

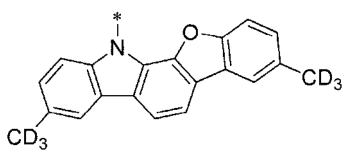
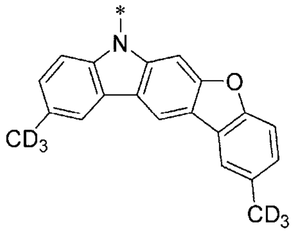
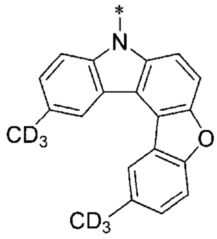
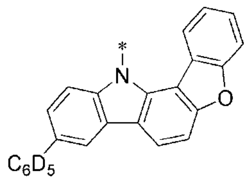

D466 D467 D468 D469

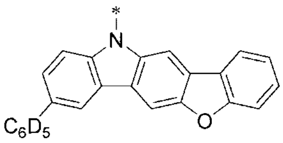
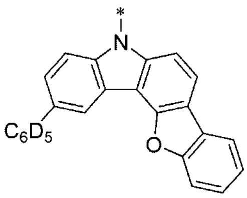
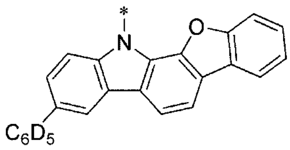
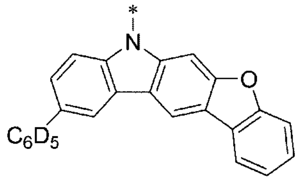

D470 D471 D472 D473

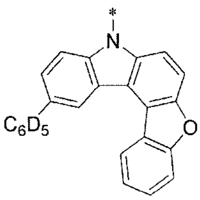
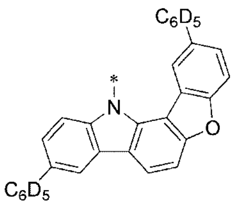
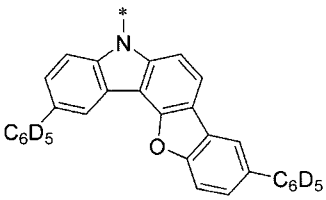
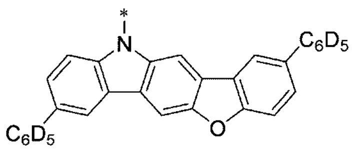

D474 D475 D476 D477

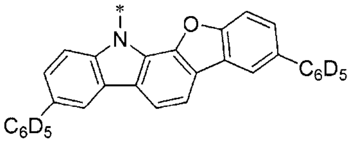
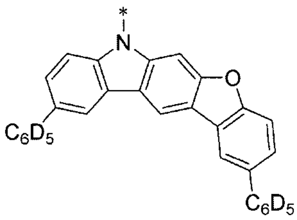
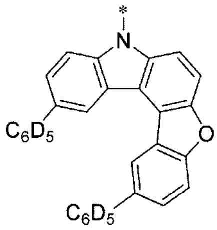
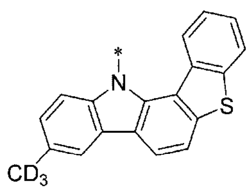

D478 D479 D480 D481

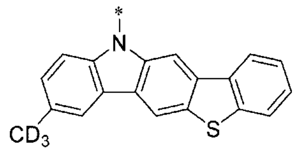

D482

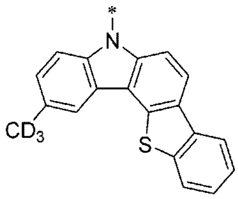

D483

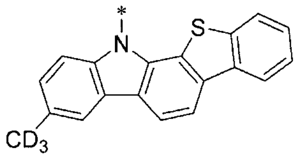

D484

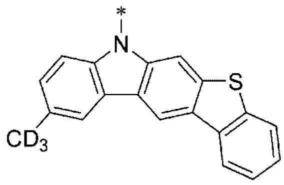

D485

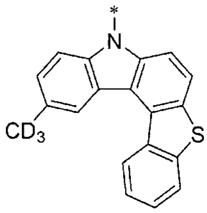

D486

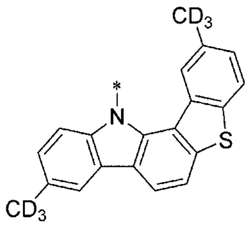

D487

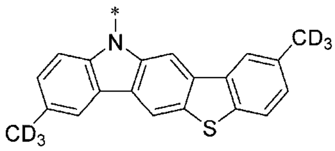

D488

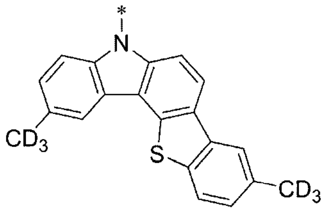

D489

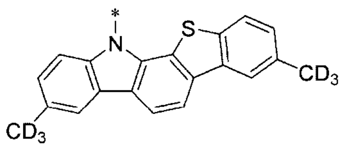

D490

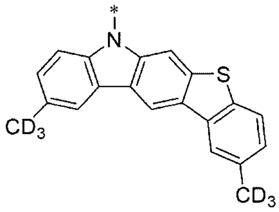

D491

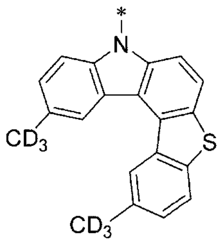

D492

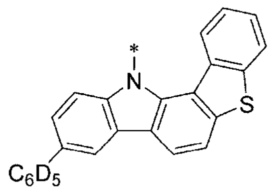

D493

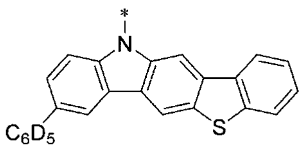

D494

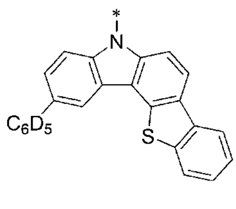

D495

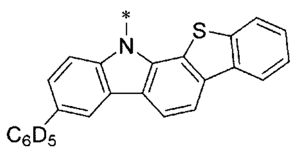

D496

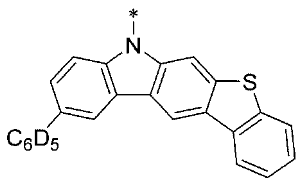

D497

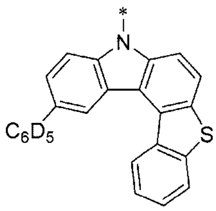

D498

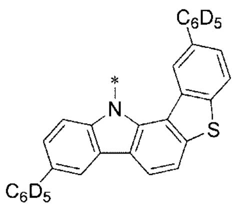

D499

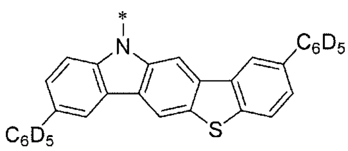

D500

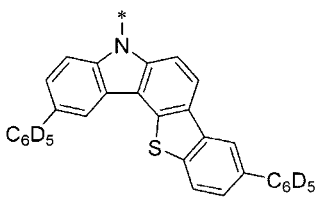

D501

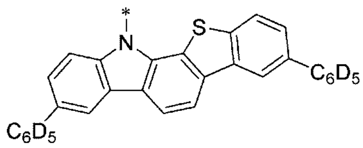

D502

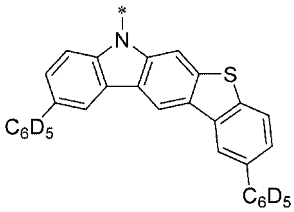

D503

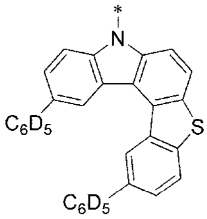

D504

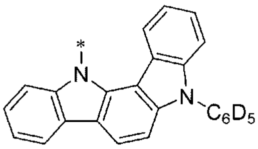

D505

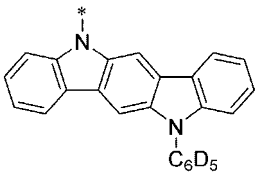

D506

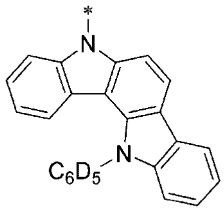

D507

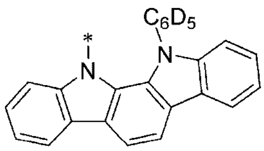

D508

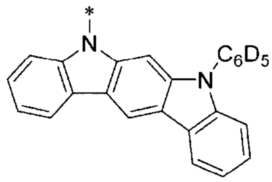

D509

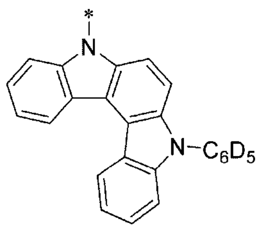

D510

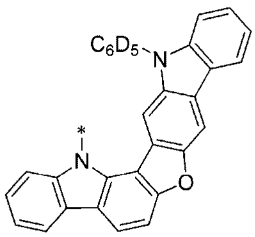

D511

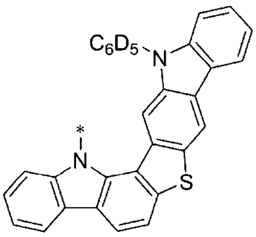

D512

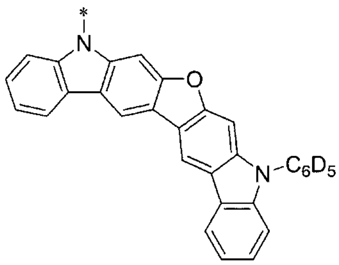

D513

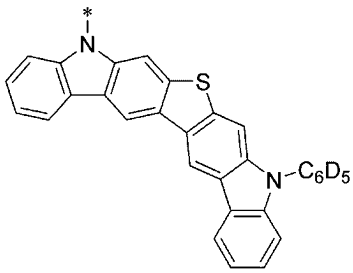

D514

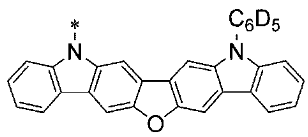

D515

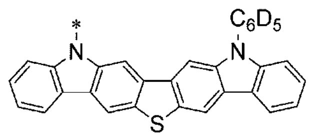

D516

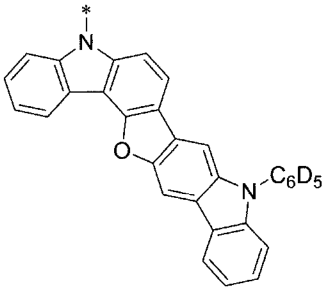

D517

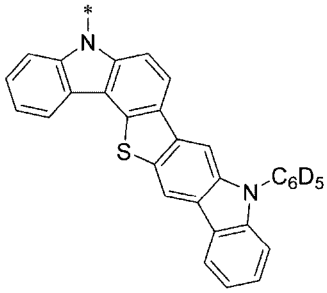

D518

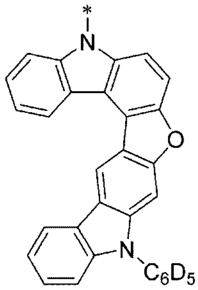

D519

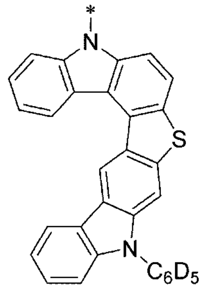

D520

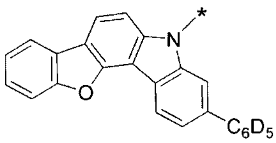 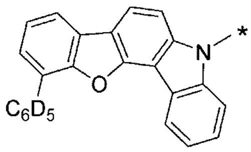 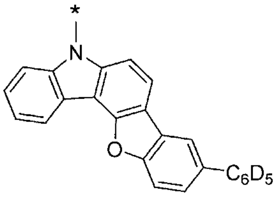 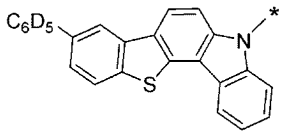

D521 D522 D523 D524

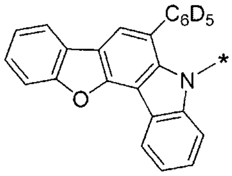 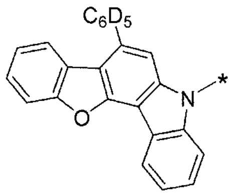 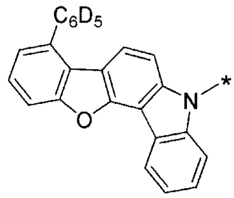 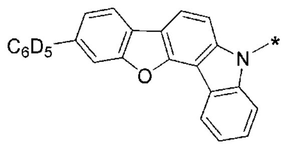

D525 D526 D527 D528

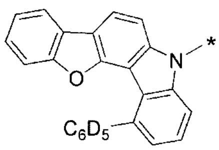 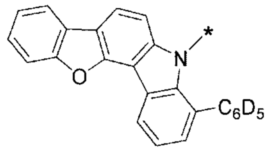 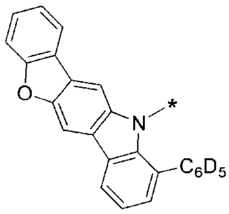 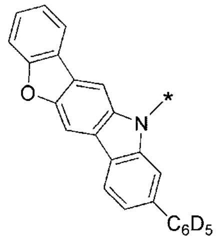 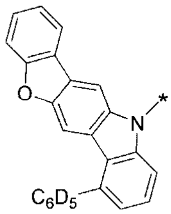

D529 D530 D531 D532 D533

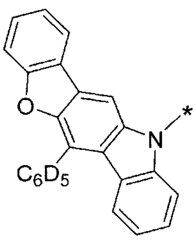 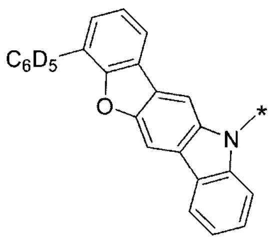 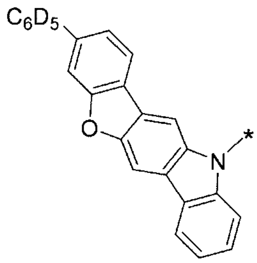 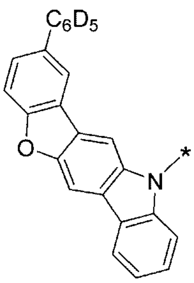

D534 D535 D536 D537

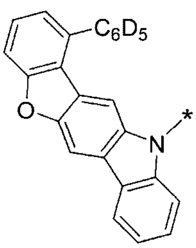 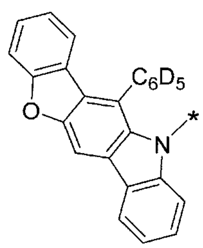 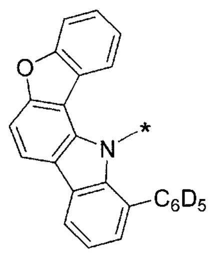 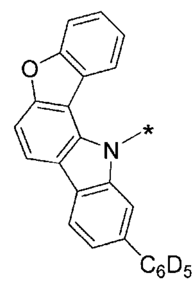

D538 D539 D540 D541

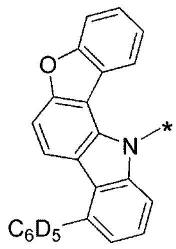
D542

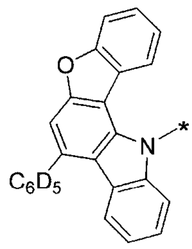
D543

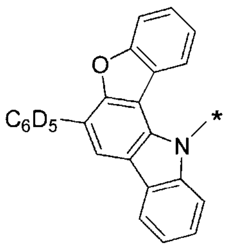
D544

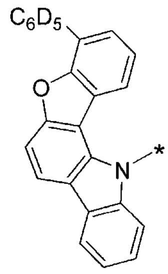
D545

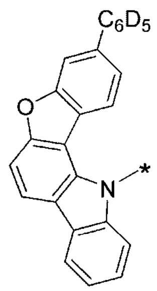
D546

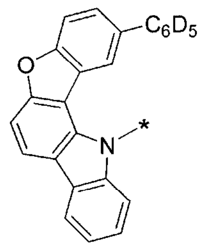
D547

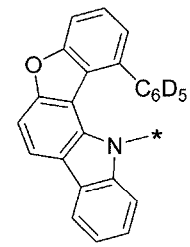
D548

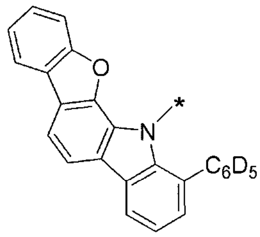
D549

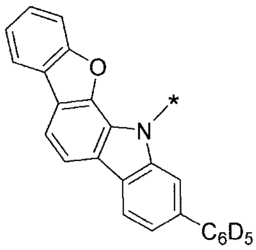
D550

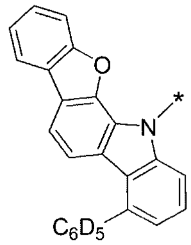
D551

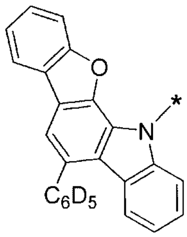
D552

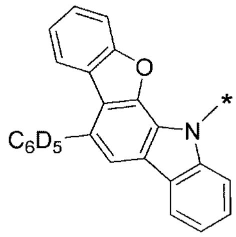
D553

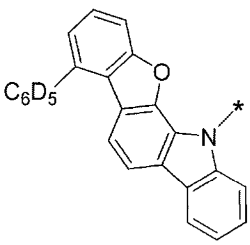
D554

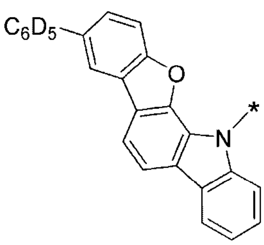
D555

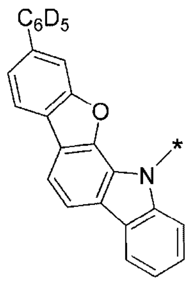
D556

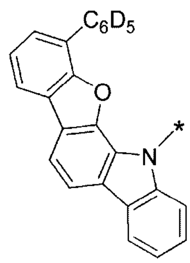
D557

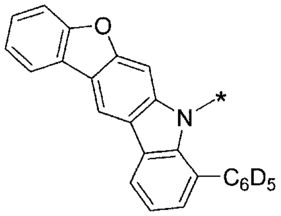 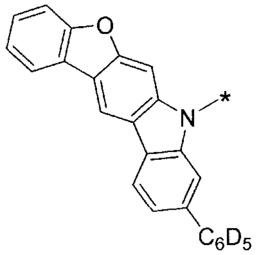 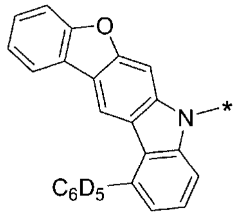 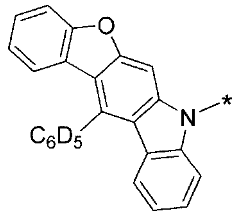

D558 D559 D560 D561

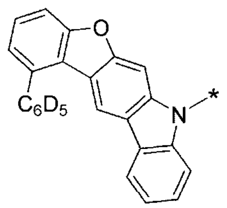 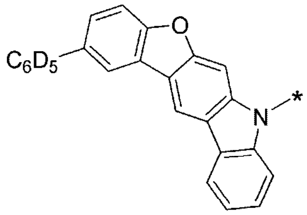 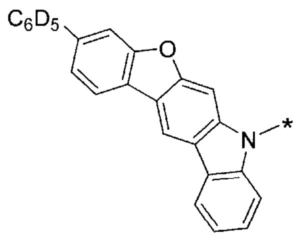 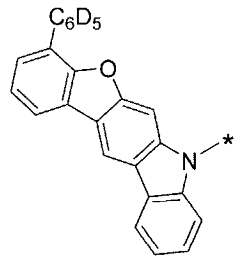

D562 D563 D564 D565

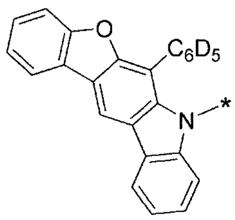 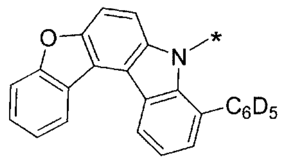 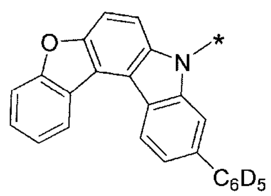 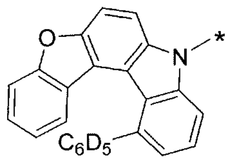

D566 D567 D568 D569

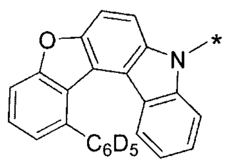 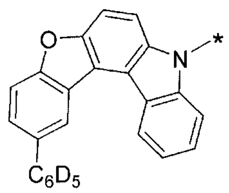 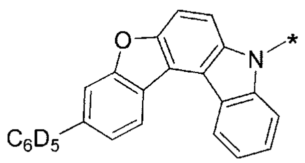 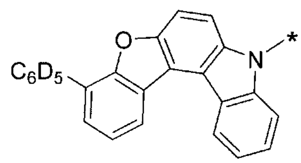

D570 D571 D572 D573

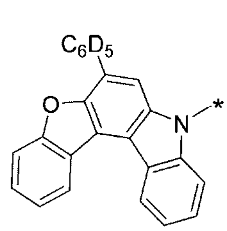 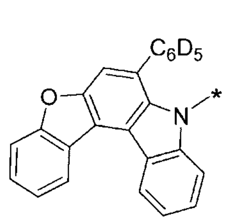 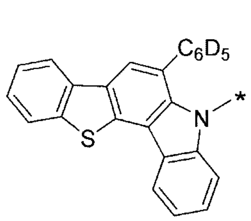 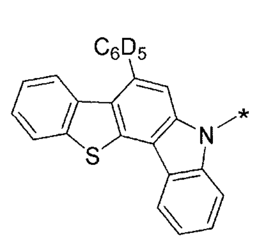

D574 D575 D576 D577

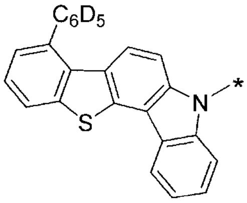
D578

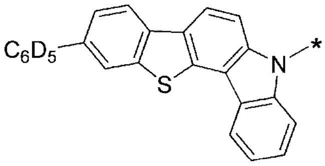
D579

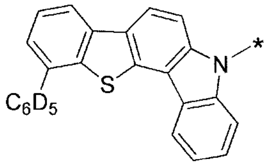
D580

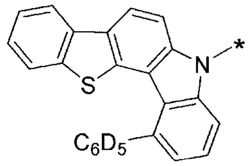
D581

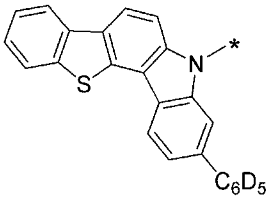
D582

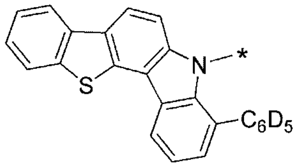
D583

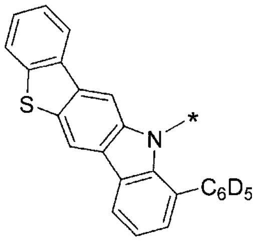
D584

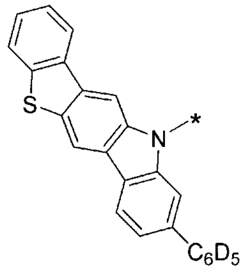
D585

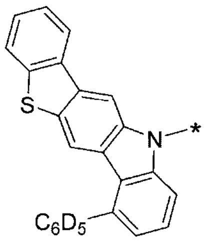
D586

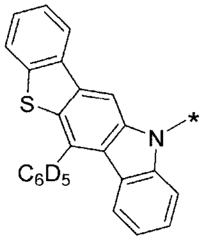
D587

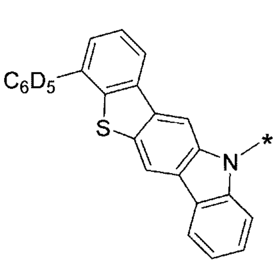
D588

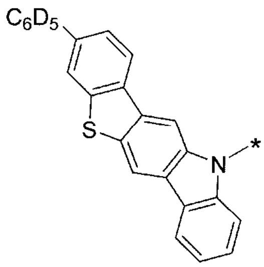
D589

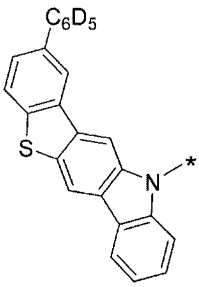
D590

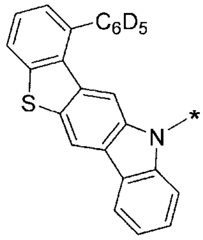
D591

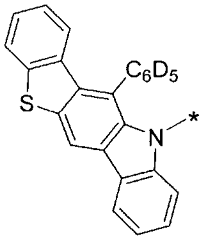
D592

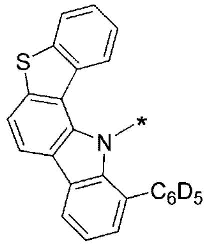
D593

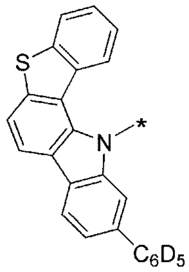
D594

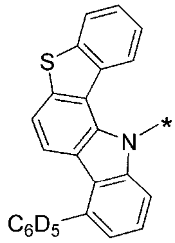
D595

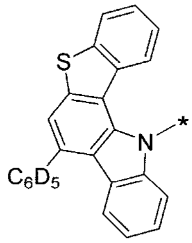
D596

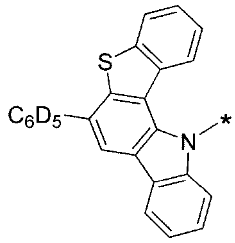
D597

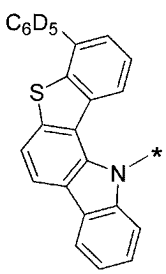
D598

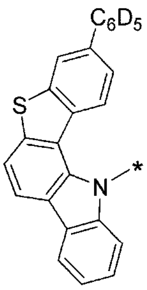
D599

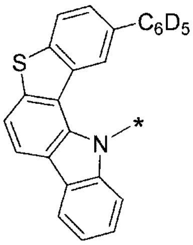
D600

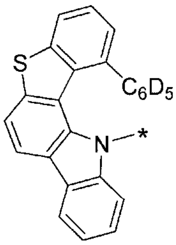
D601

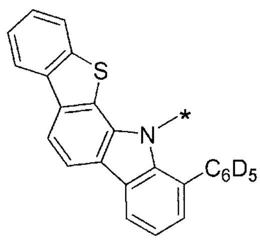
D602

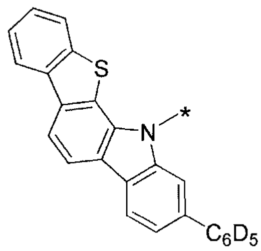
D603

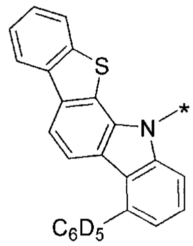
D604

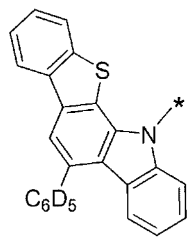
D605

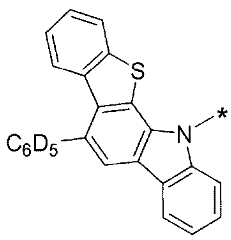
D606

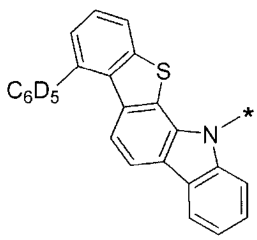
D607

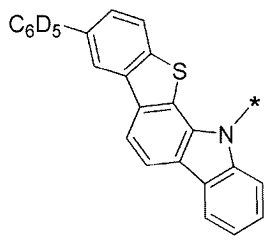
D608

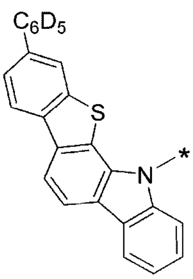
D609

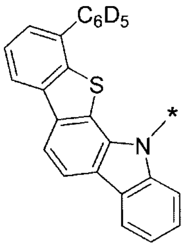
D610

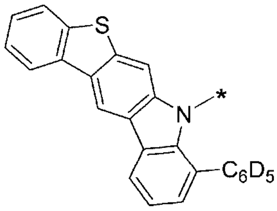
D611

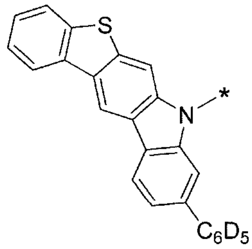
D612

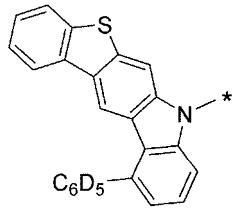
D613

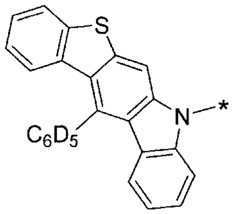
D614

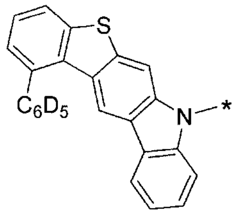
D615

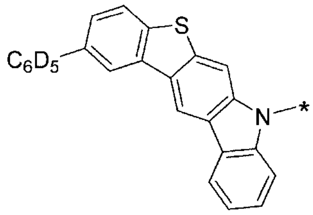
D616

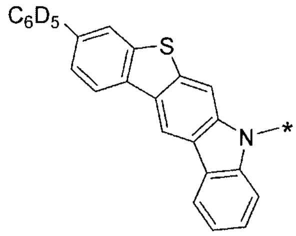
D617

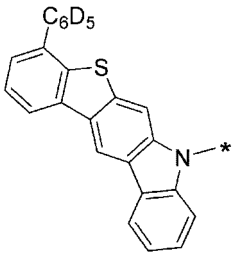
D618

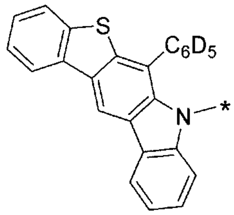
D619

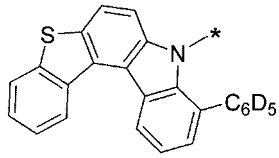
D620

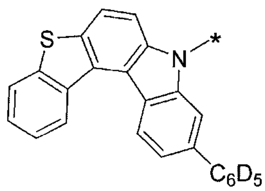
D621

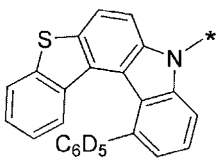
D622

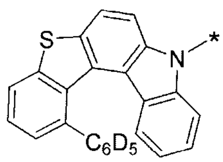
D623

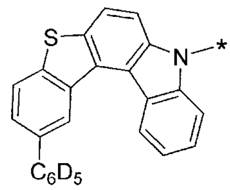
D624

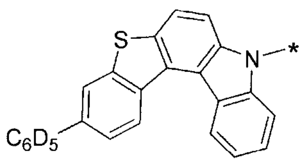
D625

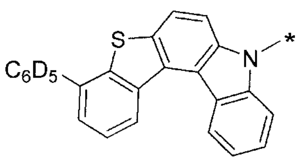
D626

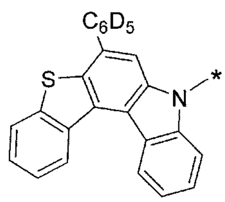
D627

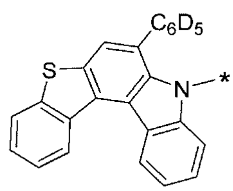
D628

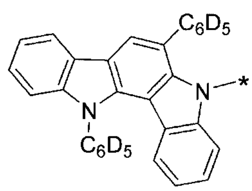
D629

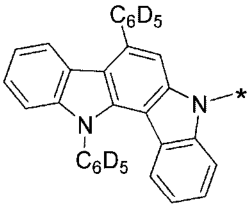 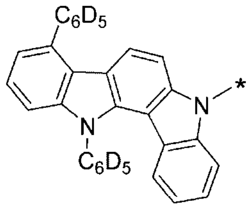 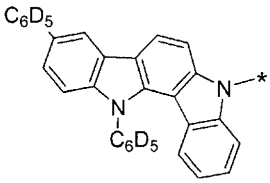 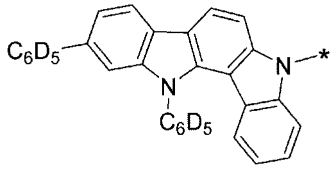

D630 D631 D632 D633

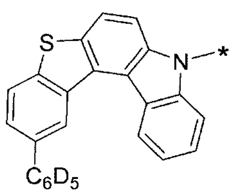 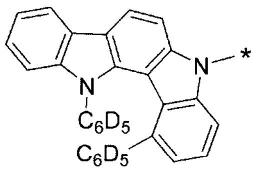 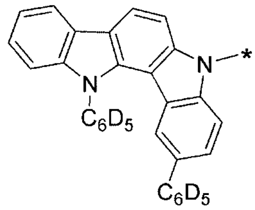 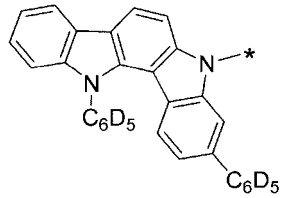

D634 D635 D636 D637

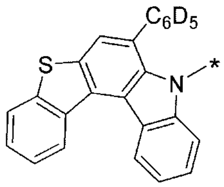 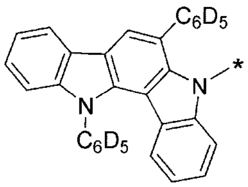 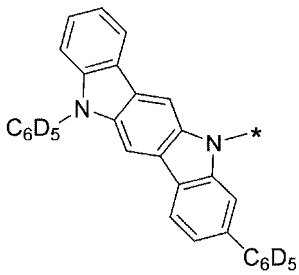 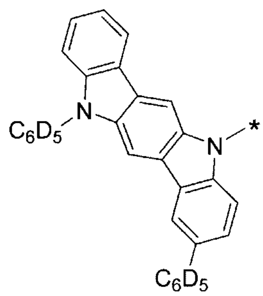

D638 D639 D640 D641

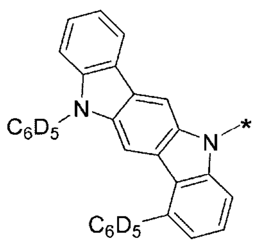 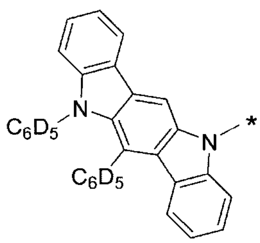 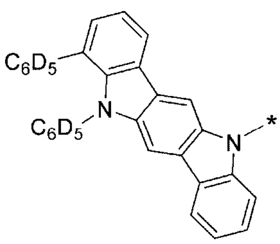 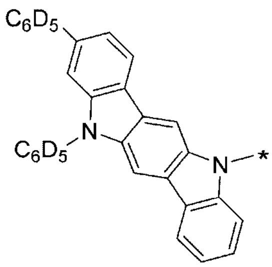

D642 D643 D644 D645

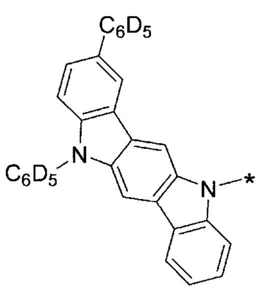 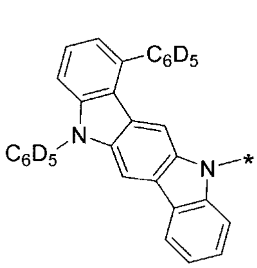 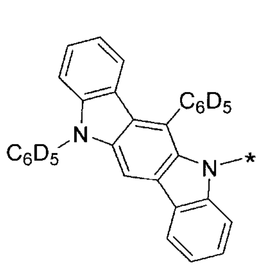 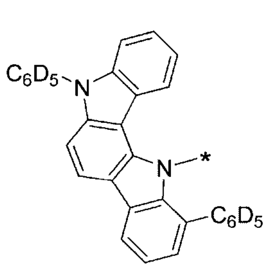

D646 D647 D648 D649

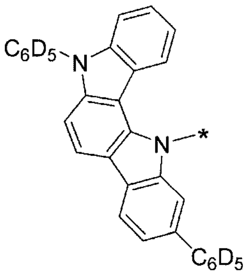
D650

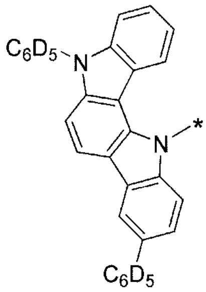
D651

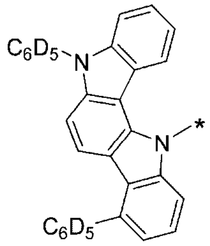
D652

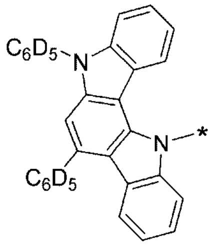
D653

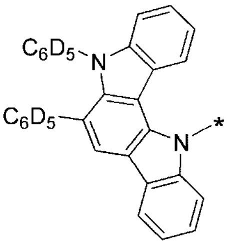
D654

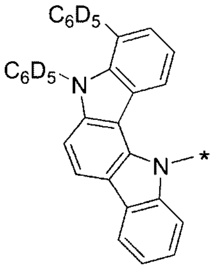
D655

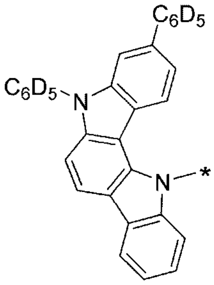
D656

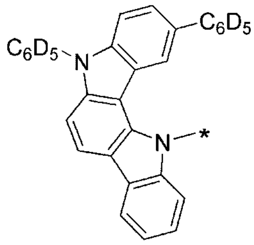
D657

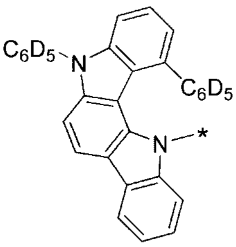
D658

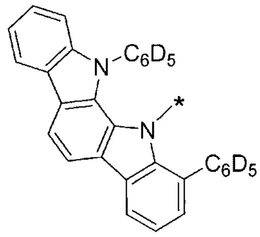
D659

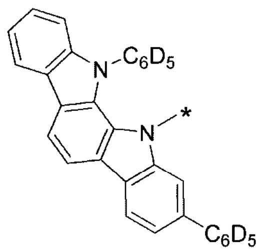
D660

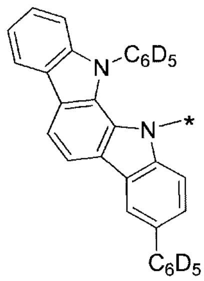
D661

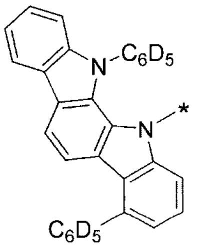
D662

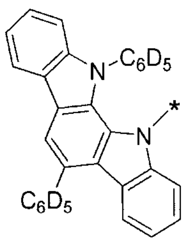
D663

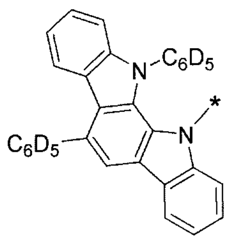
D664

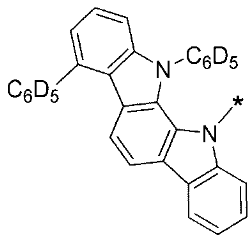
D665

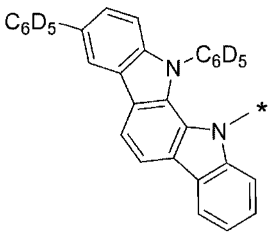
D666

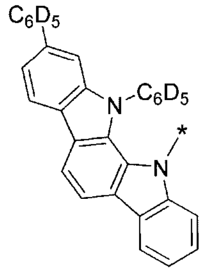
D667

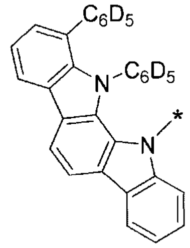
D668

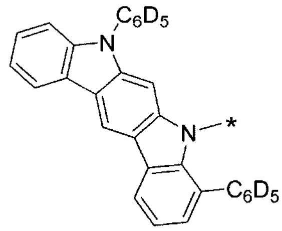
D669

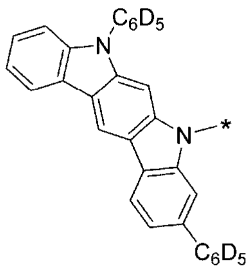
D670

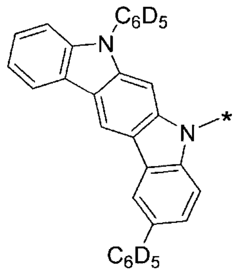
D671

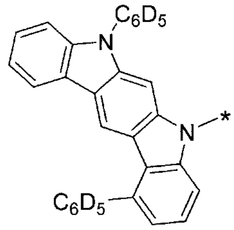
D672

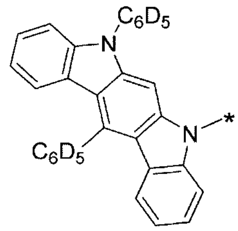
D673

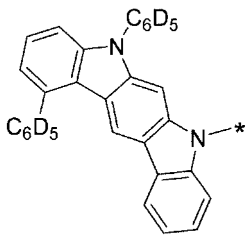
D674

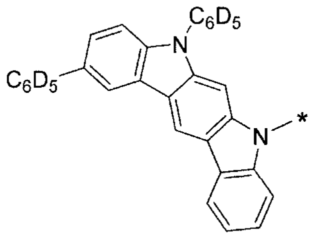
D675

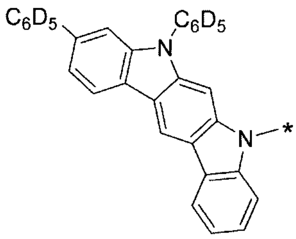
D676

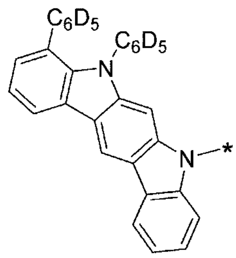
D677

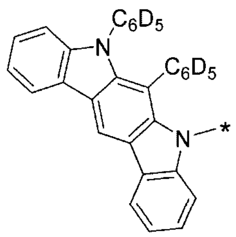
D678

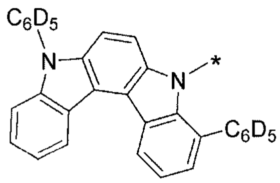
D679

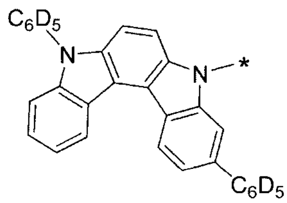
D680

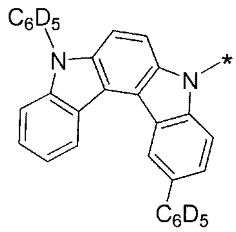
D681

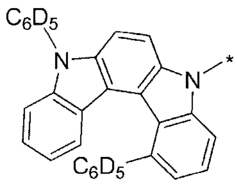
D682

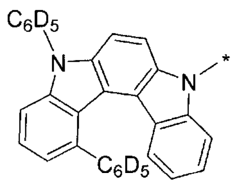
D683

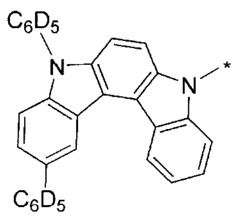
D684

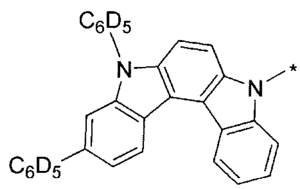
D685

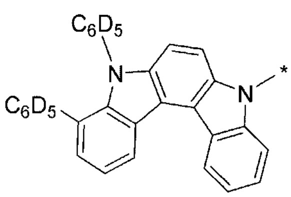
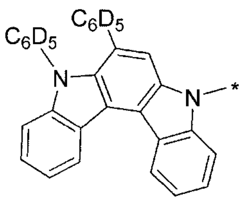
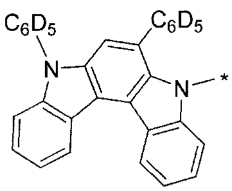
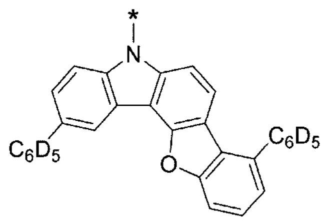

D686 D687 D688 D689

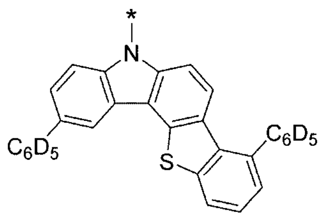
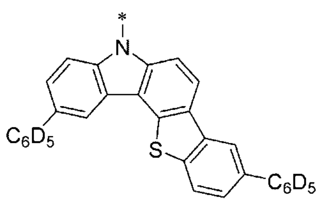
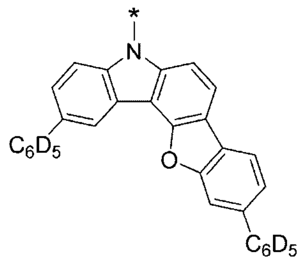

D690 D691 D692

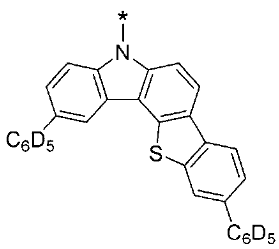
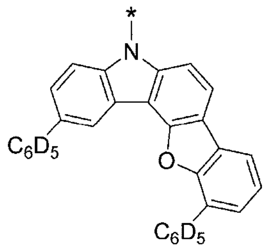
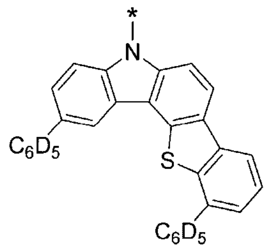

D693 D694 D695

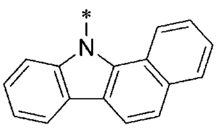
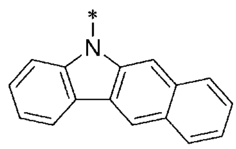
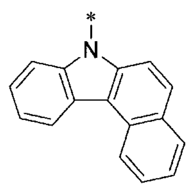
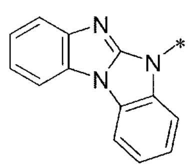

D696 D697 D698 D699

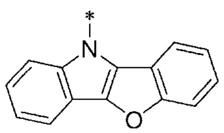
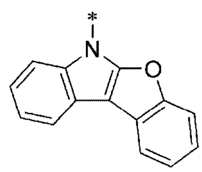
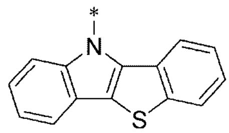
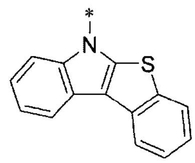

D700 D701 D702 D703

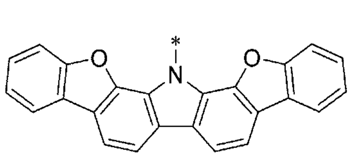

D704

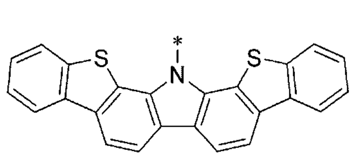

D705

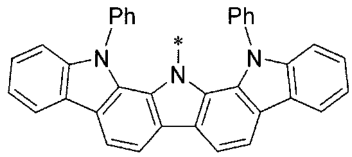

D706

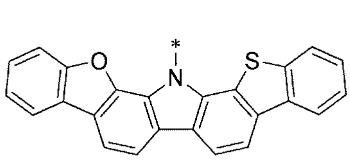

D707

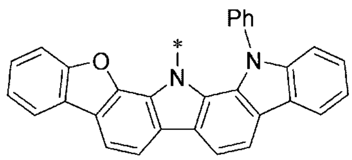

D708

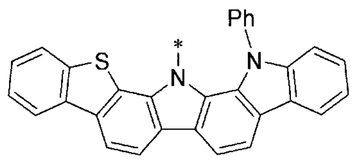

D709

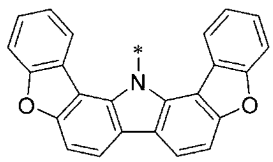

D710

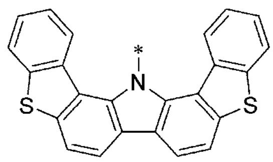

D711

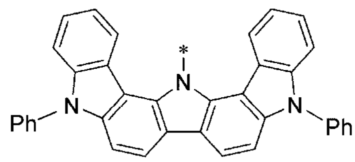

D712

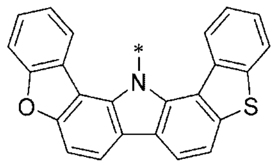

D713

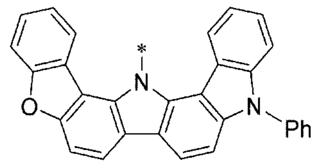

D714

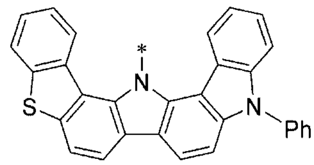

D715

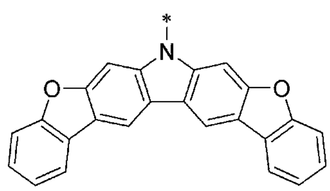

D716

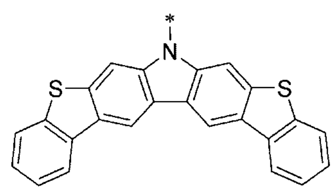

D717

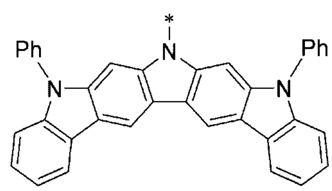

D718

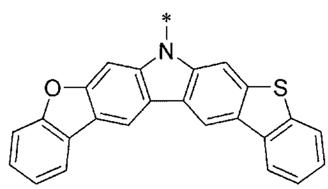

D719

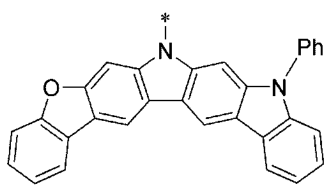

D720

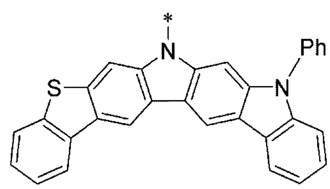

D721

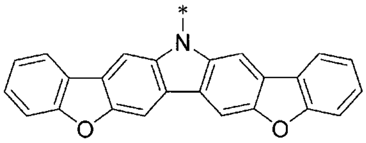
D722

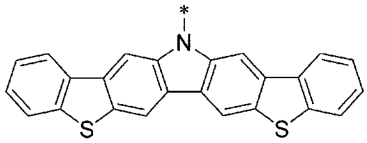
D723

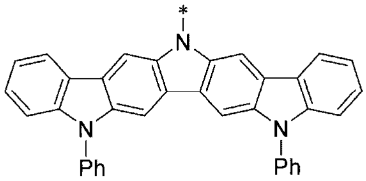
D724

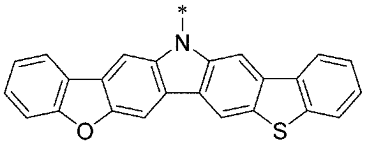
D725

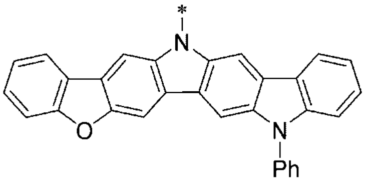
D726

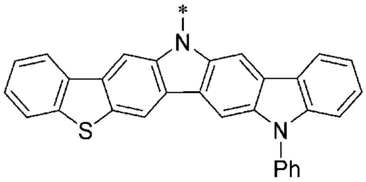
D727

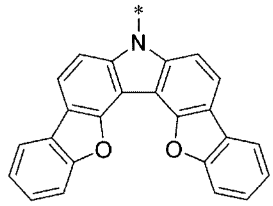
D728

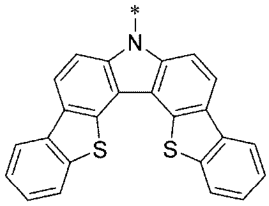
D729

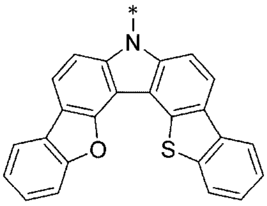
D730

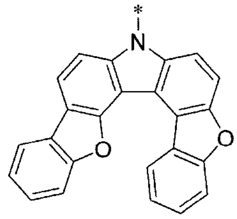
D731

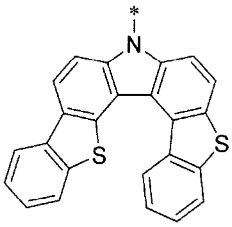
D732

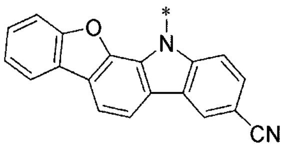
D733

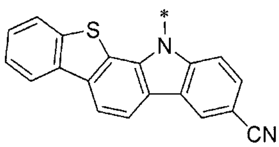
D734

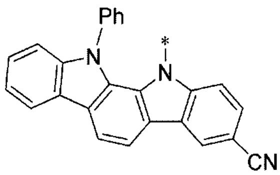
D735

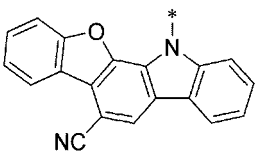
D736

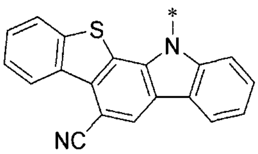
D737

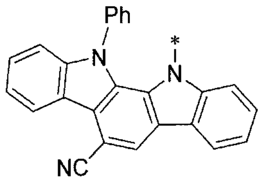
D738

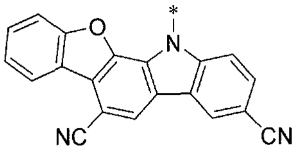
D739

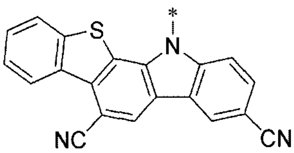
D740

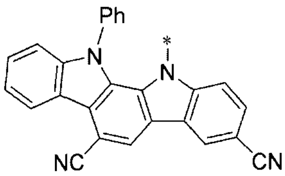
D741

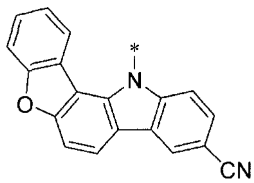
D742

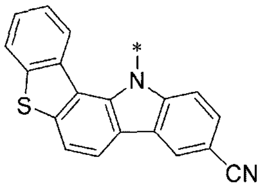
D743

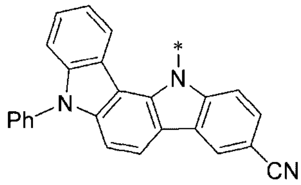

D744

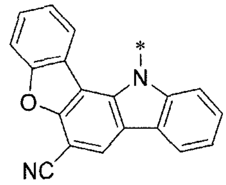

D745

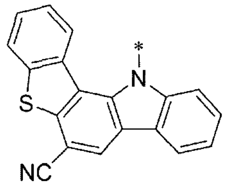

D746

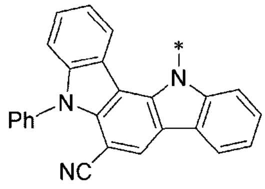

D747

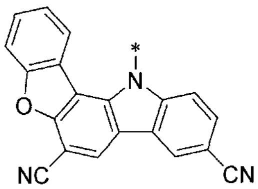

D748

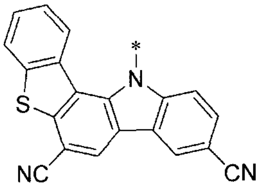

D749

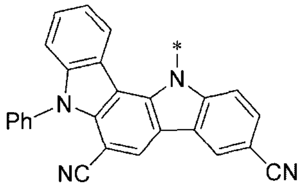

D750

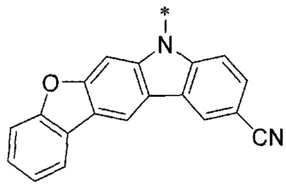

D751

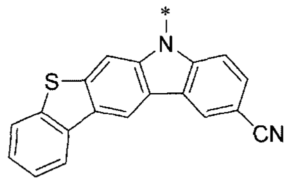

D752

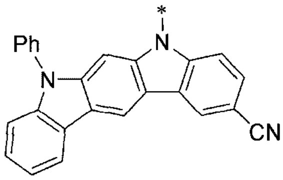

D753

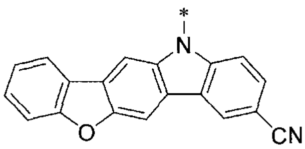

D754

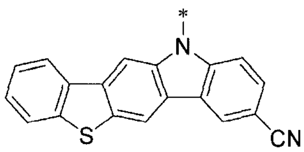

D755

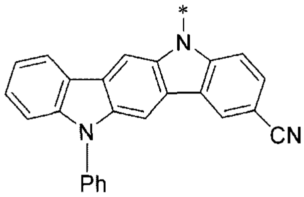

D756

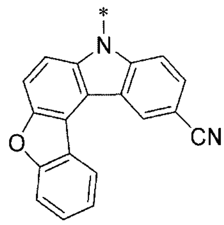

D757

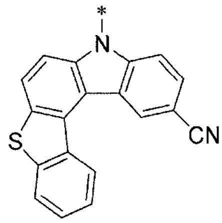

D758

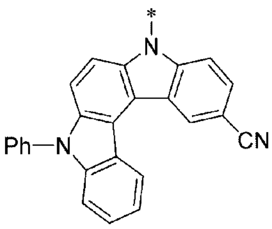

D759

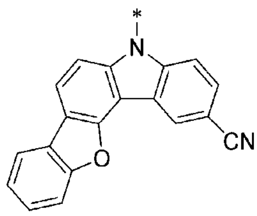

D760

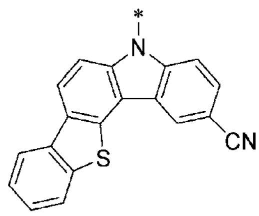

D761

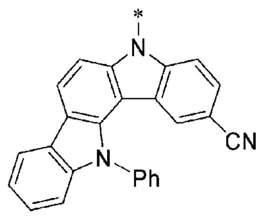

D762

Groups obtained by substituting all hydrogen atoms present in the above D86 to D456 and D696 to D762 with deuterium atoms are disclosed as D763 to D1210.

In one aspect of the present invention, the donor group that $R^1$ to $R^5$ can adopt is selected from the group consisting of D1 to D1210. In one aspect of the present invention, the donor group that $R^1$ to $R^5$ can adopt is selected from the group consisting of D1 to D85. In one aspect of the present invention, the donor group that $R^1$ to $R^5$ can adopt is selected from the group consisting of D86 to D1210. In one aspect of the present invention, the donor group that $R^1$ to $R^5$ can adopt is selected from the group consisting of D86 to D695, D704 to D1133, and D1142 to D1210. In one aspect of the present invention, the donor group that $R^1$ to $R^5$ can adopt is selected from the group consisting of D696 to D703, and D1134 to D1141.

Two or more of $R^1$ to $R^5$ in the general formula (1) are donor groups. In one aspect of the present invention, two or three of $R^1$ to $R^5$ are donor groups. In one preferred aspect of the present invention, two of $R^1$ to $R^5$ are donor groups. In one preferred aspect of the present invention, three of $R^1$ to $R^5$ are donor groups. In one aspect of the present invention, at least $R^3$ is a donor group. In one aspect of the present invention, at least $R^4$ is a donor group. In one aspect of the present invention, at least $R^5$ is a donor group. In one aspect of the present invention, only $R^3$ is a donor group. In one aspect of the

present invention, only $R^4$ is a donor group. In one aspect of the present invention, only $R^5$ is a donor group. In one aspect of the present invention, only $R^3$ and $R^5$ are donor groups. In one aspect of the present invention, only $R^2$ and $R^5$ are donor groups. In one aspect of the present invention, only $R^2$ and $R^4$ are donor groups. In one aspect of the present invention, only $R^3$, $R^4$, and $R^5$ are donor groups. In one aspect of the present invention, only $R^2$, $R^4$, and $R^5$ are donor groups. When two or more of $R^1$ to $R^5$ are donor groups, these may be the same or different.

The number of $R^1$ to $R^5$ that are a hydrogen atom or a deuterium atom is 0 to 2, preferably 0 or 1, and is, for example, 1, or for example, 0. For example, $R^1$ is a hydrogen atom or a deuterium atom. These show more excellent light emission characteristics than the compounds where the number of $R^1$ to $R^5$ that are a hydrogen atom or a deuterium atom is 3. The number of $R^1$ to $R^5$ that are a substituted or unsubstituted aryl group is 0 or 1, and is preferably 1. The number may be 0. The number of $R^1$ to $R^5$ that are a substituted or unsubstituted alkyl group is 0 to 3, preferably 0 to 2, and may be 1 or may be 0.

In one aspect of the present invention, $R^1$ to $R^5$ are each independently selected from a hydrogen atom, a deuterium atom, a substituted or unsubstituted alkyl group, a donor group, and a group represented by the general formula (2). In one aspect of the present invention, $R^1$ to $R^5$ are each independently selected from a hydrogen atom, a deuterium atom, a donor group, and a group represented by the general formula (2). In one aspect of the present invention, $R^1$ to $R^5$ are each independently selected from a hydrogen atom, a deuterium atom, a substituted or unsubstituted carbazol-9-yl group which may be fused, and a group represented by the general formula (2).

The heteroaryl group that $Ar^1$ and $Ar^2$ in the general formula (1) can adopt may be a monocyclic ring or may be a fused ring in which two or more rings are fused. In the case of a fused ring, the number of fused rings is preferably 2 to 6, and can be selected from, for example, 2 to 4. Specific examples of the ring include a pyridine ring, a pyrimidine ring and a pyrrole ring, and these rings may be fused with any other ring. Specific examples of the heteroaryl group include a 2-pyridyl group, a 3-pyridyl group, a 4-pyridyl group, a carbazol-9-yl group, a carbazol-1-yl group, a carbazol-2-yl group, a carbazol-3-yl group, and a carbazol-4-yl group. The number of the ring skeleton-constituting atoms of the heteroaryl group is preferably 4 to 40, more preferably 5 to 20, and may be selected from a range of 5 to 16, or may be selected from a range of 5 to 12.

In one aspect of the present invention, $Ar^1$ and $Ar^2$ are substituted or unsubstituted aryl groups.

In the general formula (1), $R^2$ or $R^3$ is a group represented by the following general formula (2). In addition, $R^1$, $R^4$, and $R^5$ can also adopt a group represented by the following general formula (2).

General Formula (2)

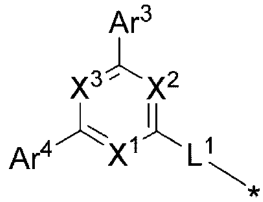

in which $X^1$ to $X^3$ each independently represent N or C(R), and at least one of $X^1$ to $X^3$ is N; R represents a hydrogen atom, a deuterium atom, or a substituent; $Ar^3$ and $Ar^4$ each independently represent a substituted or unsubstituted aryl group, or a substituted or unsubstituted heteroaryl group; $L^1$ represents a single bond, a substituted or unsubstituted arylene group, or a substituted or unsubstituted heteroarylene group; and * represents a bonding position.

In the general formula (2), $L^1$ represents a single bond or a divalent linking group. Examples of the divalent linking group include a substituted or unsubstituted arylene group, and a substituted or unsubstituted heteroarylene group. In one preferred aspect of the present invention, $L^1$ represents a single bond. In one aspect of the present invention, $L^1$ represents a substituted or unsubstituted arylene group. In one aspect of the present invention, $L^1$ represents a substituted or unsubstituted heteroarylene group. Regarding the aryl moiety constituting the arylene group, reference can be made to the description and the preferred range of the aryl group in the description section of $R^1$ to $R^5$ mentioned above. Examples of the heteroarylene group include a linking group formed by substituting at least one ring skeleton carbon atom constituting the arylene group with a nitrogen atom.

Specific examples of $L^1$ are shown below. Here, $L^1$ which can be employed in the present invention shall not be construed as being limited by these specific examples. In the following specific examples, the expression of a methyl group is omitted. Therefore, for example, L3 to L5 are substituted with a methyl group. * indicates a bonding position. L1 is a single bond.

L1

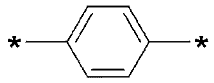
L2

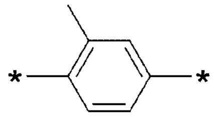
L3

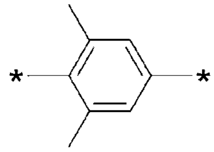
L4

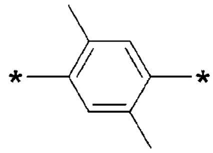
L5

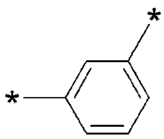
L6

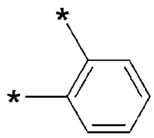
L7

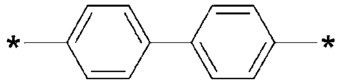
L8

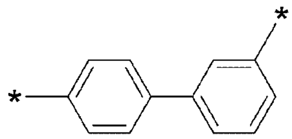
L9

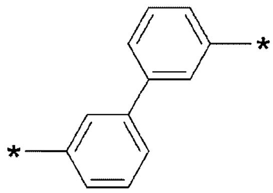
L10

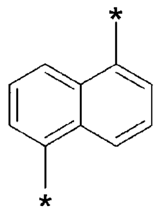
L11

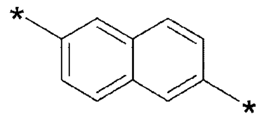
L12

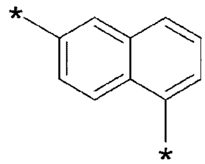
L13

Groups obtained by substituting all hydrogen atoms present in the above L2 to L13 with deuterium atoms are disclosed as L14 to L25. In one aspect of the present invention, $L^1$ is selected from the group consisting of L1 to L25. In one aspect of the present invention, $L^1$ is selected from the group consisting of L1 to L7, and L14 to L19. In one aspect of the present invention, $L^1$ is selected from the group consisting of L1, L8 to L13, and L20 to L25. In one aspect of the present invention, $L^1$ is selected from the group consisting of L2 to L25.

In the general formula (2), $X^1$ to $X^3$ each independently represent N or C(R). Here, at least one of $X^1$ to $X^3$ is N. R represents a hydrogen atom, a deuterium atom, or a substituent. The substituent referred to herein may be selected from Substituent Group A, may be selected from Substituent Group B, may be selected from Substituent Group C, may be selected from Substituent Group D, or may be selected from Substituent Group E. In one preferred aspect of the present invention, $X^1$ to $X^3$ are N. In one aspect of the present invention, $X^1$ and $X^3$ are N, and $X^2$ is C(R). In one aspect of the present invention, $X^1$ and $X^2$ are N, and $X^3$ is C(R). In one aspect of the present invention, $X^1$ is N, and $X^2$ and $X^3$ are C(R). In one aspect of the present invention, $X^2$ is N, and $X^1$ and $X^3$ are C(R). In one aspect of the present invention, R is a hydrogen atom or a deuterium atom. In one aspect of the present invention, R is an alkyl group optionally substituted with a deuterium atom. In one aspect of the present invention, R is an aryl group optionally substituted with a deuterium atom, an alkyl group, or an aryl group.

Regarding specific examples and preferred ranges of $Ar^3$ and $Ar^4$ in the general formula (2), reference can be made to the corresponding description of $Ar^1$ and $Ar^2$ in the general formula (1). In one aspect of the present invention, $Ar^1$ and $Ar^3$ are the same. In one preferred aspect of the present invention, $Ar^1$ and $Ar^3$ are the same, and $Ar^2$ and $Ar^4$ are the same. In one aspect of the present invention, $Ar^1$ to $Ar^4$ are the same.

In one preferred aspect of the present invention, $X^1$ to $X^3$ are N, and $L^1$ is a single bond. In one aspect of the present invention, $X^1$ to $X^3$ are N, and $L^1$ is a substituted or unsubstituted arylene group, preferably a substituted or unsubstituted phenylene group, and further preferably an unsubstituted phenylene group (for example, L2, for example, L6).

In one aspect of the present invention, $X^1$ to $X^3$ are N, $L^1$ is a single bond, and $Ar^3$ and $Ar^4$ are the same. In one aspect of the present invention, $X^1$ to $X^3$ are N, $L^1$ is a single bond, and $Ar^3$ and $Ar^4$ are each independently a substituted or unsubstituted aryl group. In one aspect of the present invention, $X^1$ to $X^3$ are N, $L^1$ is a single bond, and $Ar^3$ and $Ar^4$ are each independently a substituted or unsubstituted heteroaryl group (excluding a nitrogen-containing 6-membered ring group), preferably a donor heteroaryl group bonded via a nitrogen atom, and more preferably a substituted or unsubstituted carbazol-9-yl group. In one aspect of the present invention, $X^1$ to $X^3$ are N, $L^1$ is a single bond, $Ar^3$ is a substituted or unsubstituted heteroaryl group (excluding a nitrogen-containing 6-membered ring group), preferably a donor heteroaryl group bonded via a nitrogen atom, and more preferably a substituted or unsubstituted carbazol-9-yl group, and $Ar^4$ is a substituted or unsubstituted aryl group.

In one preferred aspect of the present invention, only $R^2$ is a group represented by the general formula (2). In one

preferred aspect of the present invention, only $R^3$ is a group represented by the general formula (2). In one aspect of the present invention, only one of $R^1$, $R^3$, $R^4$, and $R^5$ and $R^2$ are each independently a group represented by the general formula (2). In one aspect of the present invention, only one of $R^1$, $R^2$, $R^4$, and $R^5$ and $R^3$ are each independently a group represented by the general formula (2).

In the compound represented by the general formula (1), at least one of $R^1$ to $R^5$ is a donor group having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions.

The "donor group having a fused ring structure of four or more rings" as referred to herein is a donor group having a fused ring structure of four or more rings and bonding via one atom of the ring skeleton-constituting atoms of the fused ring structure. The one atom is preferably a carbon atom or a nitrogen atom, and more preferably a nitrogen atom. The donor group as referred to herein means a group having a negative Hammett's $\sigma p$ value.

The donor group having a fused ring structure of four or more rings preferably has a fused ring structure of five or more rings, and more preferably has a fused ring structure of five to seven rings. Examples thereof include groups having a fused ring structure of five rings and groups having a fused ring structure of seven rings.

As the donor group having a fused ring structure of four or more rings, a ring-fused carbazol-9-yl group is preferable. Specific examples thereof include D86 to D1210.

In particular, a group having a structure represented by the following general formula (3) is preferable.

General Formula (3)

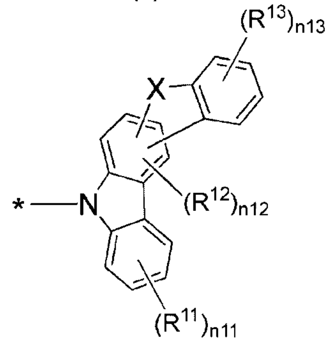

In the general formula (3), X represents O, S, or N-$R^{14}$. $R^{11}$ to $R^{13}$ each independently represent a deuterium atom, or a substituent. $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group. $R^{11}$'s, $R^{12}$'s, and $R^{13}$'s each may be bonded to each other to form a cyclic structure. n11 and n13 each independently represent an integer of 0 to 4, and n12 represents an integer of 0 to 2.

In the general formula (3), X represents O, S, or N-$R^{14}$. $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group. Here, the aryl group as a substituent for the aryl group and the alkyl group can be selected from, for example, an aryl group having 6 to 22 carbon atoms, and the alkyl group as a substituent for the alkyl group and the aryl group can be selected from, for example, an alkyl group having 1 to 20 carbon atoms. In one preferred aspect of the present invention, X is O. In one preferred aspect of the present invention, X is N-$R^{14}$. For example $R^{14}$ of N-$R^{14}$ is an aryl group (for example, having 6 to 22 carbon atoms). The aryl group may be not substituted with or may be substituted with at least one atom or group selected from the group consisting of a deuterium atom, an alkyl group (for example, having 1 to 20 carbon atoms), and an aryl group (for example, having 6 to 22 carbon atoms). X may be an oxygen atom or a sulfur atom.

In the general formula (3), the two bonds bonding to one benzene ring of the carbazole ring bond to the neighboring positions of the benzene ring to form a fused ring structure of the carbazole ring and the hetero-fused ring containing X. For example, in the case where X is O, a benzofurocarbazole ring is formed as a fused ring structure, in the case where X is S, a benzothienocarbazole ring is formed as a fused ring structure, and in the case where X is N-$R^{14}$, an indolocarbazole ring is formed as a fused ring structure. The positions to which the two bonds bond may be the 1-position and the 2-position of the carbazole ring, or may be the 2-position and the 3-position of the carbazole ring, or may be the 3-position and the 4-position of the carbazole ring. In the case where the bonding positions of the two bonds are the 1-position and the 2-position, the position at which the bond of X bonds may be the 1-position or the 2-position, in the case where the bonding positions of the two bonds are the 2-position and the 3-position, the position at which the bond of X bonds may be the 2-position or the 3-position, and in the case where the bonding positions of the two bonds are the 3-position and the 4-position, the position at which the bond of X bonds may be the 3-position or the 4-position.

In the general formula (3), * represents a bonding position.

In the general formula (3), $R^{11}$ to $R^{13}$ each independently represent a deuterium atom or a substituent. $R^{11}$'s, $R^{12}$'s, and $R^{13}$'s each may be bonded to each other to form a cyclic structure, but $R^{11}$ is not bonded to any one of $R^{12}$ to $R^{14}$ to form a cyclic structure, $R^{12}$ is not bonded to any one of $R^{13}$ and $R^{14}$ to form a cyclic structure, and $R^{13}$ is not bonded to $R^{14}$ to form a cyclic structure. For example, the substituent may be selected from Substituent Group A, may be selected from Substituent Group B, may be selected from Substituent Group C, may be selected from Substituent Group D, or may be selected from Substituent Group E. In one preferred aspect of the present invention, the substituent means one group or a combination of two or more groups selected from the group consisting of an alkyl group (for example, having 1 to 20 carbon atoms), and an aryl group (for example, having 6 to 22 carbon atoms).

n11 and n13 each independently represent an integer of 0 to 4, and n12 represents an integer of 0 to 2. When n11 is 2 or more, two or more $R^{11}$'s may be the same or different. When n13 is 2 or more, two or more $R^{13}$'s may be the same or different. When n12 is 2, two $R^{12}$'s may be the same or different. n11 and n13 may be any number of 0, 1, 2, 3, or 4, and n12 may be any number of 0, 1, or 2. When n11 is 1, $R^{11}$ may be a deuterium atom, or may be a substituent. When n11 is 2 or more, all two or more $R^{11}$'s may be deuterium atoms, or all may be substituents, or a part thereof may be deuterium atoms and the remaining ones may be substituents. When n13 is 1, $R^{13}$ may be a deuterium atom, or may be a substituent. When n13 is 2 or more, all two or more $R^{13}$'s may be deuterium atoms, or all may be substituents, or a part thereof may be deuterium atoms and the remaining ones may be substituents. When n12 is 1, $R^{12}$ may be a deuterium atom, or may be a substituent. When n12 is 2, both two $R^{12}$'s may be deuterium atoms, or both may be substituents, or one of the two may be a deuterium atom and the other may be a substituent.

In the case of having a donor group having a fused ring structure of four or more rings, two or more of $R^1$ to $R^5$ are preferably donor groups having a fused ring structure of four or more rings, and for example, two of R1 to R5 are donor groups having a fused ring structure of four or more rings, or for example, three of of R1 to R5 are donor groups having a fused ring structure of four or more rings. When two or more of $R^1$ to $R^5$ are donor groups having a fused ring structure of four or more rings, they may be the same or different, but preferably they are the same. In one aspect of the present invention, at least $R^3$ is a donor group having a fused ring structure of four or more rings. In one aspect of the present invention, at least $R^4$ is a donor group having a fused ring structure of four or more rings. In one aspect of the present invention, at least $R^5$ is a donor group having a fused ring structure of four or more rings. In one aspect of the present invention, $R^3$ and $R^5$ are donor groups having a fused ring structure of four or more rings. In one aspect of the present invention, $R^3$, $R^4$, and $R^5$ are donor groups having a fused ring structure of four or more rings.

In one preferred aspect of the present invention, at least one of $Ar^1$ to $Ar^4$ is a substituted or unsubstituted heteroaryl group (excluding a nitrogen-containing 6-membered ring group). The number of the substituted or unsubstituted heteroaryl groups (excluding a nitrogen-containing 6-membered ring group) may be only one, may be two, may be three, or may be all of $Ar^1$ to $Ar^4$. When there are two or more, they may be the same or different, but are preferably the same. The substituted or unsubstituted heteroaryl group (excluding a nitrogen-containing 6-membered ring group) is preferably a heteroaryl group bonded via a nitrogen atom which is one of ring skeleton-constituting atoms, more preferably a heteroaryl group having a 5-membered ring bonded via a nitrogen atom (a group bonded via a nitrogen atom of a pyrrole ring, and the pyrrole ring may be substituted and is preferably fused), and still more preferably a substituted or unsubstituted carbazol-9-yl group. In one preferred aspect of the present invention, the carbazol-9-yl group as referred to herein may be substituted with an alkyl group or an aryl group optionally substituted with a group selected from the group consisting of a deuterium atom, an alkyl group and an aryl group, and can also be substituted with a deuterium atom. For the description and details of the carbazol-9-yl group, reference can be made to the description of the carbazol-9-yl group that $R^1$ to $R^5$ can adopt, the description of the ring-fused carbazol-9-yl group, and specific examples D1 to D1210.

In one preferred aspect of the present invention, $Ar^1$ to $Ar^4$ are each independently a substituted or unsubstituted aryl group. All of $Ar^1$ to $Ar^4$ may be the same, and examples thereof include an unsubstituted phenyl group and a perdeuterated phenyl group. When $Ar^1$ to $Ar^4$ are each independently a substituted or unsubstituted aryl group, at least one of $R^1$ to $R^5$ is a donor group having a fused ring structure of four or more rings. The number of the donor groups having a fused ring structure of four or more rings may be 2 or more, for example, 2, and for example, 3. As the donor group having a fused ring structure of four or more rings, specific examples D86 to D1210 can be referred to. Among these, a donor group having a fused ring structure of five or more rings (for example, five rings, for example, seven rings) can be preferably employed. In one preferred aspect of the present invention, when $Ar^1$ to $Ar^4$ are each independently a substituted or unsubstituted aryl group, at least one of $R^1$ to $R^5$ has a deuterium atom. For example, the compound can have a deuterated donor group having a fused ring structure of three rings (for example, a perdeuterated carbazol-9-yl group), or can have a deuterated aryl group (for example, a perdeuterated phenyl group).

The compound represented by the general formula (1) preferably does not contain a metal atom, and may be a compound composed only of atoms selected from the group consisting of a carbon atom, a hydrogen atom, a deuterium atom, a nitrogen atom, an oxygen atom, and a sulfur atom. In one preferred aspect of the present invention, the compound represented by the general formula (1) is composed only of atoms selected from the group consisting of a carbon atom, a hydrogen atom, a deuterium atom, a nitrogen atom, and an oxygen atom. In addition, the compound represented by the

general formula (1) may be a compound composed only of atoms selected from the group consisting of a carbon atom, a hydrogen atom, a deuterium atom, a nitrogen atom, and a sulfur atom. The compound represented by the general formula (1) may be a compound composed only of atoms selected from the group consisting of a carbon atom, a hydrogen atom, a deuterium atom, and a nitrogen atom. The compound represented by the general formula (1) may be a compound composed only of atoms selected from the group consisting of a carbon atom, a hydrogen atom, and a nitrogen atom. Further, the compound represented by the general formula (1) may be a compound which does not contain a hydrogen atom but contains a deuterium atom.

In the description herein, the term "Substituent Group A" means one atom or group or a combination of two or more thereof selected from the group consisting of a deuterium atom, a hydroxyl group, a halogen atom (for example, a fluorine atom, a chlorine atom, a bromine atom, and an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), a heteroarylthio group (for example, having 5 to 30 ring skeleton-constituting atoms), an acyl group (for example, having 1 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 1 to 40 carbon atoms), a heteroaryloxycarbonyl group (for example, having 1 to 40 carbon atoms), a silyl group (for example, a trialkylsilyl group having 1 to 40 carbon atoms), and a nitro group.

In the description herein, the term "Substituent Group B" means one atom or group or a combination of two or more thereof selected from the group consisting of a deuterium atom, an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having 5 to 30 ring skeleton-constituting atoms), a heteroaryloxy group (for example, having 5 to 30 ring skeleton-constituting atoms), and a diarylaminoamino group (for example, having 0 to 20 carbon atoms).

In the description herein, the term "Substituent Group C" means one atom or group or a combination of two or more thereof selected from the group consisting of a deuterium atom, an alkyl group (for example, having 1 to 20 carbon atoms), an aryl group (for example, having 6 to 22 carbon atoms), a heteroaryl group (for example, having 5 to 20 ring skeleton-constituting atoms), and a diarylamino group (for example, having 12 to 20 carbon atoms).

In the description herein, the term "Substituent Group D" means one atom or group or a combination of two or more thereof selected from the group consisting of a deuterium atom, an alkyl group (for example, having 1 to 20 carbon atoms), an aryl group (for example, having 6 to 22 carbon atoms), and a heteroaryl group (for example, having 5 to 20 ring skeleton-constituting atoms).

In the description herein, the term "Substituent Group E" means one atom or group or a combination of two or more groups selected from the group consisting of a deuterium atom, an alkyl group (for example, having 1 to 20 carbon atoms), and an aryl group (for example, having 6 to 22 carbon atoms).

In the description herein, the substituent meant by an expression of "substituted or unsubstituted" or "optionally substituted" may be selected, for example, from Substituent Group A, may be selected from Substituent Group B, may be selected from Substituent Group C, may be selected from Substituent Group D, or may be selected from Substituent Group E.

Specific examples of the compound represented by the general formula (1) are shown in the following Tables 1 to 8. Here, the compound represented by the general formula (1) that can be used in the present invention should not be construed as being limited by these specific examples.

In Table 1, structures of the compounds are individually shown by specifying $R^3$ to $R^5$ of the following general formula (1a) for each compound. That is, structures in which $R^2$ is a group represented by the general formula (2), $Ar^1$ and $Ar^3$ are perdeuterated carbazol-9-yl groups (D55), $Ar^2$ and $Ar^4$ are perdeuterated phenyl groups (Ar47), $X^1$ to $X^3$ are nitrogen atoms (N), $L^1$ is a single bond (L1), $R^1$ is a hydrogen atom, and $R^3$ to $R^5$ are groups specified in Table 1 are individually shown as structures of Compounds 1 to 170.

General Formula (1a)

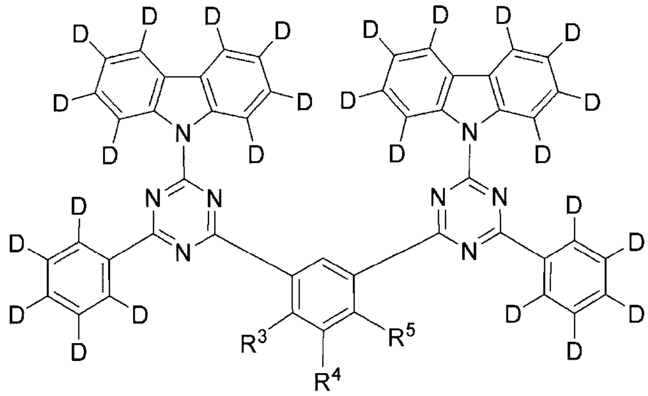

[Table 1]

| No. | $R^3$ | $R^4$ | $R^5$ |
|---|---|---|---|
| 1 | D1 | D1 | D1 |
| 2 | D2 | D1 | D2 |
| 3 | D3 | D1 | D3 |
| 4 | D4 | D1 | D4 |
| 5 | D5 | D1 | D5 |
| 6 | D6 | D1 | D6 |
| 7 | D7 | D1 | D7 |
| 8 | D8 | D1 | D8 |
| 9 | D9 | D1 | D9 |
| 10 | D10 | D1 | D10 |
| 11 | D11 | D1 | D11 |
| 12 | D12 | D1 | D12 |
| 13 | D13 | D1 | D13 |
| 14 | D14 | D1 | D14 |
| 15 | D15 | D1 | D15 |
| 16 | D16 | D1 | D16 |
| 17 | D17 | D1 | D17 |
| 18 | D18 | D1 | D18 |
| 19 | D19 | D1 | D19 |
| 20 | D20 | D1 | D20 |
| 21 | D21 | D1 | D21 |
| 22 | D22 | D1 | D22 |
| 23 | D23 | D1 | D23 |
| 24 | D24 | D1 | D24 |
| 25 | D25 | D1 | D25 |
| 26 | D26 | D1 | D26 |
| 27 | D27 | D1 | D27 |
| 28 | D28 | D1 | D28 |

(continued)

| No. | $R^3$ | $R^4$ | $R^5$ |
|---|---|---|---|
| 29 | D29 | D1 | D29 |
| 30 | D30 | D1 | D30 |
| 31 | D31 | D1 | D31 |
| 32 | D32 | D1 | D32 |
| 33 | D33 | D1 | D33 |
| 34 | D34 | D1 | D34 |
| 35 | D35 | D1 | D35 |
| 36 | D36 | D1 | D36 |
| 37 | D37 | D1 | D37 |
| 38 | D38 | D1 | D38 |
| 39 | D39 | D1 | D39 |
| 40 | D40 | D1 | D40 |
| 41 | D41 | D1 | D41 |
| 42 | D42 | D1 | D42 |
| 43 | D43 | D1 | D43 |
| 44 | D44 | D1 | D44 |
| 45 | D45 | D1 | D45 |
| 46 | D46 | D1 | D46 |
| 47 | D47 | D1 | D47 |
| 48 | D48 | D1 | D48 |
| 49 | D49 | D1 | D49 |
| 50 | D50 | D1 | D50 |
| 51 | D51 | D1 | D51 |
| 52 | D52 | D1 | D52 |
| 53 | D53 | D1 | D53 |
| 54 | D54 | D1 | D54 |
| 55 | D55 | D1 | D55 |
| 56 | D56 | D1 | D56 |
| 57 | D57 | D1 | D57 |
| 58 | D58 | D1 | D58 |
| 59 | D59 | D1 | D59 |
| 60 | D60 | D1 | D60 |
| 61 | D61 | D1 | D61 |
| 62 | D62 | D1 | D62 |
| 63 | D63 | D1 | D63 |
| 64 | D64 | D1 | D64 |
| 65 | D65 | D1 | D65 |
| 66 | D66 | D1 | D66 |
| 67 | D67 | D1 | D67 |

(continued)

| No. | $R^3$ | $R^4$ | $R^5$ |
|---|---|---|---|
| 68 | D68 | D1 | D68 |
| 69 | D69 | D1 | D69 |
| 70 | D70 | D1 | D70 |
| 71 | D71 | D1 | D71 |
| 72 | D72 | D1 | D72 |
| 73 | D73 | D1 | D73 |
| 74 | D74 | D1 | D74 |
| 75 | D75 | D1 | D75 |
| 76 | D76 | D1 | D76 |
| 77 | D77 | D1 | D77 |
| 78 | D78 | D1 | D78 |
| 79 | D79 | D1 | D79 |
| 80 | D80 | D1 | D80 |
| 81 | D81 | D1 | D81 |
| 82 | D82 | D1 | D82 |
| 83 | D83 | D1 | D83 |
| 84 | D84 | D1 | D84 |
| 85 | D85 | D1 | D85 |
| 86 | D1 | D2 | D1 |
| 87 | D2 | D2 | D2 |
| 88 | D3 | D2 | D3 |
| 89 | D4 | D2 | D4 |
| 90 | D5 | D2 | D5 |
| 91 | D6 | D2 | D6 |
| 92 | D7 | D2 | D7 |
| 93 | D8 | D2 | D8 |
| 94 | D9 | D2 | D9 |
| 95 | D10 | D2 | D10 |
| 96 | D11 | D2 | D11 |
| 97 | D12 | D2 | D12 |
| 98 | D13 | D2 | D13 |
| 99 | D14 | D2 | D14 |
| 100 | D15 | D2 | D15 |
| 101 | D16 | D2 | D16 |
| 102 | D17 | D2 | D17 |
| 103 | D18 | D2 | D18 |
| 104 | D19 | D2 | D19 |
| 105 | D20 | D2 | D20 |
| 106 | D21 | D2 | D21 |

(continued)

| No. | $R^3$ | $R^4$ | $R^5$ |
|---|---|---|---|
| 107 | D22 | D2 | D22 |
| 108 | D23 | D2 | D23 |
| 109 | D24 | D2 | D24 |
| 110 | D25 | D2 | D25 |
| 111 | D26 | D2 | D26 |
| 112 | D27 | D2 | D27 |
| 113 | D28 | D2 | D28 |
| 114 | D29 | D2 | D29 |
| 115 | D30 | D2 | D30 |
| 116 | D31 | D2 | D31 |
| 117 | D32 | D2 | D32 |
| 118 | D33 | D2 | D33 |
| 119 | D34 | D2 | D34 |
| 120 | D35 | D2 | D35 |
| 121 | D36 | D2 | D36 |
| 122 | D37 | D2 | D37 |
| 123 | D38 | D2 | D38 |
| 124 | D39 | D2 | D39 |
| 125 | D40 | D2 | D40 |
| 126 | D41 | D2 | D41 |
| 127 | D42 | D2 | D42 |
| 128 | D43 | D2 | D43 |
| 129 | D44 | D2 | D44 |
| 130 | D45 | D2 | D45 |
| 131 | D46 | D2 | D46 |
| 132 | D47 | D2 | D47 |
| 133 | D48 | D2 | D48 |
| 134 | D49 | D2 | D49 |
| 135 | D50 | D2 | D50 |
| 136 | D51 | D2 | D51 |
| 137 | D52 | D2 | D52 |
| 138 | D53 | D2 | D53 |
| 139 | D54 | D2 | D54 |
| 140 | D55 | D2 | D55 |
| 141 | D56 | D2 | D56 |
| 142 | D57 | D2 | D57 |
| 143 | D58 | D2 | D58 |
| 144 | D59 | D2 | D59 |
| 145 | D60 | D2 | D60 |

(continued)

| No. | $R^3$ | $R^4$ | $R^5$ |
|---|---|---|---|
| 146 | D61 | D2 | D61 |
| 147 | D62 | D2 | D62 |
| 148 | D63 | D2 | D63 |
| 149 | D64 | D2 | D64 |
| 150 | D65 | D2 | D65 |
| 151 | D66 | D2 | D66 |
| 152 | D67 | D2 | D67 |
| 153 | D68 | D2 | D68 |
| 154 | D69 | D2 | D69 |
| 155 | D70 | D2 | D70 |
| 156 | D71 | D2 | D71 |
| 157 | D72 | D2 | D72 |
| 158 | D73 | D2 | D73 |
| 159 | D74 | D2 | D74 |
| 160 | D75 | D2 | D75 |
| 161 | D76 | D2 | D76 |
| 162 | D77 | D2 | D77 |
| 163 | D78 | D2 | D78 |
| 164 | D79 | D2 | D79 |
| 165 | D80 | D2 | D80 |
| 166 | D81 | D2 | D81 |
| 167 | D82 | D2 | D82 |
| 168 | D83 | D2 | D83 |
| 169 | D84 | D2 | D84 |
| 170 | D85 | D2 | D85 |

In Table 2, structures of Compounds 1 to 442987 are shown by collectively displaying $R^3$ to $R^5$ of a plurality of compounds in each row. For example, in the row of Compounds 1 to 85 in Table 2, compounds in which $R^4$ is fixed to D1 (carbazol-9-yl group), $R^3$ and $R^5$ are the same, and D1 to D85 are referred to as Compounds 1 to 85, respectively. Similarly, in the row of Compounds 86 to 170 in Table 2, compounds in which $R^4$ is fixed to D2 (3-methylcarbazol-9-yl group), $R^3$ and $R^5$ are the same, and D1 to D85 are referred to as Compounds 86 to 170, respectively. That is, the row of Compounds 1 to 85 and the row of Compounds 86 to 170 in Table 2 collectively represent Compounds 1 to 170 specified in Table 1 in two rows. Similarly, in the row of Compounds 171 to 255 in Table 2, compounds in which $R^4$ is fixed to D3 (3,6-dimethylcarbazol-9-yl group), $R^3$ and $R^5$ are the same, and D1 to D85 are referred to as Compounds 171 to 255, respectively. In the same manner, Compounds 256 to 442987 in Table 2 are also specified.

[Table 2]

| No.<br>1 ~ 85 | $R^3$<br>D1~D85 | $R^4$<br>D1 | $R^5$<br>D1~D85 | = |
|---|---|---|---|---|
| 86 ~ 170 | D1~D85 | D2 | D1~D85 | |
| 171 ~ 255 | D1~D85 | D3 | D1~D85 | |
| 256 ~ 340 | D1~D85 | D4 | D1~D85 | |
| 341 ~ 425 | D1~D85 | D5 | D1~D85 | |

(continued)

| No. | $R^3$ | $R^4$ | $R^5$ | = |
|---|---|---|---|---|
| 426 ~ 510 | D1~D85 | D6 | D1~D85 | $R^3 = R^5$ |
| 511 ~ 595 | D1~D85 | D7 | D1~D85 | |
| 596 ~ 680 | D1~D85 | D8 | D1~D85 | |
| 681 ~ 765 | D1~D85 | D9 | D1~D85 | |
| 766 ~ 850 | D1~D85 | D10 | D1~D85 | |
| 851 ~ 935 | D1~D85 | D11 | D1~D85 | |
| 936 ~ 1020 | D1~D85 | D12 | D1~D85 | |
| 1021 ~ 1105 | D1~D85 | D13 | D1~D85 | |
| 1106 ~ 1190 | D1~D85 | D14 | D1~D85 | |
| 1191 ~ 1275 | D1~D85 | D15 | D1~D85 | |
| 1276 ~ 1360 | D1~D85 | D16 | D1~D85 | |
| 1361 ~ 1445 | D1~D85 | D17 | D1~D85 | |
| 1446 ~ 1530 | D1~D85 | D18 | D1~D85 | |
| 1531 ~ 1615 | D1~D85 | D19 | D1~D85 | |
| 1616 ~ 1700 | D1~D85 | D20 | D1~D85 | |
| 1701 ~ 1785 | D1~D85 | D21 | D1~D85 | |
| 1786 ~ 1870 | D1~D85 | D22 | D1~D85 | |
| 1871 ~ 1955 | D1~D85 | D23 | D1~D85 | |
| 1956 ~ 2040 | D1~D85 | D24 | D1~D85 | |
| 2041 ~ 2125 | D1~D85 | D25 | D1~D85 | |
| 2126 ~ 2210 | D1~D85 | D26 | D1~D85 | |
| 2211 ~ 2295 | D1~D85 | D27 | D1~D85 | |
| 2296 ~ 2380 | D1~D85 | D28 | D1~D85 | |
| 2381 ~ 2465 | D1~D85 | D29 | D1~D85 | |
| 2466 ~ 2550 | D1~D85 | D30 | D1~D85 | |
| 2551 ~ 2635 | D1~D85 | D31 | D1~D85 | |
| 2636 ~ 2720 | D1~D85 | D32 | D1~D85 | |
| 2721 ~ 2805 | D1~D85 | D33 | D1~D85 | |
| 2806 ~ 2890 | D1~D85 | D34 | D1~D85 | |
| 2891 ~ 2975 | D1~D85 | D35 | D1~D85 | |
| 2976 ~ 3060<br>3061 ~ 3145 | D1~D85<br>D1~D85 | D36<br>D37 | D1~D85<br>D1~D85 | |
| 3146 ~ 3230 | D1~D85 | D38 | D1~D85 | |
| 3231 ~ 3315 | D1~D85 | D39 | D1~D85 | |
| 3316 ~ 3400 | D1~D85 | D40 | D1~D85 | |
| 3401 ~ 3485 | D1~D85 | D41 | D1~D85 | |
| 3486 ~ 3570 | D1~D85 | D42 | D1~D85 | |
| 3571 ~ 3655 | D1~D85 | D43 | D1~D85 | |
| 3656 ~ 3740<br>3741 ~ 3825 | D1~D85<br>D1~D85 | D44<br>D45 | D1~D85<br>D1~D85 | |

(continued)

| No. | $R^3$ | $R^4$ | $R^5$ | = |
|---|---|---|---|---|
| 3826 ~ 3910 | D1~D85 | D46 | D1~D85 | |
| 3911 ~ 3995 | D1~D85 | D47 | D1~D85 | |
| 3996 ~ 4080 | D1~D85 | D48 | D1~D85 | |
| 4081 ~ 4165 | D1~D85 | D49 | D1~D85 | |
| 4166 ~ 4250 | D1~D85 | D50 | D1~D85 | |
| 4251 ~ 4335 | D1~D85 | D51 | D1~D85 | |
| 4336 ~ 4420 | D1~D85 | D52 | D1~D85 | |
| 4421 ~ 4505 | D1~D85 | D53 | D1~D85 | |
| 4506 ~ 4590 | D1~D85 | D54 | D1~D85 | |
| 4591 ~ 4675 | D1~D85 | D55 | D1~D85 | |
| 4676 ~ 4760 | D1~D85 | D56 | D1~D85 | |
| 4761 ~ 4845 | D1~D85 | D57 | D1~D85 | |
| 4846 ~ 4930 | D1~D85 | D58 | D1~D85 | |
| 4931 ~ 5015 | D1~D85 | D59 | D1~D85 | |
| 5016 ~ 5100 | D1~D85 | D60 | D1~D85 | |
| 5101 ~ 5185 | D1~D85 | D61 | D1~D85 | $R^3 = R^5$ |
| 5186 ~ 5270 | D1~D85 | D62 | D1~D85 | |
| 5271 ~ 5355 | D1~D85 | D63 | D1~D85 | |
| 5356 ~ 5440 | D1~D85 | D64 | D1~D85 | |
| 5441 ~ 5525 | D1~D85 | D65 | D1~D85 | |
| 5526 ~ 5610 | D1~D85 | D66 | D1~D85 | |
| 5611 ~ 5695 | D1~D85 | D67 | D1~D85 | |
| 5696 ~ 5780 | D1~D85 | D68 | D1~D85 | |
| 5781 ~ 5865 | D1~D85 | D69 | D1~D85 | |
| 5866 ~ 5950 | D1~D85 | D70 | D1~D85 | |
| 5951 ~ 6035 | D1~D85 | D71 | D1~D85 | |
| 6036 ~ 6120 | D1~D85 | D72 | D1~D85 | |
| 6121 ~ 6205 | D1~D85 | D73 | D1~D85 | |
| 6206 ~ 6290 | D1~D85 | D74 | D1~D85 | |
| 6291 ~ 6375 | D1~D85 | D75 | D1~D85 | |
| 6376 ~ 6460 | D1~D85 | D76 | D1~D85 | |
| 6461 ~ 6545 | D1~D85 | D77 | D1~D85 | |
| 6546 ~ 6630 | D1~D85 | D78 | D1~D85 | |
| 6631 ~ 6715 | D1~D85 | D79 | D1~D85 | |
| 6716 ~ 6800 | D1~D85 | D80 | D1~D85 | |
| 6801 ~ 6885 | D1~D85 | D81 | D1~D85 | |
| 6886 ~ 6970 | D1~D85 | D82 | D1~D85 | |
| 6971 ~ 7055 | D1~D85 | D83 | D1~D85 | |
| 7056 ~ 7140 | D1~D85 | D84 | D1~D85 | |
| 7141 ~ 7225 | D1~D85 | D85 | D1~D85 | |

(continued)

| No. | $R^3$ | $R^4$ | $R^5$ | = |
|---|---|---|---|---|
| 7226 ~ 7310 | D1~D85 | Ar1 | D1~D85 | |
| 7311 ~ 7395 | D1~D85 | Ar14 | D1~D85 | |
| 7396 ~ 7480 | D1~D85 | Ar15 | D1~D85 | |
| 7481 ~ 7565 | D1~D85 | Ar39 | D1~D85 | |
| 7566 ~ 7650 | D1~D85 | Ar40 | D1~D85 | |
| 7651 ~ 7735 | D1~D85 | Ar47 | D1~D85 | |
| 7736 ~ 7820 | Ar1 | D1~D85 | D1~D85 | $R^4 = R^5$ |
| 7821 ~ 7905 | Ar14 | D1~D85 | D1~D85 | |
| 7906 ~ 7990 | Ar15 | D1~D85 | D1~D85 | |
| 7991 ~ 8075 | Ar39 | D1~D85 | D1~D85 | |
| 8076 ~ 8160 | Ar40 | D1~D85 | D1~D85 | |
| 8161 ~ 8245 | Ar47 | D1~D85 | D1~D85 | |
| 8246 ~ 8330 | D1~D85 | D1~D85 | Ar1 | $R^3 = R^4$ |
| 8331 ~ 8415 | D1~D85 | D1~D85 | Ar14 | |
| 8416 ~ 8500 | D1~D85 | D1~D85 | Ar15 | |
| 8501 ~ 8585 | D1~D85 | D1~D85 | Ar39 | |
| 8586 ~ 8670 | D1~D85 | D1~D85 | Ar40 | |
| 8671 ~ 8755 | D1~D85 | D1~D85 | Ar47 | |
| 8756 ~ 9880 | D86~D1210 | D86~D1210 | D86~D1210 | $R^3 = R^4 = R^5$ |
| 9881 ~ 11005 | D86~D1210 | H | D86~D1210 | $R^3 = R^5$ |
| 11006 ~ 12130 | D86~D1210 | D1 | D86~D1210 | |
| 12131 ~ 13255 | D86~D1210 | D2 | D86~D1210 | |
| 13256 ~ 14380 | D86~D1210 | D3 | D86~D1210 | |
| 14381 ~ 15505 | D86~D1210 | D4 | D86~D1210 | |
| 15506 ~ 16630 | D86~D1210 | D5 | D86~D1210 | |
| 16631 ~ 17755 | D86~D1210 | D6 | D86~D1210 | |
| 17756 ~ 18880 | D86~D1210 | D7 | D86~D1210 | |
| 18881 ~ 20005 | D86~D1210 | D8 | D86~D1210 | |
| 20006 ~ 21130 | D86~D1210 | D9 | D86~D1210 | |
| 21131 ~ 22255 | D86~D1210 | D10 | D86~D1210 | |
| 22256 ~ 23380 | D86~D1210 | D11 | D86~D1210 | |
| 23381 ~ 24505 | D86~D1210 | D12 | D86~D1210 | |
| 24506 ~ 25630 | D86~D1210 | D13 | D86~D1210 | |
| 25631 ~ 26755 | D86~D1210 | D14 | D86~D1210 | |
| 26756 ~ 27880 | D86~D1210 | D15 | D86~D1210 | |
| 27881 ~ 29005 | D86~D1210 | D16 | D86~D1210 | |
| 29006 ~ 30130 | D86~D1210 | D17 | D86~D1210 | |
| 30131 ~ 31255 | D86~D1210 | D18 | D86~D1210 | |
| 31256 ~ 32380 | D86~D1210 | D19 | D86~D1210 | |
| 32381 ~ 33505 | D86~D1210 | D20 | D86-D1210 | |

(continued)

| No. | $R^3$ | $R^4$ | $R^5$ | = |
|---|---|---|---|---|
| 33506 ~ 34630 | D86~D1210 | D21 | D86~D1210 | |
| 34631 ~ 35755 | D86~D1210 | D22 | D86~D1210 | |
| 35756 ~ 36880 | D86~D1210 | D23 | D86~D1210 | |
| 36881 ~ 38005 | D86~D1210 | D24 | D86~D1210 | |
| 38006 ~ 39130 | D86~D1210 | D25 | D86~D1210 | |
| 39131 ~ 40255 | 086~01210 | D26 | D86~D1210 | |
| 40256 ~ 41380 | D86~D1210 | D27 | D86~D1210 | |
| 41381 ~ 42505 | D86~D1210 | D28 | D86~D1210 | |
| 42506 ~ 43630 | D86~D1210 | D29 | D86~D1210 | |
| 43631 ~ 44755 | D86~D1210 | D30 | D86~D1210 | |
| 44756 ~ 45880 | D86~D1210 | D31 | D86~D1210 | |
| 45881 ~ 47005 | D86~D1210 | D32 | D86~D1210 | |
| 47006 ~ 48130 | D86~D1210 | D33 | D86~D1210 | |
| 48131 ~ 49255 | D86~D1210 | D34 | D86~D1210 | |
| 49256 ~ 50380 | D86~D1210 | D35 | D86~D1210 | |
| 50381 ~ 51505 | D86~D1210 | D36 | D86~D1210 | |
| 51506 ~ 52630 | D86~D1210 | D37 | D86~D1210 | |
| 52631 ~ 53755 | D86~D1210 | D38 | D86~D1210 | |
| 53756 ~ 54880 | D86~D1210 | D39 | D86~D1210 | |
| 54881 ~ 56005 | D86~D1210 | D40 | D86~D1210 | |
| 56006 ~ 57130 | D86~D1210 | D41 | D86~D1210 | |
| 57131 ~ 58255 | D86~D1210 | D42 | D86~D1210 | |
| 58256 ~ 59380 | D86~D1210 | D43 | D86~D1210 | |
| 59381 ~ 60505 | D86~D1210 | D44 | D86~D1210 | |
| 60506 ~ 61630 | D86~D1210 | D45 | D86~D1210 | |
| 61631 ~ 62755 | D86~D1210 | D46 | D86~D1210 | |
| 62756 ~ 63880 | D86~D1210 | D47 | D86~D1210 | |
| 63881 ~ 65005 | D86~D1210 | D48 | D86~D1210 | $R^3 = R^5$ |
| 65006 ~ 66130 | D86~D1210 | D49 | D86~D1210 | |
| 66131 ~ 67255 | D86~D1210 | D50 | D86~D1210 | |
| 67256 ~ 68380 | D86~D1210 | D51 | D86~D1210 | |
| 68381 ~ 69505 | D86~D1210 | D52 | D86~D1210 | |
| 69506 ~ 70630 | D86~D1210 | D53 | D86~D1210 | |
| 70631 ~ 71755 | D86~D1210 | D54 | D86~D1210 | |
| 71756 ~ 72880 | D86~D1210 | D55 | D86~D1210 | |
| 72881 ~ 74005 | D86~D1210 | D56 | D86~D1210 | |
| 74006 ~ 75130 | D86~D1210 | D57 | D86~D1210 | |
| 75131 ~ 76255 | D86~D1210 | D58 | D86~D1210 | |
| 76256 ~ 77380 | D86~D1210 | D59 | D86~D1210 | |
| 77381 ~ 78505 | D86~D1210 | D60 | D86~D1210 | |

(continued)

| No. | $R^3$ | $R^4$ | $R^5$ | = |
|---|---|---|---|---|
| 78506 ~ 79630 | D86~D1210 | D61 | D86~D1210 | |
| 79631 ~ 80755 | D86~D1210 | D62 | D86~D1210 | |
| 80756 ~ 81880 | D86~D1210 | D63 | D86~D1210 | |
| 81881 ~ 83005 | D86~D1210 | D64 | D86~D1210 | |
| 83006 ~ 84130 | D86~D1210 | D65 | D86~D1210 | |
| 84131 ~ 85255 | D86~D1210 | D66 | D86~D1210 | |
| 85256 ~ 86380 | D86~D1210 | D67 | D86~D1210 | |
| 86381 ~ 87505 | D86~D1210 | D68 | D86~D1210 | |
| 87506 ~ 88630 | D86~D1210 | D69 | D86~D1210 | |
| 88631 ~ 89755 | D86~D1210 | D70 | D86~D1210 | |
| 89756 ~ 90880 | D86~D1210 | D71 | D86~D1210 | |
| 90881 ~ 92005 | D86~D1210 | D72 | D86~D1210 | |
| 92006 ~ 93130 | D86~D1210 | D73 | D86~D1210 | |
| 93131 ~ 94255 | D86~D1210 | D74 | D86~D1210 | |
| 94256 ~ 95380 | D86~D1210 | D75 | D86~D1210 | |
| 95381 ~ 96505 | D86~D1210 | D76 | D86~D1210 | |
| 96506 ~ 97630 | D86~D1210 | D77 | D86~D1210 | |
| 97631 ~ 98755 | D86~D1210 | D78 | D86~D1210 | |
| 98756 ~ 99880 | D86~D1210 | D79 | D86~D1210 | |
| 99881 ~ 101005 | D86~D1210 | D80 | D86~D1210 | |
| 101006 ~ 102130 | D86~D1210 | D81 | D86~D1210 | |
| 102131 ~ 103255 | D86~D1210 | D82 | D86~D1210 | |
| 103256 ~ 104380 | D86~D1210 | D83 | D86~D1210 | |
| 104381 ~ 105505 | D86~D1210 | D84 | D86~D1210 | |
| 105506 ~ 106630 | D86~D1210 | D85 | D86~D1210 | |
| 106631 ~ 107755 | D86~D1210 | Ar1 | D86~D1210 | |
| 107756 ~ 108880 | D86~D1210 | Ar14 | D86~D1210 | $R^3 = R^5$ |
| 108881 ~ 110005 | D86~D1210 | Ar15 | D86~D1210 | |
| 110006 ~ 111130 | D86~D1210 | Ar39 | D86~D1210 | |
| 111131 ~ 112255 | D86~D1210 | Ar40 | D86~D1210 | |
| 112256 ~ 113380 | D86~D1210 | Ar47 | D86~D1210 | |
| 113381 ~ 114505 | H | D86~D1210 | D86~D1210 | |
| 114506 ~ 115630 | D1 | D86~D1210 | D86~D1210 | |
| 115631 ~ 116755 | D2 | D86~D1210 | D86~D1210 | |
| 116756 ~ 117880 | D3 | D86~D1210 | D86~D1210 | |
| 117881 ~ 119005 | D4 | D86~D1210 | D86~D1210 | |
| 119006 ~ 120130 | D5 | D86~D1210 | D86~D1210 | |
| 120131 ~ 121255 | D6 | D86~D1210 | D86~D1210 | $R^4 = R^5$ |
| 121256 ~ 122380 | D7 | D86~D1210 | D86~D1210 | |
| 122381 ~ 123505 | D8 | D86~D1210 | D86~D1210 | |

(continued)

| No. | R3 | R4 | R5 | = |
|---|---|---|---|---|
| 123506~124630 | D9 | D86~D1210 | D86~D1210 | |
| 124631~125755 | D10 | D86~D1210 | D86~D1210 | |
| 125756~126880 | D11 | D86~D1210 | D86~D1210 | |
| 126881~128005 | D12 | D86~D1210 | D86~D1210 | |
| 128006~129130 | D13 | D86~D1210 | D86~D1210 | |
| 129131~130255 | D14 | D86~D1210 | D86~D1210 | |
| 130256~131380 | D15 | D86~D1210 | D86~D1210 | |
| 131381~132505 | D16 | D86~D1210 | D86~D1210 | |
| 132506~133630 | D17 | D86~D1210 | D86~D1210 | |
| 133631~134755 | D18 | D86~D1210 | D86~D1210 | |
| 134756~135880 | D19 | D86~D1210 | D86~D1210 | |
| 135881~137005 | D20 | D86~D1210 | D86~D1210 | |
| 137006~138130 | D21 | D86~D1210 | D86~D1210 | |
| 138131~139255 | D22 | D86~D1210 | D86~D1210 | |
| 139256~140380 | D23 | D86~D1210 | D86~D1210 | |
| 140381~141505 | D24 | D86~D1210 | D86~D1210 | |
| 141506~142630 | D25 | D86~D1210 | D86~D1210 | |
| 142631~143755 | D26 | D86~D1210 | D86~D1210 | |
| 143756~144880 | D27 | D86~D1210 | D86~D1210 | |
| 144881~146005 | D28 | D86~D1210 | D86~D1210 | |
| 146006~147130 | D29 | D86~D1210 | D86~D1210 | |
| 147131~148255 | D30 | D86~D1210 | D86~D1210 | |
| 148256~149380 | D31 | D86~D1210 | D86~D1210 | |
| 149381~150505 | D32 | D86~D1210 | D86~D1210 | |
| 150506~151630 | D33 | D86~D1210 | D86~D1210 | |
| 151631~152755 | D34 | D86~D1210 | D86~D1210 | |
| 152756~153880 | D35 | D86~D1210 | D86~D1210 | |
| 153881~155005 | D36 | D86~D1210 | D86~D1210 | |
| 155006~156130 | D37 | D86~D1210 | D86~D1210 | |
| 156131~157255 | D38 | D86~D1210 | D86~D1210 | |
| 157256~158380 | D39 | D86~D1210 | D86~D1210 | |
| 158381~159505 | D40 | D86~D1210 | D86~D1210 | |
| 159506~160630 | D41 | D86~D1210 | D86~D1210 | |
| 160631~161755 | D42 | D86~D1210 | D86~D1210 | |
| 161756~162880 | D43 | D86~D1210 | D86~D1210 | |
| 162881~164005 | D44 | D86~D1210 | D86~D1210 | |
| 164006~165130 | D45 | D86~D1210 | D86~D1210 | |
| 165131~166255 | D46 | D86~D1210 | D86~D1210 | |
| 166256~167380 | D47 | D86~D1210 | D86~D1210 | |
| 167381~168505 | D48 | D86~D1210 | D86~D1210 | |
| 168506~169630 | D49 | D86~D1210 | D86~D1210 | |

(continued)

| No. | $R^3$ | $R^4$ | $R^5$ | = |
|---|---|---|---|---|
| 169631 ~ 170755 | D50 | D86~D1210 | D86~D1210 | $R^4 = R^5$ |
| 170756 ~ 171880 | D51 | D86~D1210 | D86~D1210 | |
| 171881 ~ 173005 | D52 | D86~D1210 | D86~D1210 | |
| 173006 ~ 174130 | D53 | D86~D1210 | D86~D1210 | |
| 174131 ~ 175255 | D54 | D86~D1210 | D86~D1210 | |
| 175256 ~ 176380 | D55 | D86~D1210 | D86~D1210 | |
| 176381 ~ 177505 | D56 | D86~D1210 | D86~D1210 | |
| 177506 ~ 178630 | D57 | D86~D1210 | D86~D1210 | |
| 178631 ~ 179755 | D58 | D86~D1210 | D86~D1210 | |
| 179756 ~ 180880 | D59 | D86~D1210 | D86~D1210 | |
| 180881 ~ 182005 | D60 | D86~D1210 | D86~D1210 | |
| 182006 ~ 183130 | D61 | D86~D1210 | D86~D1210 | |
| 183131 ~ 184255 | D62 | D86~D1210 | D86~D1210 | |
| 184256 ~ 185380 | D63 | D86~D1210 | D86~D1210 | |
| 185381 ~ 186505 | D64 | D86~D1210 | D86~D1210 | |
| 186506 ~ 187630 | D65 | D86~D1210 | D86~D1210 | |
| 187631 ~ 188755 | D66 | D86~D1210 | D86~D1210 | |
| 188756 ~ 189880 | D67 | D86~D1210 | D86~D1210 | |
| 189881 ~ 191005 | D68 | D86~D1210 | D86~D1210 | |
| 191006 ~ 192130 | D69 | D86~D1210 | D86~D1210 | |
| 192131 ~ 193255 | D70 | D86~D1210 | D86~D1210 | |
| 193256 ~ 194380 | D71 | D86~D1210 | D86~D1210 | |
| 194381 ~ 195505 | D72 | D86~D1210 | D86~D1210 | |
| 195506 ~ 196630 | D73 | D86~D1210 | D86~D1210 | |
| 196631 ~ 197755 | D74 | D86~D1210 | D86~D1210 | |
| 197756 ~ 198880 | D75 | D86~D1210 | D86~D1210 | |
| 198881 ~ 200005 | D76 | D86~D1210 | D86~D1210 | |
| 200006 ~ 201130 | D77 | D86~D1210 | D86~D1210 | |
| 201131 ~ 202255 | D78 | D86~D1210 | D86~D1210 | |
| 202256 ~ 203380 | D79 | D86~D1210 | D86~D1210 | |
| 203381 ~ 204505 | D80 | D86~D1210 | D86~D1210 | |
| 204506 ~ 205630 | D81 | D86~D1210 | D86~D1210 | |
| 205631 ~ 206755 | D82 | D86~D1210 | D86~D1210 | |
| 206756 ~ 207880 | D83 | D86~D1210 | D86~D1210 | |
| 207881 ~ 209005 | D84 | D86~D1210 | D86~D1210 | |
| 209006 ~ 210130 | D85 | D86~D1210 | D86~D1210 | |
| 210131 ~ 211255 | Ar1 | D86~D1210 | D86~D1210 | |
| 211256 ~ 212380 | Ar14 | D86~D1210 | D86~D1210 | |
| 212381 ~ 213505 | Ar15 | D86~D1210 | D86~D1210 | |
| 213506 ~ 214630 | Ar39 | D86~D1210 | D86~D1210 | |

(continued)

| No. | $R^3$ | $R^4$ | $R^5$ | = |
|---|---|---|---|---|
| 214631～215755 | Ar40 | D86~D1210 | D86~D1210 | |
| 215756～216880 | Ar47 | D86~D1210 | D86~D1210 | |
| 216881～218005 | D86~D1210 | D86~D1210 | H | $R^3 = R^4$ |
| 218006～219130 | D86~D1210 | D86~D1210 | D1 | |
| 219131～220255 | D86~D1210 | D86~D1210 | D2 | |
| 220256～221380 | D86~D1210 | D86~D1210 | D3 | |
| 221381～222505 | D86~D1210 | D86~D1210 | D4 | |
| 222506～223630 | D86~D1210 | D86~D1210 | D5 | |
| 223631～224755 | D86~D1210 | D86~D1210 | D6 | |
| 224756～225880 | D86~D1210 | D86~D1210 | D7 | |
| 225881～227005 | D86~D1210 | D86~D1210 | D8 | |
| 227006～228130 | D86~D1210 | D86~D1210 | D9 | |
| 228131～229255 | D86~D1210 | D86~D1210 | D10 | |
| 229256～230380 | D86~D1210 | D86~D1210 | D11 | |
| 230381～231505 | D86~D1210 | D86~D1210 | D12 | |
| 231506～232630 | D86~D1210 | D86~D1210 | D13 | |
| 232631～233755 | D86~D1210 | D86~D1210 | D14 | |
| 233756～234880 | D86~D1210 | D86~D1210 | D15 | |
| 234881～236005 | D86~D1210 | D86~D1210 | D16 | |
| 236006～237130 | D86~D1210 | D86~D1210 | D17 | |
| 237131～238255 | D86~D1210 | D86~D1210 | D18 | |
| 238256～239380 | D86~D1210 | D86~D1210 | D19 | |
| 239381～240505 | D86~D1210 | D86~D1210 | D20 | |
| 240506～241630 | D86~D1210 | D86~D1210 | D21 | |
| 241631～242755 | D86~D1210 | D86~D1210 | D22 | |
| 242756～243880 | D86~D1210 | D86~D1210 | D23 | |
| 243881～245005 | D86~D1210 | D86~D1210 | D24 | |
| 245006～246130 | D86~D1210 | D86~D1210 | D25 | |
| 246131～247255 | D86~D1210 | D86~D1210 | D26 | |
| 247256～248380 | D86~D1210 | D86~D1210 | D27 | |
| 248381～249505 | D86~D1210 | D86~D1210 | D28 | |
| 249506～250630 | D86~D1210 | D86~D1210 | D29 | |
| 250631～251755 | D86~D1210 | D86~D1210 | D30 | |
| 251756～252880 | D86~D1210 | D86~D1210 | D31 | |
| 252881～254005 | D86~D1210 | D86~D1210 | D32 | |
| 254006～255130 | D86~D1210 | D86~D1210 | D33 | |
| 255131～256255 | D86~D1210 | D86~D1210 | D34 | |
| 256256～257380 | D86~D1210 | D86~D1210 | D35 | |
| 257381～258505 | D86~D1210 | D86~D1210 | D36 | |
| 258506～259630 | D86~D1210 | D86~D1210 | D37 | |

(continued)

| No. | $R^3$ | $R^4$ | $R^5$ | = |
|---|---|---|---|---|
| 259631～260755 | D86~D1210 | D86~D1210 | D38 | $R^3 = R^4$ |
| 260756～261880 | D86~D1210 | D86~D1210 | D39 | |
| 261881～263005 | D86~D1210 | D86~D1210 | D40 | |
| 263006～264130 | D86~D1210 | D86~D1210 | D41 | |
| 264131～265255 | D86~D1210 | D86~D1210 | D42 | |
| 265256～266380 | D86~D1210 | D86~D1210 | D43 | |
| 266381～267505 | D86~D1210 | D86~D1210 | D44 | |
| 267506～268630 | D86~D1210 | D86~D1210 | D45 | |
| 268631～269755 | D86~D1210 | D86~D1210 | D46 | |
| 269756～270880 | D86~D1210 | D86~D1210 | D47 | |
| 270881～272005 | D86~D1210 | D86~D1210 | D48 | |
| 272006～273130 | D86~D1210 | D86~D1210 | D49 | |
| 273131～274255 | D86~D1210 | D86~D1210 | D50 | |
| 274256～275380 | D86~D1210 | D86~D1210 | D51 | |
| 275381～276505 | D86~D1210 | D86~D1210 | D52 | |
| 276506～277630 | D86~D1210 | D86~D1210 | D53 | |
| 277631～278755 | D86~D1210 | D86~D1210 | D54 | |
| 278756～279880 | D86~D1210 | D86~D1210 | D55 | |
| 279881～281005 | D86~D1210 | D86~D1210 | D56 | |
| 281006～282130 | D86~D1210 | D86~D1210 | D57 | |
| 282131～283255 | D86~D1210 | D86~D1210 | D58 | |
| 283256～284380 | D86~D1210 | D86~D1210 | D59 | |
| 284381～285505 | D86~D1210 | D86~D1210 | D60 | |
| 285506～286630 | D86~D1210 | D86~D1210 | D61 | |
| 286631～287755 | D86~D1210 | D86~D1210 | D62 | |
| 287756～288880 | D86~D1210 | D86~D1210 | D63 | |
| 288881～290005 | D86~D1210 | D86~D1210 | D64 | |
| 290006～291130 | D86~D1210 | D86~D1210 \| | D65 | |
| 291131～292255 | D86~D1210 | D86~D1210 | D66 | |
| 292256～293380 | D86~D1210 | D86~D1210 | D67 | |
| 293381～294505 | D86~D1210 | D86~D1210 | D68 | |
| 294506～295630 | D86~D1210 | D86~D1210 | D69 | |
| 295631～296755 | D86~D1210 | D86~D1210 | D70 | |
| 296756～297880 | D86~D1210 | D86~D1210 | D71 | |
| 297881～299005 | D86~D1210 | D86~D1210 | D72 | |
| 299006～300130 | D86~D1210 | D86~D1210 | D73 | |
| 300131～301255 | D86~D1210 | D86~D1210 | D74 | |
| 301256～302380 | D86~D1210 | D86~D1210 | D75 | |
| 302381～303505 | D86~D1210 | D86~D1210 | D76 | |
| 303506～304630 | D86~D1210 | D86~D1210 | D77 | |

(continued)

| No. | $R^3$ | $R^4$ | $R^5$ | = |
|---|---|---|---|---|
| 304631～305755 | D86~D1210 | D86~D1210 | D78 | |
| 305756～306880 | D86~D1210 | D86~D1210 | D79 | |
| 306881～308005 | D86~D1210 | D86~D1210 | D80 | |
| 308006～309130 | D86~D1210 | D86~D1210 | D81 | |
| 309131～310255 | D86~D1210 | D86~D1210 | D82 | $R^3 = R^4$ |
| 310256～311380 | D86~D1210 | D86~D1210 | D83 | |
| 311381～312505 | D86~D1210 | D86~D1210 | D84 | |
| 312506～313630 | D86~D1210 | D86~D1210 | D85 | |
| 313631～314755 | D86~D1210 | D86~D1210 | Ar1 | |
| 314756～315880 | D86~D1210 | D86~D1210 | Ar14 | |
| 315881～317005 | D86~D1210 | D86~D1210 | Ar15 | |
| 317006～318130 | D86~D1210 | D86~D1210 | Ar39 | |
| 318131～319255 | D86~D1210 | D86~D1210 | Ar40 | |
| 319256～320380 | D86~D1210 | D86~D1210 | Ar47 | |
| 320381～321505 | D1 | D86~D1210 | D1 | $R^3 = R^5$ |
| 321506～322630 | D2 | D86~D1210 | D2 | |
| 322631～323755 | D3 | D86~D1210 | D3 | |
| 323756～324880 | D4 | D86~D1210 | D4 | |
| 324881～326005 | D5 | D86~D1210 | D5 | |
| 326006～327130 | D6 | D86~D1210 | D6 | |
| 327131～328255 | D7 | D86~D1210 | D7 | |
| 328256～329380 | D8 | D86~D1210 | D8 | |
| 329381～330505 | D9 | D86~D1210 | D9 | |
| 330506～331630 | D10 | D86~D1210 | D10 | |
| 331631～332755 | D11 | D86~D1210 | D11 | |
| 332756～333880<br>333881～335005 | D12<br>D13 | D86~D1210<br>D86~D1210 | D12<br>D13 | |
| 335006～336130 | D14 | D86~D1210 | D14 | |
| 336131～337255 | D15 | D86~D1210 | D15 | |
| 337256～338380 | D16 | D86~D1210 | D16 | |
| 338381～339505 | D17 | D86~D1210 | D17 | |
| 339506～340630<br>340631～341755 | D18<br>D19 | D86~D1210<br>D86~D1210 | D18<br>D19 | |
| 341756～342880 | D20 | D86~D1210 | D20 | |
| 342881～344005 | D21 | D86~D1210 | D21 | |
| 344006～345130<br>345131～346255 | D22<br>D23 | D86~D1210<br>D86~D1210 | D22<br>D23 | |
| 346256～347380 | D24 | D86~D1210 | D24 | |
| 347381～348505 | D25 | D86~D1210 | D25 | |
| 348506～349630 | D26 | D86~D1210 | D26 | |

(continued)

| No. | $R^3$ | $R^4$ | $R^5$ | = |
|---|---|---|---|---|
| 349631 ~ 350755 | D27 | D86~D1210 | D27 | |
| 350756 ~ 351880 | D28 | D86~D1210 | D28 | |
| 351881 ~ 353005 | D29 | D86~D1210 | D29 | |
| 353006 ~ 354130 | D30 | D86~D1210 | D30 | |
| 354131 ~ 355255 | D31 | D86~D1210 | D31 | |
| 355256 ~ 356380 | D32 | D86~D1210 | D32 | |
| 356381 ~ 357505 | D33 | D86~D1210 | D33 | |
| 357506 ~ 358630 | D34 | D86~D1210 | D34 | |
| 358631 ~ 359755 | D35 | D86~D1210 | D35 | |
| 359756 ~ 360880 | D36 | D86~D1210 | D36 | |
| 360881 ~ 362005 | D37 | D86~D1210 | D37 | |
| 362006 ~ 363130 | D38 | D86~D1210 | D38 | |
| 363131 ~ 364255 | D39 | D86~D1210 | D39 | |
| 364256 ~ 365380 | D40 | D86~D1210 | D40 | |
| 365381 ~ 366505 | D41 | D86~D1210 | D41 | |
| 366506 ~ 367630 | D42 | D86~D1210 | D42 | |
| 367631 ~ 368755 | D43 | D86~D1210 | D43 | |
| 368756 ~ 369880 | D44 | D86~D1210 | D44 | |
| 369881 ~ 371005 | D45 | D86~D1210 | D45 | |
| 371006 ~ 372130 | D46 | D86~D1210 | D46 | |
| 372131 ~ 373255 | D47 | D86~D1210 | D47 | |
| 373256 ~ 374380 | D48 | D86~D1210 | D48 | |
| 374381 ~ 375505 | D49 | D86~D1210 | D49 | |
| 375506 ~ 376630 | D50 | D86~D1210 | D50 | |
| 376631 ~ 377755 | D51 | D86~D1210 | D51 | |
| 377756 ~ 378880 | D52 | D86~D1210 | D52 | |
| 378881 ~ 380005 | D53 | D86~D1210 | D53 | $R^3 = R^5$ |
| 380006 ~ 381130 | D54 | D86~D1210 | D54 | |
| 381131 ~ 382255 | D55 | D86~D1210 | D55 | |
| 382256 ~ 383380 | D56 | D86~D1210 | D56 | |
| 383381 ~ 384505 | D57 | D86~D1210 | D57 | |
| 384506 ~ 385630 | D58 | D86~D1210 | D58 | |
| 385631 ~ 386755 | D59 | D86~D1210 | D59 | |
| 386756 ~ 387880 | D60 | D86~D1210 | D60 | |
| 387881 ~ 389005 | D61 | D86~D1210 | D61 | |
| 389006 ~ 390130 | D62 | D86~D1210 | D62 | |
| 390131 ~ 391255 | D63 | D86~D1210 | D63 | |
| 391256 ~ 392380 | D64 | D86~D1210 | D64 | |
| 392381 ~ 393505 | D65 | D86~D1210 | D65 | |
| 393506 ~ 394630 | D66 | D86~D1210 | D66 | |

(continued)

| No. | $R^3$ | $R^4$ | $R^5$ | = |
|---|---|---|---|---|
| 394631～395755 | D67 | D86~D1210 | D67 | |
| 395756～396880 | D68 | D86~D1210 | D68 | |
| 396881～398005 | D69 | D86~D1210 | D69 | |
| 398006～399130 | D70 | D86~D1210 | D70 | |
| 399131～400255 | D71 | D86~D1210 | D71 | |
| 400256～401380 | D72 | D86~D1210 | D72 | |
| 401381～402505 | D73 | D86~D1210 | D73 | |
| 402506～403630 | D74 | D86~D1210 | D74 | |
| 403631～404755 | D75 | D86~D1210 | D75 | |
| 404756～405880 | D76 | D86~D1210 | D76 | |
| 405881～407005 | D77 | D86~D1210 | D77 | |
| 407006～408130 | D78 | D86~D1210 | D78 | |
| 408131～409255 | D79 | D86~D1210 | D79 | |
| 409256～410380 | D80 | D86~D1210 | D80 | |
| 410381～411505 | D81 | D86~D1210 | D81 | |
| 411506～412630 | D82 | D86~D1210 | D82 | |
| 412631～413755 | D83 | D86~D1210 | D83 | |
| 413756～414880 | D84 | D86~D1210 | D84 | |
| 414881～416005 | D85 | D86~D1210 | D85 | |
| 416006～417130 | Ar1 | D86~D1210 | Ar1 | |
| 417131～418255 | Ar14 | D86~D1210 | Ar14 | |
| 418256～419380 | Ar15 | D86~D1210 | Ar15 | |
| 419381～420505 | Ar39 | D86~D1210 | Ar39 | |
| 420506～421630 | Ar40 | D86~D1210 | Ar40 | |
| 421631～422755 | Ar47 | D86~D1210 | Ar47 | |
| 422756～423879 | D88 | D86, D87, D89~D1210 | D88 | $R^3 = R^5$ |
| 423880～425003 | D106 | D86~D105, D107~D1210 | D106 | |
| 425004～426127 | D119 | D86~D118,D120~D1210 | D119 | |
| 426128～427251 | D471 | D86~D470, D472~D1210 | D471 | |
| 427252～428375 | D705 | D86~D704, D706~D1210 | D705 | |
| 428376～429499 | D737 | D86~D736, D738~D1210 | D737 | |
| 429500～430623 | D760 | D86~D759, D761~D1210 | D760 | |
| 430624～431747 | D763 | D86~D762, D764~D1210 | D763 | |
| 431748～432871 | D783 | D86~D782, D784~D1210 | D783 | |
| 432872～433995 | D86, D87, D89~D1210 | D88 | D86, D87, D89~D1210 | |
| 433996～435119 | D86~D105, D107~D1210 | D106 | D86~D105, D107~D1210 | |
| 435120～436243 | D86~D118, D120~D1210 | D119 | D86~D118, D120~D1210 | |
| 436244～437367 | D86~D470, D472~D1210 | D471 | D86~D470, D472~D1210 | |

(continued)

| No. | $R^3$ | $R^4$ | $R^5$ | = |
|---|---|---|---|---|
| 437368 ~ 438491 | D86~D704, D706~D1210 | D705 | D86~D704, D706~D1210 | $R^3 = R^5$ |
| 438492 ~ 439615 | D86~D736, D738~D1210 | D737 | D86~D736, D738~D1210 | |
| 439616 ~ 440739 | D86~D759, D761~D1210 | D760 | D86~D759, D761~D1210 | |
| 440740 ~ 441863 | D86~D762, D764~D1210 | D763 | D86~D762, D764~D1210 | |
| 441864 ~ 442987 | D86~D782, D784~D1210 | D783 | D86~D782. D784~D1210 | |

Next, specific examples of the compound having a structure represented by the following general formula (1b) are shown in Table 3. In Table 3, structures of the compounds are shown in the same manner as in Table 2.

General Formula (1b)

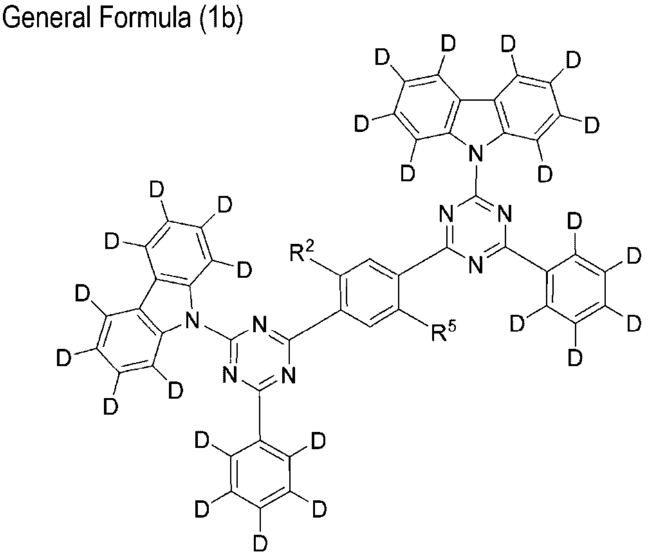

[Table 3]

| No. 442988 ~ 444197 | $R^2$ D1~D1210 | $R^5$ D1~D1210 | = $R^2 = R^5$ |
|---|---|---|---|
| 444198 ~ 445322 | D86~D1210 | D1 | |
| 445323 ~ 446447 | D86~D1210 | D2 | |
| 446448 ~ 447572 | D86~D1210 | D3 | |
| 447573 ~ 448697 | D86~D1210 | D4 | |
| 448698 ~ 449822 | D86~D1210 | D5 | |
| 449823 ~ 450947 | D86~D1210 | D6 | |
| 450948 ~ 452072 | D86~D1210 | D7 | |
| 452073 ~ 453197 | D86~D1210 | D8 | |
| 453198 ~ 454322 | D86~D1210 | D9 | |
| 454323 ~ 455447 | D86~D1210 | D10 | |
| 455448 ~ 456572 | D86~D1210 | D11 | |

(continued)

| No.<br>442988 ~ 444197 | $R^2$<br>D1~D1210 | $R^5$<br>D1~D1210 | =<br>$R^2 = R^5$ |
|---|---|---|---|
| 456573 ~ 457697 | D86~D1210 | D12 | $R^2 \neq R^5$ |
| 457698 ~ 458822 | D86~D1210 | D13 | |
| 458823 ~ 459947 | D86~D1210 | D14 | |
| 459948 ~ 461072 | D86~D1210 | D15 | |
| 461073 ~ 462197 | 086~01210 | D16 | |
| 462198 ~ 463322 | D86~D1210 | D17 | |
| 463323 ~ 464447 | D86~D1210 | D18 | |
| 464448 ~ 465572 | D86~D1210 | D19 | |
| 465573 ~ 466697 | 086~01210 | D20 | |
| 466698 ~ 467822 | D86~D1210 | D21 | |
| 467823 ~ 468947 | D86~D1210 | D22 | |
| 468948 ~ 470072 | D86~D1210 | D23 | |
| 470073 ~ 471197 | D86~D1210 | D24 | |
| 471198 ~ 472322 | D86~D1210 | D25 | |
| 472323 ~ 473447 | D86~D1210 | D26 | |
| 473448 ~ 474572 | D86~D1210 | D27 | |
| 474573 ~ 475697 | D86~D1210 | D28 | |
| 475698 ~ 476822 | D86~D1210 | D29 | |
| 476823 ~ 477947 | D86~D1210 | D30 | |
| 477948 ~ 479072 | D86~D1210 | D31 | |
| 479073 ~ 480197 | D86~D1210 | D32 | |
| 480198 ~ 481322 | D86~D1210 | D33 | |
| 481323 ~ 482447 | D86~D1210 | D34 | |
| 482448 ~ 483572 | D86~D1210 | D35 | |
| 483573 ~ 484697 | D86~D1210 | D36 | |
| 484698 ~ 485822 | D86~D1210 | D37 | |
| 485823 ~ 486947 | D86~D1210 | D38 | |
| 486948 ~ 488072 | D86~D1210 | D39 | |
| 488073 ~ 489197 | D86~D1210 | D40 | |
| 489198 ~ 490322 | D86~D1210 | D41 | |
| 490323 ~ 491447 | D86~D1210 | D42 | |
| 491448 ~ 492572 | D86~D1210 | D43 | |
| 492573 ~ 493697 | D86~D1210 | D44 | |
| 493698 ~ 494822 | D86~D1210 | D45 | |
| 494823 ~ 495947 | D86~D1210 | D46 | |
| 495948 ~ 497072 | D86~D1210 | D47 | |
| 497073 ~ 498197 | D86~D1210 | D48 | |
| 498198 ~ 499322 | D86~D1210 | D49 | |
| 499323 ~ 500447 | D86~D1210 | D50 | |

(continued)

| No.<br>442988 ~ 444197 | $R^2$<br>D1~D1210 | $R^5$<br>D1~D1210 | =<br>$R^2 = R^5$ |
|---|---|---|---|
| 500448 ~ 501572 | D86~D1210 | D51 | |
| 501573 ~ 502697 | D86~D1210 | D52 | |
| 502698 ~ 503822, | D86~D1210 | D53 | |
| 503823 ~ 504947 | D86~D1210 | D54 | |
| 504948 ~ 506072 | D86~D1210 | D55 | |
| 506073 ~ 507197 | D86~D1210 | D56 | |
| 507198 ~ 508322 | 086~01210 | D57 | |
| 508323 ~ 509447 | 086~01210 | D58 | |
| 509448 ~ 510572 | 086~01210 | D59 | |
| 510573 ~ 511697 | D86~D1210 | D60 | $R^2 \neq R^5$ |
| 511698 ~ 512822<br>512823 ~ 513947 | D86~D1210<br>D86~D1210 | D61<br>D62 | |
| 513948 ~ 515072 | D86~D1210 | D63 | |
| 515073 ~ 516197 | D86~D1210 | D64 | |
| 516198 ~ 517322 | D86~D1210 | D65 | |
| 517323 ~ 518447 | D86~D1210 | D66 | |
| 518448 ~ 519572 | D86~D1210 | D67 | |
| 519573 ~ 520697 | D86~D1210 | D68 | |
| 520698 ~ 521822 | D86~D1210 | D69 | |
| 521823 ~ 522947 | D86~D1210 | D70 | |
| 522948 ~ 524072 | D86~D1210 | D71 | |
| 524073 ~ 525197 | D86~D1210 | D72 | |
| 525198 ~ 526322 | D86~D1210 | D73 | |
| 526323 ~ 527447 | D86~D1210 | D74 | |
| 527448 ~ 528572 | D86~D1210 | D75 | |
| 528573 ~ 529697 | D86~D1210 | D76 | |
| 529698 ~ 530822 | D86~D1210 | D77 | |
| 530823 ~ 531947 | D86~D1210 | D78 | |
| 531948 ~ 533072<br>533073 ~ 534197 | D86~D1210<br>D86~D1210 | D79<br>D80 | |
| 534198 ~ 535322 | D86~D1210 | D81 | |
| 535323 ~ 536447 | D86~D1210 | D82 | |
| 536448 ~ 537572 | D86~D1210 | D83 | |
| 537573 ~ 538697 | D86~D1210 | D84 | |
| 538698 ~ 539822 | D86~D1210 | D85 | |

Next, specific examples of the compound having a structure represented by the following general formula (1c) are shown in Table 4. In Table 4, structures of the compounds are shown in the same manner as in Table 2.

General Formula (1c)

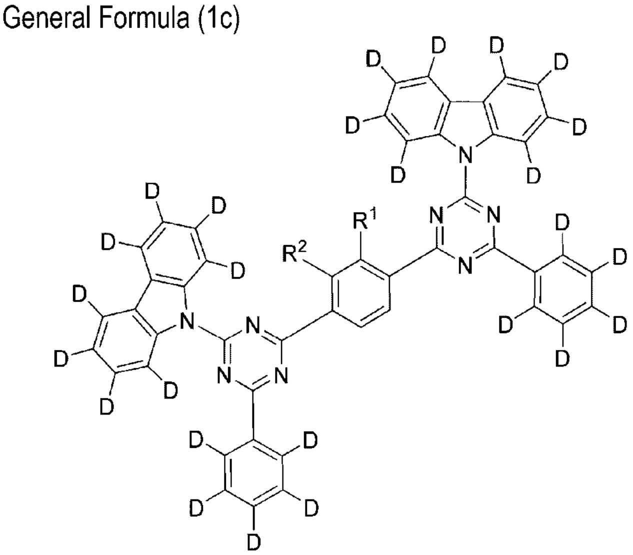

[Table 4]

| No. | $R^1$ | $R^2$ | = |
|---|---|---|---|
| 539823 ~ 541032 | D1~D1210 | D1~D1210 | $R^1 = R^2$ |
| 541033 ~ 542157 | D86~D1210 | D1 | |
| 542158 ~ 543282 | D86~D1210 | D2 | |
| 543283 ~ 544407 | D86~D1210 | D3 | |
| 544408 ~ 545532 | D86~D1210 | D4 | |
| 545533 ~ 546657 | D86~D1210 | D5 | |
| 546658 ~ 547782 | D86~D1210 | D6 | |
| 547783 ~ 548907 | D86~D1210 | D7 | |
| 548908 ~ 550032 | D86~D1210 | D8 | |
| 550033 ~ 551157 | D86~D1210 | D9 | |
| 551158 ~ 552282 | D86~D1210 | D10 | |
| 552283 ~ 553407 | D86~D1210 | D11 | |
| 553408 ~ 554532 | D86~D1210 | D12 | |
| 554533 ~ 555657 | D86~D1210 | D13 | |
| 555658 ~ 556782 | D86~D1210 | D14 | |
| 556783 ~ 557907 | D86~D1210 | D15 | |
| 557908 ~ 559032 | D86~D1210 | D16 | |
| 559033 ~ 560157 | D86~D1210 | D17 | |
| 560158 ~ 561282 | D86~D1210 | D18 | |
| 561283 ~ 562407 | D86~D1210 | D19 | |
| 562408 ~ 563532 | D86~D1210 | D20 | |
| 563533 ~ 564657 | D86~D1210 | D21 | |
| 564658 ~ 565782 | D86~D1210 | D22 | |
| 565783 ~ 566907 | D86~D1210 | D23 | |
| 566908 ~ 568032 | D86~D1210 | D24 | |

(continued)

| No. | $R^1$ | $R^2$ | = |
|---|---|---|---|
| 568033 ~ 569157 | D86~D1210 | D25 | $R^1 \neq R^2$ |
| 569158 ~ 570282 | D86~D1210 | D26 | |
| 570283 ~ 571407 | D86~D1210 | D27 | |
| 571408 ~ 572532 | D86~D1210 | D28 | |
| 572533 ~ 573657 | D86~D1210 | D29 | |
| 573658 ~ 574782 | D86~D1210 | D30 | |
| 574783 ~ 575907 | D86~D1210 | D31 | |
| 575908 ~ 577032 | D86~D1210 | D32 | |
| 577033 ~ 578157 | D86~D1210 | D33 | |
| 578158 ~ 579282 | D86~D1210 | D34 | |
| 579283 ~ 580407 | D86~D1210 | D35 | |
| 580408 ~ 581532 | D86~D1210 | D36 | |
| 581533 ~ 582657 | D86~D1210 | D37 | |
| 582658 ~ 583782 | D86~D1210 | D38 | |
| 583783 ~ 584907 | D86~D1210 | D39 | |
| 584908 ~ 586032 | D86~D1210 | D40 | |
| 586033 ~ 587157 | D86~D1210 | D41 | |
| 587158 ~ 588282 | D86~D1210 | D42 | |
| 588283 ~ 589407 | D86~D1210 | D43 | |
| 589408 ~ 590532 | D86~D1210 | D44 | |
| 590533 ~ 591657 | D86~D1210 | D45 | |
| 591658 ~ 592782 | D86~D1210 | D46 | |
| 592783 ~ 593907 | D86~D1210 | D47 | |
| 593908 ~ 595032 | D86~D1210 | D48 | |
| 595033 ~ 596157 | D86~D1210 | D49 | |
| 596158 ~ 597282 | D86~D1210 | D50 | |
| 597283 ~ 598407 | D86~D1210 | D51 | |
| 598408 ~ 599532 | D86~D1210 | D52 | |
| 599533 ~ 600657 | D86~D1210 | D53 | |
| 600658 ~ 601782 | D86~D1210 | D54 | |
| 601783 ~ 602907 | D86~D1210 | D55 | |
| 602908 ~ 604032 | D86~D1210 | D56 | |
| 604033 ~ 605157 | D86~D1210 | D57 | |
| 605158 ~ 606282 | D86~D1210 | D58 | |
| 606283 ~ 607407 | D86~D1210 | D59 | |
| 607408 ~ 608532 | D86~D1210 | D60 | |
| 608533 ~ 609657 | D86~D1210 | D61 | |
| 609658 ~ 610782 | D86~D1210 | D62 | |
| 610783 ~ 611907 | D86~D1210 | D63 | |
| 611908 ~ 613032 | D86~D1210 | D64 | |

(continued)

| No. | $R^1$ | $R^2$ | = |
|---|---|---|---|
| 613033 ~ 614157 | D86~D1210 | D65 | $R^1 \neq R^2$ |
| 614158 ~ 615282 | D86~D1210 | D66 | |
| 615283 ~ 616407 | D86~D1210 | D67 | |
| 616408 ~ 617532 | D86~D1210 | D68 | |
| 617533 ~ 618657 | D86~D1210 | D69 | |
| 618658 ~ 619782 | D86~D1210 | D70 | |
| 619783 ~ 620907 | D86~D1210 | D71 | |
| 620908 ~ 622032 | D86~D1210 | D72 | |
| 622033 ~ 623157 | D86~D1210 | D73 | |
| 623158 ~ 624282 | D86~D1210 | D74 | |
| 624283 ~ 625407 | D86~D1210 | D75 | |
| 625408 ~ 626532 | D86~D1210 | D76 | |
| 626533 ~ 627657 | D86~D1210 | D77 | |
| 627658 ~ 628782 | D86~D1210 | D78 | |
| 628783 ~ 629907 | D86~D1210 | D79 | |
| 629908 ~ 631032 | D86~D1210 | D80 | |
| 631033 ~ 632157 | D86~D1210 | D81 | |
| 632158 ~ 633282 | D86~D1210 | D82 | |
| 633283 ~ 634407 | D86~D1210 | D83 | |
| 634408 ~ 635532 | D86~D1210 | D84 | |
| 635533 ~ 636657 | D86~D1210 | D85 | |

Next, specific examples of the compound having a structure represented by the following general formula (1d) are shown in Table 5. In Table 5, structures of the compounds are shown in the same manner as in Table 2.

General Formula (1d)

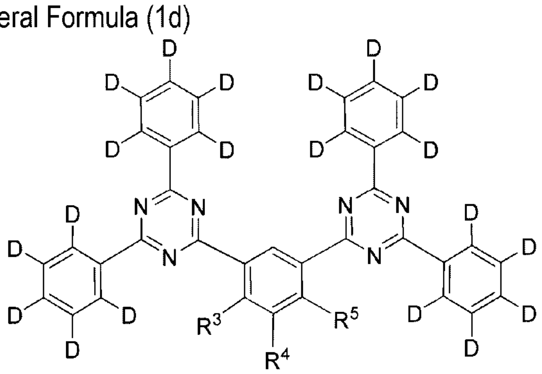

[Table 5]

| No. | $R^3$ | $R^4$ | $R^5$ | = |
|---|---|---|---|---|
| 636658 ~ 637782 | D86~D1210 | D86~D1210 | D86~D1210 | $R^3 = R^4 = R^5$ |
| 637783 ~ 638907 | D86~D1210 | H | D86~D1210 | |
| 638908 ~ 640032 | D86~D1210 | D1 | D86~D1210 | |

(continued)

| No. | $R^3$ | $R^4$ | $R^5$ | = |
|---|---|---|---|---|
| 640033 ~ 641157 | D86~D1210 | D2 | D86~D1210 | |
| 641158 ~ 642282 | D86~D1210 | D3 | D86~D1210 | |
| 642283 ~ 643407 | D86~D1210 | D4 | D86~D1210 | |
| 643408 ~ 644532 | D86~D1210 | D5 | D86~D1210 | |
| 644533 ~ 645657 | D86~D1210 | D6 | D86~D1210 | |
| 645658 ~ 646782 | D86~D1210 | D7 | D86~D1210 | |
| 646783 ~ 647907 | D86~D1210 | D8 | D86~D1210 | |
| 647908 ~ 649032 | D86~D1210 | D9 | D86~D1210 | |
| 649033 ~ 650157 | D86~D1210 | D10 | D86~D1210 | |
| 650158 ~ 651282 | D86~D1210 | D11 | D86~D1210 | |
| 651283 ~ 652407 | D86~D1210 | D12 | D86~D1210 | |
| 652408 ~ 653532 | D86~D1210 | D13 | D86~D1210 | |
| 653533 ~ 654657 | D86~D1210 | D14 | D86~D1210 | |
| 654658 ~ 655782 | D86~D1210 | D15 | D86~D1210 | |
| 655783 ~ 656907 | D86~D1210 | D16 | D86~D1210 | |
| 656908 ~ 658032 | D86~D1210 | D17 | D86~D1210 | |
| 658033 ~ 659157 | D86~D1210 | D18 | D86~D1210 | |
| 659158 ~ 660282 | D86~D1210 | D19 | D86~D1210 | |
| 660283 ~ 661407 | D86~D1210 | D20 | D86~D1210 | |
| 661408 ~ 662532 | D86~D1210 | D21 | D86~D1210 | |
| 662533 ~ 663657 | D86~D1210 | D22 | D86~D1210 | |
| 663658 ~ 664782 | D86~D1210 | D23 | D86~D1210 | |
| 664783 ~ 665907 | D86~D1210 | D24 | D86~D1210 | |
| 665908 ~ 667032 | D86~D1210 | D25 | D86~D1210 | |
| 667033 ~ 668157 | D86~D1210 | D26 | D86~D1210 | |
| 668158 ~ 669282 | D86~D1210 | D27 | D86~D1210 | |
| 669283 ~ 670407 | D86~D1210 | D28 | D86~D1210 | |
| 670408 ~ 671532 | D86~D1210 | D29 | D86~D1210 | $R^3 = R^5$ |
| 671533 ~ 672657 | D86~D1210 | D30 | D86~D1210 | |
| 672658 ~ 673782 | D86~D1210 | D31 | D86~D1210 | |
| 673783 ~ 674907 | D86~D1210 | D32 | D86~D1210 | |
| 674908 ~ 676032 | D86~D1210 | D33 | D86~D1210 | |
| 676033 ~ 677157 | D86~D1210 | D34 | D86~D1210 | |
| 677158 ~ 678282 | D86~D1210 | D35 | D86~D1210 | |
| 678283 ~ 679407 | D86~D1210 | D36 | D86~D1210 | |
| 679408 ~ 680532 | D86~D1210 | D37 | D86~D1210 | |
| 680533 ~ 681657 | D86~D1210 | D38 | D86~D1210 | |
| 681658 ~ 682782 | D86~D1210 | D39 | D86~D1210 | |
| 682783 ~ 683907 | D86~D1210 | D40 | D86~D1210 | |

(continued)

| No. | $R^3$ | $R^4$ | $R^5$ | = |
|---|---|---|---|---|
| 683908 ~ 685032 | D86~D1210 | D41 | D86~D1210 | |
| 685033 ~ 686157 | D86~D1210 | D42 | D86~D1210 | |
| 686158 ~ 687282 | D86~D1210 | D43 | D86~D1210 | |
| 687283 ~ 688407 | D86~D1210 | D44 | D86~D1210 | |
| 688408 ~ 689532 | D86~D1210 | D45 | D86~D1210 | |
| 689533 ~ 690657 | D86~D1210 | D46 | D86~D1210 | |
| 690658 ~ 691782 | D86~D1210 | D47 | D86~D1210 | |
| 691783 ~ 692907 | D86~D1210 | D48 | D86~D1210 | |
| 692908 ~ 694032 | D86~D1210 | D49 | D86~D1210 | |
| 694033 ~ 695157 | D86~D1210 | D50 | D86~D1210 | |
| 695158 ~ 696282 | D86~D1210 | D51 | D86~D1210 | |
| 696283 ~ 697407 | D86~D1210 | D52 | D86~D1210 | |
| 697408 ~ 698532 | D86~D1210 | D53 | D86~D1210 | |
| 698533 ~ 699657 | D86~D1210 | D54 | D86~D1210 | |
| 699658 ~ 700782 | D86~D1210 | D55 | D86~D1210 | |
| 700783 ~ 701907 | D86~D1210 | D56 | D86~D1210 | |
| 701908 ~ 703032 | D86~D1210 | D57 | D86~D1210 | |
| 703033 ~ 704157 | D86~D1210 | D58 | D86~D1210 | |
| 704158 ~ 705282 | D86~D1210 | D59 | D86~D1210 | $R^3 = R^5$ |
| 705283 ~ 706407 | D86~D1210 | D60 | D86~D1210 | |
| 706408 ~ 707532 | D86~D1210 | D61 | D86~D1210 | |
| 707533 ~ 708657 | D86~D1210 | D62 | D86~D1210 | |
| 708658 ~ 709782 | D86~D1210 | D63 | D86~D1210 | |
| 709783 ~ 710907 | D86~D1210 | D64 | D86~D1210 | |
| 710908 ~ 712032 | D86~D1210 | D65 | D86~D1210 | |
| 712033 ~ 713157 | D86~D1210 | D66 | D86~D1210 | |
| 713158 ~ 714282 | D86~D1210 | D67 | D86~D1210 | |
| 714283 ~ 715407 | D86~D1210 | D68 | D86~D1210 | |
| 715408 ~ 716532 | D86~D1210 | D69 | D86~D1210 | |
| 716533 ~ 717657 | D86~D1210 | D70 | D86~D1210 | |
| 717658 ~ 718782 | D86~D1210 | D71 | D86~D1210 | |
| 718783 ~ 719907 | D86~D1210 | D72 | D86~D1210 | |
| 719908 ~ 721032 | D86~D1210 | D73 | D86~D1210 | |
| 721033 ~ 722157 | D86~D1210 | D74 | D86~D1210 | |
| 722158 ~ 723282 | D86~D1210 | D75 | D86~D1210 | |
| 723283 ~ 724407 | D86~D1210 | D76 | D86~D1210 | |
| 724408 ~ 725532 | D86~D1210 | D77 | D86~D1210 | |
| 725533 ~ 726657 | D86~D1210 | D78 | D86~D1210 | |
| 726658 ~ 727782 | D86~D1210 | D79 | D86~D1210 | |

(continued)

| No. | $R^3$ | $R^4$ | $R^5$ | = |
|---|---|---|---|---|
| 727783 ~ 728907 | D86~D1210 | D80 | D86~D1210 | |
| 728908 ~ 730032 | D86~D1210 | D81 | D86~D1210 | |
| 730033 ~ 731157 | D86~D1210 | D82 | D86~D1210 | |
| 731158 ~ 732282 | D86~D1210 | D83 | D86~D1210 | |
| 732283 ~ 733407 | D86~D1210 | D84 | D86~D1210 | |
| 733408 ~ 734532 | D86~D1210 | D85 | D86~D1210 | |
| 734533 ~ 735657 | D86~D1210 | Ar1 | D86~D1210 | |
| 735658 ~ 736782 | D86~D1210 | Ar14 | D86~D1210 | |
| 736783 ~ 737907 | D86~D1210 | Ar15 | D86~D1210 | |
| 737908 ~ 739032 | D86~D1210 | Ar39 | D86~D1210 | |
| 739033 ~ 740157 | D86~D1210 | Ar40 | D86~D1210 | |
| 740158 ~ 741282 | D86~D1210 | Ar47 | D86~D1210 | |

(continued)

| No. | $R^3$ | $R^4$ | $R^5$ | = |
|---|---|---|---|---|
| 741283 ~ 742407 | H | D86~D1210 | D86~D1210 | $R^4 = R^5$ |
| 742408 ~ 743532 | D1 | D86~D1210 | D86~D1210 | |
| 743533 ~ 744657 | D2 | D86~D1210 | D86~D1210 | |
| 744658 ~ 745782 | D3 | D86~D1210 | D86~D1210 | |
| 745783 ~ 746907 | D4 | D86~D1210 | D86~D1210 | |
| 746908 ~ 748032 | D5 | D86~D1210 | D86~D1210 | |
| 748033 ~ 749157 | D6 | D86~D1210 | D86~D1210 | |
| 749158 ~ 750282 | D7 | D86~D1210 | D86~D1210 | |
| 750283 ~ 751407 | D8 | D86~D1210 | D86~D1210 | |
| 751408 ~ 752532 | D9 | D86~D1210 | D86~D1210 | |
| 752533 ~ 753657 | D10 | D86~D1210 | D86~D1210 | |
| 753658 ~ 754782 | D11 | D86~D1210 | D86~D1210 | |
| 754783 ~ 755907 | D12 | D86~D1210 | D86~D1210 | |
| 755908 ~ 757032 | D13 | D86~D1210 | D86~D1210 | |
| 757033 ~ 758157 | D14 | D86~D1210 | D86~D1210 | |
| 758158 ~ 759282 | D15 | D86~D1210 | D86~D1210 | |
| 759283 ~ 760407 | D16 | D86~D1210 | D86~D1210 | |
| 760408 ~ 761532 | D17 | D86~D1210 | D86~D1210 | |
| 761533 ~ 762657 | D18 | D86~D1210 | D86~D1210 | |
| 762658 ~ 763782 | D19 | D86~D1210 | D86~D1210 | |
| 763783 ~ 764907 | D20 | D86~D1210 | D86~D1210 | |
| 764908 ~ 766032 | D21 | D86~D1210 | D86~D1210 | |
| 766033 ~ 767157 | D22 | D86~D1210 | D86~D1210 | |
| 767158 ~ 768282 | D23 | D86~D1210 | D86~D1210 | |
| 768283 ~ 769407 | D24 | D86~D1210 | D86~D1210 | |
| 769408 ~ 770532 | D25 | D86~D1210 | D86~D1210 | |
| 770533 ~ 771657 | D26 | D86~D1210 | D86~D1210 | |
| 771658 ~ 772782 | D27 | D86~D1210 | D86~D1210 | |
| 772783 ~ 773907 | D28 | D86~D1210 | D86~D1210 | |
| 773908 ~ 775032 | D29 | D86~D1210 | D86~D1210 | |
| 775033 ~ 776157 | D30 | D86~D1210 | D86~D1210 | |
| 776158 ~ 777282 | D31 | D86~D1210 | D86~D1210 | |
| 777283 ~ 778407 | D32 | D86~D1210 | D86~D1210 | |
| 778408 ~ 779532 | D33 | D86~D1210 | D86~D1210 | |
| 779533 ~ 780657 | D34 | D86~D1210 | D86~D1210 | |
| 780658 ~ 781782 | D35 | D86~D1210 | D86~D1210 | |
| 781783 ~ 782907 | D36 | D86~D1210 | D86~D1210 | |
| 782908 ~ 784032 | D37 | D86~D1210 | D86~D1210 | |
| 784033 ~ 785157 | D38 | D86~D1210 | D86~D1210 | |
| 785158 ~ 786282 | D39 | D86~D1210 | D86~D1210 | |

(continued)

| No. | $R^3$ | $R^4$ | $R^5$ | = |
|---|---|---|---|---|
| 786283 ~ 787407 | D40 | D86~D1210 | D86~D1210 | $R^4 = R^5$ |
| 787408 ~ 788532 | D41 | D86~D1210 | D86~D1210 | |
| 788533 ~ 789657 | D42 | D86~D1210 | D86~D1210 | |
| 789658 ~ 790782 | D43 | D86~D1210 | D86~D1210 | |
| 790783 ~ 791907 | D44 | D86~D1210 | D86~D1210 | |
| 791908 ~ 793032 | D45 | D86~D1210 | D86~D1210 | |
| 793033 ~ 794157 | D46 | D86~D1210 | D86~D1210 | |
| 794158 ~ 795282 | D47 | D86~D1210 | D86~D1210 | |
| 795283 ~ 796407 | D48 | D86~D1210 | D86~D1210 | |
| 796408 ~ 797532 | D49 | D86~D1210 | D86~D1210 | |
| 797533 ~ 798657 | D50 | D86~D1210 | D86~D1210 | |
| 798658 ~ 799782 | D51 | D86~D1210 | D86~D1210 | |
| 799783 ~ 800907 | D52 | D86~D1210 | D86~D1210 | |
| 800908 ~ 802032 | D53 | D86~D1210 | D86~D1210 | |
| 802033 ~ 803157 | D54 | D86~D1210 | D86~D1210 | |
| 803158 ~ 804282 | D55 | D86~D1210 | D86~D1210 | |
| 804283 ~ 805407 | D56 | D86~D1210 | D86~D1210 | |
| 805408 ~ 806532 | D57 | D86~D1210 | D86~D1210 | |
| 806533 ~ 807657<br>807658 ~ 808782 | D58<br>D59 | D86~D1210<br>D86~D1210 | D86~D1210<br>D86~D1210 | |
| 808783 ~ 809907 | D60 | D86~D1210 | D86~D1210 | |
| 809908 ~ 811032 | D61 | D86~D1210 | D86~D1210 | |
| 811033 ~ 812157 | D62 | D86~D1210 | D86~D1210 | |
| 812158 ~ 813282 | D63 | D86~D1210 | D86~D1210 | |
| 813283 ~ 814407 | D64 | D86~D1210 | D86~D1210 | |
| 814408 ~ 815532 | D65 | D86~D1210 | D86~D1210 | |
| 815533 ~ 816657 | D66 | D86~D1210 | D86~D1210 | |
| 816658 ~ 817782 | D67 | D86~D1210 | D86~D1210 | |
| 817783 ~ 818907 | D68 | D86~D1210 | D86~D1210 | |
| 818908 ~ 820032 | D69 | D86~D1210 | D86~D1210 | |
| 820033 ~ 821157 | D70 | D86~D1210 | D86~D1210 | |
| 821158 ~ 822282 | D71 | D86~D1210 | D86~D1210 | |
| 822283 ~ 823407 | D72 | D86~D1210 | D86~D1210 | |
| 823408 ~ 824532 | D73 | D86~D1210 | D86~D1210 | |
| 824533 ~ 825657 | D74 | D86~D1210 | D86~D1210 | |
| 825658 ~ 826782 | D75 | D86~D1210 | D86~D1210 | |
| 826783 ~ 827907 | D76 | D86~D1210 | D86~D1210 | |
| 827908 ~ 829032 | D77 | D86~D1210 | D86~D1210 | |
| 829033 ~ 830157 | D78 | D86~D1210 | D86~D1210 | |
| 830158 ~ 831282 | D79 | D86~D1210 | D86~D1210 | |

(continued)

| No. | $R^3$ | $R^4$ | $R^5$ | = |
|---|---|---|---|---|
| 831283 ~ 832407 | D80 | D86~D1210 | D86~D1210 | |
| 832408 ~ 833532 | D81 | D86~D1210 | D86~D1210 | |
| 833533 ~ 834657 | D82 | D86~D1210 | D86~D1210 | |
| 834658 ~ 835782 | D83 | D86~D1210 | D86~D1210 | |
| 835783 ~ 836907 | D84 | D86~D1210 | D86~D1210 | |
| 836908 ~ 838032 | D85 | D86~D1210 | D86~D1210 | |
| 838033 ~ 839157 | Ar1 | D86~D1210 | D86~D1210 | |
| 839158 ~ 840282 | Ar14 | D86~D1210 | D86~D1210 | |
| 840283 ~ 841407 | Ar15 | D86~D1210 | D86~D1210 | |
| 841408 ~ 842532 | Ar39 | D86~D1210 | D86~D1210 | |
| 842533 ~ 843657 | Ar40 | D86~D1210 | D86~D1210 | |
| 843658 ~ 844782 | Ar47 | D86~D1210 | D86~D1210 | $R^4 = R^5$ |
| 844783 ~ 845907<br>845908 ~ 847032 | D86~D1210<br>D86~D1210 | D86~D1210<br>D86 ~ D1210 | H<br>D1 | |
| 847033 ~ 848157 | D86~D1210 | D86~D1210 | D2 | |
| 848158 ~ 849282 | D86~D1210 | D86~D1210 | D3 | |
| 849283 ~ 850407 | D86~D1210 | D86~D1210 | D4 | |
| 850408 ~ 851532<br>851533 ~ 852657 | D86~D1210<br>D86~D1210 | D86~D1210<br>D86 ~ D1210 | D5<br>D6 | |
| 852658 ~ 853782 | D86~D1210 | D86~D1210 | D7 | |
| 853783 ~ 854907 | D86~D1210 | D86~D1210 | D8 | |
| 854908 ~ 856032 | D86~D1210 | D86~D1210 | D9 | |
| 856033 ~ 857157 | D86~D1210 | D86~D1210 | D10 | |
| 857158 ~ 858282 | D86~D1210 | D86~D1210 | D11 | |
| 858283 ~ 859407<br>859408 ~ 860532 | D86~D1210<br>D86~D1210 | D86~D1210<br>D86~D1210 | D12<br>D13 | |
| 860533 ~ 861657 | D86~D1210 | D86~D1210 | D14 | |
| 861658 ~ 862782 | D86~D1210 | D86~D1210 | D15 | |
| 862783 ~ 863907 | D86~D1210 | D86~D1210 | D16 | |
| 863908 ~ 865032 | D86~D1210 | D86~D1210 | D17 | |
| 865033 ~ 866157 | D86~D1210 | D86~D1210 | D18 | |
| 866158 ~ 867282 | D86~D1210 | D86~D1210 | D19 | |
| 867283 ~ 868407 | D86~D1210 | D86~D1210 | D20 | |
| 868408 ~ 869532 | D86~D1210 | D86~D1210 | D21 | |
| 869533 ~ 870657 | D86~D1210 | D86~D1210 | D22 | |
| 870658 ~ 871782 | D86~D1210 | D86~D1210 | D23 | |
| 871783 ~ 872907 | D86~D1210 | D86~D1210 | D24 | |
| 872908 ~ 874032 | D86~D1210 | D86~D1210 | D25 | |
| 874033 ~ 875157 | D86~D1210 | D86~D1210 | D26 | |
| 875158 ~ 876282 | D86~D1210 | D86~D1210 | D27 | |

(continued)

| No. | $R^3$ | $R^4$ | $R^5$ | = |
|---|---|---|---|---|
| 876283 ~ 877407<br>877408 ~ 878532 | D86~D1210<br>D86~D1210 | D86~D1210<br>D86~D1210 | D28<br>D29 | $R^3 = R^4$ |
| 878533 ~ 879657 | D86~D1210 | D86~D1210 | D30 | |
| 879658 ~ 880782 | D86~D1210 | D86~D1210 | D31 | |
| 880783 ~ 881907 | D86~D1210 | D86~D1210 | D32 | |
| 881908 ~ 883032 | D86~D1210 | D86~D1210 | D33 | |
| 883033 ~ 884157<br>884158 ~ 885282 | D86~D1210<br>D86~D1210 | D86~D1210<br>D86~D1210 | D34<br>D35 | |
| 885283 ~ 886407 | D86~D1210 | D86~D1210 | D36 | |
| 886408 ~ 887532 | D86~D1210 | D86~D1210 | D37 | |
| 887533 ~ 888657 | D86~D1210 | D86~D1210 | D38 | |
| 888658 ~ 889782 | D86~D1210 | D86~D1210 | D39 | |
| 889783 ~ 890907 | D86~D1210 | D86~D1210 | D40 | |
| 890908 ~ 892032 | D86~D1210 | D86~D1210 | D41 | |
| 892033 ~ 893157 | D86~D1210 | D86~D1210 | D42 | |
| 893158 ~ 894282 | D86~D1210 | D86~D1210 | D43 | |
| 894283 ~ 895407 | D86~D1210 | D86~D1210 | D44 | |
| 895408 ~ 896532 | D86~D1210 | D86~D1210 | D45 | |
| 896533 ~ 897657 | D86~D1210 | D86~D1210 | D46 | |
| 897658 ~ 898782 | D86~D1210 | D86~D1210 | D47 | |
| 898783 ~ 899907 | D86~D1210 | D86~D1210 | D48 | |
| 899908 ~ 901032 | D86~D1210 | D86~D1210 | D49 | |
| 901033 ~ 902157 | D86~D1210 | D86~D1210 | D50 | |
| 902158 ~ 903282 | D86~D1210 | D86~D1210 | D51 | |
| 903283 ~ 904407 | D86~D1210 | D86~D1210 | D52 | |
| 904408 ~ 905532<br>905533 ~ 906657 | D86~D1210<br>D86~D1210 | D86~D1210<br>D86~D1210 | D53<br>D54 | |
| 906658 ~ 907782 | D86~D1210 | D86~D1210 | D55 | |
| 907783 ~ 908907 | D86~D1210 | D86~D1210 | D56 | |
| 908908 ~ 910032<br>910033 ~ 911157 | D86~D1210<br>D86~D1210 | D86 ~ D1210<br>D86~D1210 | D57<br>D58 | |
| 911158 ~ 912282 | D86~D1210 | D86~D1210 | D59 | |
| 912283 ~ 913407 | D86~D1210 | D86~D1210 | D60 | |
| 913408 ~ 914532 | D86~D1210 | D86~D1210 | D61 | |
| 914533 ~ 915657 | D86~D1210 | D86~D1210 | D62 | |
| 915658 ~ 916782 | D86~D1210 | ~ D86~D1210 | D63 | |
| 916783 ~ 917907 | D86~D1210 | D86~D1210 | D64 | |
| 917908 ~ 919032 | D86~D1210 | D86~D1210 | D65 | |
| 919033 ~ 920157 | D86~D1210 | D86~D1210 | D66 | |
| 920158 ~ 921282 | D86~D1210 | D86~D1210 | D67 | |

(continued)

| No. | $R^3$ | $R^4$ | $R^5$ | = |
|---|---|---|---|---|
| 921283 ~ 922407 | D86~D1210 | D86~D1210 | D68 | |
| 922408 ~ 923532 | D86~D1210 | D86~D1210 | D69 | |
| 923533 ~ 924657 | D86~D1210 | D86~D1210 | D70 | |
| 924658 ~ 925782 | D86~D1210 | D86~D1210 | D71 | |
| 925783 ~ 926907 | D86~D1210 | D86~D1210 | D72 | |
| 926908 ~ 928032 | D86~D1210 | D86~D1210 | D73 | |
| 928033 ~ 929157 | D86~D1210 | D86~D1210 | D74 | |
| 929158 ~ 930282 | D86~D1210 | D86~D1210 | D75 | |
| 930283 ~ 931407 | D86~D1210 | D86~D1210 | D76 | $R^3 = R^4$ |
| 931408 ~ 932532 | D86~D1210 | D86~D1210 | D77 | |
| 932533 ~ 933657 | D86~D1210 | D86~D1210 | D78 | |
| 933658 ~ 934782 | D86~D1210 | D86~D1210 | D79 | |
| 934783 ~ 935907 | D86~D1210 | D86~D1210 | D80 | |
| 935908 ~ 937032 | D86~D1210 | D86~D1210 | D81 | |
| 937033 ~ 938157 | D86~D1210 | D86~D1210 | D82 | |
| 938158 ~ 939282 | D86~D1210 | D86~D1210 | D83 | |
| 939283 ~ 940407 | D86~D1210 | D86~D1210 | D84 | |
| 940408 ~ 941532 | D86~D1210 | D86~D1210 | D85 | |
| 941533 ~ 942657 | D86~D1210 | D86~D1210 | Ar1 | |
| 942658 ~ 943782<br>943783 ~ 944907 | D86~D1210<br>D86~D1210 | D86~D1210<br>D86~D1210 | Ar14<br>Ar15 | |
| 944908 ~ 946032 | D86~D1210 | D86~D1210 | Ar39 | |
| 946033 ~ 947157<br>947158 ~ 948282<br>948283 ~ 949407 | D86~D1210<br>D86~D1210<br>D1 | D86~D1210<br>D86~D1210<br>D86~D1210 | Ar40<br>Ar47<br>D1 | |
| 949408 ~ 950532 | D2 | D86~D1210 | D2 | |
| 950533 ~ 951657 | D3 | D86~D1210 | D3 | |
| 951658 ~ 952782 | D4 | D86~D1210 | D4 | |
| 952783 ~ 953907 | D5 | D86~D1210 | D5 | |
| 953908 ~ 955032 | D6 | D86~D1210 | D6 | |
| 955033 ~ 956157 | D7 | D86~D1210 | D7 | |
| 956158 ~ 957282 | D8 | D86~D1210 | D8 | |
| 957283 ~ 958407 | D9 | D86~D1210 | D9 | |
| 958408 ~ 959532<br>959533 ~ 960657 | D10<br>D11 | ~ D86~D1210<br>D86~D1210 | D10<br>D11 | |
| 960658 ~ 961782 | D12 | D86~D1210 | D12 | |
| 961783 ~ 962907<br>962908 ~ 964032 | D13<br>D14 | D86~D1210<br>D86~D1210 | D13<br>D14 | |
| 964033 ~ 965157 | D15 | D86~D1210 | D15 | |
| 965158 ~ 966282 | D16 | D86~D1210 | D16 | $R^3 = R^5$ |

(continued)

| No. | $R^3$ | $R^4$ | $R^5$ | = |
|---|---|---|---|---|
| 966283 ~ 967407 | D17 | D86~D1210 | D17 | |
| 967408 ~ 968532 | D18 | D86~D1210 | D18 | |
| 968533 ~ 969657 | D19 | D86~D1210 | D19 | |
| 969658 ~ 970782 | D20 | D86~D1210 | D20 | |
| 970783 ~ 971907 | D21 | D86~D1210 | D21 | |
| 971908 ~ 973032 | D22 | D86~D1210 | D22 | |
| 973033 ~ 974157 | D23 | D86~D1210 | D23 | |
| 974158 ~ 975282 | D24 | D86~D1210 | D24 | |
| 975283 976407<br>976408 ~ 977532 | D25<br>D26 | D86~D1210<br>~ D86~D1210 | D25<br>D26 | |
| 977533 ~ 978657<br>978658 ~ 979782 | D27<br>D28 | D86~D1210<br>D86~D1210 | D27<br>D28 | |
| 979783 ~ 980907 | D29 | D86~D1210 | D29 | |
| 980908 ~ 982032 | D30 | D86~D1210 | D30 | |
| 982033 ~ 983157 | D31 | D86~D1210 | D31 | |
| 983158 ~ 984282 | D32 | D86~D1210 | D32 | |
| 984283 ~ 985407 | D33 | D86~D1210 | D33 | |
| 985408 ~ 986532 | D34 | D86~D1210 | D34 | |
| 986533 ~ 987657 | D35 | D86~D1210 | D35 | |
| 987658 ~ 988782 | D36 | D86~D1210 | D36 | |
| 988783 ~ 989907 | D37 | D86~D1210 | D37 | |
| 989908 ~ 991032 | D38 | D86~D1210 | D38 | |
| 991033 ~ 992157 | D39 | D86~D1210 | D39 | |
| 992158 ~ 993282 | D40 | D86~D1210 | D40 | |
| 993283 ~ 994407 | D41 | D86~D1210 | D41 | |
| 994408 ~ 995532 | D42 | D86~D1210 | D42 | |
| 995533 ~ 996657 | D43 | D86~D1210 | D43 | |
| 996658 ~ 997782 | D44 | D86~D1210 | D44 | |
| 997783 ~ 998907 | D45 | D86~D1210 | D45 | |
| 998908 ~ 1000032 | D46 | D86~D1210 | D46 | |
| 1000033 ~ 1001157 | D47 | D86~D1210 | D47 | |
| 1001158 ~ 1002282 | D48 | D86~D1210 | D48 | |
| 1002283 ~ 1003407 | D49 | D86~D1210 | D49 | |
| 1003408 ~ 1004532 | D50 | D86~D1210 | D50 | |
| 1004533 ~ 1005657 | D51 | D86~D1210 | D51 | |
| 1005658 ~ 1006782 | D52 | D86~D1210 | D52 | |
| 1006783 ~ 1007907 | D53 | D86~D1210 | D53 | |
| 1007908 ~ 1009032 | D54 | D86~D1210 | D54 | |
| 1009033 ~ 1010157 | D55 | D86~D1210 | D55 | |
| 1010158 ~ 1011282 | D56 | D86~D1210 | D56 | |

(continued)

| No. | $R^3$ | $R^4$ | $R^5$ | = |
|---|---|---|---|---|
| 1011283 ~ 1012407 | D57 | D86~D1210 | D57 | $R^3 = R^5$ |
| 1012408 ~ 1013532<br>1013533 ~ 1014657 | D58<br>D59 | D86~D1210<br>D86~D1210 | D58<br>D59 | |
| 1014658 ~ 1015782<br>1015783 ~ 1016907 | D60<br>D61 | D86~D1210<br>D86~D1210 | D60<br>D61 | |
| 1016908 ~ 1018032 | D62 | D86~D1210 | D62 | |
| 1018033 ~ 1019157 | D63 | D86~D1210 | D63 | |
| 1019158 ~ 1020282 | D64 | D86~D1210 | D64 | |
| 1020283 ~ 1021407 | D65 | D86~D1210 | D65 | |
| 1021408 ~ 1022532 | D66 | D86~D1210 | D66 | |
| 1022533 ~ 1023657 | D67 | D86~D1210 | D67 | |
| 1023658 ~ 1024782 | D68 | D86~D1210 | D68 | |
| 1024783 ~ 1025907<br>1025908 ~ 1027032 | D69<br>D70 | D86~D1210<br>D86~D1210 | D69<br>D70 | |
| 1027033 ~ 1028157 | D71 | D86~D1210 | D71 | |
| 1028158 ~ 1029282 | D72 | D86~D1210 | D72 | |
| 1029283 ~ 1030407 | D73 | D86~D1210 | D73 | |
| 1030408 ~ 1031532 | D74 | D86~D1210 | D74 | |
| 1031533 ~ 1032657 | D75 | D86~D1210 | D75 | |
| 1032658 ~ 1033782 | D76 | D86~D1210 | D76 | |
| 1033783 ~ 1034907 | D77 | D86~D1210 | D77 | |
| 1034908 ~ 1036032 | D78 | D86~D1210 | D78 | |
| 1036033 ~ 1037157 | D79 | D86~D1210 | D79 | |
| 1037158 ~ 1038282 | D80 | D86~D1210 | D80 | |
| 1038283 ~ 1039407 | D81 | D86~D1210 | D81 | |
| 1039408 ~ 1040532 | D82 | D86~D1210 | D82 | |
| 1040533 ~ 1041657<br>1041658 ~ 1042782 | D83<br>D84 | D86~D1210<br>D86~D1210 | D83<br>D84 | |
| 1042783 ~ 1043907 | D85 | D86~D1210 | D85 | |
| 1043908 ~ 1045031 | D88 | D86, D87, D89~D1210 | D88 | $R^3 = R^5$ |
| 1045032 ~ 1046155 | D106 | D86~D105,<br>D107~D1210 | D106 | |
| 1046156 ~ 1047279 | D119 | D86~D118,<br>D120~D1210 | D119 | |
| 1047280 ~ 1048403 | D471 | D86~D470,<br>D472~D1210 | D471 | |
| 1048404 ~ 1049527 | D705 | D86~D704,<br>D706~D1210 | D705 | |
| 1049528 ~ 1050651<br>1050652 ~ 1051775 | D737<br>D760 | D86~D736,<br>D738~D1210<br>D86-D759,<br>D761~D1210 | D737<br>D760 | |

(continued)

| No. | $R^3$ | $R^4$ | $R^5$ | = |
|---|---|---|---|---|
| 1051776 ~ 1052899 | D763 | D86~D762, D764~D1210 | D763 | |
| 1052900 ~ 1054023 | D783 | D86~D782, D784~D1210 | D783 | |
| | | | | |
| No. | $R^3$ | $R^4$ | $R^5$ | |
| 1054024 ~ 1055147 | D86, D87, D89~D1210 | D88 | D86, D87, D89~D1210 | $R^3 = R^5$ |
| 1055148 ~ 1056271 | D86~D105, D107~D1210 | D106 | D86~D105, D107~D1210 | |
| 1056272 ~ 1057395 | D86~D118, D120~D1210 | D119 | D86~D118, D120~D1210 | |
| 1057396 ~ 1058519 | D86~D470, D472~D1210 | D471 | D86~D470, D472~D1210 | |
| 1058520 ~ 1059643 | D86~D704, D706~D1210 | D705 | D86~D704, D706~D1210 | |
| 1059644 ~ 1060767 | D86~D736, D738~D1210 | D737 | D86~D736, D738~D1210 | |
| 1060768 ~ 1061891 | D86~D759, D761~D1210 | D760 | D86~D759, D761~D1210 | |
| 1061892 ~ 1063015 | D86~D762, D764~D1210 | D763 | D86~D762, D764~D1210 | |
| 1063016 ~ 1064139 | D86~D782, D784~D1210 | D783 | D86~D782, D784~D1210 | |

Next, specific examples of the compound having a structure represented by the following general formula (1e) are shown in Table 6. In Table 6, structures of the compounds are shown in the same manner as in Table 2.

General Formula (1e)

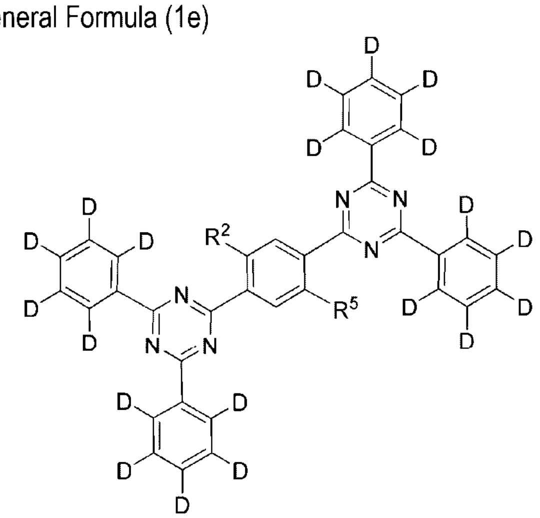

[Table 6]

| No. | $R^2$ | $R^5$ | = |
|---|---|---|---|
| 1064140 ~ 1065264 | D86~D1210 | D86~D1210 | $R^2 = R^5$ |

(continued)

| No. | $R^2$ | $R^5$ | = |
|---|---|---|---|
| 1065265 ~ 1066389 | D86~D1210 | D1 | |
| 1066390 ~ 1067514 | D86~D1210 | D2 | |
| 1067515 ~ 1068639 | D86~D1210 | D3 | |
| 1068640 ~ 1069764 | D86~D1210 | D4 | |
| 1069765 ~ 1070889 | D86~D1210 | D5 | |
| 1070890 ~ 1072014 | D86~D1210 | D6 | |
| 1072015 ~ 1073139 | D86~D1210 | D7 | |
| 1073140 ~ 1074264 | D86~D1210 | D8 | |
| 1074265 ~ 1075389 | D86~D1210 | D9 | |
| 1075390 ~ 1076514<br>1076515 ~ 1077639 | D86~D1210<br>D86~D1210 | D10<br>D11 | |
| 1077640 ~ 1078764 | D86~D1210 | D12 | |
| 1078765 ~ 1079889 | D86~D1210 | D13 | |
| 1079890 ~ 1081014 | D86~D1210 | D14 | |
| 1081015 ~ 1082139 | D86~D1210 | D15 | |
| 1082140 ~ 1083264 | D86~D1210 | D16 | |
| 1083265 ~ 1084389 | D86~D1210 | D17 | |
| 1084390 ~ 1085514 | D86~D1210 | D18 | |
| 1085515 ~ 1086639 | D86~D1210 | D19 | |
| 1086640 ~ 1087764 | D86~D1210 | D20 | |
| 1087765 ~ 1088889<br>1088890 ~ 1090014 | D86~D1210<br>D86~D1210 | D21<br>D22 | |
| 1090015 ~ 1091139 | D86~D1210 | D23 | |
| 1091140 ~ 1092264 | D86~D1210 | D24 | |
| 1092265 ~ 1093389<br>1093390 ~ 1094514 | D86~D1210<br>D86~D1210 | D25<br>D26 | |
| 1094515 ~ 1095639 | D86~D1210 | D27 | |
| 1095640 ~ 1096764 | D86~D1210 | D28 | |
| 1096765 ~ 1097889 | D86~D1210 | D29 | |
| 1097890 ~ 1099014 | D86~D1210 | D30 | $R^2 \neq R^5$ |
| 1099015 ~ 1100139 | D86~D1210 | D31 | |
| 1100140 ~ 1101264 | D86~D1210 | D32 | |
| 1101265 ~ 1102389 | D86~D1210 | D33 | |
| 1102390 ~ 1103514 | D86~D1210 | D34 | |
| 1103515 ~ 1104639 | D86~D1210 | D35 | |
| 1104640 ~ 1105764 | D86~D1210 | D36 | |
| 1105765 ~ 1106889 | D86~D1210 | D37 | |
| 1106890 ~ 1108014 | D86~D1210 | D38 | |
| 1108015 ~ 1109139 | D86~D1210 | D39 | |
| 1109140 ~ 1110264 | D86~D1210 | D40 | |

(continued)

| No. | $R^2$ | $R^5$ | = |
|---|---|---|---|
| 1110265 ~ 1111389 | D86~D1210 | D41 | |
| 1111390 ~ 1112514 | D86~D1210 | D42 | |
| 1112515 ~ 1113639 | D86~D1210 | D43 | |
| 1113640 ~ 1114764 | D86~D1210 | D44 | |
| 1114765~ 1115889 | D86~D1210 | D45 | |
| 1115890 ~ 1117014 | D86~D1210 | D46 | |
| 1117015 ~ 1118139 | D86~D1210 | D47 | |
| 1118140 ~ 1119264 | D86~D1210 | D48 | |
| 1119265 ~ 1120389 | D86~D1210 | D49 | |
| 1120390 ~ 1121514 | D86~D1210 | D50 | |
| 1121515 ~ 1122639 | D86~D1210 | D51 | |
| 1122640 ~ 1123764 | D86~D1210 | D52 | |
| 1123765 ~ 1124889 | D86~D1210 | D53 | |
| 1124890 ~ 1126014 | D86~D1210 | D54 | |
| 1126015 ~ 1127139 | D86~D1210 | D55 | |
| 1127140 ~ 1128264 | D86~D1210 | D56 | |
| 1128265 ~ 1129389 | D86~D1210 | D57 | |
| 1129390 ~ 1130514 | D86~D1210 | D58 | |
| 1130515 ~ 1131639 | D86~D1210 | D59 | |
| 1131640 ~ 1132764 | D86~D1210 | D60 | $R^2 \neq R^5$ |
| 1132765 ~ 1133889 | D86~D1210 | D61 | |
| 1133890 ~ 1135014 | D86~D1210 | D62 | |
| 1135015 ~ 1136139 | D86~D1210 | D63 | |
| 1136140 ~ 1137264 | D86~D1210 | D64 | |
| 1137265 ~ 1138389<br>1138390 ~ 1139514 | D86~D1210<br>D86~D1210 | D65<br>D66 | |
| 1139515 ~ 1140639 | D86~D1210 | D67 | |
| 1140640 ~ 1141764 | D86~D1210 | D68 | |
| 1141765 ~ 1142889 | D86~D1210 | D69 | |
| 1142890 ~ 1144014 | D86~D1210 | D70 | |
| 1144015 ~ 1145139 | D86~D1210 | D71 | |
| 1145140 ~ 1146264 | D86~D1210 | D72 | |
| 1146265 ~ 1147389<br>1147390 ~ 1148514 | D86~D1210<br>D86~D1210 | D73<br>D74 | |
| 1148515 ~ 1149639 | D86~D1210 | D75 | |
| 1149640 ~ 1150764 | D86~D1210 | D76 | |
| 1150765 ~ 1151889 | D86~D1210 | D77 | |
| 1151890 ~ 1153014 | D86~D1210 | D78 | |
| 1153015 ~ 1154139 | D86~D1210 | D79 | |
| 1154140 ~ 1155264 | D86~D1210 | D80 | |

(continued)

| No. | $R^2$ | $R^5$ | = |
|---|---|---|---|
| 1155265 ~ 1156389 | D86~D1210 | D81 | |
| 1156390 ~ 1157514 | D86~D1210 | D82 | |
| 1157515 ~ 1158639 | D86~D1210 | D83 | |
| 1158640 ~ 1159764 | D86~D1210 | D84 | |
| 1159765 ~ 1160889 | D86~D1210 | D85 | |

Next, specific examples of the compound having a structure represented by the following general formula (1f) are shown in Table 7. In Table 7, structures of the compounds are shown in the same manner as in Table 2.

General Formula (1f)

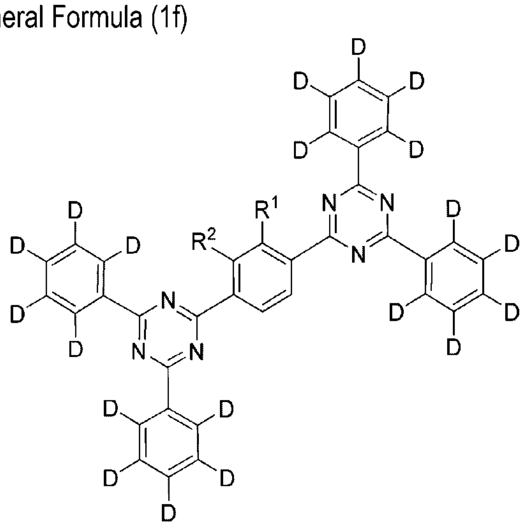

[Table 7]

| No. | $R^1$ | $R^2$ | = |
|---|---|---|---|
| 1160890 ~ 1162014 | D86~D1210 | D86~D1210 | $R^1 = R^2$ |
| 1162015 ~ 1163139 | D86~D1210 | D1 | |
| 1163140 ~ 1164264 | D86~D1210 | D2 | |
| 1164265 ~ 1165389 | D86~D1210 | D3 | |
| 1165390 ~ 1166514 | D86~D1210 | D4 | |
| 1166515 ~ 1167639 | D86~D1210 | D5 | |
| 1167640 ~ 1168764 | D86~D1210 | D6 | |
| 1168765~ 1169889 | D86~D1210 | D7 | |
| 1169890 ~ 1171014 | D86~D1210 | D8 | |
| 1171015 ~ 1172139 | D86~D1210 | D9 | |
| 1172140 ~ 1173264 | D86~D1210 | D10 | |
| 1173265 ~ 1174389 | D86~D1210 | D11 | |
| 1174390 ~ 1175514 | D86~D1210 | D12 | |
| 1175515 ~ 1176639 | D86~D1210 | D13 | |
| 1176640 ~ 1177764 | D86~D1210 | D14 | |
| 1177765 ~ 1178889 | D86~D1210 | D15 | |

(continued)

| No. | $R^1$ | $R^2$ | = |
|---|---|---|---|
| 1178890 ~ 1180014 | D86~D1210 | D16 | $R^1 \neq R^2$ |
| 1180015 ~ 1181139 | D86~D1210 | D17 | |
| 1181140 ~ 1182264 | D86~D1210 | D18 | |
| 1182265 ~ 1183389 | D86~D1210 | D19 | |
| 1183390 ~ 1184514 | D86~D1210 | D20 | |
| 1184515 ~ 1185639 | D86~D1210 | D21 | |
| 1185640 ~ 1186764 | D86~D1210 | D22 | |
| 1186765 ~ 1187889 | D86~D1210 | D23 | |
| 1187890 ~ 1189014 | D86~D1210 | D24 | |
| 1189015 ~ 1190139 | D86~D1210 | D25 | |
| 1190140 ~ 1191264 | D86~D1210 | D26 | |
| 1191265 ~ 1192389 | D86~D1210 | D27 | |
| 1192390 ~ 1193514 | D86~D1210 | D28 | |
| 1193515 ~ 1194639 | D86~D1210 | D29 | |
| 1194640 ~ 1195764 | D86~D1210 | D30 | |
| 1195765 ~ 1196889 | D86~D1210 | D31 | |
| 1196890 ~ 1198014 | D86~D1210 | D32 | |
| 1198015 ~ 1199139 | D86~D1210 | D33 | |
| 1199140 ~ 1200264 | D86~D1210 | D34 | |
| 1200265 ~ 1201389 | D86~D1210 | D35 | |
| 1201390 ~ 1202514 | D86~D1210 | D36 | |
| 1202515 ~ 1203639 | D86~D1210 | D37 | |
| 1203640 ~ 1204764 | D86~D1210 | D38 | |
| 1204765 ~ 1205889 | D86~D1210 | D39 | |
| 1205890 ~ 1207014 | D86~D1210 | D40 | |
| 1207015 ~ 1208139 | D86~D1210 | D41 | |
| 1208140 ~ 1209264 | D86~D1210 | D42 | |
| 1209265 ~ 1210389 | D86~D1210 | D43 | |
| 1210390 ~ 1211514 | D86~D1210 | D44 | |
| 1211515 ~ 1212639 | D86~D1210 | D45 | |
| 1212640 ~ 1213764 | D86~D1210 | D46 | |
| 1213765 ~ 1214889 | D86~D1210 | D47 | |
| 1214890 ~ 1216014 | D86~D1210 | D48 | |
| 1216015 ~ 1217139 | D86~D1210 | D49 | |
| 1217140 ~ 1218264 | D86~D1210 | D50 | |
| 1218265 ~ 1219389 | D86~D1210 | D51 | |
| 1219390 ~ 1220514 | D86~D1210 | D52 | |
| 1220515 ~ 1221639 | D86~D1210 | D53 | |
| 1221640 ~ 1222764 | D86~D1210 | D54 | |

(continued)

| No. | $R^1$ | $R^2$ | = |
|---|---|---|---|
| 1222765 ~ 1223889 | D86~D1210 | D55 | |
| 1223890 ~ 1225014 | D86~D1210 | D56 | |
| 1225015 ~ 1226139 | D86~D1210 | D57 | |
| 1226140 ~ 1227264 | D86~D1210 | D58 | |
| 1227265 ~ 1228389 | D86~D1210 | D59 | |
| 1228390 ~ 1229514<br>1229515 ~ 1230639 | D86~D1210<br>D86~D1210 | D60<br>D61 | $R^1 \neq R^2$ |
| 1230640 ~ 1231764 | D86~D1210 | D62 | |
| 1231765 ~ 1232889<br>1232890 ~ 1234014 | D86~D1210<br>D86~D1210 | D63<br>D64 | |
| 1234015 ~ 1235139 | D86~D1210 | D65 | |
| 1235140 ~ 1236264<br>1236265 ~ 1237389 | D86~D1210<br>D86~D1210 | D66<br>D67 | |
| 1237390 ~ 1238514 | D86~D1210 | D68 | |
| 1238515 ~ 1239639<br>1239640 ~ 1240764 | D86~D1210<br>D86~D1210 | D69<br>D70 | |
| 1240765 ~ 1241889 | D86~D1210 | D71 | |
| 1241890 ~ 1243014 | D86~D1210 | D72 | |
| 1243015 ~ 1244139 | D86~D1210 | D73 | |
| 1244140 ~ 1245264<br>1245265 ~ 1246389 | D86~D1210<br>D86~D1210 | D74<br>D75 | |
| 1246390 ~ 1247514 | D86~D1210 | D76 | |
| 1247515 ~ 1248639 | D86~D1210 | D77 | |
| 1248640 ~ 1249764 | D86~D1210 | D78 | |
| 1249765 ~ 1250889 | D86~D1210 | D79 | |
| 1250890 ~ 1252014 | D86~D1210 | D80 | |
| 1252015 ~ 1253139 | D86~D1210 | D81 | |
| 1253140 ~ 1254264 | D86~D1210 | D82 | |
| 1254265 ~ 1255389 | D86~D1210 | D83 | |
| 1255390 ~ 1256514<br>1256515 ~ 1257639 | D86~D1210<br>D86~D1210 | D84<br>D85 | |

In Tables 1 to 4, structures in which $Ar^1$ and $Ar^3$ in the general formula (1) are perdeuterated carbazol-9-yl groups (D55) and $Ar^2$ and $Ar^4$ are perdeuterated phenyl groups (Ar47) were specified as structures of Compounds 1 to 636657. In Tables 5 to 7, structures in which $Ar^1$ to $Ar^4$ in the general formula (1) are perdeuterated phenyl groups (Ar47) were specified as structures of Compounds 636658 to 1257639.

In the following Table 8, together with these Compounds 1 to 1257639, compounds obtained by changing $Ar^1$ to $Ar^4$ of all or a part of Compounds 1 to 636657 as shown in Table 8 are shown in order in the form of a table. In Table 8, Compounds 1 to 636657 are shown in the first row and Compounds 636658 to 1257639 are shown in the second row for clarifying the correspondence relationship. In the third row of Table 8, the compounds in which both $Ar^1$ and $Ar^3$ of Compounds 1 to 636657 are D1 are referred to as Compounds 1(1) to 636657(1), respectively. For example, Compound 1(1) indicates a compound having a structure in which $Ar^1$ and $Ar^3$ of Compound 1 are substituted with D1. Compound 2(1) indicates a compound having a structure in which $Ar^1$ and $Ar^3$ of Compound 2 are substituted with D1. Compound 636657(1) indicates a compound having a structure in which $Ar^1$ and $Ar^3$ of Compound 636657 are substituted with D1. In

the fourth row of Table 8, the compounds in which both $Ar^1$ and $Ar^3$ of Compounds 1 to 636657 are D7 are referred to as Compounds 1(2) to 636657(2), respectively. In this manner, compounds in which $Ar^1$ and $Ar^3$, and $Ar^2$ and $Ar^4$ of Compounds 1 to 636657 are listed in Table 8 are specified in order. $X^1$ to $X^3$ of the compounds specified in Table 8 all represent a nitrogen atom (N), $L^1$ represents a single bond (L1), and $R^1$ represents a hydrogen atom. $Ar^1$ and $Ar^3$ are the same, and $Ar^2$ and $Ar^4$ are the same.

[Table 8]

| No. | $Ar^1$,$Ar^3$ | $Ar^2$,$Ar^4$ |
|---|---|---|
| 1 ~ 636657 | D55 | Ar47 |
| 636658 ~ 1257639<br>1(1) ~ 636657(1) | Ar47<br>D1 | Ar47<br>Ar47 |
| 1(2) ~ 636657(2) | D7 | Ar47 |
| 1(3) ~ 636657(3) | D8 | Ar47 |
| 1(4) ~ 636657(4) | D9 | Ar47 |
| 1(5) ~ 636657(5) | D17 | Ar47 |
| 1(6) ~ 636657(6) | D27 | Ar47 |
| 1(7) ~ 636657(7) | D37 | Ar47 |
| 1(8) ~ 636657(8) | D39 | Ar47 |
| 1(9) ~ 636657(9) | D47 | Ar47 |
| 1(10) ~ 636657(10) | D51 | Ar47 |
| 1(11) ~ 636657(11) | D61 | Ar47 |
| 1(12) ~ 636657(12) | D62 | Ar47 |
| 1(13) ~ 636657(13) | D63 | Ar47 |
| 1(14) ~ 636657(14) | D71 | Ar47 |
| 1(15) ~ 636657(15) | D81 | Ar47 |
| 1(16) ~ 636657(16) | D88 | Ar47 |
| 1(17) ~ 636657(17) | D106 | Ar47 |
| 1(18) ~ 636657(18) | D119 | Ar47 |
| 1(19) ~ 636657(19) | D765 | Ar47 |
| 1(20) ~ 636657(20) | D1 | Ar1 |
| 1(21) ~ 636657(21) | D7 | Ar1 |
| 1(22) ~ 636657(22) | D8 | Ar1 |
| 1(23) ~ 636657(23) | D9 | Ar1 |
| 1(24) ~ 636657(24) | D17 | Ar1 |
| 1(25) ~ 636657(25) | D27 | Ar1 |
| 1(26) ~ 636657(26) | D37 | Ar1 |
| 1(27) ~ 636657(27)<br>1(28) ~ 636657(28) | D39<br>D47 | Ar1<br>Ar1 |
| 1(29) ~ 636657(29) | D51 | Ar1 |
| 1(30) ~ 636657(30) | D55 | Ar1 |
| 1(31) ~ 636657(31)<br>1(32) ~ 636657(32) | D61<br>D62 | Ar1<br>Ar1 |
| 1(33) ~ 636657(33) | D63 | Ar1 |
| 1(34) ~ 636657(34) | D71 | Ar1 |

(continued)

| No. | $Ar^1$,$Ar^3$ | $Ar^2$,$Ar^4$ |
|---|---|---|
| 1(35) ~ 636657(35) | D81 | Ar1 |
| 1(36) ~ 636657(36) | D88 | Ar1 |
| 1(37) ~ 636657(37) | D106 | Ar1 |
| 1(38) ~ 636657(38) | D119 | Ar1 |
| 1(39) ~ 636657(39)<br>1(40) ~ 636657(40) | D765<br>D1 | Ar1<br>Ar10 |
| 1(41) ~ 636657(41) | D7 | Ar10 |
| 1(42) ~ 636657(42) | D8 | Ar10 |
| 1(43) ~ 636657(43) | D9 | Ar10 |
| 1(44) ~ 636657(44) | D17 | Ar10 |
| 1(45) ~ 636657(45) | D27 | Ar10 |
| 1(46) ~ 636657(46) | D37 | Ar10 |
| 1(47) ~ 636657(47) | D39 | Ar10 |
| 1(48) ~ 636657(48) | D47 | Ar10 |
| 1(49) ~ 636657(49) | D51 | Ar10 |
| 1(50) ~ 636657(50) | D55 | Ar10 |
| 1(51) ~ 636657(51) | D61 | Ar10 |
| 1(52) ~ 636657(52) | D62 | Ar10 |
| 1(53) ~ 636657(53) | D63 | Ar10 |
| 1(54) ~ 636657(54) | D71 | Ar10 |
| 1(55) ~ 636657(55) | D81 | Ar10 |
| 1(56) ~ 636657(56) | D88 | Ar10 |
| 1(57) ~ 636657(57) | D106 | Ar10 |
| 1(58) ~ 636657(58) | D119 | Ar10 |
| 1(59) ~ 636657(59) | D765 | Ar10 |
| 1(60) ~ 636657(60) | D1 | Ar12 |
| 1(61) ~ 636657(61)<br>1(62) ~ 636657(62) | D7<br>D8 | Ar12<br>Ar12 |
| 1(63) ~ 636657(63) | D9 | Ar12 |
| 1(64) ~ 636657(64) | D17 | Ar12 |
| 1(65) ~ 636657(65) | D27 | Ar12 |
| 1(66) ~ 636657(66) | D37 | Ar12 |
| 1(67) ~ 636657(67) | D39 | Ar12 |
| 1(68) ~ 636657(68) | D47 | Ar12 |
| 1(69) ~ 636657(69)<br>1(70) ~ 636657(70) | D51<br>D55 | Ar12<br>Ar12 |
| 1(71) ~ 636657(71) | D61 | Ar12 |
| 1(72) ~ 636657(72) | D62 | Ar12 |
| 1(73) ~ 636657(73) | D63 | Ar12 |
| 1(74) ~ 636657(74) | D71 | Ar12 |

(continued)

| No. | $Ar^1$,$Ar^3$ | $Ar^2$,$Ar^4$ |
|---|---|---|
| 1(75) ~ 636657(75) | D81 | Ar12 |
| 1(76) ~ 636657(76) | D88 | Ar12 |
| 1(77) ~ 636657(77) | D106 | Ar12 |
| 1(78) ~ 636657(78) | D119 | Ar12 |
| 1(79) ~ 636657(79) | D765 | Ar12 |
| 1(80) ~ 636657(80) | D1 | Ar14 |
| 1(81) ~ 636657(81) | D7 | Ar14 |
| 1(82) ~ 636657(82) | D8 | Ar14 |
| 1(83) ~ 636657(83) | D9 | Ar14 |
| 1(84) ~ 636657(84)<br>1(85) ~ 636657(85) | D17<br>D27 | Ar14<br>Ar14 |
| 1(86) ~ 636657(86) | D37 | Ar14 |
| 1(87) ~ 636657(87) | D39 | Ar14 |
| 1(88) ~ 636657(88)<br>1(89) ~ 636657(89) | D47<br>D51 | Ar14<br>Ar14 |
| 1(90) ~ 636657(90) | D55 | Ar14 |
| 1(91) ~ 636657(91) | D61 | Ar14 |
| 1(92) ~ 636657(92)<br>1(93) ~ 636657(93) | D62<br>D63 | Ar14<br>Ar14 |
| 1(94) ~ 636657(94) | D71 | Ar14 |
| 1(95) ~ 636657(95) | D81 | Ar14 |
| 1(96) ~ 636657(96) | D88 | Ar14 |
| 1(97) ~ 636657(97) | D106 | Ar14 |
| 1(98) ~ 636657(98) | D119 | Ar14 |
| 1(99) ~ 636657(99) | D765 | Ar14 |
| 1(100) ~ 636657(100)<br>1(101) ~ 636657(101) | D1<br>D7 | Ar15<br>Ar15 |
| 1(102) ~ 636657(102) | D8 | Ar15 |
| 1(103) ~ 636657(103) | D9 | Ar15 |
| 1(104) ~ 636657(104) | D17 | Ar15 |
| 1(105) ~ 636657(105) | D27 | Ar15 |
| 1(106) ~ 636657(106) | D37 | Ar15 |
| 1(107) ~ 636657(107) | D39 | Ar15 |
| 1(108) ~ 636657(108) | D47 | Ar15 |
| 1(109) ~ 636657(109) | D51 | Ar15 |
| 1(110) ~ 636657(110) | D55 | Ar15 |
| 1(111) ~ 636657(111) | D61 | Ar15 |
| 1(112) ~ 636657(112) | D62 | Ar15 |
| 1(113) ~ 636657(113) | D63 | Ar15 |
| 1(114) ~ 636657(114) | D71 | Ar15 |

(continued)

| No. | $Ar^1$,$Ar^3$ | $Ar^2$,$Ar^4$ |
|---|---|---|
| 1(115) ~ 636657(115) | D81 | Ar15 |
| 1(116) ~ 636657(116) | D88 | Ar15 |
| 1(117) ~ 636657(117) | D106 | Ar15 |
| 1(118) ~ 636657(118) | D119 | Ar15 |
| 1(119) ~ 636657(119) | D765 | Ar15 |
| 1(120) ~ 636657(120) | D1 | Ar35 |
| 1(121) ~ 636657(121) | D7 | Ar35 |
| 1(122) ~ 636657(122) | D8 | Ar35 |
| 1(123) ~ 636657(123)<br>1(124) ~ 636657(124) | D9<br>D17 | Ar35<br>Ar35 |
| 1(125) ~ 636657(125) | D27 | Ar35 |
| 1(126) ~ 636657(126) | D37 | Ar35 |
| 1(127) ~ 636657(127) | D39 | Ar35 |
| 1(128) ~ 636657(128) | D47 | Ar35 |
| 1(129) ~ 636657(129) | D51 | Ar35 |
| 1(130) ~ 636657(130)<br>1(131) ~ 636657(131) | D55<br>D61 | Ar35<br>Ar35 |
| 1(132) ~ 636657(132) | D62 | Ar35 |
| 1(133) ~ 636657(133)<br>1(134) ~ 636657(134) | D63<br>D71 | Ar35<br>Ar35 |
| 1(135) ~ 636657(135)<br>1(136) ~ 636657(136) | D81<br>D88 | Ar35<br>Ar35 |
| 1(137) ~ 636657(137) | D106 | Ar35 |
| 1(138) ~ 636657(138) | D119 | Ar35 |
| 1(139) ~ 636657(139) | D765 | Ar35 |
| 1(140) ~ 636657(140) | D1 | Ar37 |
| 1(141) ~ 636657(141) | D7 | Ar37 |
| 1(142) ~ 636657(142) | D8 | Ar37 |
| 1(143) ~ 636657(143) | D9 | Ar37 |
| 1(144) ~ 636657(144) | D17 | Ar37 |
| 1(145) ~ 636657(145) | D27 | Ar37 |
| 1(146) ~ 636657(146) | D37 | Ar37 |
| 1(147) ~ 636657(147) | D39 | Ar37 |
| 1(148) ~ 636657(148) | D47 | Ar37 |
| 1(149) ~ 636657(149) | D51 | Ar37 |
| 1(150) ~ 636657(150) | D55 | Ar37 |
| 1(151) ~ 636657(151) | D61 | Ar37 |
| 1(152) ~ 636657(152) | D62 | Ar37 |
| 1(153) ~ 636657(153) | D63 | Ar37 |
| 1(154) ~ 636657(154) | D71 | Ar37 |

(continued)

| No. | $Ar^1$,$Ar^3$ | $Ar^2$,$Ar^4$ |
|---|---|---|
| 1(155) ~ 636657(155) | D81 | Ar37 |
| 1(156) ~ 636657(156) | D88 | Ar37 |
| 1(157) ~ 636657(157) | D106 | Ar37 |
| 1(158) ~ 636657(158) | D119 | Ar37 |
| 1(159) ~ 636657(159) | D765 | Ar37 |
| 1(160) ~ 636657(160) | D1 | Ar39 |
| 1(161) ~ 636657(161)<br>1(162) ~ 636657(162) | D7<br>D8 | Ar39<br>Ar39 |
| 1(163) ~ 636657(163) | D9 | Ar39 |
| 1(164) ~ 636657(164) | D17 | Ar39 |
| 1(165) ~ 636657(165) | D27 | Ar39 |
| 1(166) ~ 636657(166)<br>1(167) ~ 636657(167) | D37<br>D39 | Ar39<br>Ar39 |
| 1(168) ~ 636657(168) | D47 | Ar39 |
| 1(169) ~ 636657(169) | D51 | Ar39 |
| 1(170) ~ 636657(170) | D55 | Ar39 |
| 1(171) ~ 636657(171) | D61 | Ar39 |
| 1(172) ~ 636657(172) | D62 | Ar39 |
| 1(173) ~ 636657(173) | D63 | Ar39 |
| 1(174) ~ 636657(174) | D71 | Ar39 |
| 1(175) ~ 636657(175) | D81 | Ar39 |
| 1(176) ~ 636657(176) | D88 | Ar39 |
| 1(177) ~ 636657(177) | D106 | Ar39 |
| 1(178) ~ 636657(178) | D119 | Ar39 |
| 1(179) ~ 636657(179) | D765 | Ar39 |
| 1(180) ~ 636657(180) | D1 | Ar40 |
| 1(181) ~ 636657(181) | D7 | Ar40 |
| 1(182) ~ 636657(182) | D8 | Ar40 |
| 1(183) ~ 636657(183)<br>1(184) ~ 636657(184) | D9<br>D17 | Ar40<br>Ar40 |
| 1(185) ~ 636657(185) | D27 | Ar40 |
| 1(186) ~ 636657(186) | D37 | Ar40 |
| 1(187) ~ 636657(187) | D39 | Ar40 |
| 1(188) ~ 636657(188) | D47 | Ar40 |
| 1(189) ~ 636657(189) | D51 | Ar40 |
| 1(190) ~ 636657(190)<br>1(191) ~ 636657(191) | D55<br>D61 | Ar40<br>Ar40 |
| 1(192) ~ 636657(192) | D62 | Ar40 |
| 1(193) ~ 636657(193)<br>1(194) ~ 636657(194) | D63<br>D71 | Ar40<br>Ar40 |

(continued)

| No. | $Ar^1$,$Ar^3$ | $Ar^2$,$Ar^4$ |
|---|---|---|
| 1(195) ~ 636657(195) | D81 | Ar40 |
| 1(196) ~ 636657(196) | D88 | Ar40 |
| 1(197) ~ 636657(197) | D106 | Ar40 |
| 1(198) ~ 636657(198) | D119 | Ar40 |
| 1(199) ~ 636657(199) | D765 | Ar40 |
| 1(200) ~ 636657(200) | D1 | Ar56 |
| 1(201) ~ 636657(201) | D7 | Ar56 |
| 1(202) ~ 636657(202) | D8 | Ar56 |
| 1(203) ~ 636657(203) | D9 | Ar56 |
| 1(204) ~ 636657(204) | D17 | Ar56 |
| 1(205) ~ 636657(205) | - D27 | Ar56 |
| 1(206) ~ 636657(206) | D37 | Ar56 |
| 1(207) ~ 636657(207) | D39 | Ar56 |
| 1(208) ~ 636657(208) | D47 | Ar56 |
| 1(209) ~ 636657(209) | D51 | Ar56 |
| 1(210) ~ 636657(210) | D55 | Ar56 |
| 1(211) ~ 636657(211) | D61 | Ar56 |
| 1(212) ~ 636657(212) | D62 | Ar56 |
| 1(213) ~ 636657(213) | D63 | Ar56 |
| 1(214) ~ 636657(214) | D71 | Ar56 |
| 1(215) ~ 636657(215) | D81 | Ar56 |
| 1(216) ~ 636657(216) | D88 | Ar56 |
| 1(217) ~ 636657(217) | D106 | Ar56 |
| 1(218) ~ 636657(218) | D119 | Ar56 |
| 1(219) ~ 636657(219) | D765 | Ar56 |
| 1(220) ~ 636657(220) | D1 | Ar58 |
| 1(221) ~ 636657(221) | D7 | Ar58 |
| 1(222) ~ 636657(222) | D8 | Ar58 |
| 1(223) ~ 636657(223) | D9 | Ar58 |
| 1(224) ~ 636657(224) | D17 | Ar58 |
| 1(225) ~ 636657(225) | D27 | Ar58 |
| 1(226) ~ 636657(226) | D37 | Ar58 |
| 1(227) ~ 636657(227) | D39 | Ar58 |
| 1(228) ~ 636657(228) | D47 | Ar58 |
| 1(229) ~ 636657(229) | D51 | Ar58 |
| 1(230) ~ 636657(230) | D55 | Ar58 |
| 1(231) ~ 636657(231) | D61 | Ar58 |
| 1(232) ~ 636657(232) | D62 | Ar58 |
| 1(233) ~ 636657(233) | D63 | Ar58 |
| 1(234) ~ 636657(234) | D71 | Ar58 |

(continued)

| No. | $Ar^1$,$Ar^3$ | $Ar^2$,$Ar^4$ |
|---|---|---|
| 1(235) ~ 636657(235) | D81 | Ar58 |
| 1(236) ~ 636657(236) | D88 | Ar58 |
| 1(237) ~ 636657(237) | D106 | Ar58 |
| 1(238) ~ 636657(238) | D119 | Ar58 |
| 1(239) ~ 636657(239) | D765 | Ar58 |
| 1(240) ~ 636657(240) | D1 | Ar60 |
| 1(241) ~ 636657(241) | D7 | Ar60 |
| 1(242) ~ 636657(242) | D8 | Ar60 |
| 1(243) ~ 636657(243) | D9 | Ar60 |
| 1(244) ~ 636657(244) | D17 | Ar60 |
| 1(245) ~ 636657(245) | D27 | Ar60 |
| 1(246) ~ 636657(246) | D37 | Ar60 |
| 1(247) ~ 636657(247) | D39 | Ar60 |
| 1(248) ~ 636657(248) | D47 | Ar60 |
| 1(249) ~ 636657(249) | D51 | Ar60 |
| 1(250) ~ 636657(250) | D55 | Ar60 |
| 1(251) ~ 636657(251) | D61 | Ar60 |
| 1(252) ~ 636657(252) | D62 | Ar60 |
| 1(253) ~ 636657(253) | D63 | Ar60 |
| 1(254) ~ 636657(254) | D71 | Ar60 |
| 1(255) ~ 636657(255) | D81 | Ar60 |
| 1(256) ~ 636657(256) | D88 | Ar60 |
| 1(257) ~ 636657(257) | D106 | Ar60 |
| 1(258) ~ 636657(258) | D119 | Ar60 |
| 1(259) ~ 636657(259) | D765 | Ar60 |
| 1(260) ~ 636657(260) | D1 | Ar61 |
| 1(261) ~ 636657(261) | D7 | Ar61 |
| 1(262) ~ 636657(262) | D8 | Ar61 |
| 1(263) ~ 636657(263) | D9 | Ar61 |
| 1(264) ~ 636657(264) | D17 | Ar61 |
| 1(265) ~ 636657(265) | D27 | Ar61 |
| 1(266) ~ 636657(266) | D37 | Ar61 |
| 1(267) ~ 636657(267) | D39 | Ar61 |
| 1(268) ~ 636657(268) | D47 | Ar61 |
| 1(269) ~ 636657(269) | D51 | Ar61 |
| 1(270) ~ 636657(270) | D55 | Ar61 |
| 1(271) ~ 636657(271) | D61 | Ar61 |
| 1(272) ~ 636657(272) | D62 | Ar61 |
| 1(273) ~ 636657(273) | D63 | Ar61 |
| 1(274) ~ 636657(274) | D71 | Ar61 |

(continued)

| No. | $Ar^1$,$Ar^3$ | $Ar^2$,$Ar^4$ |
|---|---|---|
| 1(275) ~ 636657(275) | D81 | Ar61 |
| 1(276) ~ 636657(276) | D88 | Ar61 |
| 1(277) ~ 636657(277) | D106 | Ar61 |
| 1(278) ~ 636657(278) | D119 | Ar61 |
| 1(279) ~ 636657(279) | D765 | Ar61 |
| 1(280) ~ 636657(280) | D1 | D55 |
| 1(281) ~ 636657(281) | D7 | D55 |
| 1(282) ~ 636657(282) | D8 | D55 |
| 1(283) ~ 636657(283) | D9 | D55 |
| 1(284) ~ 636657(284) | D17 | D55 |
| 1(285) ~ 636657(285) | D27 | D55 |
| 1(286) ~ 636657(286)<br>1(287) ~ 636657(287) | D37<br>D39 | D55<br>D55 |
| 1(288) ~ 636657(288) | D47 | D55 |
| 1(289) ~ 636657(289) | D51 | D55 |
| 1(290) ~ 636657(290) | D55 | D55 |
| 1(291) ~ 636657(291) | D61 | D55 |
| 1(292) ~ 636657(292) | D62 | D55 |
| 1(293) ~ 636657(293) | D63 | D55 |
| 1(294) ~ 636657(294) | D71 | D55 |
| 1(295) ~ 636657(295) | D81 | D55 |
| 1(296) ~ 636657(296) | D88 | D55 |
| 1(297) ~ 636657(297) | D106 | D55 |
| 1(298) ~ 636657(298) | D119 | D55 |
| 1(299) ~ 636657(299) | D765 | D55 |
| 8756(300) ~ 442987(300) | Ar1 | Ar1 |
| 8756(301) -- 442987(301) | Ar10 | Ar10 |
| 8756(302) ~ 442987(302) | Ar12 | Ar12 |
| 8756(303) ~ 442987(303) | Ar14 | Ar14 |
| 8756(304) ~ 442987(304) | Ar15 | Ar15 |
| 8756(305) ~ 442987(305) | Ar35 | Ar35 |
| 8756(306) ~ 442987(306) | Ar37 | Ar37 |
| 8756(307) ~ 442987(307) | Ar39 | Ar39 |
| 8756(308) ~ 442987(308) | Ar40 | Ar40 |
| 8756(309) ~ 442987(309) | Ar56 | Ar56 |
| 8756(310) ~ 442987(310) | Ar58 | Ar58 |
| 8756(311) ~\| 442987(311) | Ar61 | Ar61 |
| 443073(312) ~ 539822(312) | Ar1 | Ar1 |
| 443073(313) ~ 539822(313) | Ar10 | Ar10 |
| 443073(314) ~ 539822(314) | Ar12 | Ar12 |

(continued)

| No. | $Ar^1$,$Ar^3$ | $Ar^2$,$Ar^4$ |
|---|---|---|
| 443073(315) ~ 539822(315) | Ar14 | Ar14 |
| 443073(316) ~ 539822(316) | Ar15 | Ar15 |
| 443073(317) ~ 539822(317) | Ar35 | Ar35 |
| 443073(318) ~ 539822(318) | Ar37 | Ar37 |
| 443073(319) ~ 539822(319) | Ar39 | Ar39 |
| 443073(320) ~ 539822(320) | Ar40 | Ar40 |
| 443073(321) ~ 539822(321) | Ar56 | Ar56 |
| 443073(322) ~ 539822(322) | Ar58 | Ar58 |
| 443073(323) ~ 539822(323) | Ar61 | Ar61 |
| 539908(324) ~ 636657(324) | Ar1 | Ar1 |
| 539908(325) ~ 636657(325)<br>539908(326) ~ 636657(326)<br>539908(327) ~ 636657(327) | Ar10<br>Ar12<br>Ar14 | Ar10<br>Ar12<br>Ar14 |
| 539908(328) ~ 636657(328) | Ar15 | Ar15 |
| 539908(329) ~ 636657(329) | Ar35 | Ar35 |
| 539908(330) ~ 636657(330) | Ar37 | Ar37 |
| 539908(331) ~ 636657(331)<br>539908(332) ~ 636657(332) | Ar39<br>Ar40 | Ar39<br>Ar40 |
| 539908(333) ~ 636657(333) | Ar56 | Ar56 |
| 539908(334) ~ 636657(334) | Ar58 | Ar58 |
| 539908(335) ~ 636657(335) | Ar61 | Ar61 |
| 8756(336) ~ 442987(336) | Ar47 | Ar1 |
| 8756(337) ~ 442987(337) | Ar47 | Ar10 |
| 8756(338) ~ 442987(338) | Ar47 | Ar12 |
| 8756(339) ~ 442987(339) | Ar47 | Ar14 |
| 8756(340) ~ 442987(340) | Ar47 | Ar15 |
| 8756(341) ~ 442987(341) | Ar47 | Ar35 |
| 8756(342) ~ 442987(342) | Ar47 | Ar37 |
| 8756(343) ~ 442987(343) | Ar47 | Ar39 |
| 8756(344) ~ 442987(344) | Ar47 | Ar40 |
| 8756(345) ~ 442987(345) | Ar47 | Ar56 |
| 8756(346) ~ 442987(346)<br>8756(347) ~ 442987(347) | Ar47<br>Ar47 | Ar58<br>Ar61 |
| 443073(348) ~ 539822(348) | Ar47 | Ar1 |
| 443073(349) ~ 539822(349) | Ar47 | Ar10 |
| 443073(350) ~ 539822(350) | Ar47 | Ar12 |
| 443073(351) ~ 539822(351) | Ar47 | Ar14 |
| 443073(352) ~ 539822(352) | Ar47 | Ar15 |
| 443073(353) ~ 539822(353) | Ar47 | Ar35 |
| 443073(354) ~ 539822(354) | Ar47 | Ar37 |

(continued)

| No. | $Ar^1$,$Ar^3$ | $Ar^2$,$Ar^4$ |
|---|---|---|
| 443073(355) ~ 539822(355) | Ar47 | Ar39 |
| 443073(356) ~ 539822(356) | Ar47 | Ar40 |
| 443073(357) ~ 539822(357) | Ar47 | Ar56 |
| 443073(358) ~ 539822(358) | Ar47 | Ar58 |
| 443073(359) ~ 539822(359) | Ar47 | Ar61 |
| 539908(360) ~ 636657(360) | Ar47 | Ar1 |
| 539908(361) ~ 636657(361) | Ar47 | Ar10 |
| 539908(362) ~ 636657(362) | Ar47 | Ar12 |
| 539908(363) ~ 636657(363) | Ar47 | Ar14 |
| 539908(364) ~ 636657(364) | Ar47 | Ar15 |
| 539908(365) ~ 636657(365) | Ar47 | Ar35 |
| 539908(366) ~ 636657(366) | Ar47 | Ar37 |
| 539908(367) ~ 636657(367) | Ar47 | Ar39 |
| 539908(368) ~ 636657(368) | Ar47 | Ar40 |
| 539908(369) ~ 636657(369) | Ar47 | Ar56 |
| 539908(370) ~ 636657(370) | Ar47 | Ar58 |
| 539908(371) ~ 636657(371) | Ar47 | Ar61 |

Compound 1 to Compound 636657(371) listed in Table 8 are compounds in which $R^1$ in the general formula (1) is a hydrogen atom. Compounds having a structure in which $R^1$ of these compounds is substituted with a deuterium atom are referred to as Compound 1d to Compound 636657(371)d in order. Compounds having a structure in which $R^1$ of these compounds is substituted with a perdeuterated phenyl group (Ar47) are referred to as Compound 1D to Compound 636657(371)D in order.

All of the compounds specified by the numbers in Tables 1 to 8, Compound 1d to Compound 636657(371)d, and Compound 1D to Compound 636657(371)D are individually disclosed. In addition, among the specific examples of the compounds, in the case where a rotamer is present, a mixture of rotamers and each separated rotamer are also disclosed in the description herein.

In one aspect of the present invention, the compound is selected from the group of compounds specified in Table 8.

An example of a preferred group of compounds represented by the general formula (1) is given below.

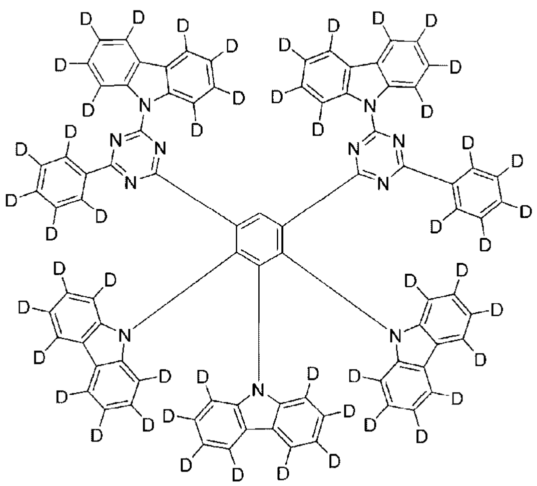

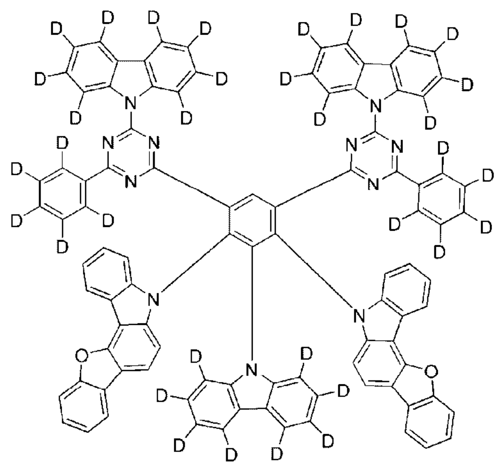

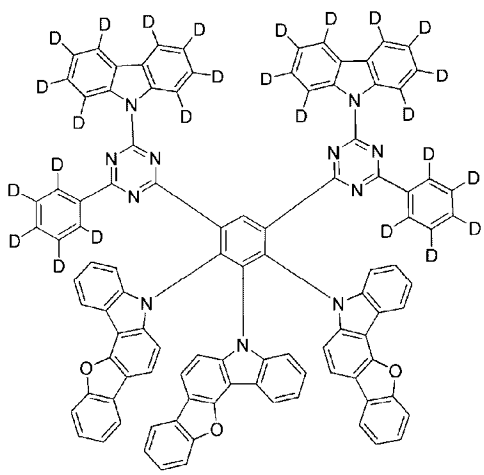

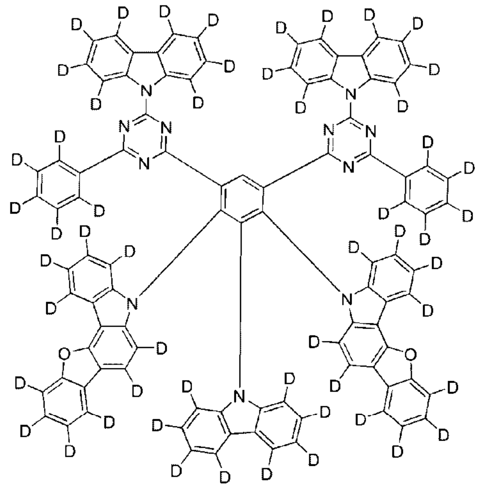

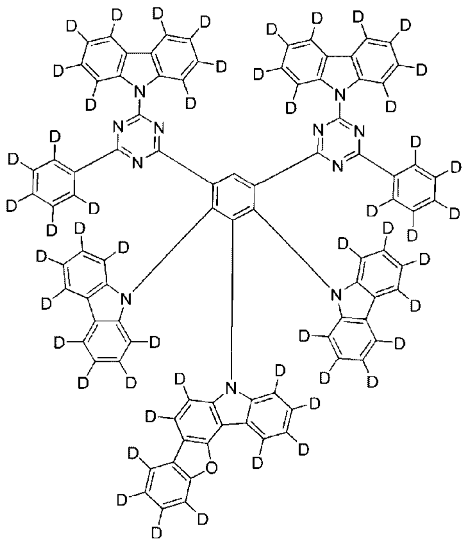

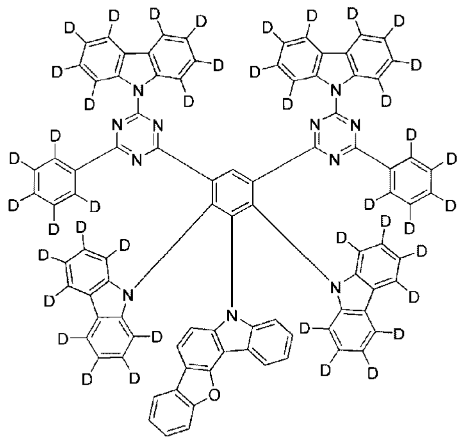

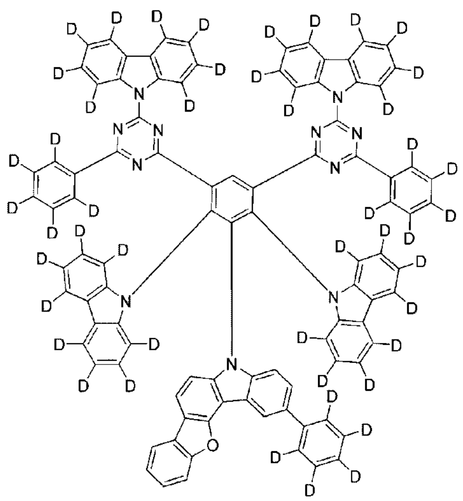

An example of another preferred group of compounds represented by the general formula (1) is given below.

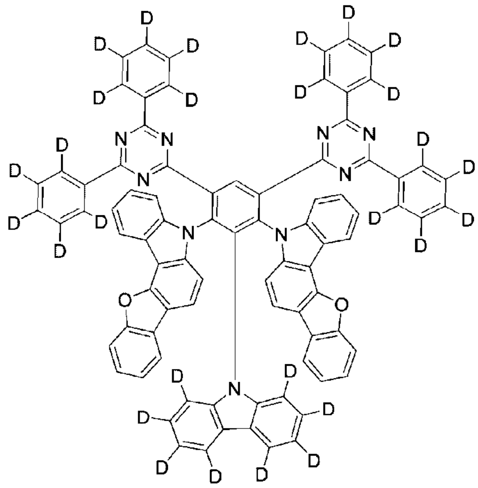

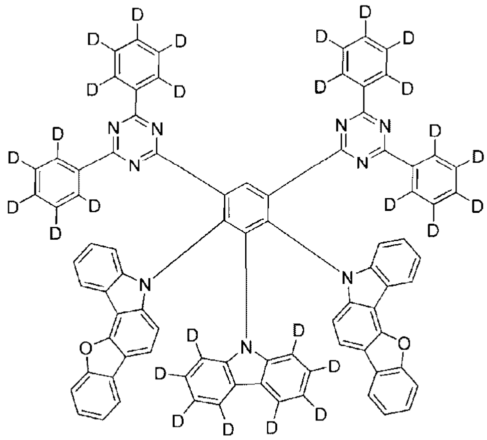

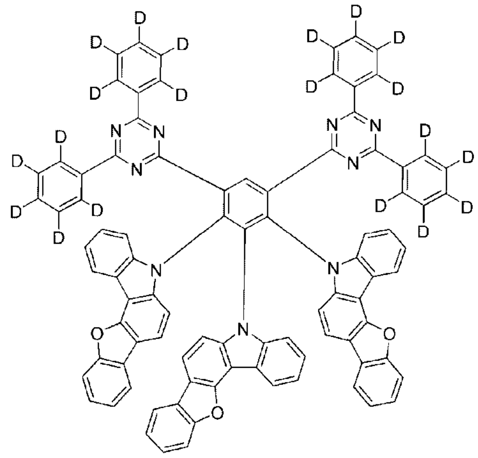

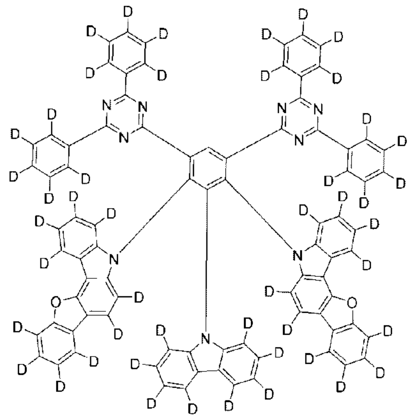

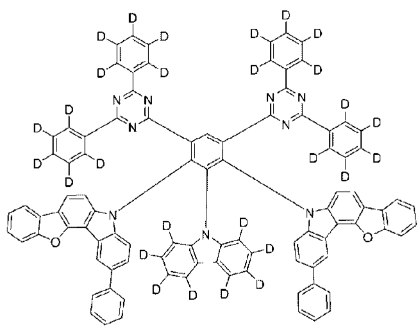

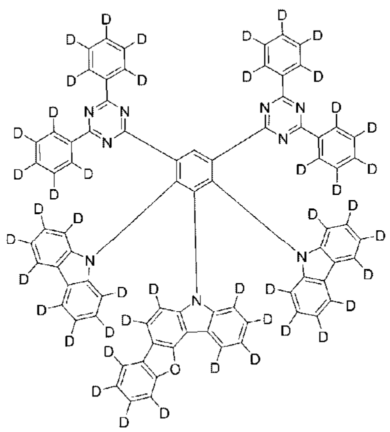

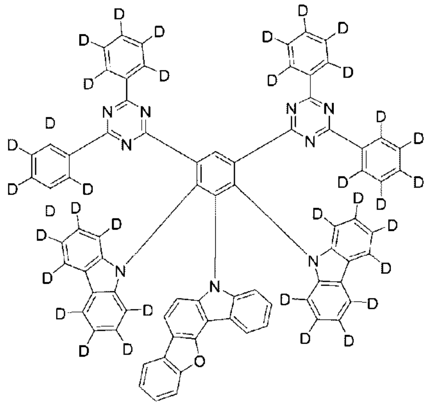

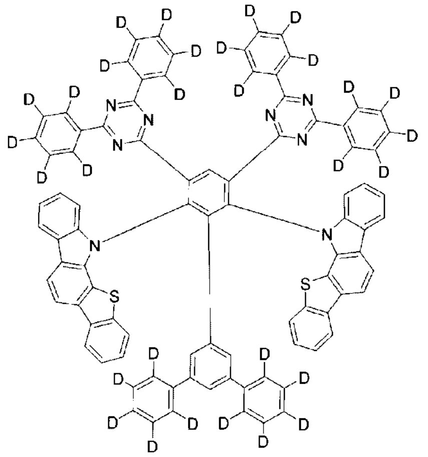

An example of another preferred group of compounds represented by the general formula (1) is given below.

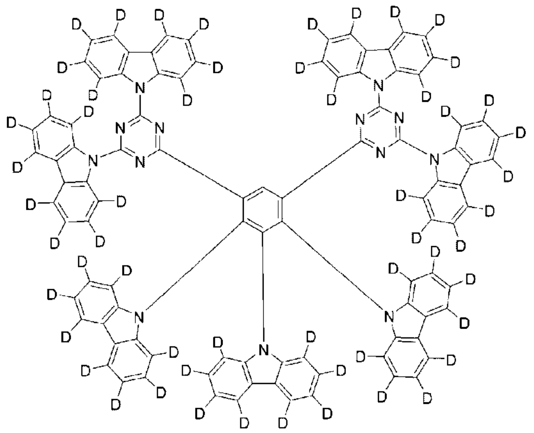

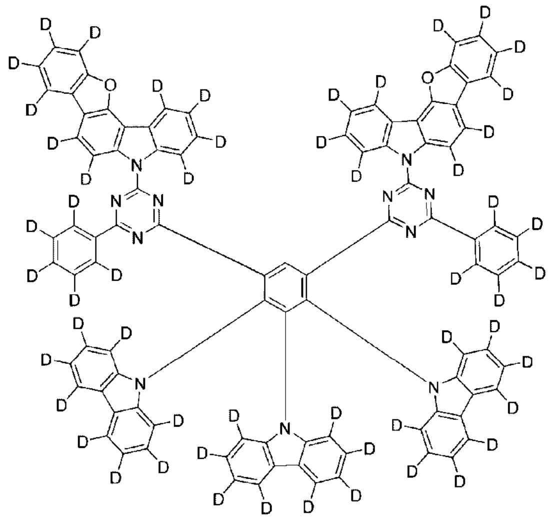

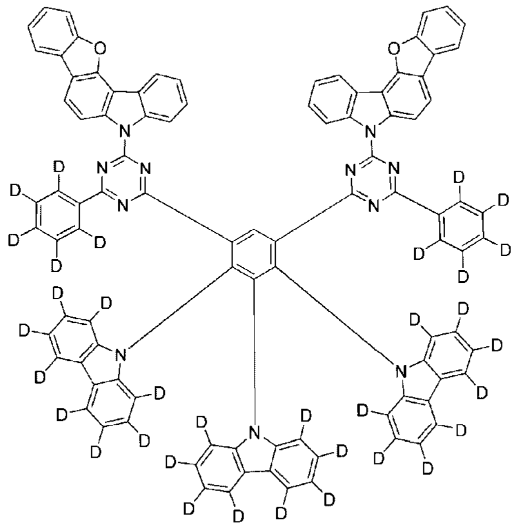

An example of another preferred group of compounds represented by the general formula (1) is given below.

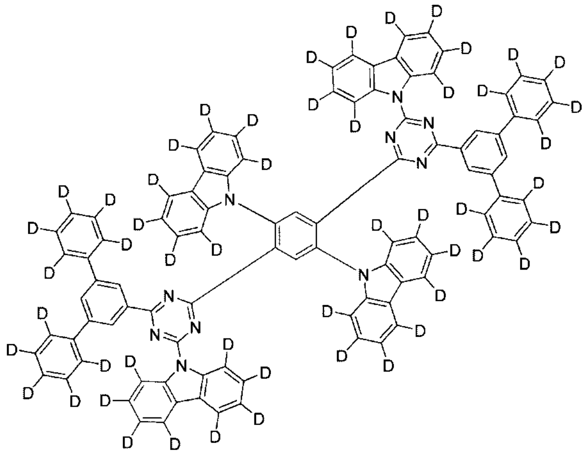

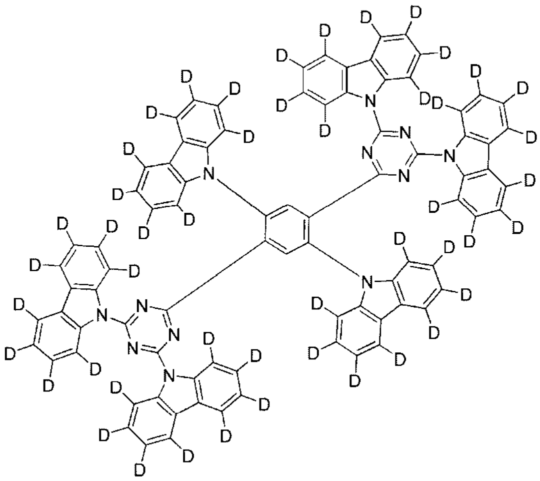

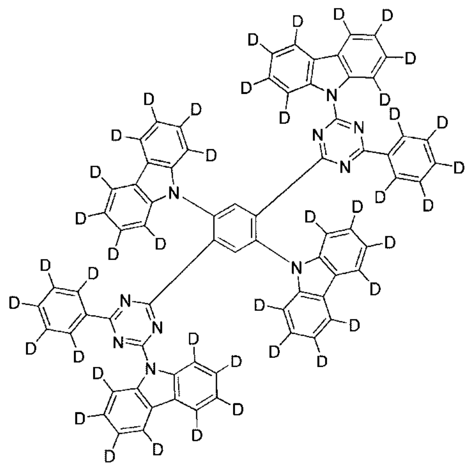

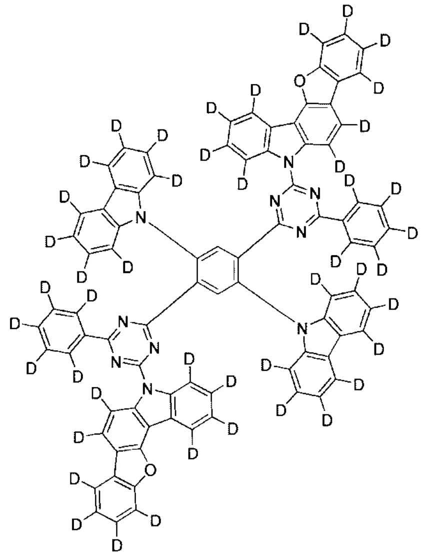

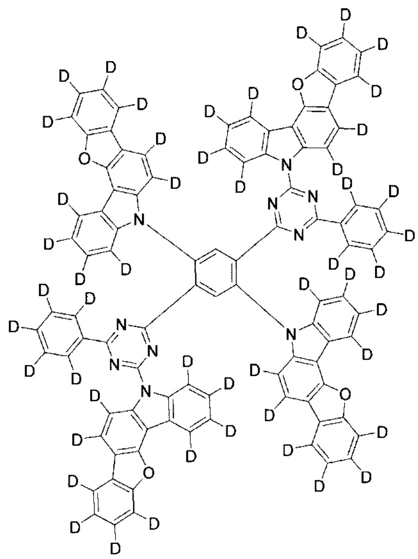

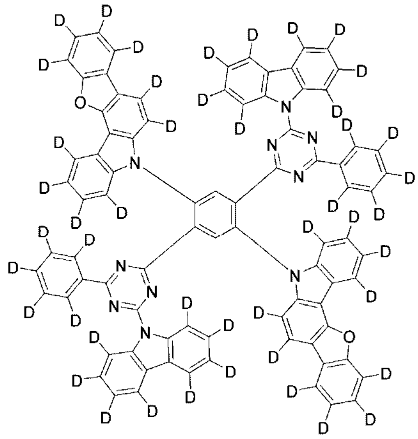

An example of another preferred group of compounds represented by the general formula (1) is given below.

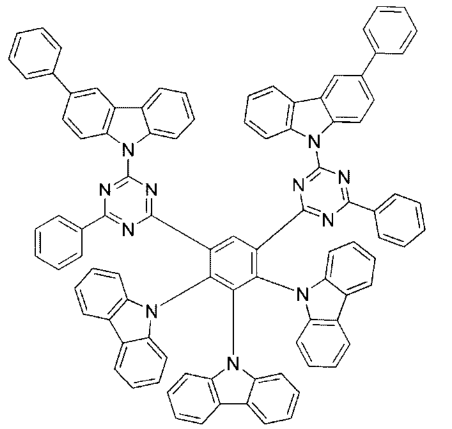

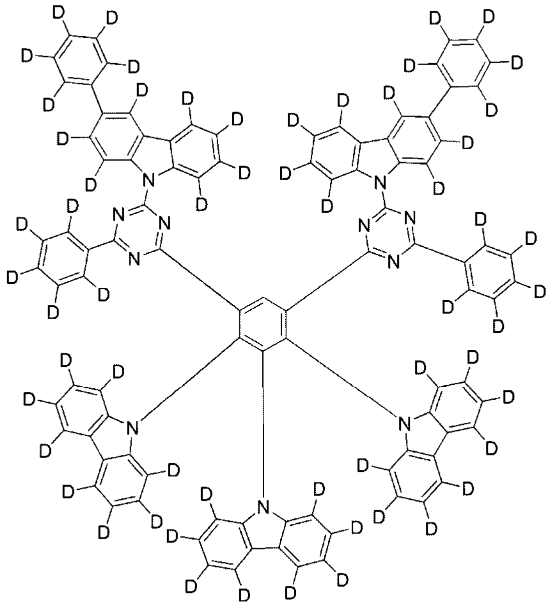

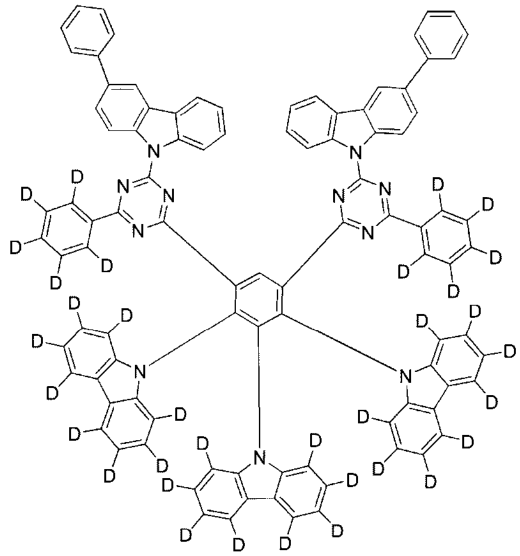

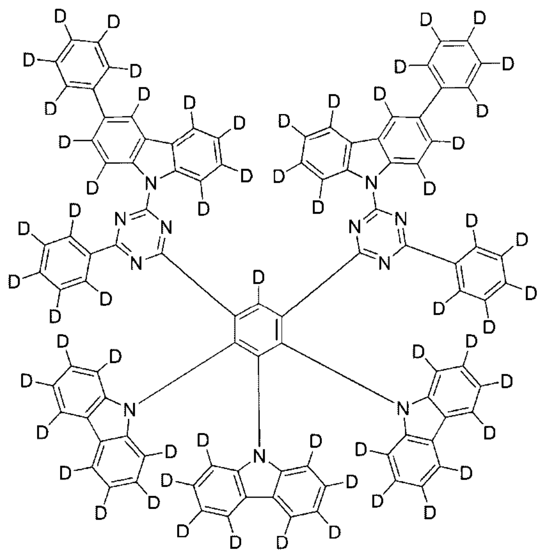

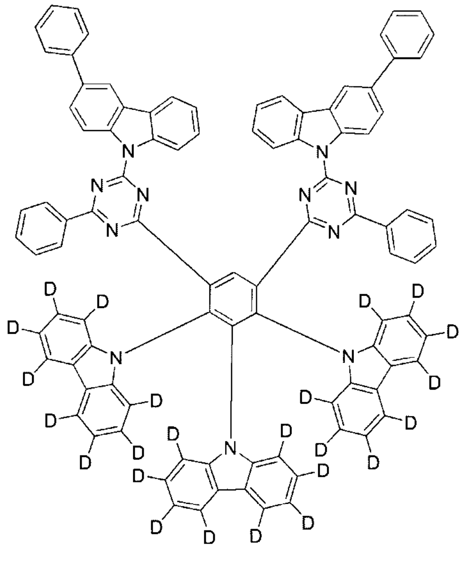

An example of another preferred group of compounds represented by the general formula (1) is given below.

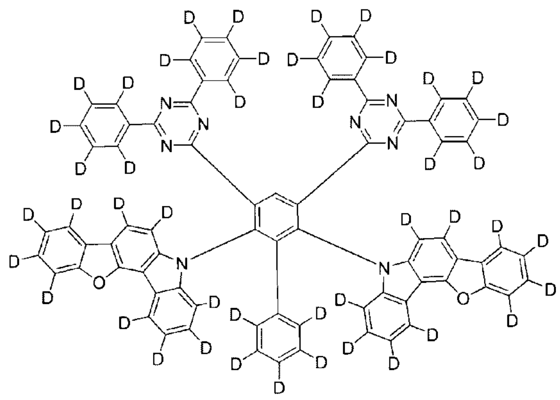

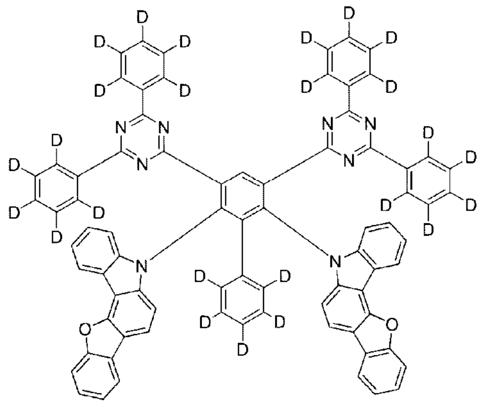

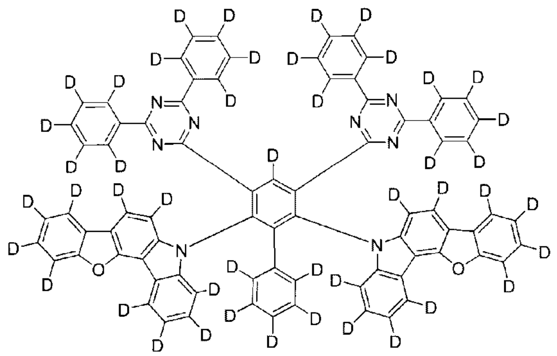

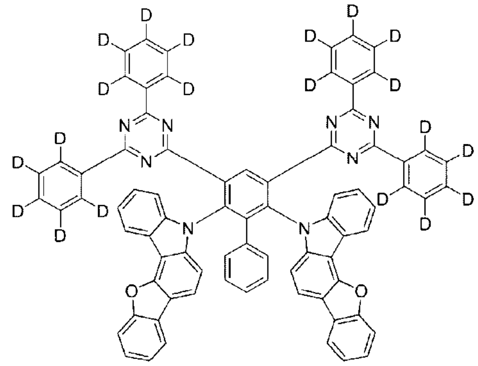

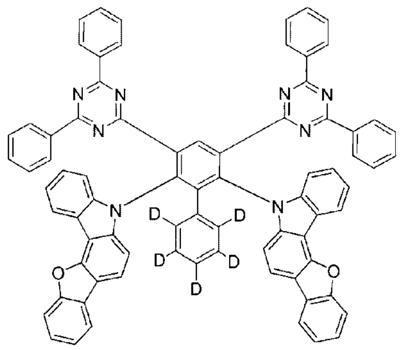

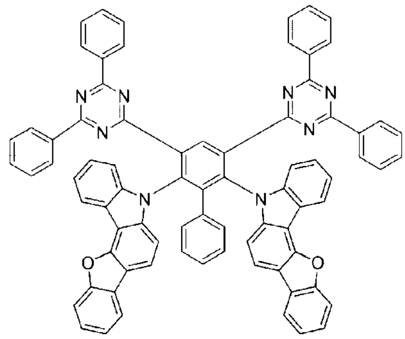

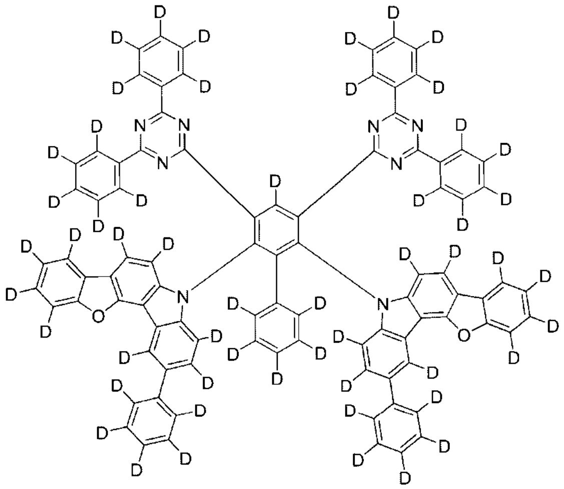

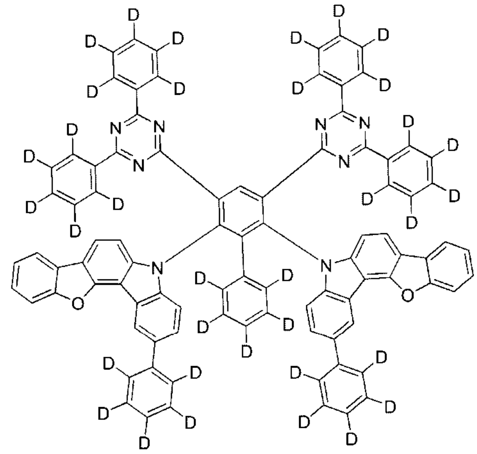

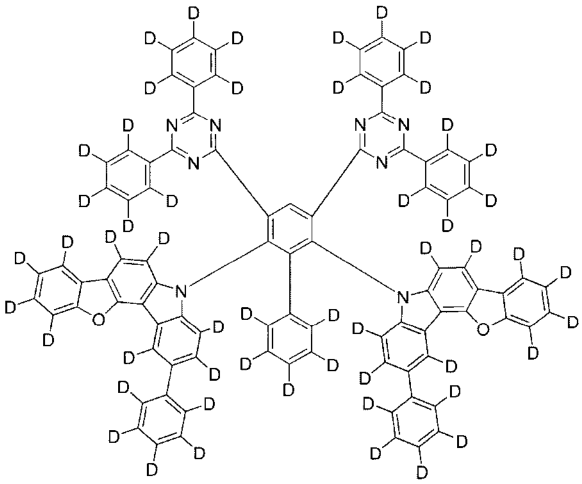

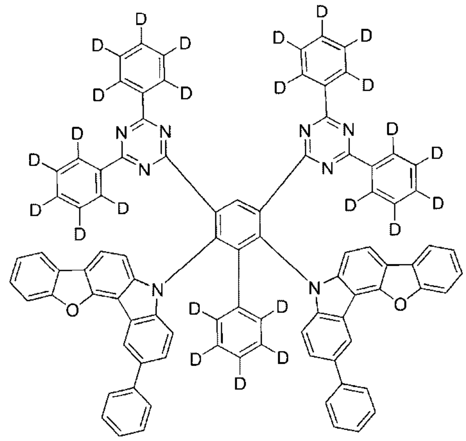

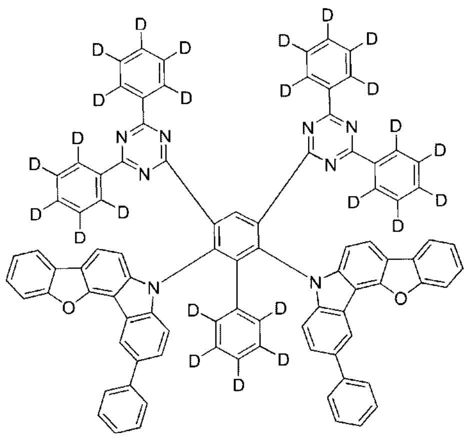

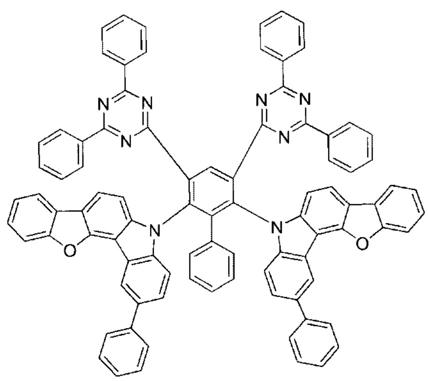

An example of another preferred group of compounds represented by the general formula (1) is given below.

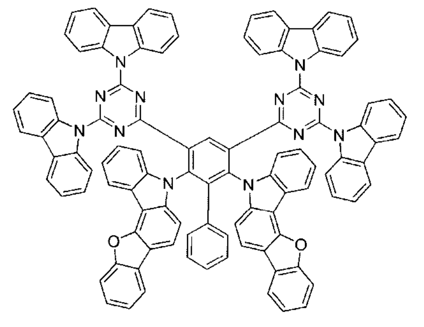
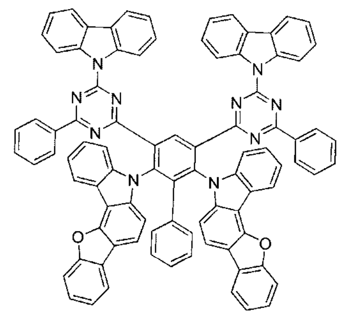

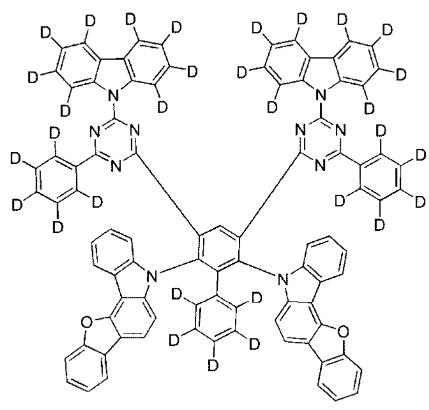
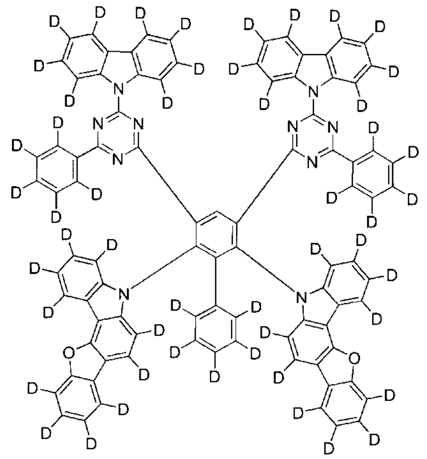

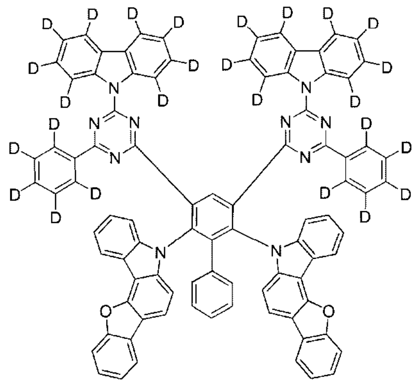
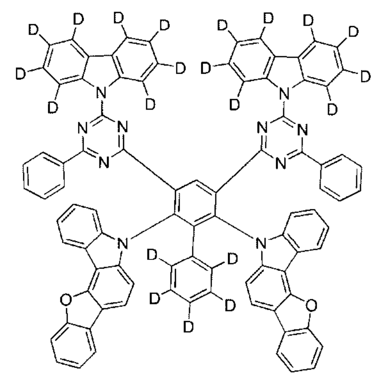

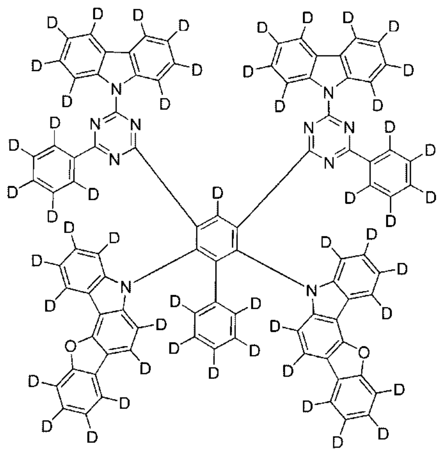
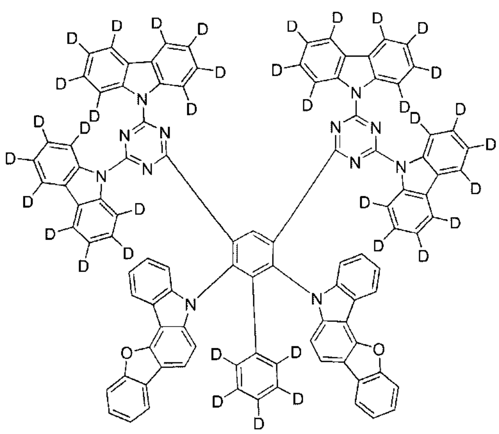

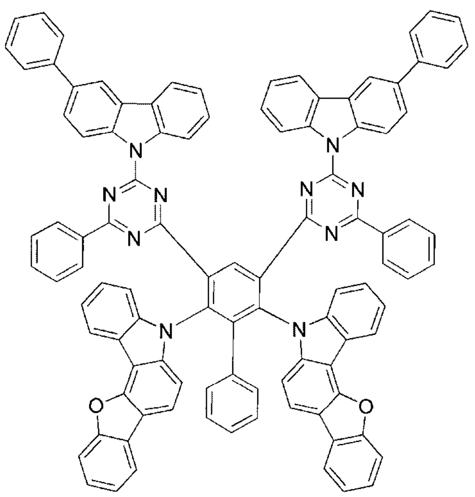

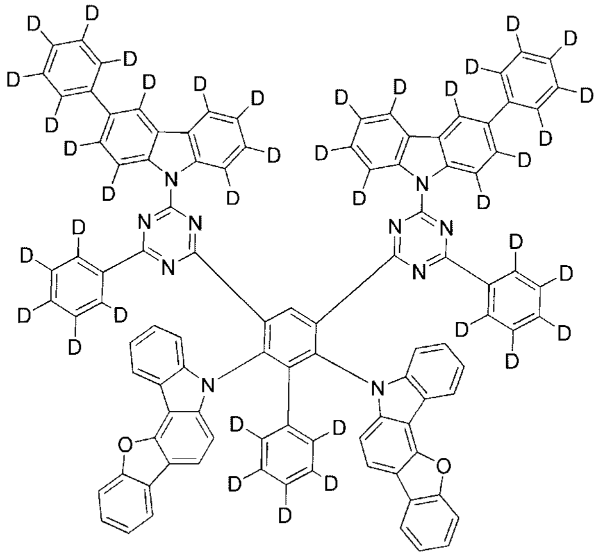

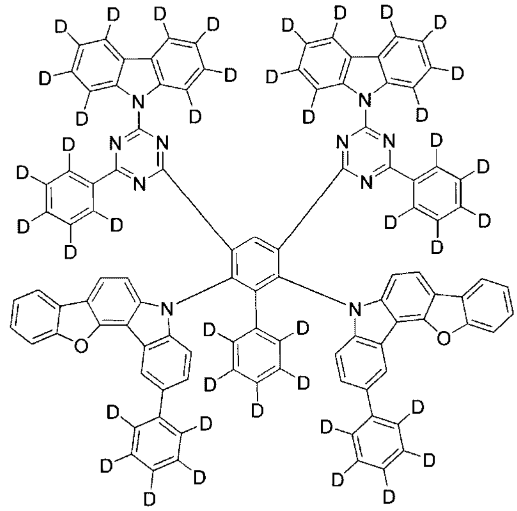

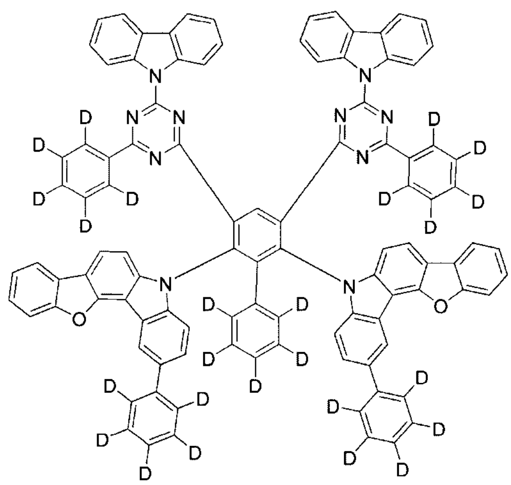

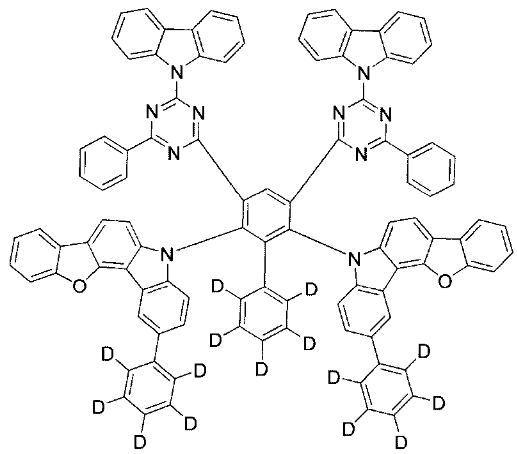

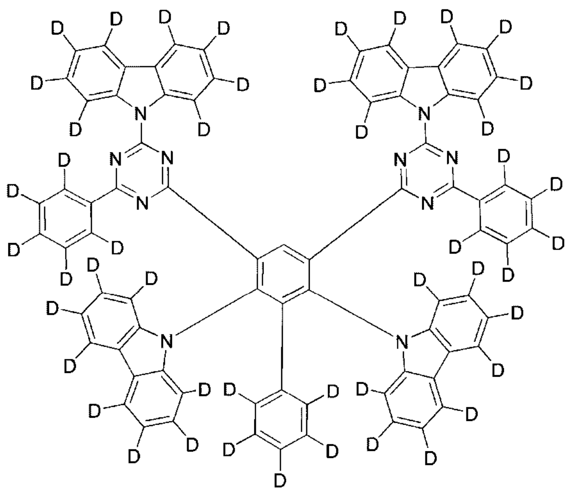

An example of another preferred group of compounds represented by the general formula (1) is given below.

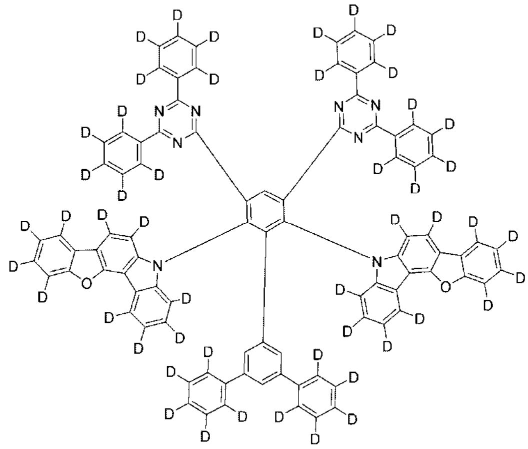

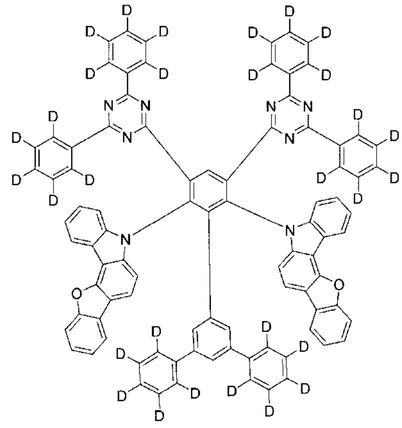

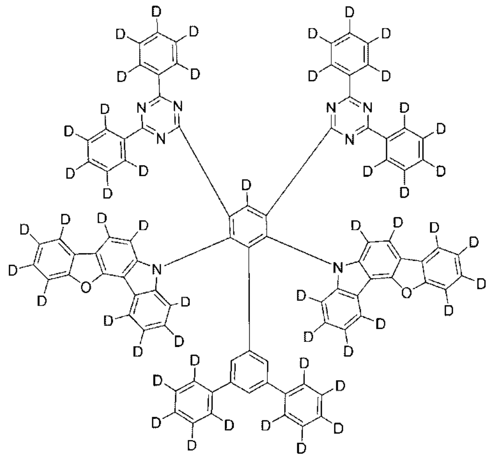

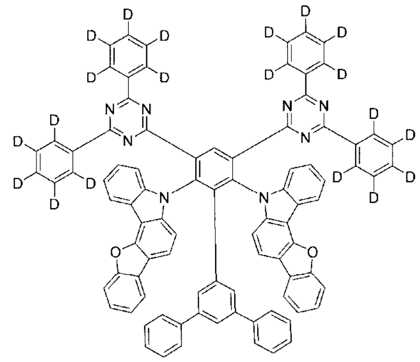

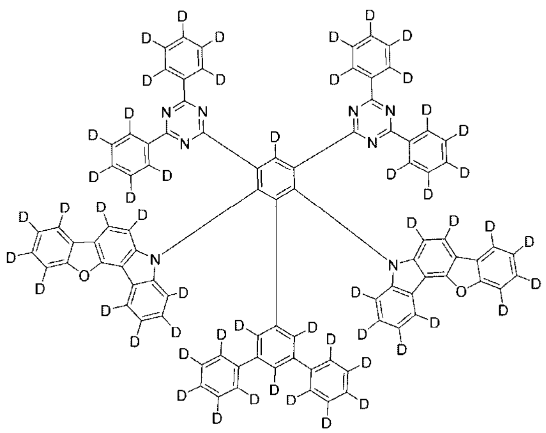

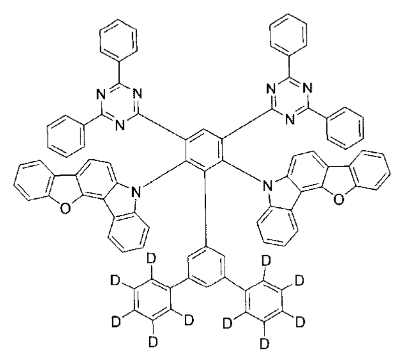

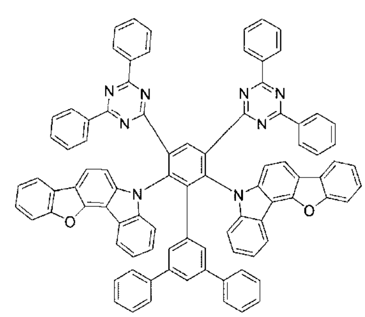

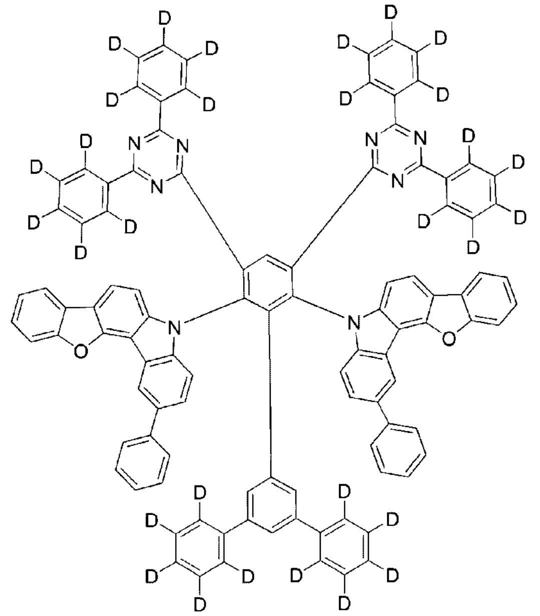

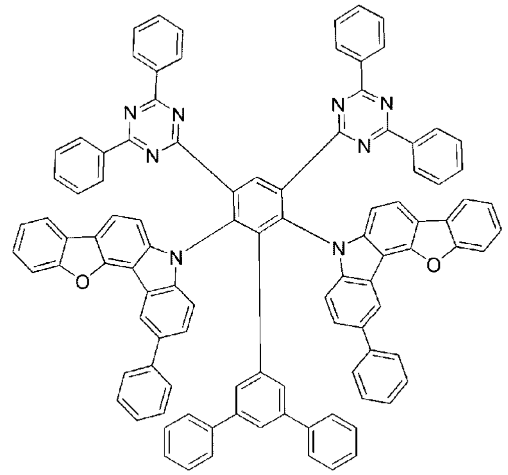

An example of another preferred group of compounds represented by the general formula (1) is given below.

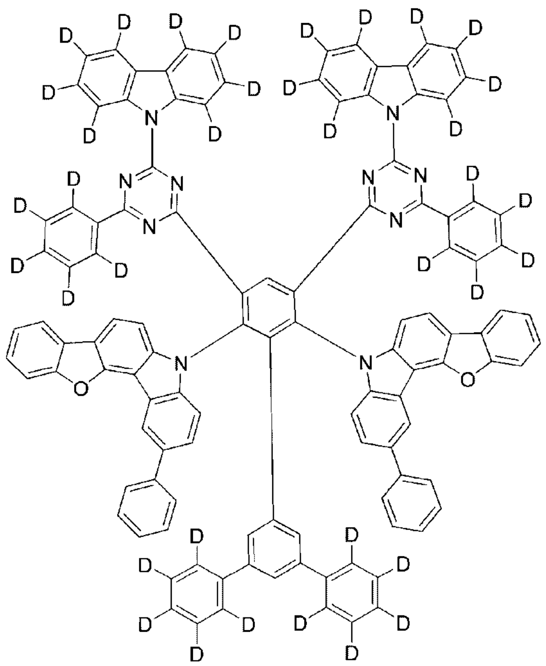

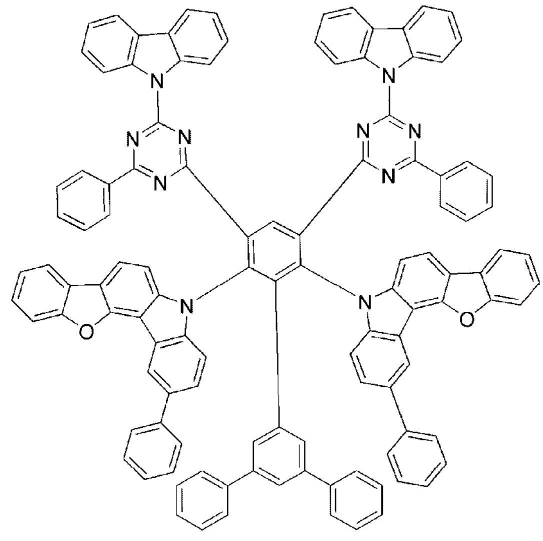

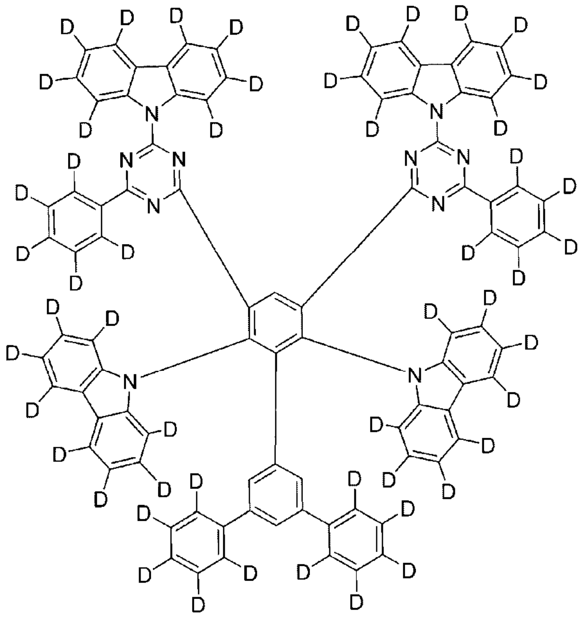

An example of another preferred group of compounds represented by the general formula (1) is given below.

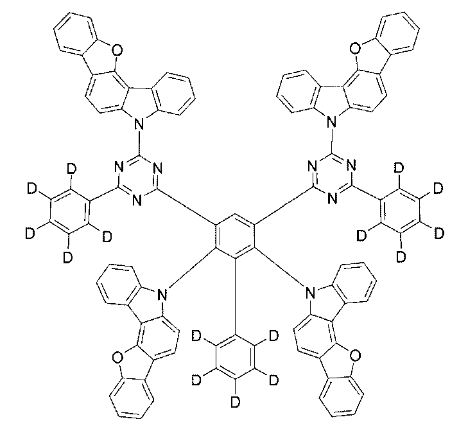

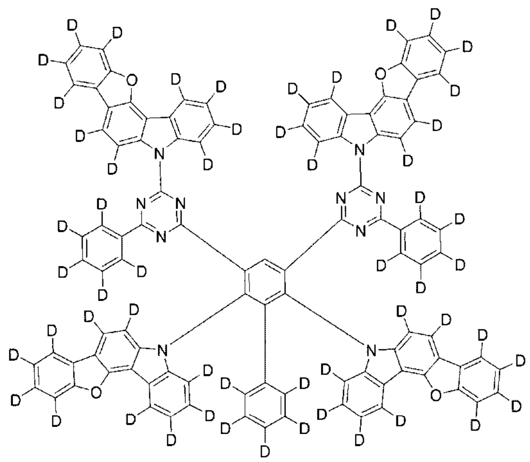

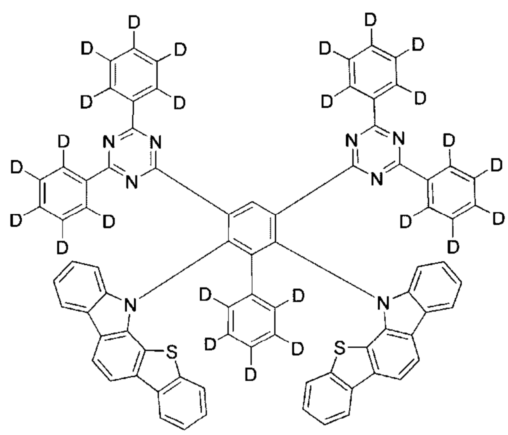

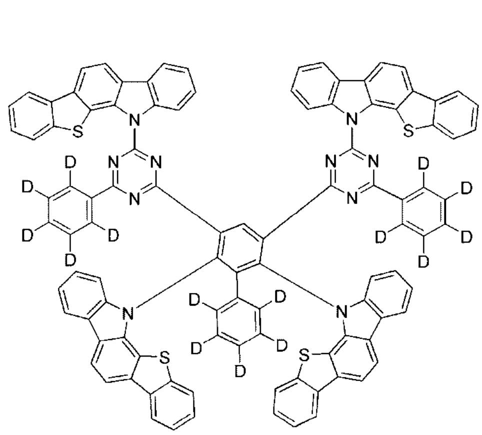

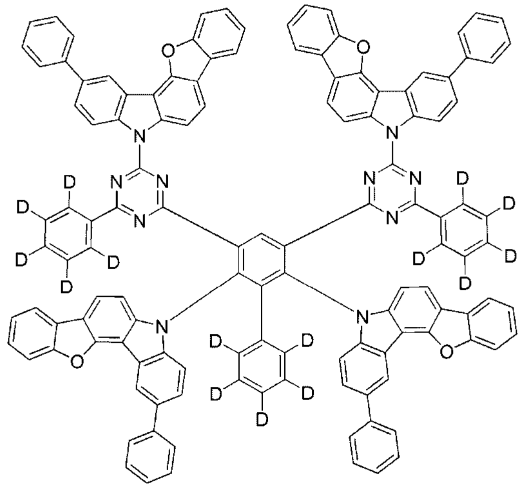

An example of another preferred group of compounds represented by the general formula (1) is given below.

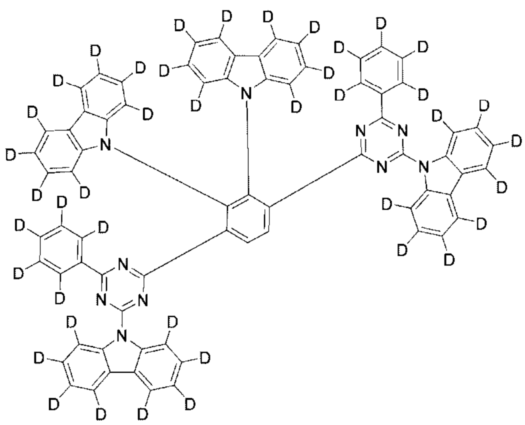

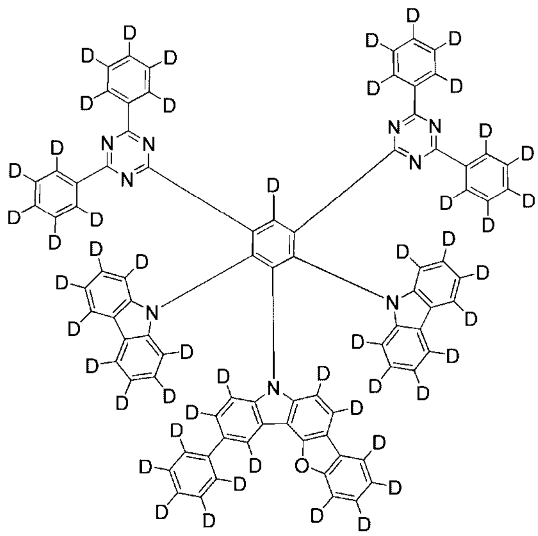

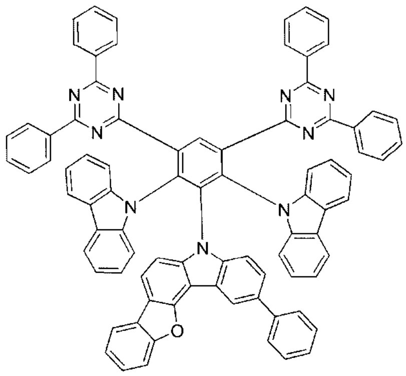

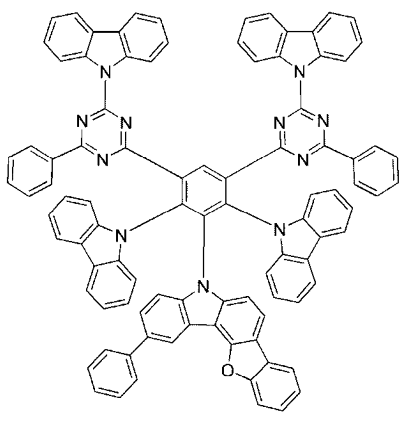

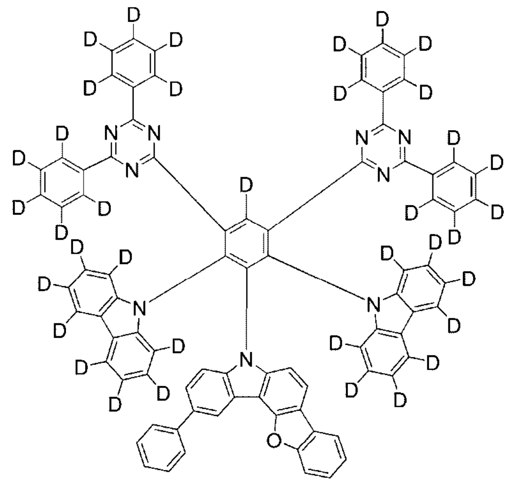

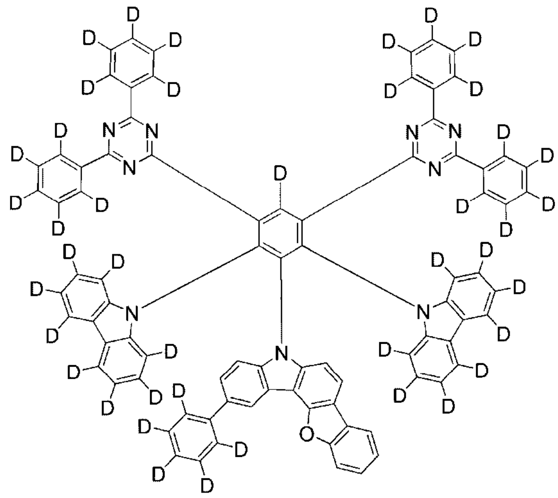

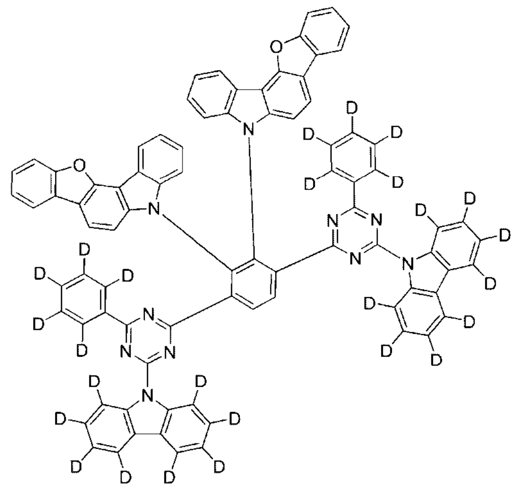

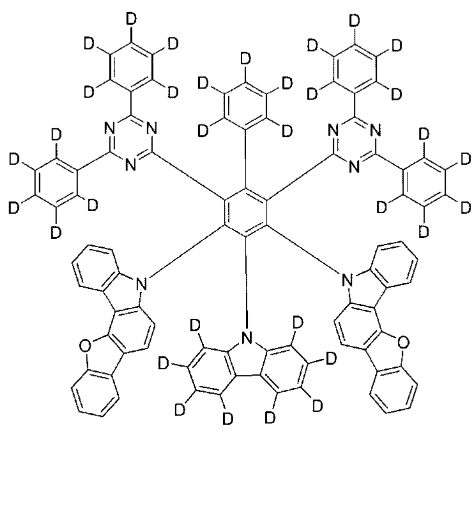

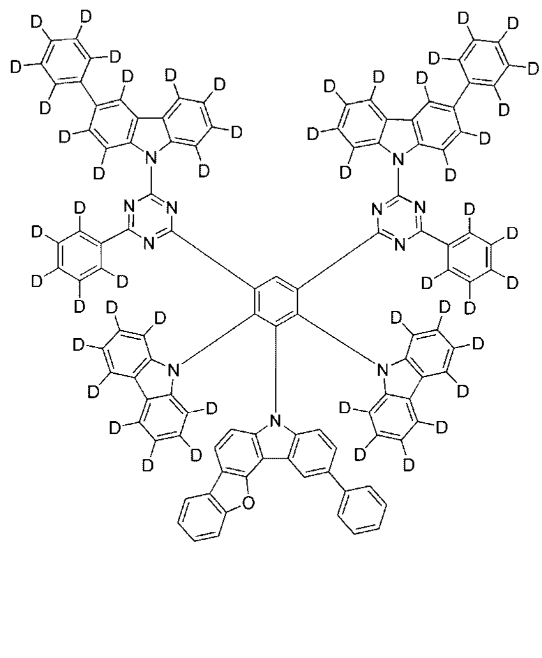

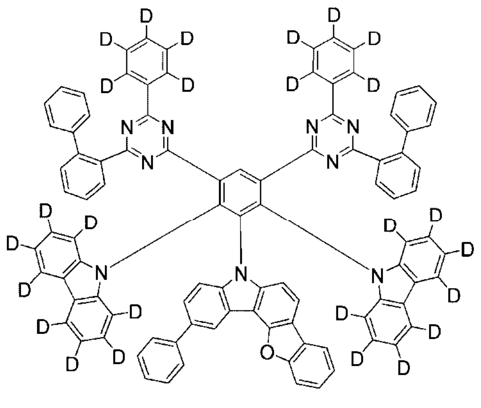

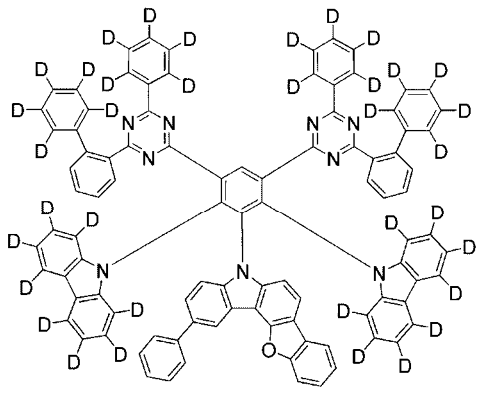

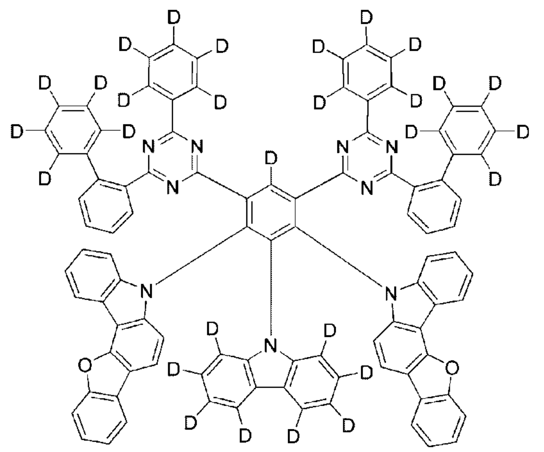

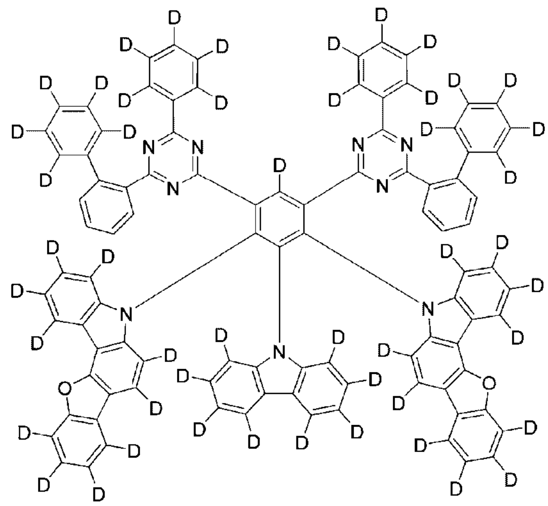

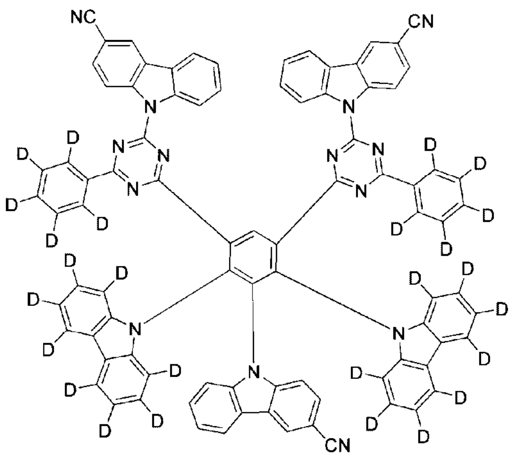

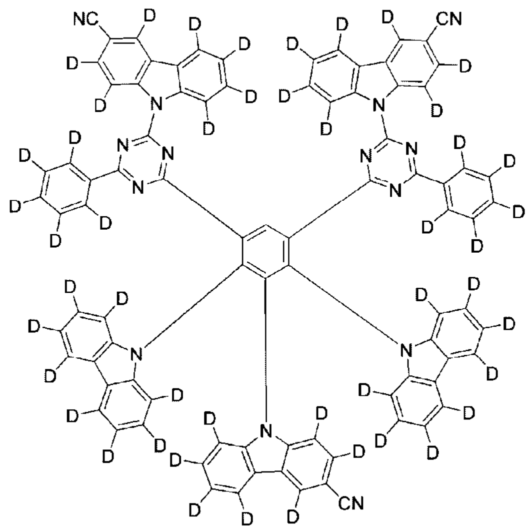

The molecular weight of the compound represented by the general formula (1) is preferably 1500 or less, more preferably 1200 or less, further preferably 1000 or less, and still further preferably 900 or less, for example, in the case where an organic layer containing the compound represented by the general formula (1) is intended to be film-formed and used by a vapor deposition method. The lower limit value of the molecular weight is the molecular weight of the minimum compound represented by the general formula (1).

The compound represented by the general formula (1) may be formed into a film by a coating method regardless of the molecular weight. When the coating method is used, even a compound having a relatively large molecular weight can be formed into a film. The compound represented by the general formula (1) has an advantage of being easily dissolved in an organic solvent. For this reason, the compound represented by the general formula (1) is easily applicable

to a coating method and is easily purified to increase its purity.

It is also conceivable to use a compound containing a plurality of structures represented by the general formula (1) in a molecule as a light-emitting material by applying the present invention.

For example, it is conceivable that a polymer obtained by allowing a polymerizable group to be present in the structure represented by the general formula (1) in advance and polymerizing the polymerizable group is used as the light-emitting material. For example, it is conceivable that a polymer having a repeating unit is obtained by preparing a monomer containing a polymerizable functional group at any site of the general formula (1) and polymerizing the monomer alone or copolymerizing the monomer with another monomer, and the polymer is used as the light-emitting material. Alternatively, it is also conceivable to obtain a dimer or a trimer by coupling compounds having a structure represented by the general formula (1) to each other and to use the dimer or the trimer as a light-emitting material.

Examples of the polymer having a repeating unit containing a structure represented by the general formula (1) include polymers containing a structure represented by any one of the following two general formulae.

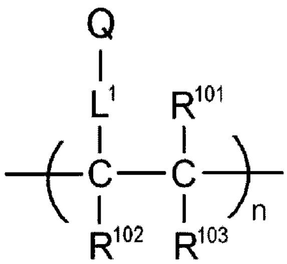
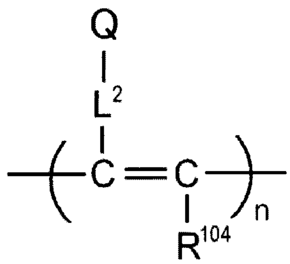

In the above general formulae, Q represents a group containing the structure represented by the general formula (1), and $L^1$ and $L^2$ each represent a linking group. The linking group preferably has 0 to 20 carbon atoms, more preferably 1 to 15 carbon atoms, and further preferably 2 to 10 carbon atoms. The linking group preferably has a structure represented by $-X^{11}-L^{11}-$. Here, $X^{11}$ represents an oxygen atom or a sulfur atom, and is preferably an oxygen atom. $L^{11}$ represents a linking group, and is preferably a substituted or unsubstituted alkylene group or a substituted or unsubstituted arylene group, and more preferably a substituted or unsubstituted alkylene group having 1 to 10 carbon atoms or a substituted or unsubstituted phenylene group.

In the above general formulae, $R^{101}$, $R^{102}$, $R^{103}$, and $R^{104}$ each independently represent a substituent. The substituent is preferably a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom, or a chlorine atom, and further preferably an unsubstituted alkyl group having 1 to 3 carbon atoms or an unsubstituted alkoxy group having 1 to 3 carbon atoms.

The linking group represented by $L^1$ and $L^2$ can be bonded to any site of the general formula (1) constituting Q. Two or more linking groups may be linked to one Q to form a cross-linked structure or a network structure.

Specific structural examples of the repeating unit include structures represented by the following formulae.

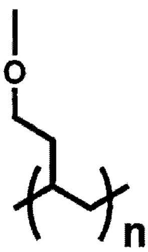
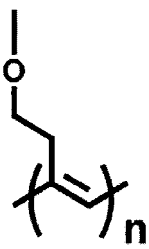
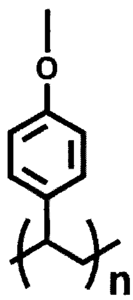
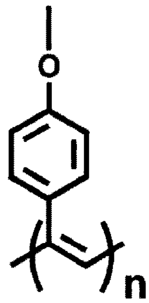

The polymer having a repeating unit including these formulae can be synthesized by introducing a hydroxyl group into any site of the general formula (1), reacting the following compound using the hydroxy group as a linker to introduce a polymerizable group, and polymerizing the polymerizable group.

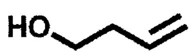
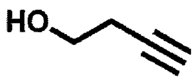
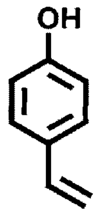
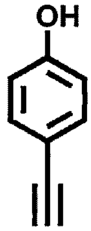

The polymer having a structure represented by the general formula (1) in the molecule may be a polymer having only a repeating unit that has the structure represented by the general formula (1), or may be a polymer containing a repeating unit that has any other structure. The repeating unit having the structure represented by the general formula (1) to be contained in the polymer may be a single kind or two or more kinds. The repeating unit not having the structure represented by the general formula (1) includes those derived from monomers used in general copolymerization. For example, it includes repeating units derived from monomers having an ethylenically unsaturated bond, such as ethylene or styrene.

With respect to the use and the like of the compound represented by the general formula (1), reference can be made to [0058] to [0059], [0061], and [0063] of WO2022/168956A1, which are incorporated as a part of this description by reference.

[Method for Synthesizing Compound Represented by General Formula (1)]

The compound represented by the general formula (1) includes a novel compound.

The compound represented by the general formula (1) can be synthesized by combining known reactions. In the compound represented by the general formula (1), two or more of $R^1$ to $R^5$ are donor groups. For example, the compound represented by the general formula (1) in which a substituted or unsubstituted carbazol-9-yl group is a donor group can be synthesized by reacting a substituted or unsubstituted carbazole with a precursor in which the site of the donor group is a fluorine atom. For details of the reaction conditions, Synthesis Examples described later can be referred to.

[Organic Light-Emitting Device]

The organic light-emitting device of the present invention contains the compound represented by the general formula (1) and a host material or a dopant material in the same layer. Here, the same layer is preferably a light-emitting layer. In one aspect of the present invention, the compound represented by the general formula (1) and the host material are contained in the same layer. The host material is used at a concentration higher than that of the compound represented by the general formula (1), and preferably has a lowest excited singlet energy higher than that of the compound represented by the general formula (1). In one aspect of the present invention, the compound represented by the general formula (1) and the dopant material are contained in the same layer. The dopant material is used at a concentration lower than that of the compound represented by the general formula (1), and preferably has a lowest excited singlet energy lower than that of the compound represented by the general formula (1).

In some embodiments, the compound represented by the general formula (1) is applied to Phosphorescence Sensitized Fluorescence.

In some embodiments, the compound represented by the general formula (1) functions as a light-emitting material in the organic light-emitting device. In some embodiments, the compound represented by the general formula (1) functions as a light-emitting material that emits delayed fluorescence in the organic light-emitting device.

In some embodiments of the present disclosure, the compound represented by the general formula (1) is, when excited thermally or by an electronic means, able to emit light in a UV region, emit light of blue, green, yellow, orange, or red in a visible spectral region (e.g., about 420 nm to about 500 nm, about 500 nm to about 600 nm, or about 600 nm to about 700 nm) or emit light in a near IR region.

In some embodiments of the present disclosure, the compound represented by the general formula (1) is, when excited thermally or by an electronic means, able to emit light of red or orange in a visible spectral region (e.g., about 620 nm to about 780 nm, about 650 nm).

In some embodiments of the present disclosure, the compound represented by the general formula (1) is, when excited thermally or by an electronic means, able to emit light of orange or yellow in a visible spectral region (e.g., about 570 nm to about 620 nm, about 590 nm, about 570 nm).

In some embodiments of the present disclosure, the compound represented by the general formula (1) is, when excited thermally or by an electronic means, able to emit light of green in a visible spectral region (e.g., about 490 nm to about 575 nm, about 510 nm).

In some embodiments of the present disclosure, the compound represented by the general formula (1) is, when excited thermally or by an electronic means, able to emit light of blue in a visible spectral region (e.g., about 400 nm to about 490 nm, about 475 nm).

In some embodiments of the present disclosure, the compound represented by the general formula (1) is, when excited thermally or by an electronic means, able to emit light in a UV spectral region (e.g., about 280 nm to 400 nm).

In some embodiments of the present disclosure, the compound represented by the general formula (1) is, when excited thermally or by an electronic means, able to emit light in an IR spectral region (e.g., about 780 nm to 2 μm).

When a layer of an organic light-emitting device is formed, a film forming technique can be used.

In some embodiments, a film containing the compound represented by the general formula (1) can be formed in a wet process. In the wet process, a solution prepared by dissolving a composition containing the compound of the present invention is applied onto a surface, and then the solvent is removed to form a film. The wet process includes a spin coating method, a slit coating method, an inkjet method (a spraying method), a gravure printing method, an offset printing method, and a flexographic printing method, which, however, are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum deposition method is employable as a dry process, which, however, is not limitative. In the case where a vacuum deposition method is employed, compounds to constitute a film may be co-deposited from individual vapor deposition sources, or may be co-deposited from a single vapor deposition source formed by mixing the compounds. In the case where a single vapor deposition source is used, a mixed powder prepared by mixing compound powders may be used, or a compression molded body prepared by compression-molding the mixed powder may be used, or a mixture prepared by heating and melting the constituent compounds and cooling the resulting melt may be used. In some embodiments, by co-deposition under the condition where the vapor deposition rate (weight reduction rate) of the plural compounds contained in a single vapor deposition source is the same or is nearly the same, a film having a compositional ratio corresponding to the compositional ratio of the plural compounds contained in the vapor deposition source can be formed. When plural compounds are mixed in the same compositional ratio as the compositional ratio of the film to be formed to prepare a vapor deposition source, a film having a desired compositional ratio can be formed in a simplified manner. In some embodiments, the temperature at which the compounds to be co-deposited have the same weight reduction ratio is specifically defined, and the temperature can be employed as the temperature of co-deposition.

In some embodiments, the organic light-emitting device is an organic photoluminescence device (organic PL device). In some embodiments, the organic light-emitting device is an organic electroluminescent device (organic EL device). In some embodiments, the compound represented by the general formula (1) assists light emission from the other light-emitting materials contained in the light-emitting layer (as a so-called assist dopant). In some embodiments, the compound represented by the general formula (1) contained in the light-emitting layer is in a lowest excited singlet energy level, and is contained between the lowest excited single energy level of the host material contained in the light-emitting layer and the lowest excited singlet energy level of the other light-emitting materials contained in the light-emitting layer.

In some embodiments, the organic photoluminescence device includes at least one light-emitting layer. In some embodiments, the organic electroluminescent device includes at least an anode, a cathode, and an organic layer between the anode and the cathode. In some embodiments, the organic layer includes at least a light-emitting layer. In some embodiments, the organic layer includes only a light-emitting layer. In some embodiments, the organic layer includes one or more organic layers as well as the light-emitting layer. Examples of the organic layer include a hole transport layer, a hole injection layer, an electron barrier layer, a hole barrier layer, an electron injection layer, an electron transport layer, and an exciton barrier layer. In some embodiments, the hole transport layer may be a hole injection transport layer having a hole injection function, and the electron transport layer may be an electron injection transport layer having an electron injection function.

Light-Emitting Layer:

In some embodiments, the light-emitting layer is a layer where holes and electrons injected from the anode and the cathode, respectively, are recombined to form excitons. In some embodiments, the layer emits light.

In some embodiments, the light-emitting layer contains a light-emitting material that is a dopant material and a host material. In some embodiments, the light-emitting material is a compound represented by the general formula (1). In some embodiments, for improving light emission efficiency of an organic electroluminescent device and an organic photoluminescence device, the singlet exciton and the triplet exciton generated in a light-emitting material are confined inside the light-emitting material. In some embodiments, a host material is used in the light-emitting layer in addition to a light-emitting material. In some embodiments, the host material is an organic compound. In some embodiments, the organic compound has an excited singlet energy and an excited triplet energy, and at least one of them is higher than those

in the light-emitting material of the present invention. In some embodiments, the singlet exciton and the triplet exciton generated in the light-emitting material of the present invention are confined in the molecules of the light-emitting material of the present invention. In some embodiments, the singlet and triplet excitons are fully confined for improving light emission efficiency. In some embodiments, although high light emission efficiency is still attained, singlet excitons and triplet excitons are not fully confined, that is, a host material capable of attaining high light emission efficiency can be used in the present invention with no specific limitation. In some embodiments, in the light-emitting material in the light-emitting layer of the device of the present invention, light emission occurs. In some embodiments, emitted light includes both fluorescence and delayed fluorescence. In some embodiments, emitted light includes emitted light from a host material. In some embodiments, emitted light is composed of emitted light from a host material. In some embodiments, emitted light includes emitted light from the compound represented by the general formula (1) and emitted light from a host material. In some embodiment, a TADF molecule and a host material are used. In some embodiments, TADF is an assist dopant and has a lower excited singlet energy than the host material in the light-emitting layer and a higher excited singlet energy than the light-emitting material in the light-emitting layer.

(Dopant Material)

When the compound represented by the general formula (1) is used as a host material or an assist dopant, various compounds can be employed as a light-emitting material (preferably a fluorescent material) which is a dopant material. As such a light-emitting material, it is possible to use an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorene derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, a derivative having a metal (Al, Zn), or the like. These exemplified skeletons may have substituents, or may not have substituents. Further, these exemplified skeletons may be combined with each other.

Hereinafter, dopant materials (light-emitting materials) which can be used in combination with the compound represented by the general formula (1) will be exemplified.

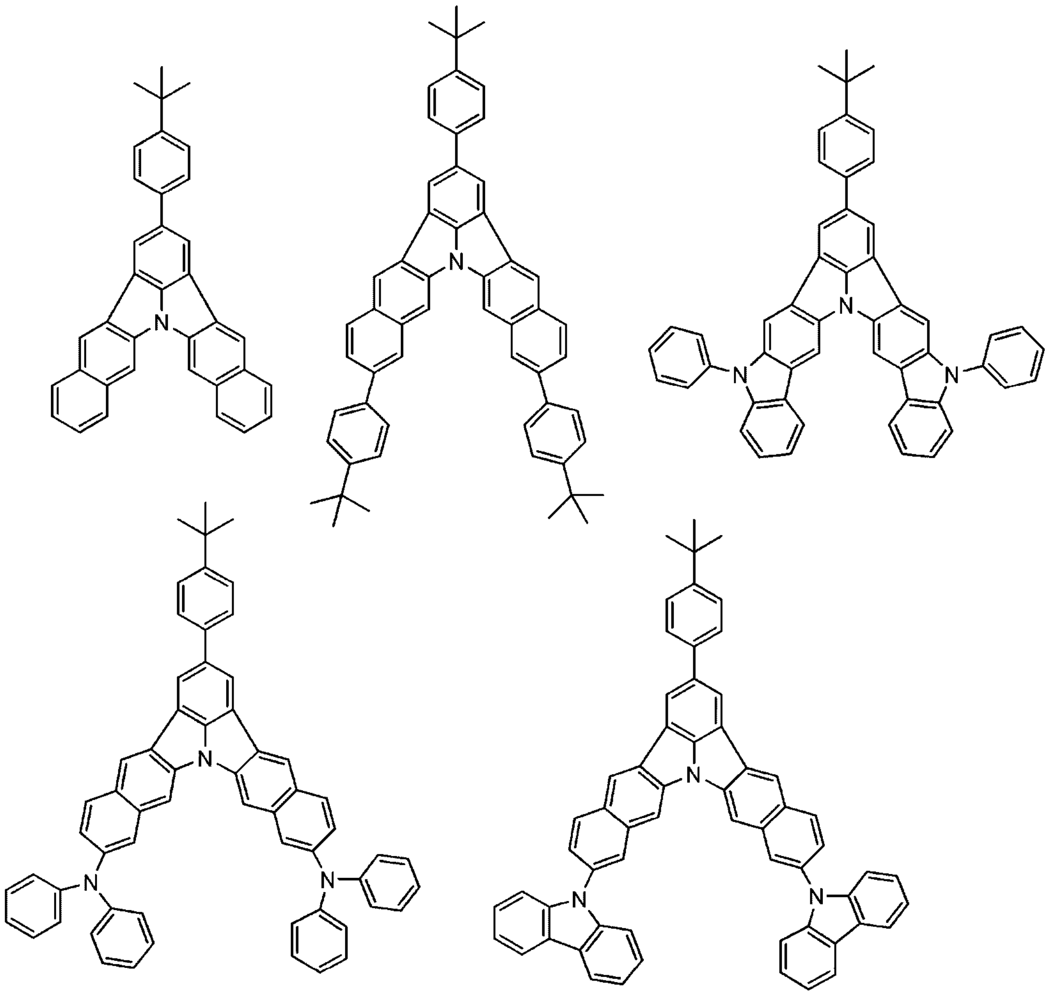

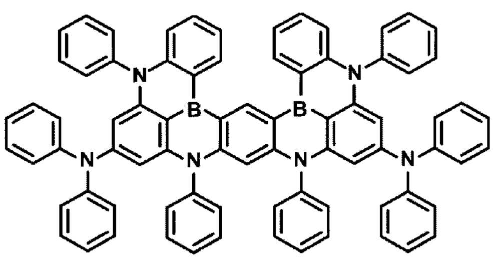

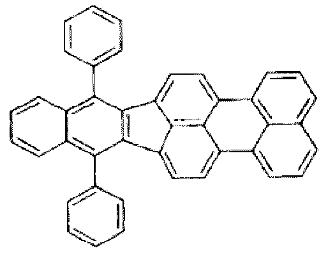

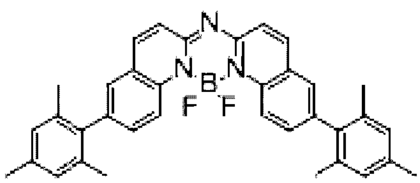

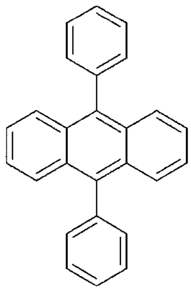
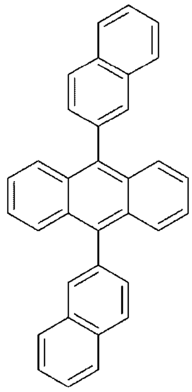
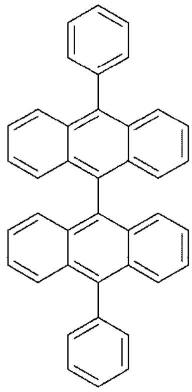
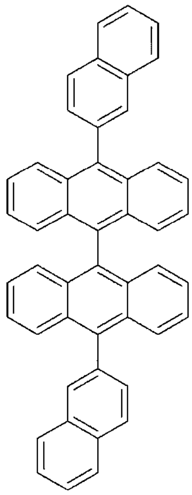
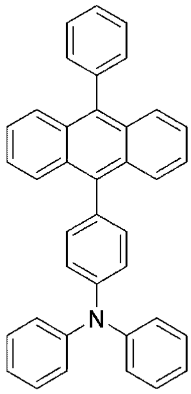
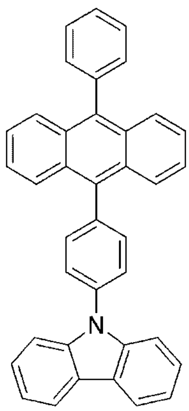
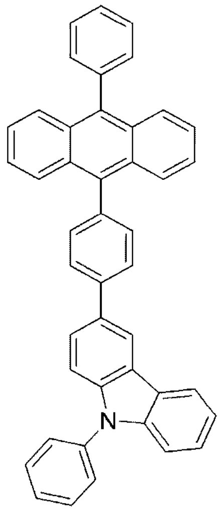
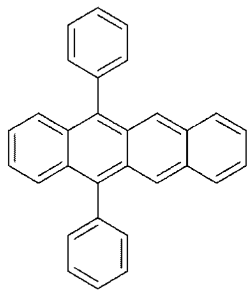
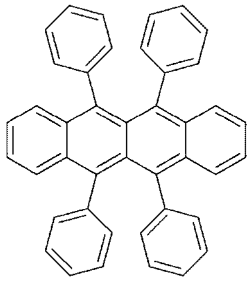
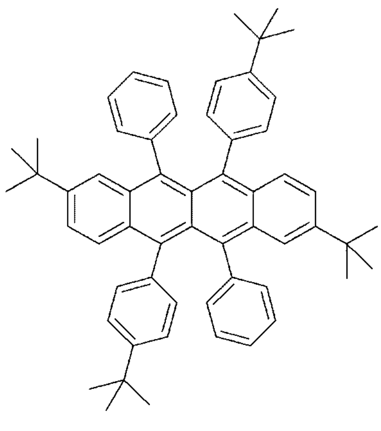
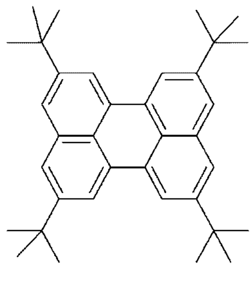

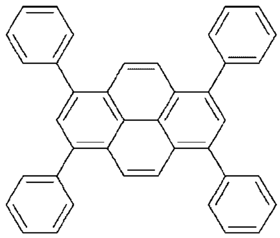
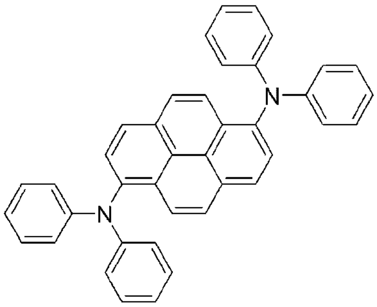
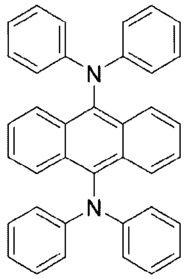
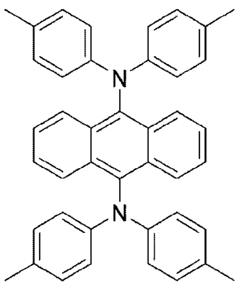
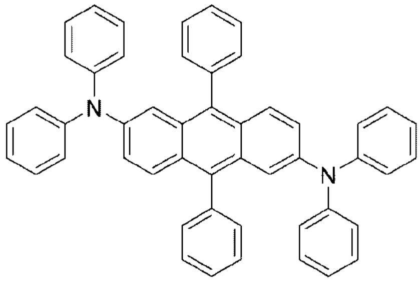
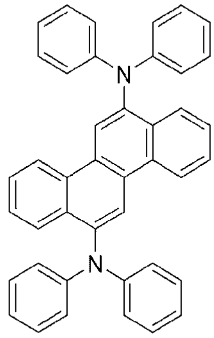
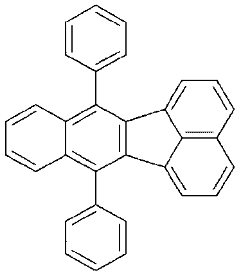
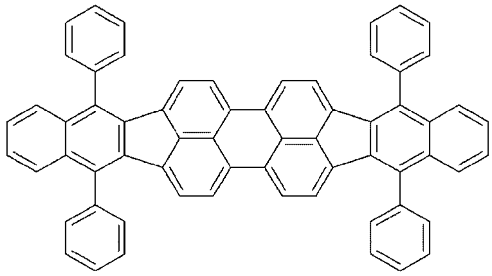
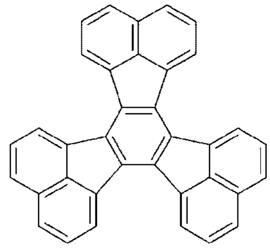
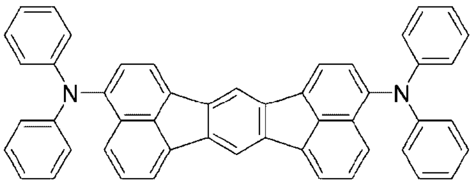
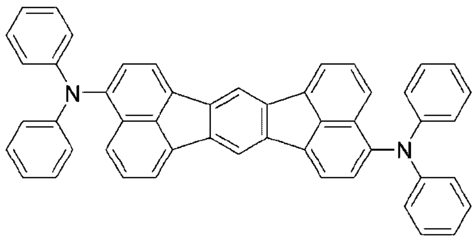
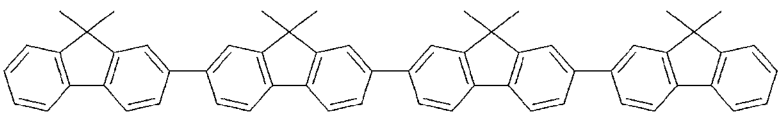
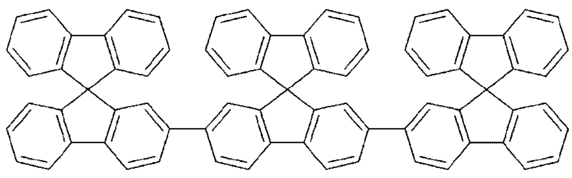
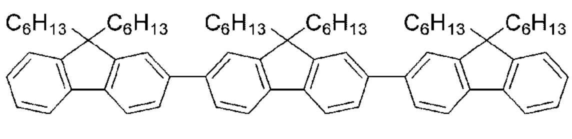

In addition to the above, the compounds described in paragraphs 0220 to 0239 of WO2015/022974 can also be employed as the light-emitting material used together with the assist dopant having the structure represented by the general formula (1).

As the dopant material (light-emitting material) which can be used in combination with the compound represented by the general formula (1), in particular, a compound represented by the following general formula (4) can be preferably used.

General Formula (4)

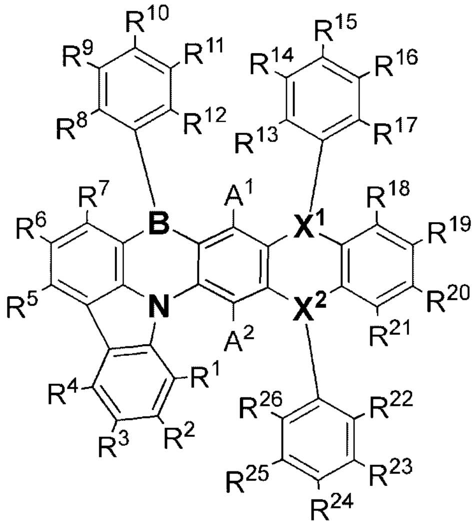

In the general formula (4), one of $X^1$ and $X^2$ is a nitrogen atom, and the other is a boron atom. In one aspect of the present invention, $X^1$ is a nitrogen atom, and $X^2$ is a boron atom. In that case, $R^{17}$ and $R^{18}$ are bonded to each other to form a single bond so as to form a pyrrole ring. In another aspect of the present invention, $X^1$ is a boron atom, and $X^2$ is a nitrogen atom. In that case, $R^{21}$ and $R^{22}$ are bonded to each other to form a single bond so as to form a pyrrole ring.

In the general formula (4), $R^1$ to $R^{26}$, $A^1$, and $A^2$ each independently represent a hydrogen atom, a deuterium atom, or a substituent.

$R^1$ and $R^2$, $R^2$ and $R^3$, $R^3$ and $R^4$, $R^4$ and $R^5$, $R^5$ and $R^6$, $R^6$ and $R^7$, $R^7$ and $R^8$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{22}$, $R^{22}$ and $R^{23}$, $R^{23}$ and $R^{24}$, $R^{24}$ and $R^{25}$, and $R^{25}$ and $R^{26}$ each may be bonded to each other to form a cyclic structure;

The cyclic structure formed by bonding $R^7$ and $R^8$ to each other includes a boron atom and four carbon atoms as ring skeleton-constituting atoms. The cyclic structure formed by bonding $R^{17}$ and $R^{18}$ to each other includes a boron atom and four carbon atoms as ring skeleton-constituting atoms when $X^1$ is a boron atom. When $X^1$ is a nitrogen atom, the cyclic structure is limited to a pyrrole ring. The cyclic structure formed by bonding $R^{21}$ and $R^{22}$ to each other includes a boron atom and four carbon atoms as ring skeleton-constituting atoms when $X^2$ is a boron atom. When $X^2$ is a nitrogen atom, the cyclic structure is limited to a pyrrole ring. When $R^7$ and $R^8$, $R^{17}$ and $R^{18}$, and $R^{21}$ and $R^{22}$ are bonded to each other to form boron atom-containing cyclic structures, the cyclic structure is preferably a 5 to 7-membered ring, more preferably a 5 or 6-membered ring, and still more preferably a 6-membered ring. When $R^7$ and $R^8$, $R^{17}$ and $R^{18}$, and $R^{21}$ and $R^{22}$ are bonded to each other, these preferably form a single bond, -O-, -S-, -N($R^{27}$)-, - C($R^{28}$)($R^{29}$)-, -Si($R^{30}$)($R^{31}$)-, -B($R^{32}$)-, -CO-, or -CS- by bonding to each other, more preferably form -O-, -S-, or -N($R^{27}$)-, and still more preferably form -N($R^{27}$)-. Here, $R^{27}$ to $R^{32}$ each independently represent a hydrogen atom, a deuterium atom, or a substituent. As the substituent, a group selected from any of Substituent Groups A to E may be employed, but a substituted or unsubstituted alkyl group, a substituted or unsubstituted aryl group, or a substituted or unsubstituted heteroaryl group is preferable. In particular, $R^{27}$ is preferably a substituted or unsubstituted aryl group. When $R^{27}$ to $R^{32}$ are substituents, $R^{27}$ to $R^{32}$ in the ring formed by bonding $R^7$ and $R^8$ to each other may further form a cyclic structure by bonding to at least one of $R^6$ and $R^9$, $R^{27}$ to $R^{32}$ in the ring formed by bonding $R^{17}$ and $R^{18}$ to each other may further form a cyclic structure by bonding to at least one of $R^{16}$ and $R^{19}$, and $R^{27}$ to $R^{32}$ in the ring formed by bonding $R^{21}$ and $R^{22}$ to each other may further form a cyclic structure by bonding to at least one of $R^{20}$ and $R^{23}$. In one aspect of the present invention, only one of the pairs $R^7$ and $R^8$, $R^{17}$ and $R^{18}$, and $R^{21}$ and $R^{22}$ are bonded to each other. In one aspect of the present invention, only two of the pairs $R^7$ and $R^8$, $R^{17}$ and $R^{18}$, and $R^{21}$ and $R^{22}$ are bonded to each other. In one aspect of the present invention, all of $R^7$ and $R^8$, $R^{17}$ and $R^{18}$, and $R^{21}$ and $R^{22}$ are bonded to each other.

The cyclic structure formed by bonding $R^1$ and $R^2$, $R^2$ and $R^3$, $R^3$ and $R^4$, $R^4$ and $R^5$, $R^5$ and $R^6$, $R^6$ and $R^7$, $R^8$ and

$R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$ to each other may be an aromatic ring or an aliphatic ring, or may contain a hetero atom, and further may be fused with at least one other ring. The hetero atom mentioned herein is preferably one selected from the group consisting of a nitrogen atom, an oxygen atom, and a sulfur atom. Examples of the cyclic structure to be formed include a benzene ring, a pyridine ring, a pyridazine ring, a pyrimidine ring, a pyrazine ring, a pyrrole ring, an imidazole ring, a pyrazole ring, a triazole ring, an imidazoline ring, a furan ring, a thiophene ring, an oxazole ring, an isoxazole ring, a thiazole ring, an isothiazole ring, a cyclohexadiene ring, a cyclohexene ring, a cyclopentene ring, a cycloheptatriene ring, a cycloheptadiene ring, a cycloheptene ring, and a ring in which one or more rings selected from the group consisting of these rings are further fused. In one preferred aspect of the present invention, the cyclic structure is a substituted or unsubstituted benzene ring (further, a ring may be fused), and is for example, a benzene ring which may be substituted with an alkyl group or an aryl group. In one preferred aspect of the present invention, the cyclic structure is a substituted or unsubstituted heteroaromatic ring, and preferably a furan ring of benzofuran, or a thiophene ring of benzothiophene. Among $R^1$ and $R^2$, $R^2$ and $R^3$, $R^3$ and $R^4$ , $R^4$ and $R^5$, $R^5$ and $R^6$, $R^6$ and $R^7$, $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{18}$ and $R^{19}$, $R^{19}$ and $R^{20}$, $R^{20}$ and $R^{21}$, $R^{22}$ and $R^{23}$, $R^{23}$ and $R^{24}$, $R^{24}$ and $R^{25}$, and $R^{25}$ and $R^{26}$, the number of combinations that are bonded to each other to form a cyclic structure may be 0, or may be, for example, any one of 1 to 6. For example, the number may be any one of 1 to 4, and 1 can be selected, 2 can be selected, or 3 or 4 can be selected. In one aspect of the present invention, a pair selected from $R^1$ and $R^2$, $R^2$ and $R^3$, and $R^3$ and $R^4$ are bonded to each other to form a cyclic structure. In one aspect of the present invention, $R^5$ and $R^6$ are bonded to each other to form a cyclic structure. In one aspect of the present invention, a pair selected from $R^9$ and $R^{10}$, $R^{10}$ and $R^{11}$, and $R^{11}$ and $R^{12}$ are bonded to each other to form a cyclic structure. In one aspect of the present invention, both $R^1$ and $R^2$, and $R^{13}$ and $R^{14}$ are bonded to each other to form a cyclic structure. In one aspect of the present invention, a pair selected from $R^1$ and $R^2$, $R^2$ and $R^3$, and $R^3$ and $R^4$ are bonded to each other to form a cyclic structure, and moreover $R^5$ and $R^6$ are bonded to each other to form a cyclic structure. In one aspect of the present invention, both $R^5$ and $R^6$, and $R^{19}$ and $R^{20}$ are bonded to each other to form a cyclic structure.

$R^1$ to $R^{26}$ which are not bonded to adjacent $R^n$ (n=1 to 26) each other are hydrogen atoms, deuterium atoms, or substituents. As the substituent, a group selected from any of Substituent Groups A to E can be employed.

Preferred substituents that $R^1$ to $R^{26}$ can adopt are substituted or unsubstituted alkyl groups, substituted or unsubstituted aryl groups, and substituted or unsubstituted heteroaryl groups, and for example, the substituent may be a substituted or unsubstituted aryl group, and for example, the substituent may be a substituted or unsubstituted alkyl group. As the substituent of the alkyl group, the aryl group, or the heteroaryl group mentioned herein, a group selected from any of Substituent Groups A to E can also be employed. Meanwhile, one or more groups selected from the group consisting of an alkyl group, an aryl group, and a heteroaryl group are preferred, and a group of Substituent Group E is more preferred, and it may be unsubstituted. In one preferred aspect of the present invention, at least one of $R^1$ to $R^6$ is a substituent, and preferably a group of Substituent Group E. For example, at least one of $R^2$ to $R^6$ is a substituent, and preferably a group of Substituent Group E. For example, at least one of $R^5$ and $R^6$ is a substituent, and preferably a group of Substituent Group E. In one preferred aspect of the present invention, at least one of $R^3$ and $R^6$ is a substituent, more preferably both are substituents, and a group of Substituent Group E is preferred. In one preferred aspect of the present invention, when $X^1$ is a nitrogen atom, at least one of $R^{15}$ and $R^{20}$ is a substituent, more preferably both are substituents, and a group of Substituent Group E is preferred. Here, $R^{17}$ and $R^{18}$ are bonded to each other to form a single bond. In one preferred aspect of the present invention, when $X^2$ is a nitrogen atom, at least one of $R^{19}$ and $R^{24}$ is a substituent, more preferably both are substituents, and a group of Substituent Group E is preferred. Here, $R^{21}$ and $R^{22}$ are bonded to each other to form a single bond. In one aspect of the present invention, at least one of $R^8$ and $R^{12}$ is a substituent, and preferably both are substituents. In one aspect of the present invention, $R^8$, $R^{10}$ and $R^{12}$ are substituents. As the substituent of $R^8$ to $R^{12}$, an unsubstituted alkyl group is preferable. In particular, the case where $R^8$ and $R^{12}$ are alkyl groups having 2 or more carbon atoms (preferably alkyl groups having 3 or more carbon atoms, more preferably alkyl groups having 3 to 8 carbon atoms, further preferably alkyl groups having 3 or 4 carbon atoms) is preferable because orientation becomes high when a film is formed. Among them, particularly preferred is a case where $R^8$ and $R^{12}$ are substituents (preferably alkyl groups, more preferably alkyl groups having 2 or more carbon atoms, further preferably alkyl groups having 3 or more carbon atoms, still further preferably alkyl groups having 3 to 8 carbon atoms, particularly preferably alkyl groups having 3 or 4 carbon atoms), and moreover, at least one of $R^1$ to $R^6$ is a substituent (preferably a group of Substituent Group E). When $X^1$ is a boron atom, at least one of $R^{13}$ and $R^{17}$ is a substituent, and preferably both are substituents. In one aspect of the present invention, when $X^1$ is a boron atom, $R^{13}$, $R^{15}$, and $R^{17}$ are substituents. When $X^1$ is a boron atom, as the substituent of $R^{13}$ to $R^{17}$, an unsubstituted alkyl group is preferable. When $X^2$ is a boron atom, at least one of $R^{22}$ and $R^{26}$ is a substituent, and preferably both are substituents. In one aspect of the present invention, when $X^2$ is a boron atom, $R^{22}$, $R^{24}$, and $R^{26}$ are substituents. When $X^2$ is a boron atom, as the substituent of $R^{22}$ to $R^{26}$, an unsubstituted alkyl group is preferable. Specific examples of the group that is bonded to the boron atom indicated as B or the boron atom represented by $X^1$ or $X^2$ in the general formula (4) are shown below. Here, groups bonded to the boron atom, which can be employed in the present invention, should not be construed as being limited by the following specific examples. In the description herein, the

expression of $CH_3$ is omitted for a methyl group. * represents a bonding position.

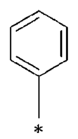 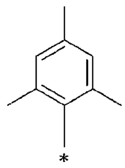 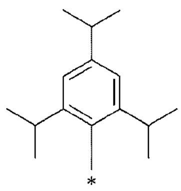 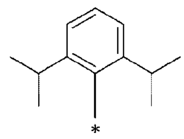 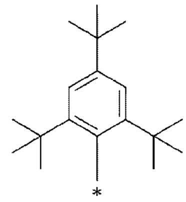

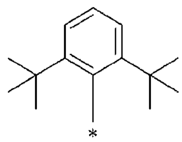 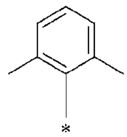 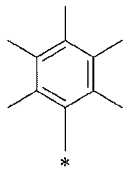

Specific examples of $R^1$ to $R^{26}$ in the general formula (4) are shown below. Y1 to Y9 are preferable as $R^1$ to $R^7$, as $R^{13}$ to $R^{21}$ when $X^1$ is a nitrogen atom, and as $R^{18}$ to $R^{26}$ when $X^2$ is a nitrogen atom, and Y1 to Y7 are preferable as $R^8$ to $R^{12}$, as $R^{22}$ to $R^{26}$ when $X^1$ is a nitrogen atom, and as $R^{13}$ to $R^{17}$ when $X^2$ is a nitrogen atom. Here, groups bonded to the boron atom, which can be employed in the present invention, should not be construed as being limited by the following specific examples. D represents a deuterium atom. * represents a bonding position.

*-H Y 1

*-D Y 2

*-$CH_3$

Y 3

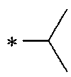 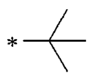 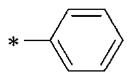

Y 4 Y 5 Y 6

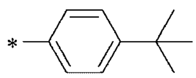 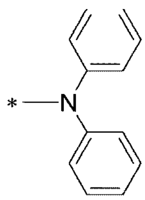 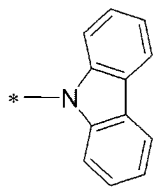

Y 7 Y 8 Y 9

$A^1$ and $A^2$ are hydrogen atoms, deuterium atoms, or substituents. As the substituent, a group selected from any of Substituent Groups A to E can be employed.

In one preferred aspect of the present invention, $A^1$ and $A^2$ are each independently a hydrogen atom or a deuterium atom. For example, $A^1$ and $A^2$ are hydrogen atoms. For example, $A^1$ and $A^2$ are deuterium atoms.

One of $A^1$ and $A^2$ may be a substituent. Further, $A^1$ and $A^2$ may be each independently a substituent. A preferable substituent that $A^1$ and $A^2$ can adopt is an acceptor group. The acceptor group is a group having a positive Hammett's $\sigma p$ value.

The acceptor group that $A^1$ and $A^2$ can adopt is more preferably a group having a Hammett's $\sigma p$ value greater than 0.2. Examples of the group having a Hammett's $\sigma p$ value greater than 0.2 include a cyano group, an aryl group substituted with at least a cyano group, a fluorine atom-containing group, and a substituted or unsubstituted heteroaryl group containing a nitrogen atom as a ring skeleton-constituting atom. The aryl group substituted with at least a cyano group, which is mentioned herein, may be substituted with a substituent other than the cyano group (for example, an alkyl

group or an aryl group), but may be an aryl group substituted with only a cyano group. The aryl group substituted with at least a cyano group is preferably a phenyl group substituted with at least a cyano group. The number of substitutions of the cyano group is preferably one or two, and, for example, may be one, or may be two. As the fluorine atom-containing group, a fluorine atom, a fluoroalkyl group, and an aryl group substituted with at least a fluorine atom or a fluoroalkyl group may be mentioned. The fluoroalkyl group is preferably a perfluoroalkyl group, and the number of carbon atoms is preferably 1 to 6, and more preferably 1 to 3. Further, the heteroaryl group containing a nitrogen atom as a ring skeleton-constituting atom may be a monocyclic ring, or may be a fused ring in which two or more rings are fused. In the case of a fused ring, the number of rings after fusing is preferably 2 to 6, and, for example, can be selected from 2 to 4, or can be 2. Specific examples of the ring constituting the heteroaryl group include a pyridine ring, a pyrimidine ring, a pyrazine ring, a triazine ring, a quinoline ring, an isoquinoline ring, a quinazoline ring, a quinoxaline ring, and a naphthyridine ring other than the quinazoline ring or the quinoxaline ring. The ring constituting the heteroaryl group may be substituted with a deuterium atom or a substituent, and examples of the substituent include one group or a group formed by combining two or more groups selected from the group consisting of an alkyl group, an aryl group, and a heteroaryl group. As the acceptor group that $A^1$ and $A^2$ can adopt, a cyano group is particularly preferable.

In one aspect of the present invention, at least one of $A^1$ and $A^2$ is an acceptor group. In one aspect of the present invention, only one of $A^1$ and $A^2$ is an acceptor group. In one aspect of the present invention, both $A^1$ and $A^2$ are the same acceptor groups. In one aspect of the present invention, $A^1$ and $A^2$ are different acceptor groups. In one aspect of the present invention, $A^1$ and $A^2$ are cyano groups. In one aspect of the present invention, $A^1$ and $A^2$ are halogen atoms, for example, bromine atoms.

Hereinafter, specific examples of the acceptor group that can be employed in the present invention will be illustrated. Here, the acceptor group that can be used in the present invention shall not be construed as being limited by the following specific examples. In the description herein, the expression of $CH_3$ is omitted for a methyl group. Thus, for example, A15 indicates a group including two 4-methylphenyl groups. Further, "D" represents a deuterium atom. * represents a bonding position.

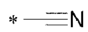 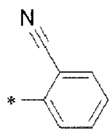 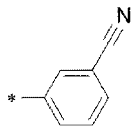 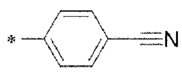 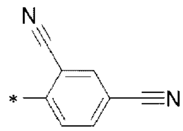 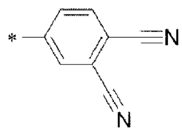

A 1 A 2 A 3 A 4 A 5 A 6

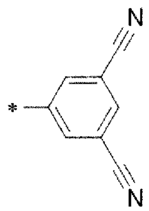 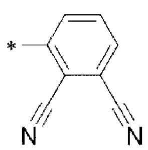 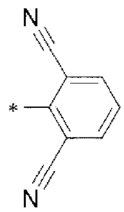 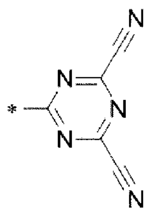 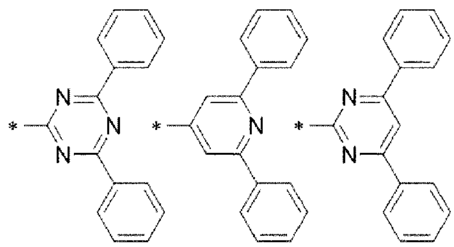

A 7 A 8 A 9 A 1 0 A 1 1 A 1 2 A 1 3

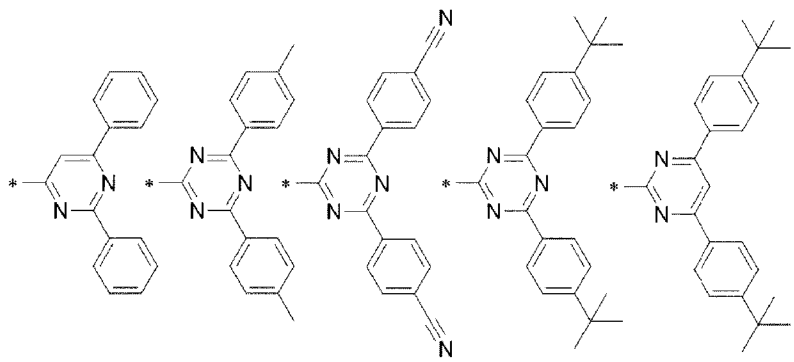

A 1 4 A 1 5 A 1 6 A 1 7 A 1 8

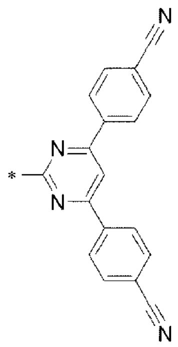 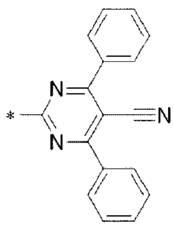 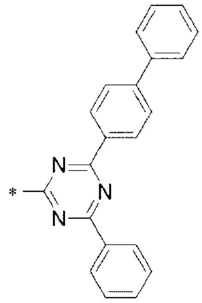 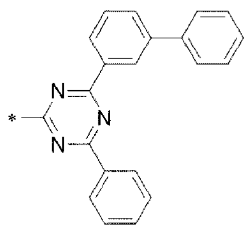

A 1 9　　A 2 0　　A 2 1　　A 2 2

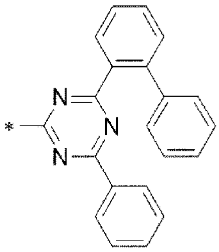 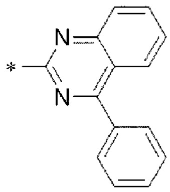 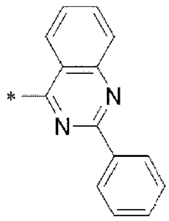

A 2 3　　A 2 4　　A 2 5

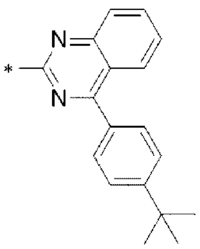 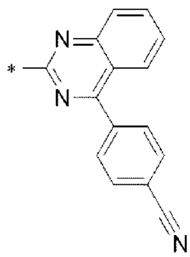 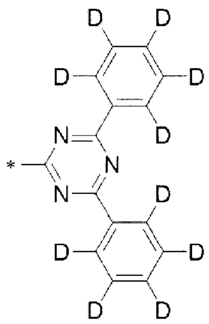 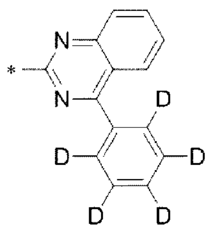

A 2 6　　A 2 7　　A 2 8　　A 2 9

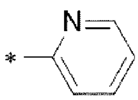 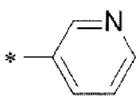 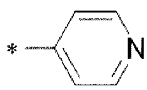 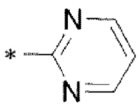 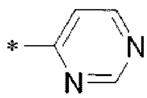

A 3 0　　A 3 1　　A 3 2　　A 3 3　　A 3 4

*-$CF_3$　　A 3 5

*-$C_2F_5$　　A36

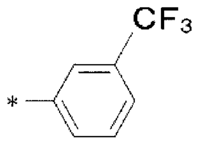 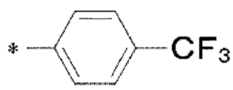

A 3 7 A 3 8

*-F A 3 9

*-Cl A40

*-Br A41

*-I A 4 2

When $X^1$ is a nitrogen atom, $R^7$ and $R^8$ are bonded via a nitrogen atom to form a 6-membered ring, $R^{21}$ and $R^{22}$ are bonded via a nitrogen atom to form a 6-membered ring, and $R^{17}$ and $R^{18}$ are bonded to each other to form a single bond, at least one of $R^1$ to $R^6$ is a substituted or unsubstituted aryl group, or any of $R^1$ and $R^2$, $R^2$ and $R^3$, $R^3$ and $R^4$, $R^4$ and $R^5$, and $R^5$ and $R^6$ are bonded to each other to form an aromatic ring (a substituted or unsubstituted benzene ring which may be fused) or a heteroaromatic ring (preferably a substituted or unsubstituted furan ring of benzofuran which may be fused, or a substituted or unsubstituted thiophene ring of benzothiophene which may be fused).

Further, when $X^1$ is a boron atom, $X^2$ is a nitrogen atom, and $R^7$ and $R^8$, and $R^{17}$ and $R^{18}$ are each bonded to each other to form a cyclic structure containing a boron atom, the cyclic structure is a 5 to 7-membered ring, and in the case of a 6-membered ring, $R^7$ and $R^8$, and $R^{17}$ and $R^{18}$ are each bonded to each other to form -B($R^{32}$)-, -CO-, -CS- or - N($R^{27}$)-. $R^{27}$ preferably represents a hydrogen atom, a deuterium atom, or a substituent.

With respect to details and specific examples of the compound represented by the general formula (4), reference can be made to [0019] to [0128] of WO2022/270354A1, which are incorporated as a part of this description by reference.

In some embodiments, the light-emitting layer contains two or more kinds of TADF molecules differing in the structure. For example, the light-emitting layer can contain three kinds of materials of a host material, a first TADF molecule, and a second TADF molecule whose excited singlet energy level is higher in that order. In that case, both the first TADF molecule and the second TADF molecule are preferably such that the difference $\Delta E_{ST}$ between the lowest excited singlet energy level and the lowest excited triplet energy level at 77 K is 0.3 eV or less, more preferably 0.25 eV or less, still more preferably 0.2 eV or less, even more preferably 0.15 eV or less, further preferably 0.1 eV or less, further more preferably 0.07 eV or less, further more preferably 0.05 eV or less, further more preferably 0.03 eV or less, and particularly preferably 0.01 eV or less. The content of the first TADF molecule in the light-emitting layer is preferably larger than the content of the second TADF molecule therein. The content of the host material in the light-emitting layer is preferably larger than the content of the second TADF molecule therein. The content of the first TADF molecule in the light-emitting layer may be larger than or may be smaller than or may be the same as the content of the host material therein. In some embodiments, the composition in the light-emitting layer may be 10% by weight to 70% by weight of a host material, 10% by weight to 80% by weight of a first TADF molecule, and 0.1% by weight to 30% by weight of a second TADF molecule. In some embodiments, the composition in the light-emitting layer may be 20% by weight to 45% by weight of a host material, 50% by weight to 75% by weight of a first TADF molecule, and 5% by weight to 20% by weight of a second TADF molecule. In some embodiments, the photoluminescence quantum yield $\varphi$PL1(A) by photo-excitation of a co-deposited film of a first TADF molecule and a host material (the content of the first TADF molecule in the co-deposited film = A% by weight) and the photoluminescence quantum yield $\varphi$PL2(A) by photo-excitation of a co-deposited film of a second TADF molecule and a host material (the content of the second TADF molecule in the co-deposited film = A% by weight) satisfy a relational formula $\varphi$PL1(A) > $\varphi$PL2(A). In some embodiments, the photoluminescence quantum yield $\varphi$PL2(B) by photo-excitation of a co-deposited film of a second TADF molecule and a host material (the content of the second TADF molecule in the co-deposited film = B% by weight) and the photoluminescence quantum yield $\varphi$PL2(100) by photo-excitation of a single film of a second TADF molecule satisfy a relational formula $\varphi$PL2(B) > $\varphi$PL2(100). In some embodiments, the light-emitting layer can contain three kinds of TADF molecules differing in the structure. The compound of the present invention may be any of the plural TADF compounds contained in the light-emitting layer.

In some embodiments, the light-emitting layer can be composed of materials selected from the group consisting of a host material, an assist dopant and a light-emitting material. In some embodiments, the light-emitting layer does not contain a metal element. In some embodiments, the light-emitting layer can be formed of a material composed of atoms alone selected from the group consisting of a carbon atom, a hydrogen atom, a deuterium atom, a nitrogen atom, an

oxygen atom, and a sulfur atom. Alternatively, the light-emitting layer can be formed of a material composed of atoms alone selected from the group consisting of a carbon atom, a hydrogen atom, a deuterium atom, a nitrogen atom, and an oxygen atom. Alternatively, the light-emitting layer can be formed of a material composed of atoms alone selected from the group consisting of a carbon atom, a hydrogen atom, a nitrogen atom, and an oxygen atom.

In the case where the light-emitting layer contains any other TADF material than the compound of the present invention, the TADF material may be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073 to 0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101 to 0121; JP 2014-9352 A, paragraphs 0007 to 0041 and 0060 to 0069; JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP 2018-100411 A, paragraphs 0012 to 0043; WO2018/047853, paragraphs 0016 to 0044; and especially, exemplary compounds therein capable of emitting delayed fluorescence. In addition, also preferably employable here are light-emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541, and WO2015/159541. These patent publications described in this paragraph are hereby incorporated as a part of this description by reference.

(Host Material)

When the compound represented by the general formula (1) is used together with a host material, the amount of the compound represented by the general formula (1) is 0.1% by weight or more. In some embodiments, in a case where a host material is used, the amount of the compound represented by the general formula (1) contained in a light-emitting layer as a light-emitting material is 1% by weight or more. In some embodiments, in a case where a host material is used, the amount of the compound represented by the general formula (1) contained in a light-emitting layer as a light-emitting material is 50% by weight or less. In some embodiments, in a case where a host material is used, the amount of the compound represented by the general formula (1) contained in a light-emitting layer as a light-emitting material is 20% by weight or less. In some embodiments, in a case where a host material is used, the amount of the compound represented by the general formula (1) contained in a light-emitting layer as a light-emitting material is 10% by weight or less.

In some embodiments, the host material in a light-emitting layer is an organic compound having a hole transporting capability and an electron transporting capability. In some embodiments, the host material in a light-emitting layer is an organic compound that prevents increase in the wavelength of emitted light. In some embodiments, the host material in a light-emitting layer is an organic compound having a high glass transition temperature.

In some embodiments, the host material is selected from the group consisting of the followings:

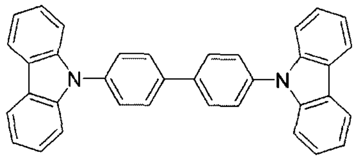
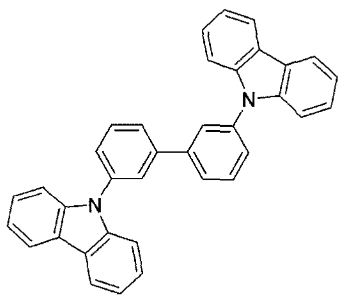
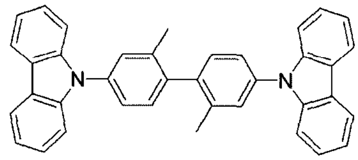

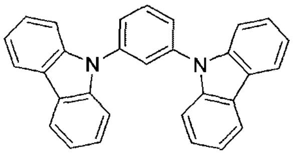
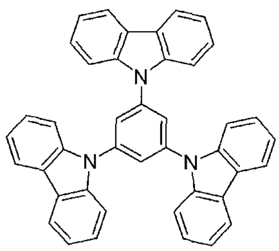
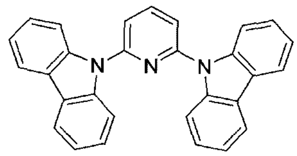

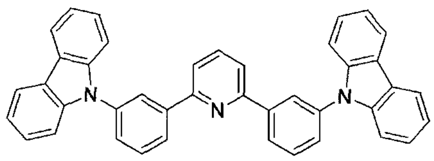
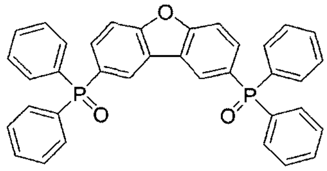

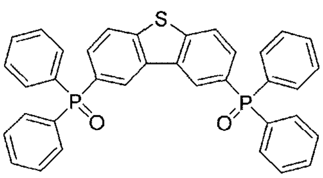
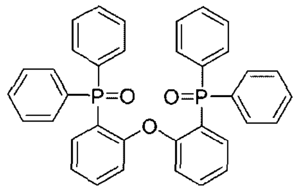
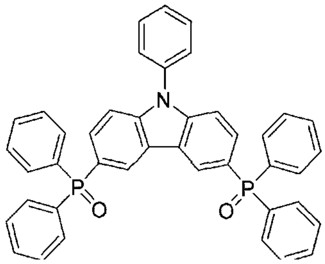

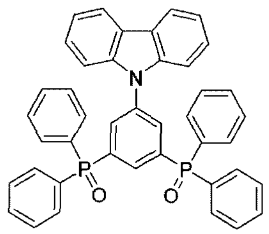
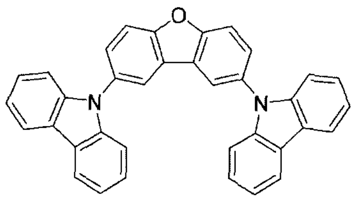
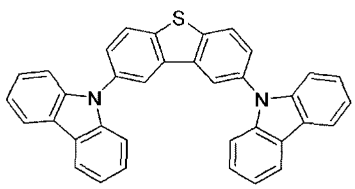

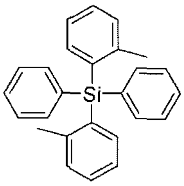
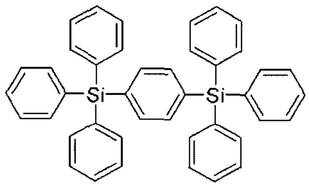
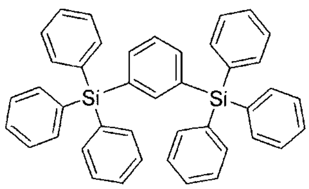
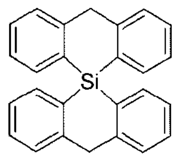

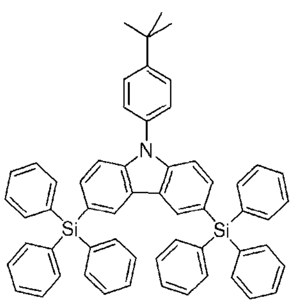
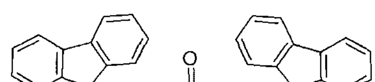
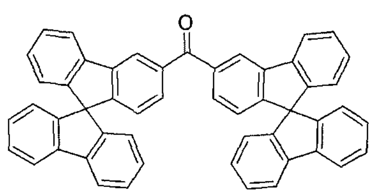

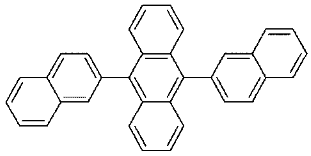
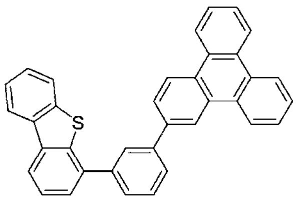
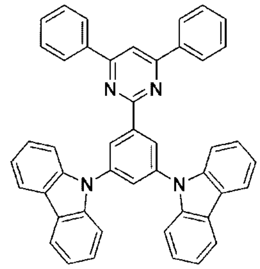

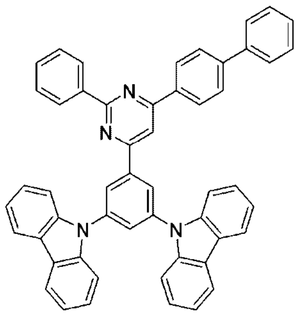
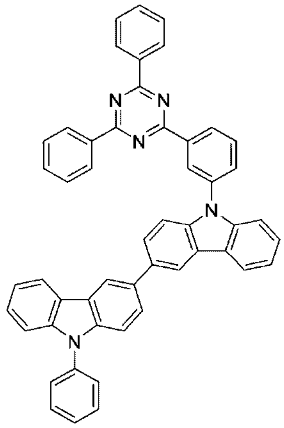
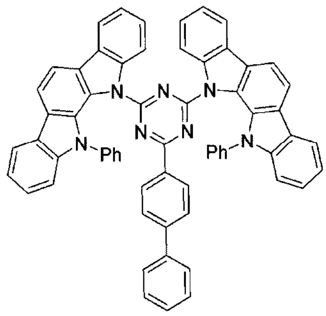

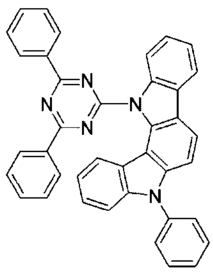
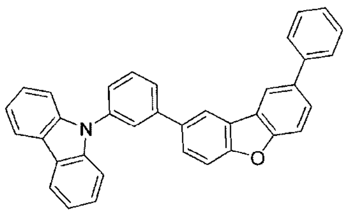
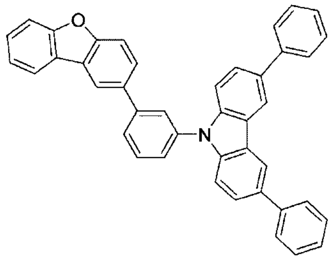

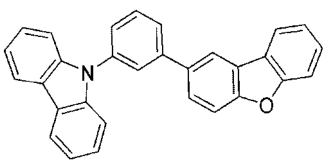
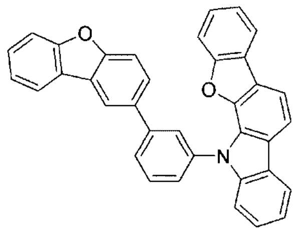
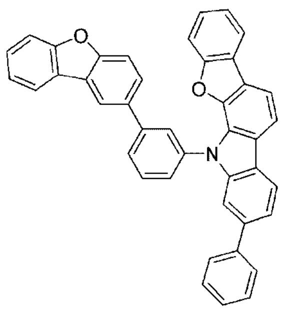

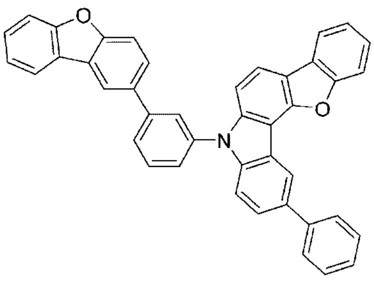
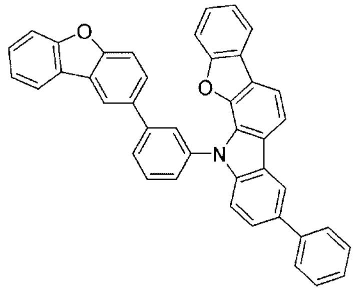
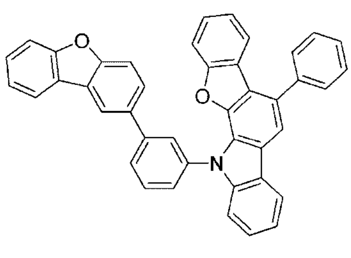

In one preferred aspect of the present invention, the host material used together with the compound represented by the general formula (1) is a compound having a structure represented by the following general formula (5).

General Formula (5)

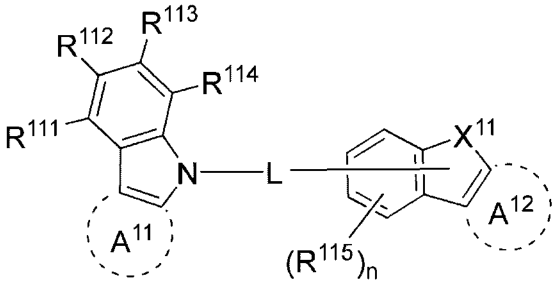

In the general formula (5), $X^{11}$ represents O, S, $N(R^A)$, or $C(R^B)(R^C)$. In one aspect of the present invention, $X^{11}$ is O, S, or $N(R^A)$. In one aspect of the present invention, $X^{11}$ is O or S. In one aspect of the present invention, $X^{11}$ is $N(R^A)$. In one aspect of the present invention, $X^{11}$ is O. In one aspect of the present invention, $X^{11}$ is S. When $X^{11}$ is O, S, or $C(R^B)(R^C)$, L is bonded to a benzene ring to which $(R^{115})n$ is bonded. When $X^{11}$ is $N(R^A)$, L is bonded to a benzene ring to which $(R^{115})n$ is bonded or N represented by $X^{11}$. As described herein, a bond elongating from L to the right side means being bonded to a benzene ring to which $(R^{115})n$ is bonded, or being bonded to $X^{11}$ (that is, N) when $X^{11}$ is N.

In the general formula (5), $A^{11}$ and $A^{12}$ are each independently a benzene ring, a furan ring, a thiol ring, a pyrrole ring, or a cyclopentadiene ring, which may have other rings further fused in such rings or may be substituted. In one preferred aspect of the present invention, $A^{11}$ is a benzene ring. In one preferred aspect of the present invention, $A^{12}$ is a benzene ring. In a further preferred aspect of the present invention, both $A^{11}$ and $A^{12}$ are benzene rings. In one aspect of the present invention, at least one of $A^{11}$ and $A^{12}$ is a furan ring, a thiol ring, a pyrrole ring, or a cyclopentadiene ring. In one aspect of the present invention, at least one of $A^{11}$ and $A^{12}$ is furan ring. In one aspect of the present invention, at least one of $A^{11}$ and $A^{12}$ is a thiol ring. In one aspect of the present invention, at least one of $A^{11}$ and $A^{12}$ is a pyrrole ring. In one aspect of the present invention, at least one of $A^{11}$ and $A^{12}$ is a cyclopentadiene ring.

The benzene ring, the furan ring, the thiol ring, the pyrrole ring, and the cyclopentadiene ring mentioned herein may have another ring further fused. The fused ring may be any of an aromatic hydrocarbon ring, an aromatic heterocyclic ring, an aliphatic hydrocarbon ring, and an aliphatic heterocyclic ring, or may be a ring in which two or more of these rings are fused. Preferably, the fused ring is an aromatic hydrocarbon ring, an aromatic heterocyclic ring, or a ring in which two or more of these rings are fused. Examples of the aromatic hydrocarbon ring include a benzene ring. The aromatic heterocyclic ring means a ring containing a hetero atom as a ring skeleton-constituting atom and exhibiting aromaticity, and is preferably a 5- to 7-membered ring, and for example, a 5-membered ring or a 6-membered ring can be employed. In one aspect of the present invention, a furan ring, a thiophene ring, or a pyrrole ring can be employed as the aromatic heterocyclic ring. The aliphatic hydrocarbon ring is preferably a hydrocarbon ring that does not exhibit aromaticity, and is preferably a 5- to 7-membered ring, and for example, a 5-membered ring or a 6-membered ring can be employed. For example, a cyclopentadiene ring can be employed. The aliphatic heterocyclic ring means a ring containing a hetero atom as a ring skeleton-constituting atom and not exhibiting aromaticity, and is preferably a 5- to 7-membered ring, and for example, a 5-membered ring or a 6-membered ring can be employed.

In one aspect of the present invention, $A^{11}$ is a benzene ring, and in the benzene ring, a benzene ring, a furan ring, a thiol ring, a pyrrole ring, or a ring in which two or more of these rings are fused is further fused. In one aspect of the present invention, $A^{11}$ is a benzene ring, and in the benzene ring, a benzene ring, a furan ring, a thiol ring, or a ring in which two or more of these rings are fused is further fused. In one aspect of the present invention, $A^{11}$ is a benzene ring, and in the benzene ring, a furan ring of benzofuran or a thiophene ring of benzothiophene is fused. In one aspect of the present invention, $A^{11}$ is fused with a furan ring of benzofuran. In one aspect of the present invention, $A^{11}$ is fused with a thiophene ring of benzothiophene. In one aspect of the present invention, $A^{12}$ is a benzene ring, and in the benzene ring, a benzene ring, a furan ring, a thiol ring, a pyrrole ring, or a ring in which two or more of these rings are fused is further fused. In one aspect of the present invention, $A^{12}$ is a benzene ring, and in the benzene ring, a benzene ring, a furan ring, a thiol ring, or a ring in which two or more of these rings are fused is further fused. In one aspect of the present invention, $A^{12}$ is a benzene ring, and in the benzene ring, a furan ring of benzofuran or a thiophene ring of benzothiophene is fused. In one aspect of the present invention, $A^{12}$ is fused with a furan ring of benzofuran. In one aspect of the present invention, $A^{12}$ is fused with a thiophene ring of benzothiophene.

The hydrogen atoms of the ring constituting $A^{11}$ or $A^{12}$ may be substituted with a deuterium atom or a substituent. The substituent can be selected from any of Substituent Groups A to E, and for example, is selected from Substituent Group E. In one aspect of the present invention, the ring constituting $A^{11}$ or $A^{12}$ may be substituted with one atom or group selected from the group consisting of a deuterium atom, a substituted or unsubstituted aryl group, a substituted or unsubstituted heteroaryl group, a substituted or unsubstituted alkyl group, and a cyano group or with a group formed by

combining two or more thereof. In one aspect of the present invention, the ring constituting $A^{11}$ or $A^{12}$ may be substituted with a deuterium atom, an alkyl group, an aryl group, or a group formed by combining thereof. In one aspect of the present invention, the ring constituting $A^{11}$ or $A^{12}$ may be substituted with at least one of a deuterium atom, an alkyl group, an aryl group, and a group formed by combining thereof. In the case where a pyrrole ring is included as a ring constituting $A^{11}$ or $A^{12}$, it is preferable that an aryl group which may be substituted with a deuterium atom, an alkyl group, or an aryl group is bonded to the ring skeleton-constituting nitrogen atom of the pyrrole ring (the same applies to a nitrogen atom of an indole ring described below). When two or more hydrogen atoms of the ring constituting $A^{11}$ or $A^{12}$ are substituted, they may be substituted with the same atom or group, or may be substituted with different atoms or groups.

In the general formula (5), $R^{111}$ to $R^{114}$, $R^B$, and $R^C$ each independently represent a hydrogen atom, a deuterium atom, a substituted or unsubstituted aryl group, a substituted or unsubstituted heteroaryl group, a substituted or unsubstituted alkyl group, or a cyano group. Each of $R^{115}$'s independently represents a hydrogen atom, a deuterium atom, a substituted or unsubstituted aryl group, a substituted or unsubstituted heteroaryl group, a substituted or unsubstituted alkyl group, a cyano group, or a bond with L (that is, a single bond to L). $R^A$ represents a substituted or unsubstituted aryl group, a substituted or unsubstituted heteroaryl group, a substituted or unsubstituted alkyl group, or a bond with L (that is, a single bond to L). For the aryl group, the heteroaryl group, and the alkyl group, the description on the "aryl group", the "heteroaryl group", and the "alkyl group" can be referred to. The aryl group preferably has 6 to 14 carbon atoms, and examples thereof include a phenyl group, a 1-naphthyl group, and a 2-naphthyl group. The heteroaryl group is preferably composed of a 5-membered ring or a 6-membered ring, and examples thereof include a 2-pyridyl group, a 3-pyridyl group, a 4-pyridyl group, a carbazol-9-yl group, a dibenzofuryl group, and a dibenzothienyl group. The alkyl group preferably has 1 to 6 carbon atoms, and examples thereof include a methyl group, an ethyl group, an isopropyl group, and a tert-butyl group. Such aryl group, heteroaryl group, and alkyl group may be substituted, and in the case of being substituted, such groups are preferably substituted with one atom or group selected from the group consisting of a deuterium atom, an aryl group, a heteroaryl group, an alkyl group, and a cyano group, or a group formed by combining two or more thereof, and more preferably substituted with one atom or group selected from the group consisting of a deuterium atom, an aryl group, a heteroaryl group, and an alkyl group, or a group formed by combining two or more thereof. In one aspect of the present invention, $R^{112}$ is a substituted or unsubstituted aryl group, a substituted or unsubstituted heteroaryl group, a substituted or unsubstituted alkyl group, or a cyano group. In one aspect of the present invention, $R^{111}$ to $R^{114}$ are each independently a hydrogen atom or a deuterium atom.

$R^{111}$ and $R^{112}$, $R^{112}$ and $R^{113}$, $R^{113}$ and $R^{114}$, adjacent two $R^{115}$'s, and $R^B$ and $R^C$ each may be bonded to each other to form a cyclic structure. For the cyclic structure mentioned herein, the description on the ring further fused in the benzene ring described for $A^{11}$ and $A^{12}$ can be referred to. In one aspect of the present invention, a pair selected from (i) $R^{111}$ and $R^{112}$, (ii) $R^{112}$ and $R^{113}$, and (iii) $R^{113}$ and $R^{114}$ are bonded to each other to form a benzofuran ring (fused with a furan ring), a benzothiophene ring (fused with a thiophene ring), or an indole ring (fused with a pyrrole ring).

In one aspect of the present invention, a group bonded to L from the left side in the general formula (5) is a substituted or unsubstituted carbazol-9-yl group. For example, the group is a carbazol-9-yl group in which at least one (preferably both) of the 3-position and the 6-position is substituted with a deuterium atom, an alkyl group, an aryl group, or a group formed by combining thereof. Further, the group may be an unsubstituted carbazol-9-yl group. In one aspect of the present invention, the group bonded to L from the left side in the general formula (5) is a substituted or unsubstituted benzofuro[2,3-a]carbazol-12-yl group, a substituted or unsubstituted benzofuro[3,2-a]carbazol-12-yl group, a substituted or unsubstituted benzofuro[2,3-b]carbazol-7-yl group, a substituted or unsubstituted benzofuro[3,2-b]carbazol-11-yl group, a substituted or unsubstituted benzofuro[2,3-c]carbazol-8-yl group, or a substituted or unsubstituted benzofuro[3,2-c]carbazol-5-yl group. In one aspect of the present invention, the group bonded to L from the left side in the general formula (5) is a substituted or unsubstituted benzothieno[2,3-a]carbazol-12-yl group, a substituted or unsubstituted benzothieno[3,2-a]carbazol-12-yl group, a substituted or unsubstituted benzothieno[2,3-b]carbazol-7-yl group, a substituted or unsubstituted benzothieno[3,2-b]carbazol-11-yl group, a substituted or unsubstituted benzothieno[2,3-c]carbazol-8-yl group, or a substituted or unsubstituted benzothieno[3,2-c]carbazol-5-yl group. In one aspect of the present invention, the group bonded to L from the left side in the general formula (5) is a substituted or unsubstituted 11-phenylindolo[2,3-a]carbazol-12-yl group, a substituted or unsubstituted 5-phenylindolo[3,2-a]carbazol-12-yl group, a substituted or unsubstituted 5-phenylindolo[2,3-b]carbazol-7-yl group, a substituted or unsubstituted 5-phenylindolo[3,2-b]carbazol-11-yl group, a substituted or unsubstituted 5-phenylindolo[2,3-c]carbazol-8-yl group, or a substituted or unsubstituted 12-phenylindolo[3,2-a]carbazol-5-yl group.

In one aspect of the present invention, as a group bonded to L from the right side in the general formula (5), the group exemplified as the group bonded to L from the left side can be employed. Here, the group bonded to L from the right side is not an unsubstituted carbazol-9-yl group.

Specific examples of the group that can be adopted as the group bonded to L from the left side in the general formula (5) are shown below. Here, the groups that can be employed in the present invention shall not be construed as being limited by these specific examples. In the following specific examples, the expression of a methyl group is omitted. Therefore, for example, Z2 or Z3 is substituted with a methyl group. * represents a bonding position to L.

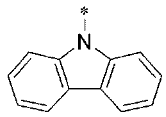
Z1

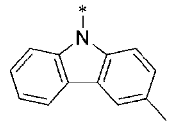
Z2

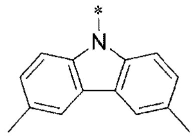
Z3

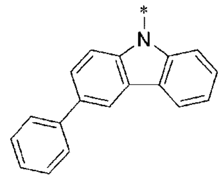
Z4

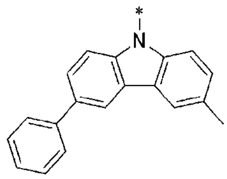
Z5

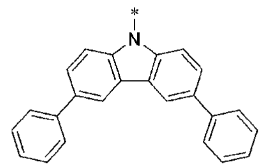
Z6

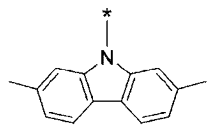
Z7

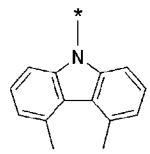
Z8

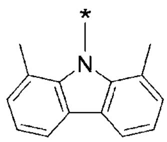
Z9

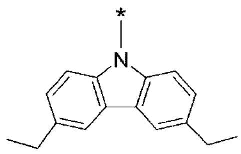
Z10

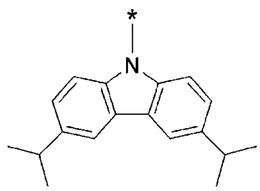
Z11

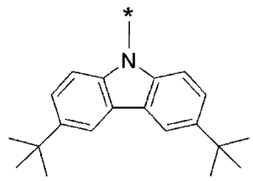
Z12

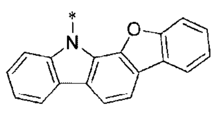
Z13

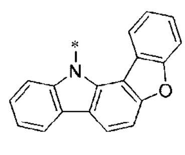
Z14

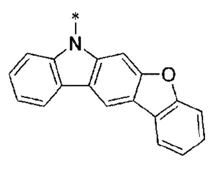
Z15

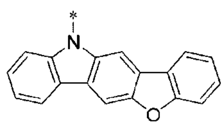
Z16

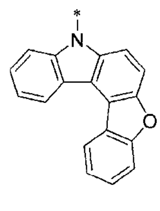
Z17

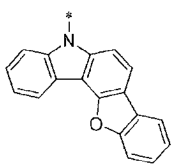
Z18

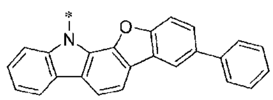
Z19

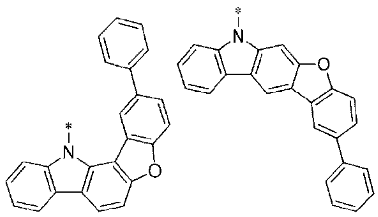
Z20

Z21

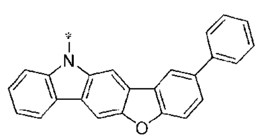
Z22

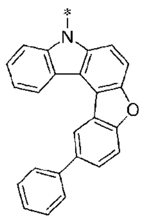
Z23

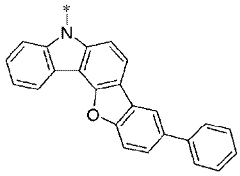
Z24

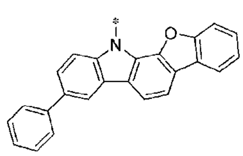
Z25

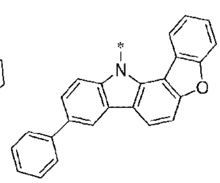
Z26

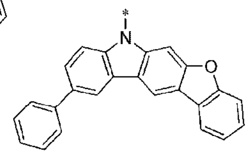
Z27

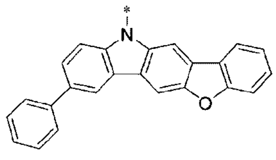
Z28

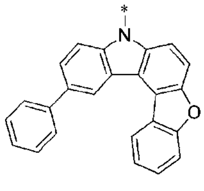
Z29

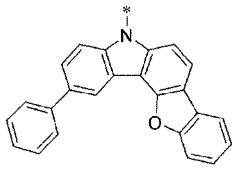
Z30

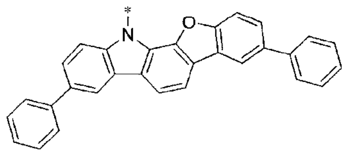
Z31

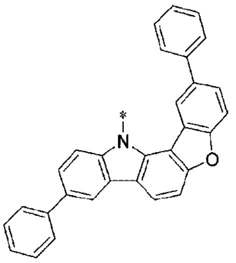
Z32

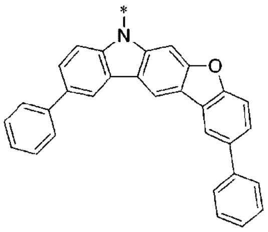
Z33

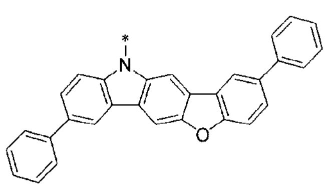
Z34

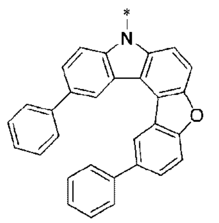
Z35

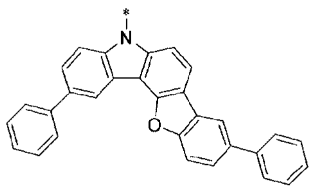
Z36

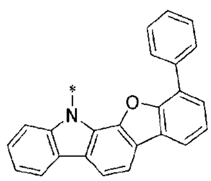
Z37

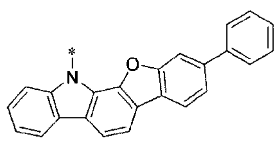
Z38

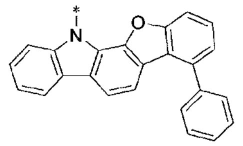
Z39

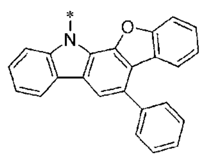
Z40

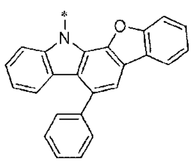
Z41

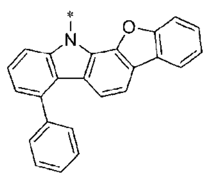
Z42

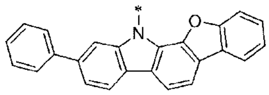
Z43

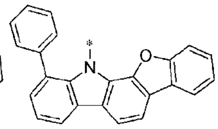
Z44

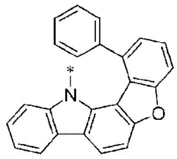
Z45

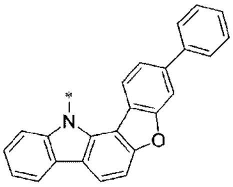
Z46

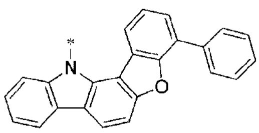
Z47

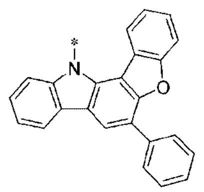
Z48

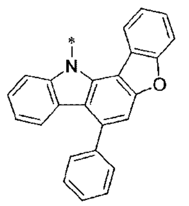
Z49

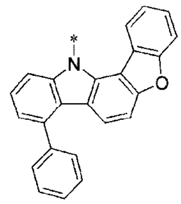
Z50

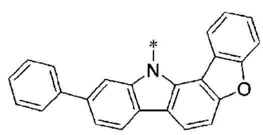
Z51

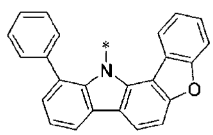
Z52

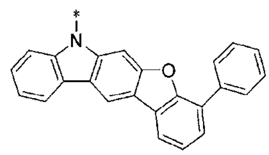
Z53

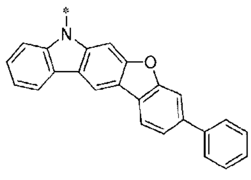
Z54

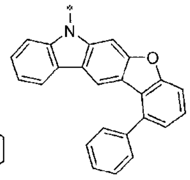
Z55

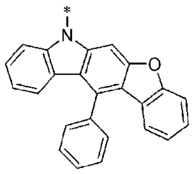
Z56

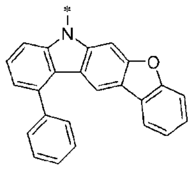
Z57

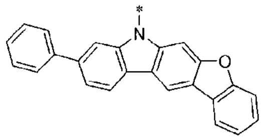
Z58

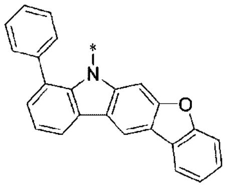
Z59

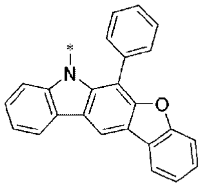
Z60

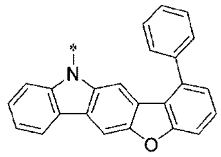
Z61

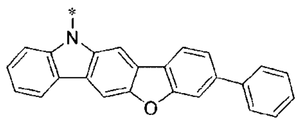
Z62

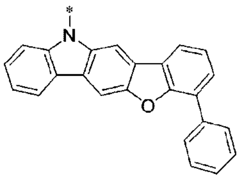
Z63

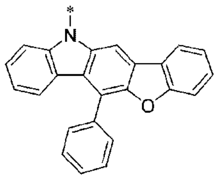
Z64

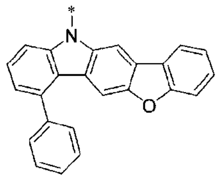
Z65

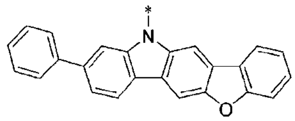
Z66

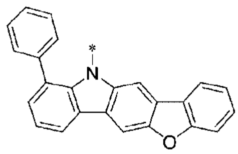
Z67

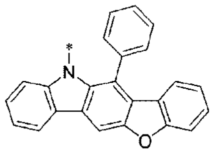
Z68

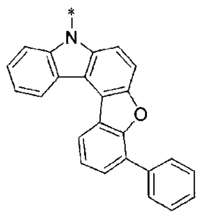
Z69

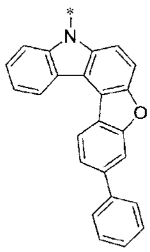
Z70

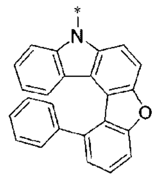
Z71

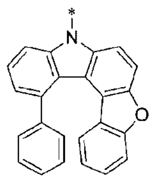
Z72

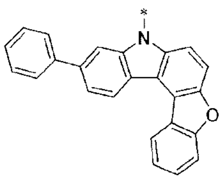
Z73

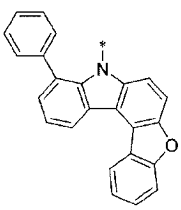
Z74

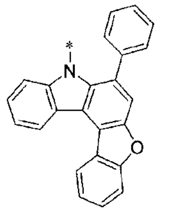
Z75

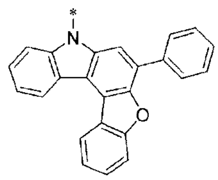
Z76

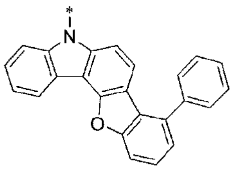
Z77

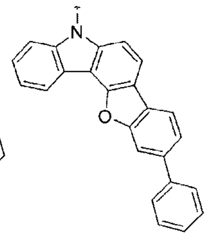
Z78

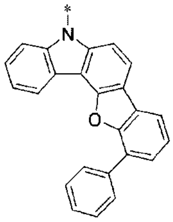
Z79

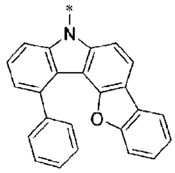
Z80

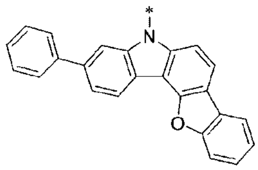
Z81

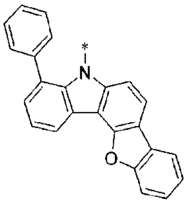
Z82

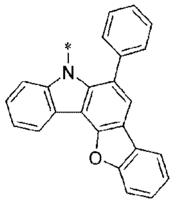
Z83

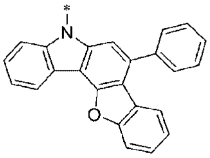
Z84

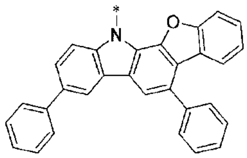
Z85

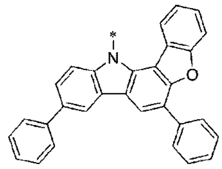
Z86

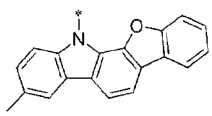
Z87

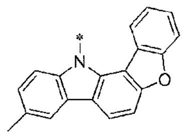
Z88

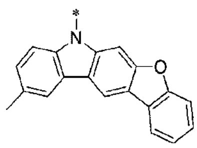
Z89

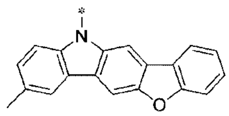
Z90

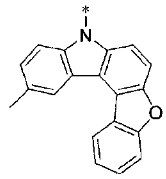
Z91

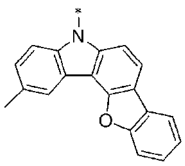
Z92

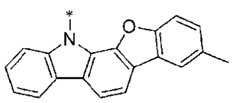
Z93

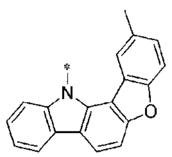
Z94

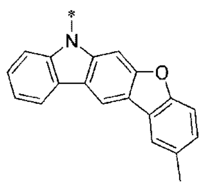
Z95

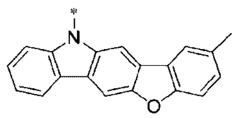
Z96

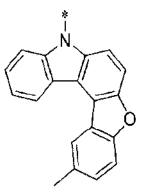
Z97

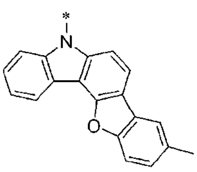
Z98

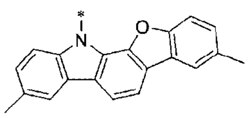
Z99

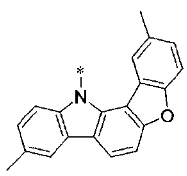
Z100

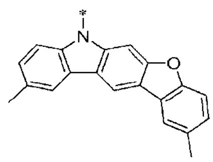
Z101

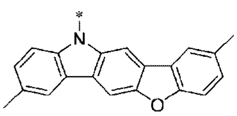
Z102

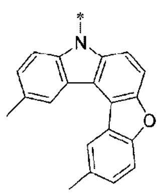
Z103

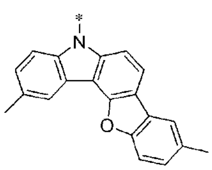
Z104

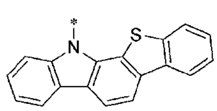
Z105

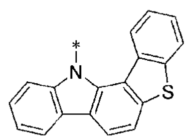
Z106

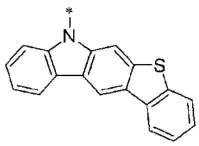
Z107

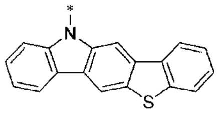
Z108

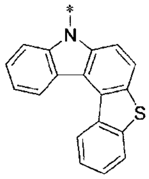
Z109

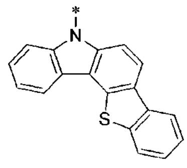
Z110

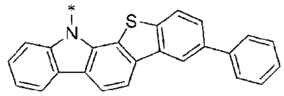
Z111

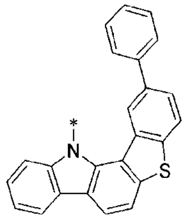
Z112

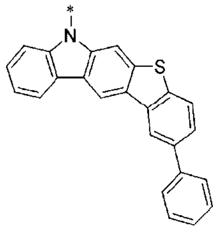
Z113

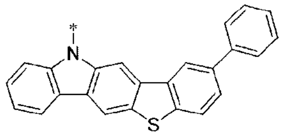
Z114

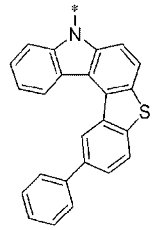
Z115

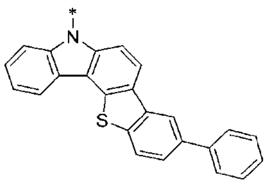
Z116

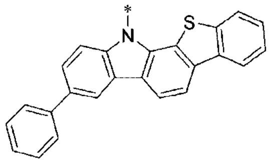
Z117

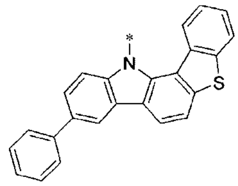
Z118

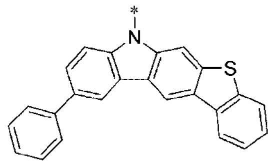
Z119

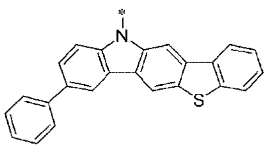
Z120

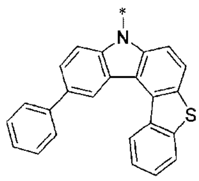
Z121

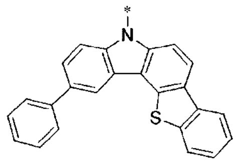
Z122

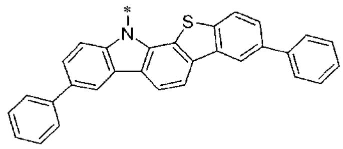
Z123

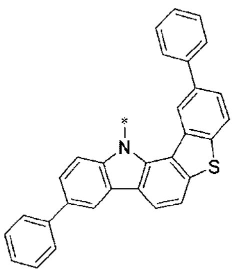
Z124

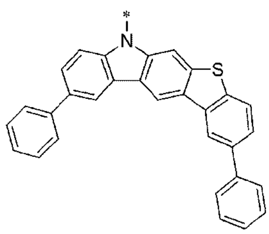
Z125

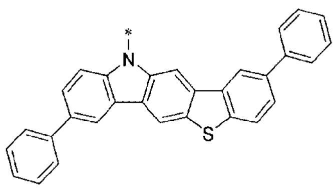
Z126

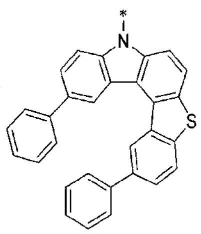
Z127

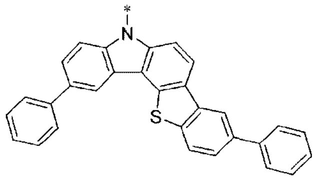
Z128

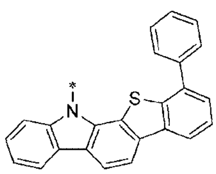
Z129

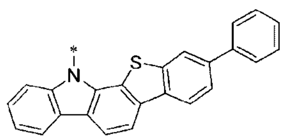
Z130

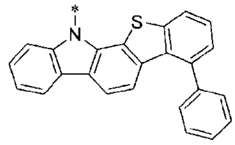
Z131

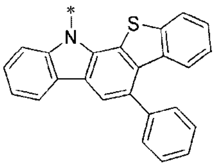
Z132

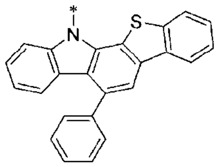
Z133

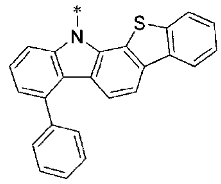
Z134

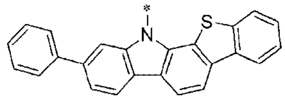
Z135

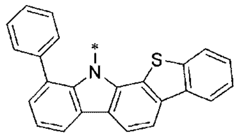
Z136

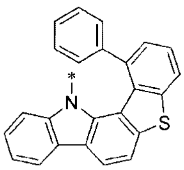
Z137

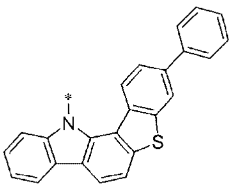
Z138

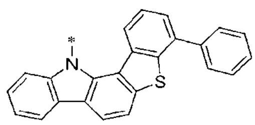
Z139

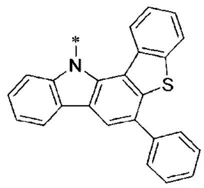
Z140

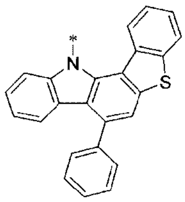
Z141

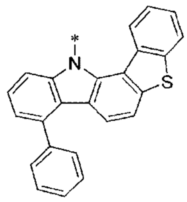
Z142

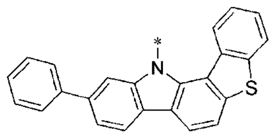
Z143

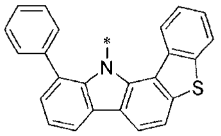
Z144

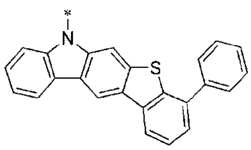
Z145

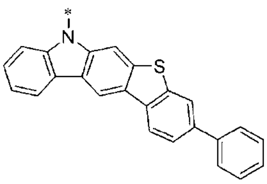
Z146

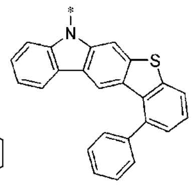
Z147

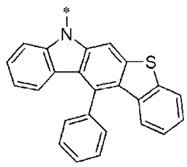
Z148

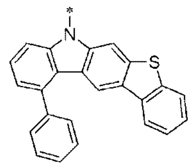
Z149

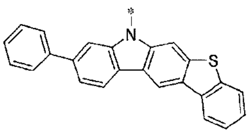
Z150

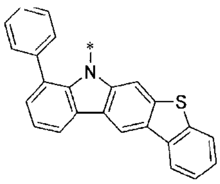
Z151

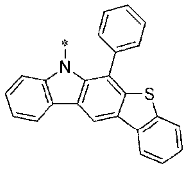
Z152

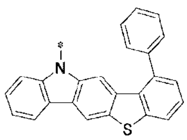
Z153

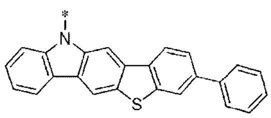
Z154

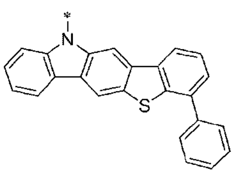
Z155

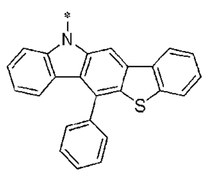
Z156

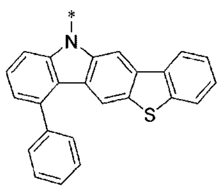
Z157

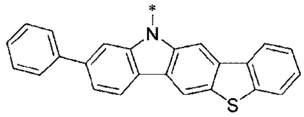
Z158

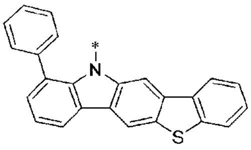
Z159
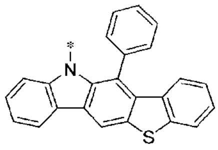
Z160
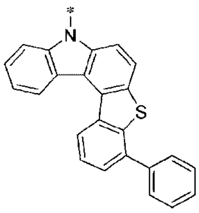
Z161
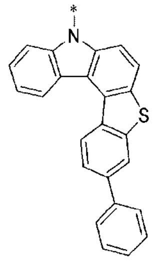
Z162
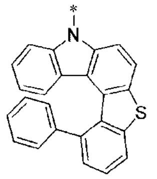
Z163

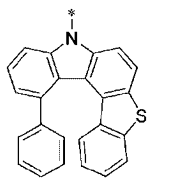
Z164
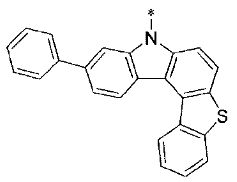
Z165
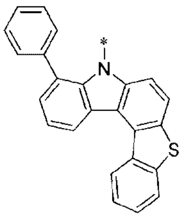
Z166
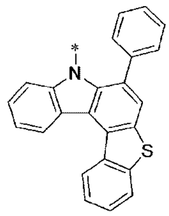
Z167
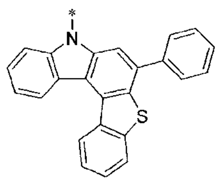
Z168

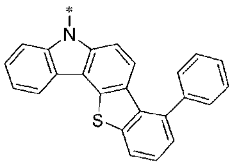
Z169
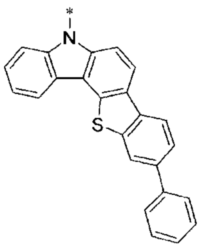
Z170
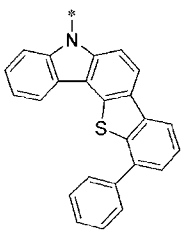
Z171
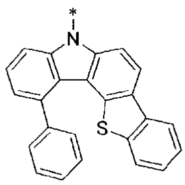
Z172
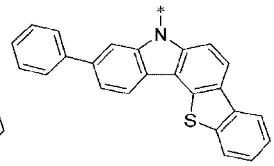
Z173

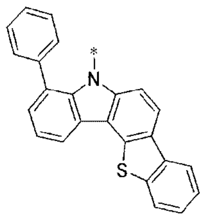
Z174
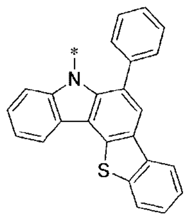
Z175
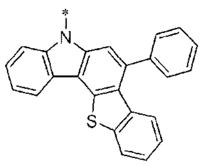
Z176
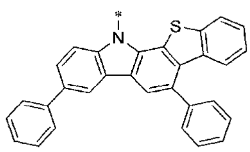
Z177
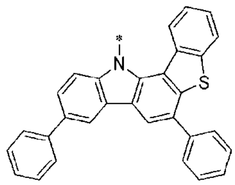
Z178

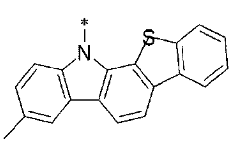
Z179
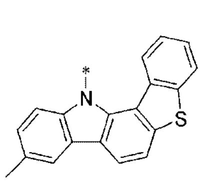
Z180
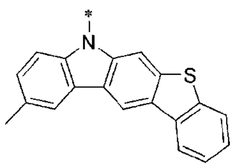
Z181
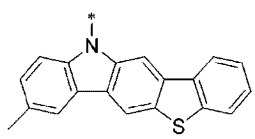
Z182

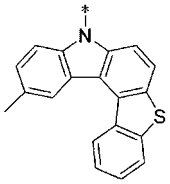
Z183

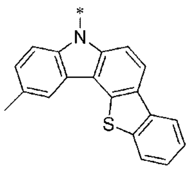
Z184

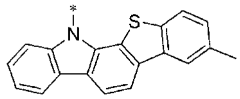
Z185

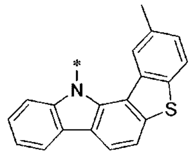
Z186

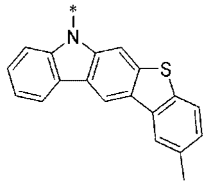
Z187

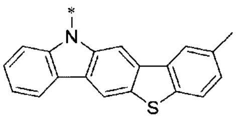
Z188

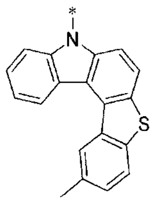
Z189

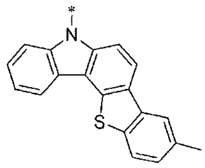
Z190

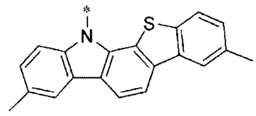
Z191

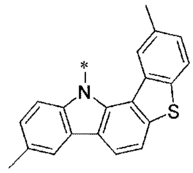
Z192

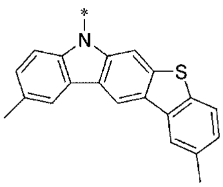
Z193

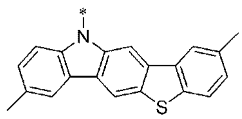
Z194

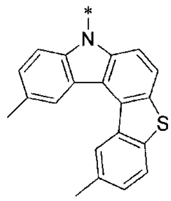
Z195

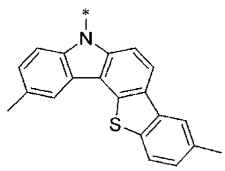
Z196

In addition to the above specific examples, groups in which all hydrogen atoms of alkyl groups of Z2, Z3, Z5, Z7 to Z12, Z87 to Z104, and Z179 to Z196 are substituted with deuterium atoms are exemplified herein as Z2(m), Z3(m), Z5(m), Z7(m) to Z12(m), Z87(m) to Z104(m), and Z179(m) to Z196(m), respectively. In addition, groups in which phenyl groups ($C_6H_5$) of Z4 to Z6, Z19 to Z86, and Z111 to Z178 are substituted with deuterated $C_6D_5$ are exemplified herein as Z4(p) to Z6(p), Z19(p) to Z86(p), and Z111(p) to Z178(p), respectively. Further, groups in which all hydrogen atoms of Z1 to Z196 are deuterated are exemplified herein as Z1(D) to Z196(D), respectively.

Specific examples of the group that can be adopted as the group bonded to L from the right side in the general formula (5) also include the following specific examples as well as Z2 to Z196 and deuterium atom substitutes thereof. Here, the groups that can be employed in the present invention shall not be construed as being limited by these specific examples. Also in the following specific examples, the expression of a methyl group is omitted. * represents a bonding position to L.

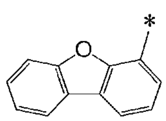
X1

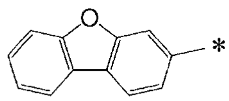
X2

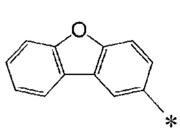
X3

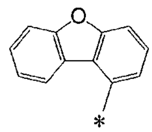
X4

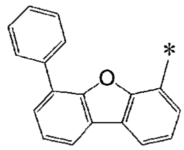
X5

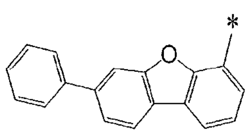
X6

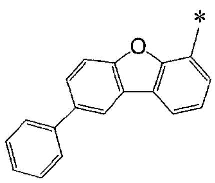
X7

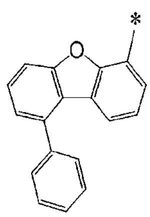
X8

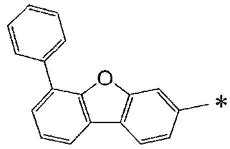
X9

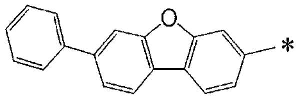 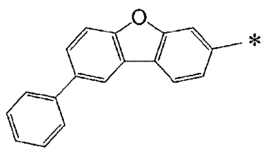 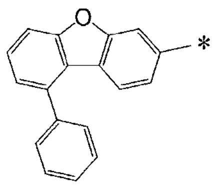 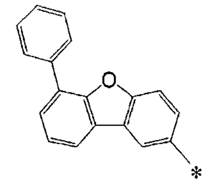

X10 X11 X12 X13

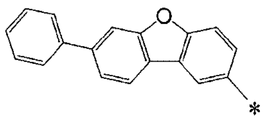 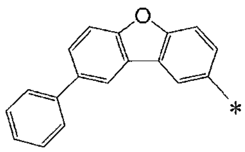 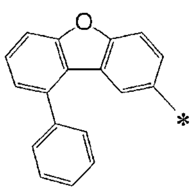 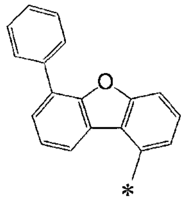

X14 X15 X16 X17

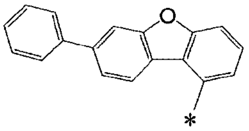 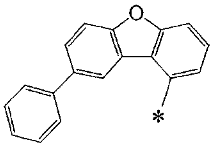 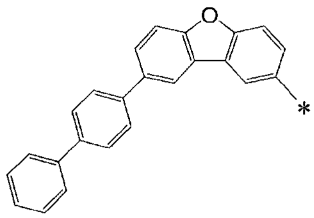 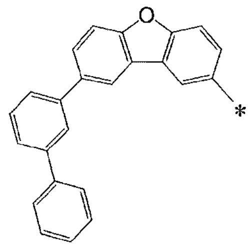

X18 X19 X20 X21

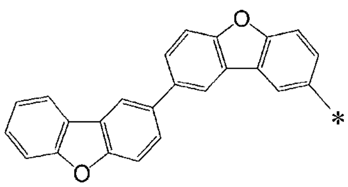 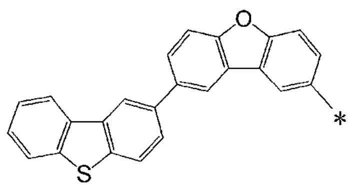 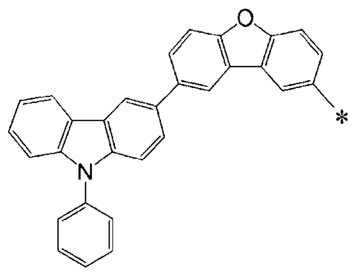

X22 X23 X24

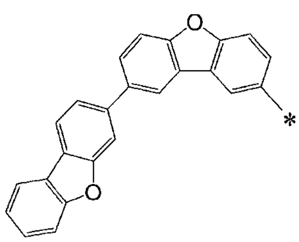 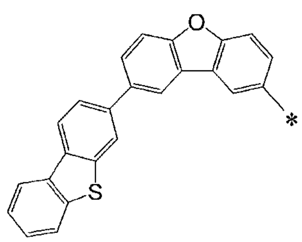 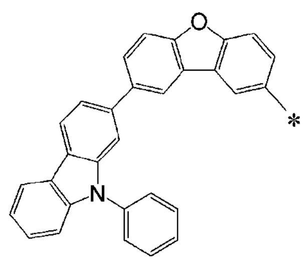

X25 X26 X27

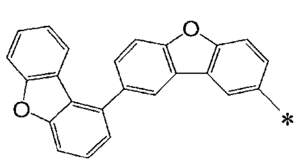
X28

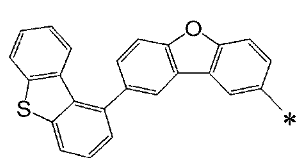
X29

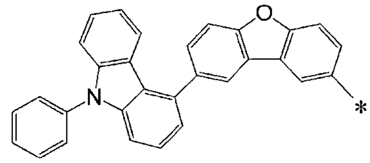
X30

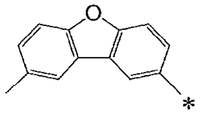
X31

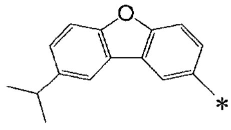
X32

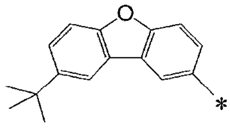
X33

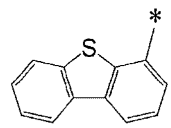
X34

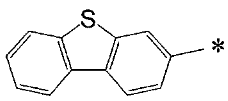
X35

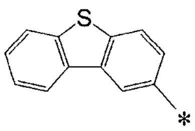
X36

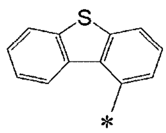
X37

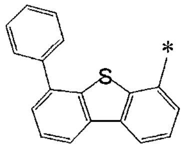
X38

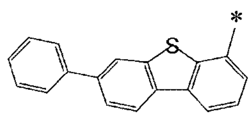
X39

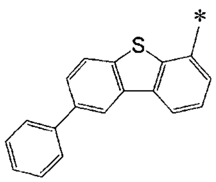
X40

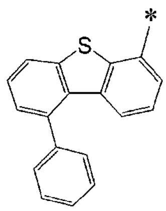
X41

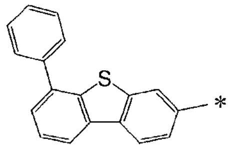
X42

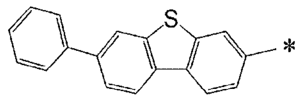
X43

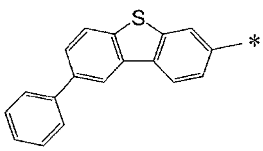
X44

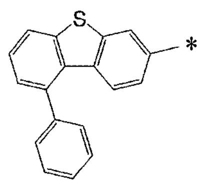
X45

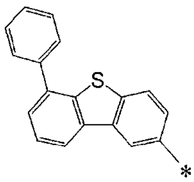
X46

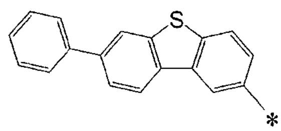
X47

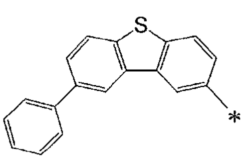
X48

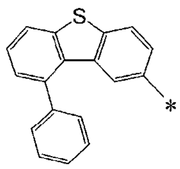
X49

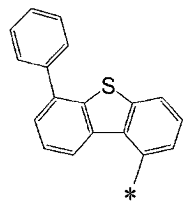
X50

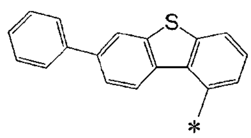
X51

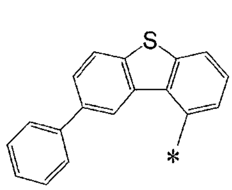
X52

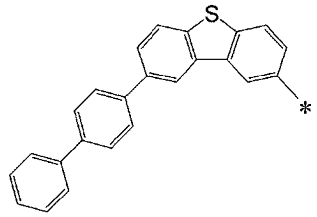
X53

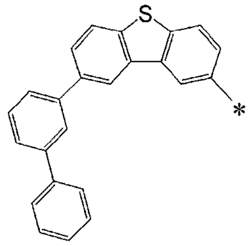
X54

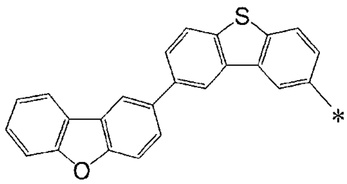
X55

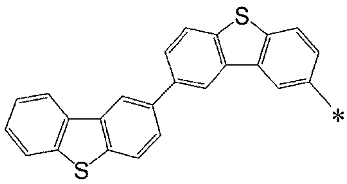
X56

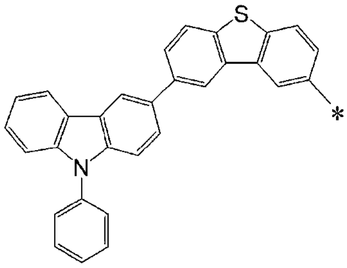
X57

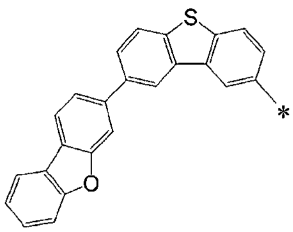
X58

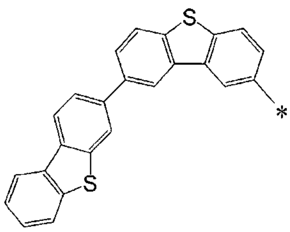
X59

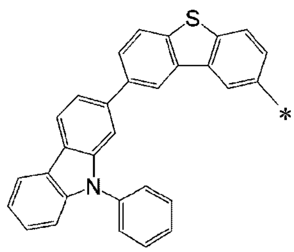
X60

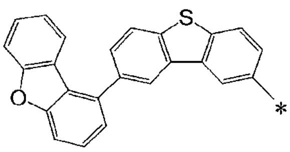
X61

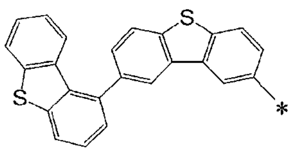
X62

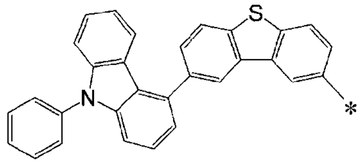
X63

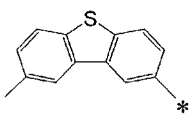
X64

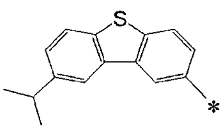
X65

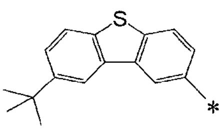
X66

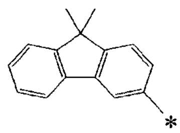
X67

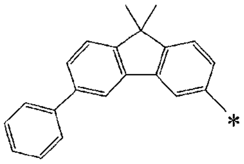

X68

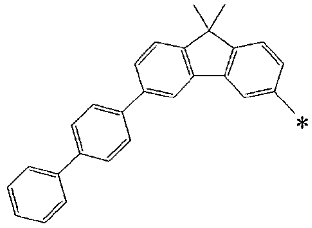

X69

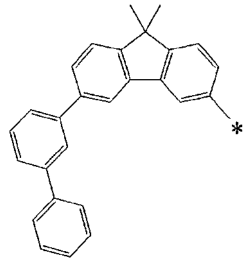

X70

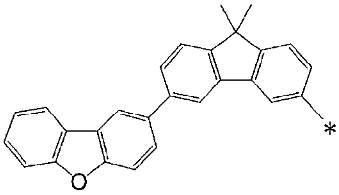

X71

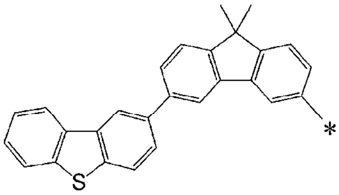

X72

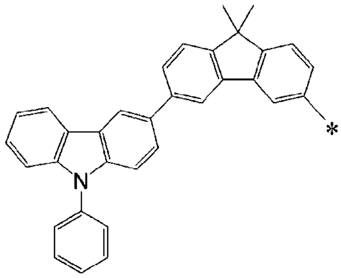

X73

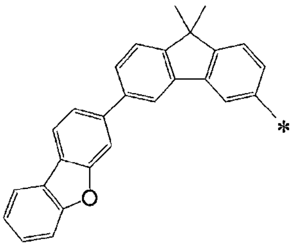

X74

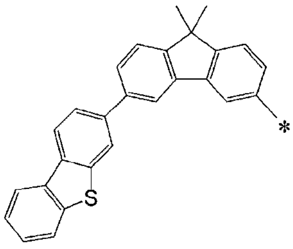

X75

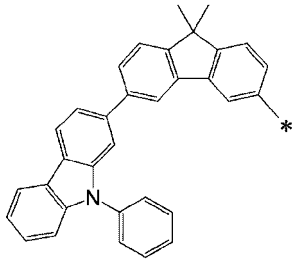

X76

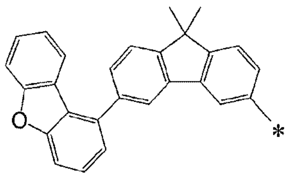

X77

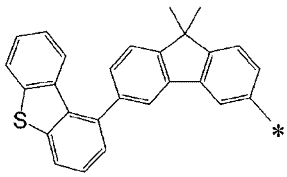

X78

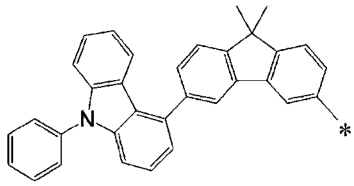

X79

Groups in which methyl groups ($CH_3$) of X31 to X33 and X64 to X79 are substituted with deuterated $CD_3$ are exemplified herein as X31(m) to X33(m), and X64(m) to X79(m), respectively, as well as the specific examples described above. Further, groups in which phenyl groups ($C_6H_5$) of X5 to X21, X38 to X54, and X68 to X70 are substituted with deuterated $C_6D_5$ are exemplified herein as X5(p) to X21(p), X38(p) to X54(p), and X68(p) to X70(p), respectively. Furthermore, groups in which all hydrogen atoms of X1 to X79 are deuterated are exemplified herein as X1(D) to X79(D), respectively.

n in the general formula (5) represents an integer of 3 or 4. When $X^{11}$ is O, S, or $C(R^B)(R^C)$, L is bonded to a benzene ring to which $(R^{115})n$ is bonded, and thus, n is 3. When $X^{11}$ is $N(R^A)$, and L is bonded to a benzene ring to which $(R^{115})n$ is bonded, n is 3, and further, when $X^{11}$ is $N(R^A)$, and L is bonded to N represented by $X^{11}$, n is 4. n $R^{115}$'s may be the same or different from each other.

L in the general formula (5) represents a single bond, a substituted or unsubstituted arylene group, a substituted or unsubstituted heteroarylene group, or a linking group in which two or more thereof are bonded. For an aryl structure of an arylene group and a heteroaryl structure of a heteroarylene group, the description on the "aryl group" and the "heteroaryl

group" can be referred to. The arylene group and the heteroarylene group may be substituted, and in the case of being substituted, the groups are preferably substituted with one atom or group selected from the group consisting of a deuterium atom, an aryl group, a heteroaryl group, an alkyl group, and a cyano group, or a group formed by combining two or more thereof, and more preferably substituted with one atom or group selected from the group consisting of a deuterium atom, an aryl group, a heteroaryl group, and an alkyl group, or a group formed by combining two or more thereof. In the case where the groups are substituted, a methyl group, an ethyl group, an isopropyl group, a tert-butyl group, a phenyl group, or deuterated products thereof are preferable. In one aspect of the present invention, L is an unsubstituted arylene group.

Specific examples of L are shown below. Here, L that can be employed in the present invention shall not be construed as being limited by these specific examples. In the following specific examples, the expression of a methyl group is omitted. Therefore, for example, L3 to L5 are substituted with a methyl group. * indicates a bonding position. L1 is a single bond.

none (direct bond)

L1

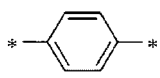

L2

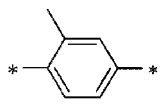

L3

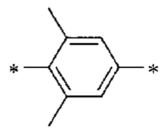

L4

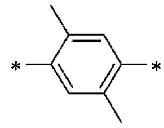

L5

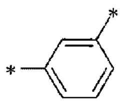

L6

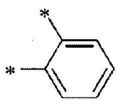

L7

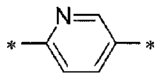

L8

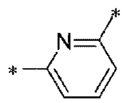

L9

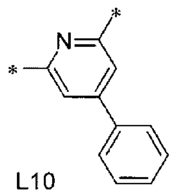

L10

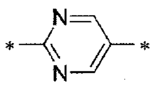

L11

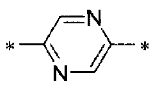

L12

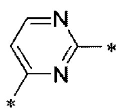

L13

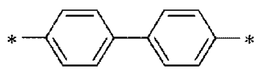

L14

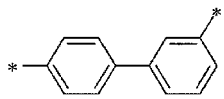

L15

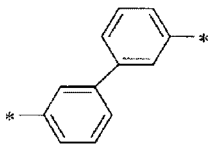

L16

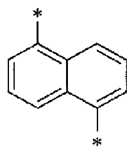

L17

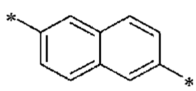

L18

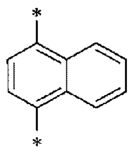

L19

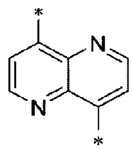

L20

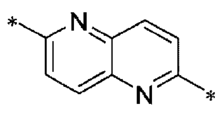

L21

In one aspect of the present invention, in the general formula (5), the group bonded to L from the left side is selected from Z1 to Z196 and deuterated products thereof, and the group bonded to L from the right side is selected from X1 to X79 and deuterated products thereof (Aspect 1). In one aspect of the present invention, the group bonded to L from the left side is selected from Z1 to Z12 and deuterated products thereof, and the group bonded to L from the right side is selected from X1 to X79 and deuterated products thereof (Aspect 2). In one aspect of the present invention, the group bonded to L from the left side is selected from Z13 to Z196 and deuterated products thereof, and the group bonded to L from the right side is selected from X1 to X79 and deuterated products thereof (Aspect 3). In one aspect of the present invention, the group bonded to L from the left side is selected from Z1 to Z196 and deuterated products thereof, and the group bonded

to L from the right side is selected from X1 to X66 and deuterated products thereof (Aspect 4). In one aspect of the present invention, the group bonded to L from the left side is selected from Z1 to Z196 and deuterated products thereof, and the group bonded to L from the right side is selected from X1 to X33 and deuterated products thereof (Aspect 5). In one aspect of the present invention, the group bonded to L from the left side is selected from Z1 to Z196 and deuterated products thereof, and the group bonded to L from the right side is selected from X1 to X21, X31 to X33, and deuterated products thereof (Aspect 6). In one aspect of the present invention, the group bonded to L from the left side is selected from Z1 to Z196 and deuterated products thereof, and the group bonded to L from the right side is selected from X22 to X30 and deuterated products thereof (Aspect 7).

In one aspect of the present invention, L in Aspect 1 is L1. In one aspect of the present invention, L in Aspect 2 is L1. In one aspect of the present invention, L in Aspect 3 is L1. In one aspect of the present invention, L in Aspect 4 is L1. In one aspect of the present invention, L in Aspect 5 is L1. In one aspect of the present invention, L in Aspect 6 is L1. In one aspect of the present invention, L in Aspect 7 is L1.

In one aspect of the present invention, L in Aspect 1 is L6. In one aspect of the present invention, L in Aspect 2 is L6. In one aspect of the present invention, L in Aspect 3 is L6. In one aspect of the present invention, L in Aspect 4 is L6. In one aspect of the present invention, L in Aspect 5 is L6. In one aspect of the present invention, L in Aspect 6 is L6. In one aspect of the present invention, L in Aspect 7 is L6.

In one aspect of the present invention, L in Aspect 1 is L14. In one aspect of the present invention, L in Aspect 2 is L14. In one aspect of the present invention, L in Aspect 3 is L14. In one aspect of the present invention, L in Aspect 4 is L14. In one aspect of the present invention, L in Aspect 5 is L14. In one aspect of the present invention, L in Aspect 6 is L14. In one aspect of the present invention, L in Aspect 7 is L14.

In one aspect of the present invention, L in Aspect 1 is L16. In one aspect of the present invention, L in Aspect 2 is L16. In one aspect of the present invention, L in Aspect 3 is L16. In one aspect of the present invention, L in Aspect 4 is L16. In one aspect of the present invention, L in Aspect 5 is L16. In one aspect of the present invention, L in Aspect 6 is L16. In one aspect of the present invention, L in Aspect 7 is L16.

Specific examples of the compound represented by the general formula (5) are shown below. Here, the compound represented by the general formula (5) that can be employed in the present invention shall not be construed as being limited by the following specific examples.

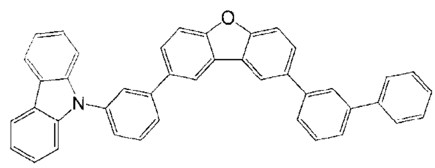

H1

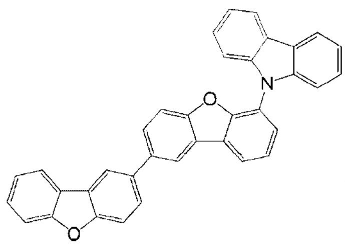

H2

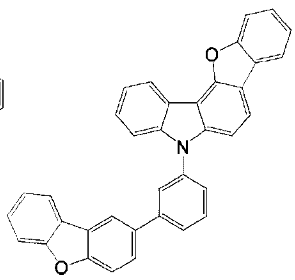

H3

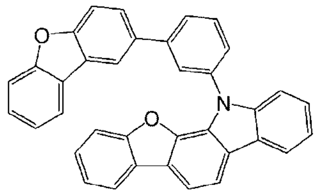

H4

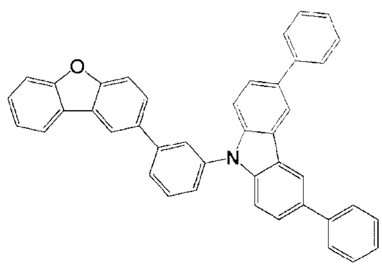

H5

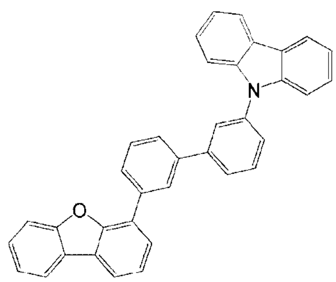

H6

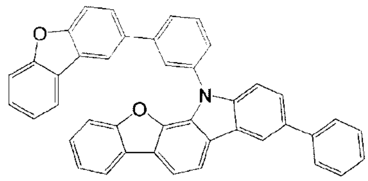

H7

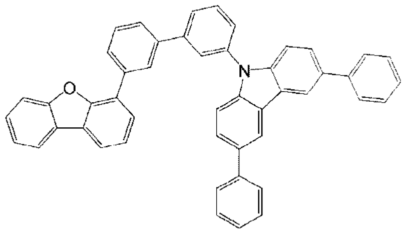

H8

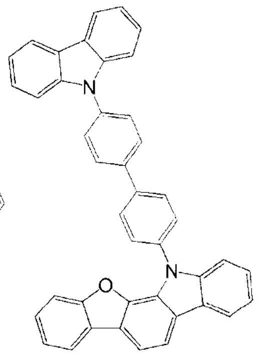

H9

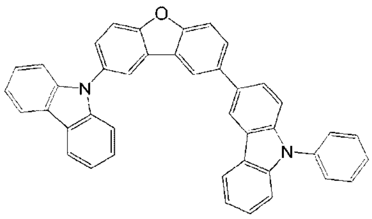

H10

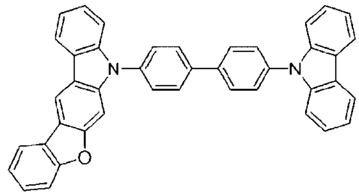

H11

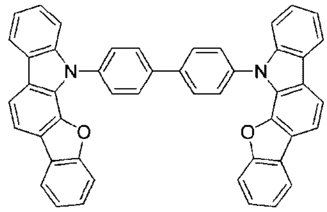

H12

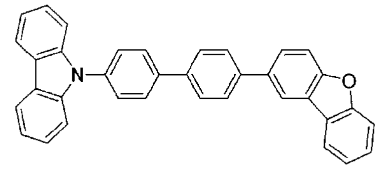

H13

Compounds in which all hydrogen atoms present in substituted or unsubstituted carbazol-9-yl groups present in H1 to H13 are substituted with deuterium atoms are exemplified herein as H1(d) to H13(d), respectively, as well as the specific examples described above. Further, compounds in which all hydrogen atoms present in H1 to H13 are substituted with deuterium atoms are exemplified herein as H1(D) to H13(D), respectively.

The molecular weight of the compound represented by the general formula (5) is preferably 1500 or less, more preferably 1200 or less, further preferably 1000 or less, still further preferably 800 or less, and for example, may be 600 or less, for example, when there is an intention to form and use a film of an organic layer containing the compound represented by the general formula (5) through a vapor deposition method. The lower limit value of the molecular weight is the molecular weight of the minimum compound in the compound group represented by the general formula (5).

The compound represented by the general formula (5) with a smaller dipole moment is preferable because the orientation when a film is formed increases. The dipole moment is preferably smaller than 2.3, more preferably smaller than 2.0, further preferably smaller than 1.7, and still further preferably smaller than 1.4.

As the compound represented by the general formula (5), a compound composed of atoms selected from the group consisting of a carbon atom, a hydrogen atom, a deuterium atom, a nitrogen atom, an oxygen atom, and a sulfur atom can be selected. For example, as the compound represented by the general formula (5), a compound composed of atoms selected from the group consisting of a carbon atom, a hydrogen atom, a deuterium atom, a nitrogen atom, and an oxygen atom can be selected. For example, as the compound represented by the general formula (5), a compound composed of atoms selected from the group consisting of a carbon atom, a hydrogen atom, a deuterium atom, a nitrogen atom, and a sulfur atom can be selected. For example, as the compound represented by the general formula (5), a compound composed of atoms selected from the group consisting of a carbon atom, a hydrogen atom, a deuterium atom, and a nitrogen atom can be selected.

Hereinafter, the respective members and the respective layers other than the light-emitting layer of the organic electroluminescent device will be described.

Substrate:

In some embodiments, the organic electroluminescent device of the present invention is supported by a substrate, and the substrate is not particularly limited, and may be any material commonly used in organic electroluminescent devices, for example, formed of glass, transparent plastic, quartz, and silicon.

Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the conductive transparent material is selected from CuI, indium tin oxide (ITO), $SnO_2$, and ZnO. In some embodiments, an amorphous material capable of forming a transparent conductive film, such as IDIXO ($In_2O_3$-ZnO), is used. In some embodiments, the anode is a thin film. In some embodiments, the thin film is made by vapor deposition or sputtering. In some embodiments, the film is patterned by a photolithography method. In some embodiments, where the pattern may not require high accuracy (for example, approximately 100 μm or more), the pattern may be formed with a mask having a desired shape on vapor deposition or sputtering of the electrode material. In some embodiments, when a coating material such as an organic electroconductive compound can be applied, a wet film forming method such as a printing method or a coating method is used. In some embodiments, when the emitted light goes through the anode, the anode has a transmittance of more than 10%, and the anode has a sheet resistance of several hundred Ohm per unit area or less. In some embodiments, the thickness of the anode is 10 to 1,000 nm. In some embodiments, the thickness of the anode is 10 to 200 nm. In some embodiments, the thickness of the anode varies depending on the material to be used.

Cathode:

In some embodiments, the cathode is made of an electrode material such as a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-copper mixture, a magnesium-silver mixture, a magnesium-aluminum mixture, a magnesium-indium mixture, an aluminum-aluminum oxide ($Al_2O_3$) mixture, indium, a lithium-aluminum mixture, and a rare earth element. In some embodiments, a mixture of an electron injection metal and a second metal that is a stable metal having a work function larger than that of the electron injection metal is used. In some embodiments, the mixture is selected from a magnesium-silver mixture, a magnesium-aluminum mixture, a magnesium-indium mixture, an aluminum-aluminum oxide ($Al_2O_3$) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per unit area or less. In some embodiments, the thickness of the cathode ranges from 10 nm to 5 μm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent device enhances the light emission luminance.

In some embodiments, the cathode is formed with an electroconductive transparent material, as described for the anode, to form a transparent or translucent cathode. In some embodiments, a device includes an anode and a cathode, both being transparent or translucent.

Injection Layer:

An injection layer is a layer between the electrode and the organic layer. In some embodiments, the injection layer decreases the drive voltage and enhances the light emission luminance. In some embodiments, the injection layer includes a hole injection layer and an electron injection layer. The injection layer can be positioned between the anode and the light-emitting layer or the hole transport layer, and between the cathode and the light-emitting layer or the electron transport layer. In some embodiments, an injection layer is present. In some embodiments, no injection layer is present.

Preferred compound examples for use as a hole injection material are shown below.

$MoO_3$,

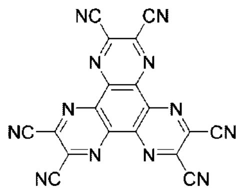 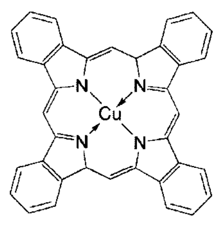 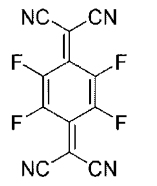 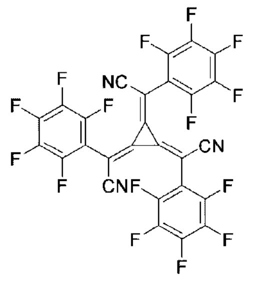

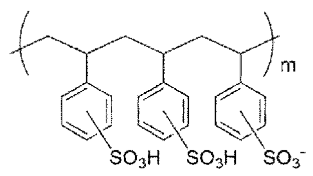 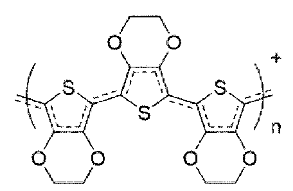

Next, preferred compound examples for use as an electron injection material are shown below.

LiF, CsF,

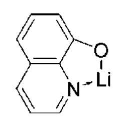

Barrier Layer:

A barrier layer is a layer capable of inhibiting charges (electrons or holes) and/or excitons present in the light-emitting layer from being diffused outside the light-emitting layer. In some embodiments, the electron barrier layer is present between the light-emitting layer and the hole transport layer, and inhibits electrons from passing through the light-emitting layer toward the hole transport layer. In some embodiments, the hole barrier layer is present between the light-emitting layer and the electron transport layer, and inhibits holes from passing through the light-emitting layer toward the electron transport layer. In some embodiments, the barrier layer inhibits excitons from being diffused outside the light-emitting layer. In some embodiments, the electron barrier layer and the hole barrier layer constitute an exciton barrier layers. The term "electron barrier layer" or "exciton barrier layer" used herein includes a layer that has both the functions of an electron barrier layer and an exciton barrier layer.

Hole Barrier Layer:

A hole barrier layer acts as an electron transport layer. In some embodiments, the hole barrier layer inhibits holes from reaching the electron transport layer while transporting electrons. In some embodiments, the hole barrier layer enhances the recombination probability of electrons and holes in the light-emitting layer. The material used for the hole barrier layer may be the same material as described above for the electron transport layer.

Preferred examples of compounds that can be used in the hole barrier layer are shown below.

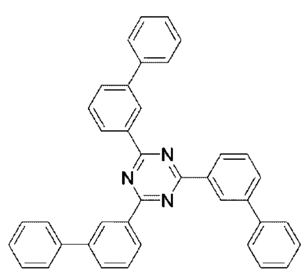 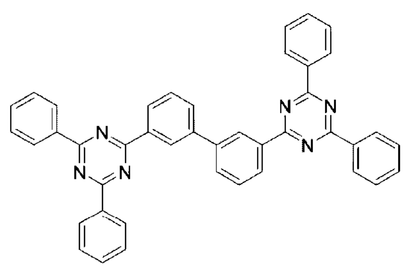 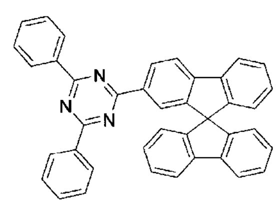

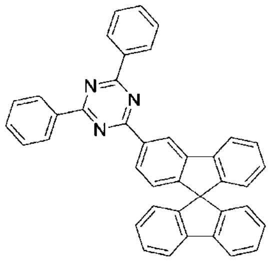 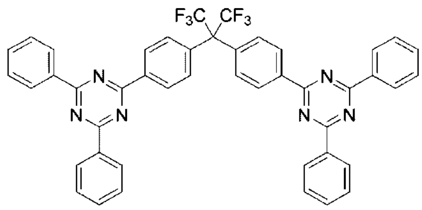 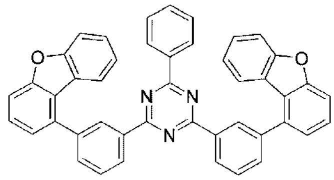

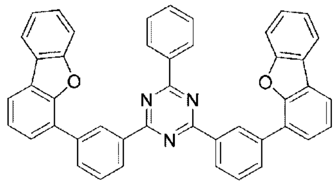 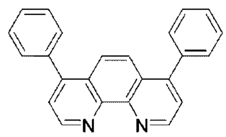 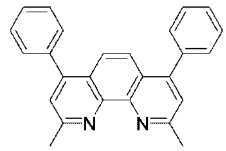

Electron Barrier Layer:

An electron barrier layer transports holes. In some embodiments, the electron barrier layer inhibits electrons from reaching the hole transport layer while transporting holes. In some embodiments, the electron barrier layer enhances the recombination probability of electrons and holes in the light-emitting layer. The material used for the electron barrier layer may be the same material as described above for the hole transport layer.

Preferred specific examples of compounds that can be used as the electron barrier material are shown below.

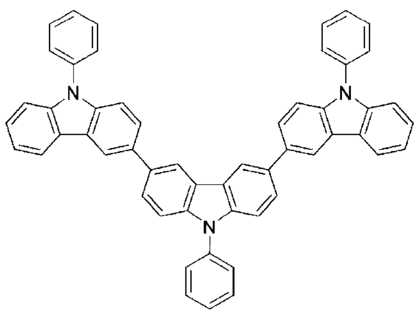 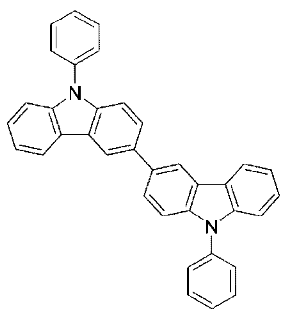 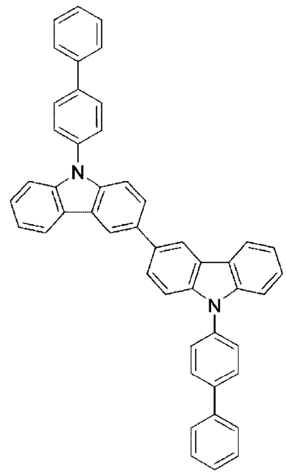

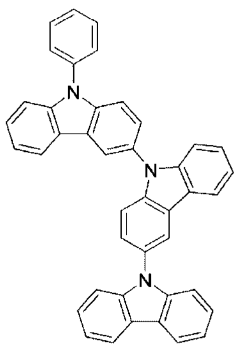 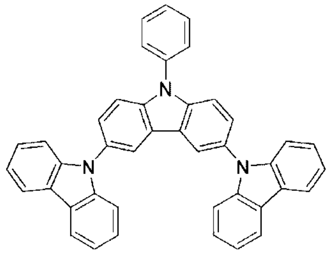 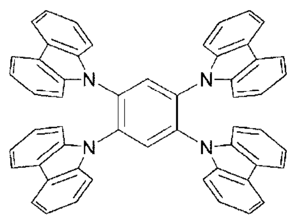

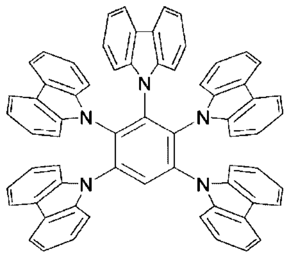
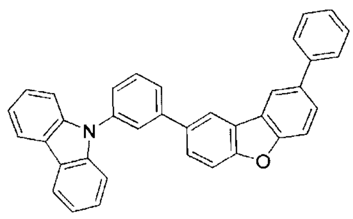
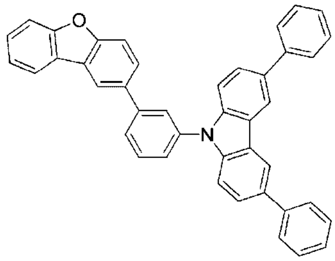
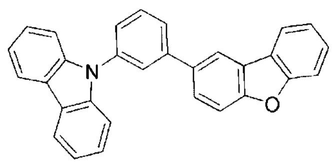
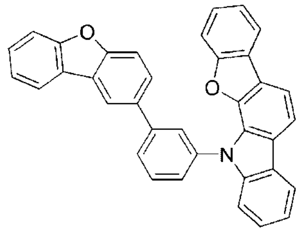
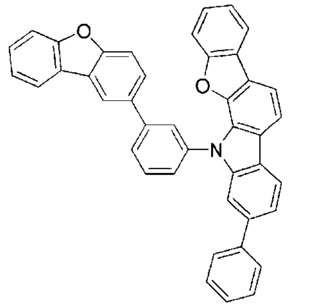
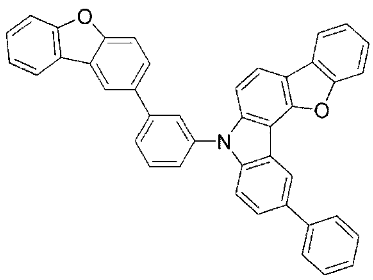
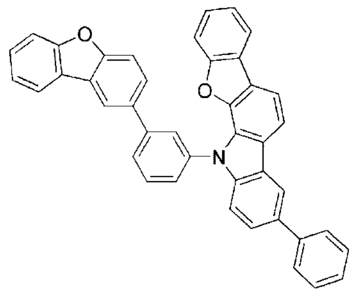
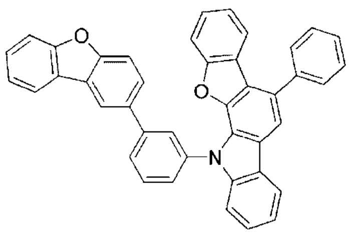

Exciton Barrier Layer:

An exciton barrier layer inhibits excitons generated through recombination of holes and electrons in the light-emitting layer from being diffused to the charge transport layer. In some embodiments, the exciton barrier layer enables effective confinement of excitons in the light-emitting layer. In some embodiments, the light emission efficiency of the device is enhanced. In some embodiments, the exciton barrier layer may be present adjacent to only one of the side of the anode and the side of the cathode of the light-emitting layer, or one exciton barrier layer may be present adjacent to the side of the anode of the light-emitting layer and another exciton barrier layer may be present adjacent to the side of the cathode of the light-emitting layer. In some embodiments, when the exciton barrier layer is present on the side of the anode, the layer may be present between the hole transport layer and the light-emitting layer and adjacent to the light-emitting layer. In some embodiments, when the exciton barrier layer is present on the side of the cathode, the layer may be present between the light-emitting layer and the cathode and adjacent to the light-emitting layer. In some embodiments, a hole injection layer, an electron barrier layer, or a similar layer is present between the anode and the exciton barrier layer that is adjacent to the light-emitting layer on the side of the anode. In some embodiments, a hole injection layer, an electron barrier layer, a hole barrier layer, or a similar layer is present between the cathode and the exciton barrier layer that is adjacent to the light-emitting layer on the side of the cathode. In some embodiments, the exciton barrier layer includes excited singlet energy and excited triplet energy, at least one of which is higher than the excited singlet energy and the excited triplet energy of the light-emitting material, respectively.

Hole Transport Layer:

The hole transport layer contains a hole transport material. In some embodiments, the hole transport layer is a single layer. In some embodiments, the hole transport layer has a plurality of layers.

In some embodiments, the hole transport material has one of injection or transport property of holes and barrier property of electrons. In some embodiments, the hole transport material is an organic material. In some embodiments, the hole transport material is an inorganic material. Examples of known hole transport materials that can be used in the present

invention include, but are not limited to, a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an allylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer (particularly, a thiophene oligomer), or a combination thereof. In some embodiments, the hole transport material is selected from a porphyrin compound, an aromatic tertiary amine compound, and a styrylamine compound. In some embodiments, the hole transport material is an aromatic tertiary amine compound. Preferred specific examples of compounds that can be used as the hole transport material are shown below.

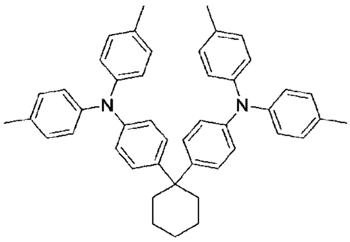
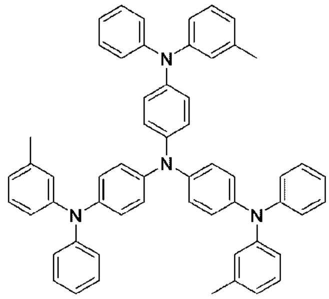
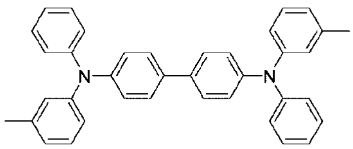

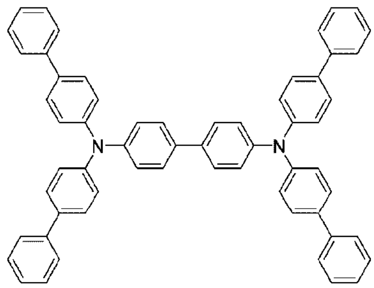
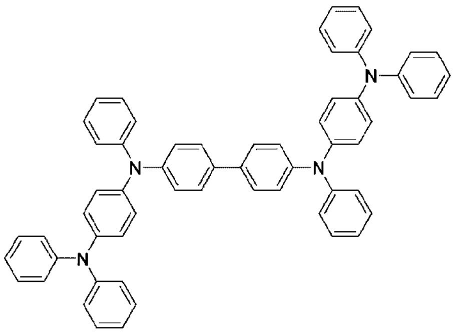

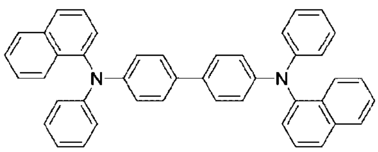
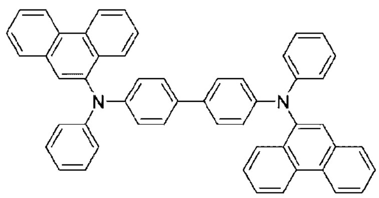

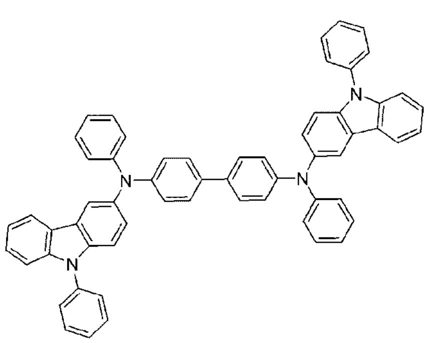
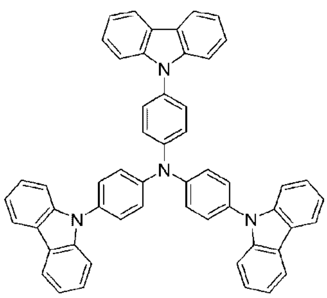
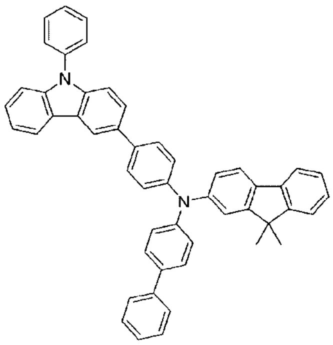

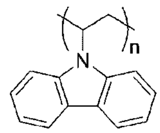
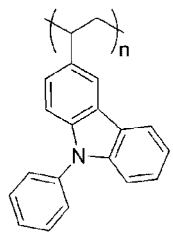
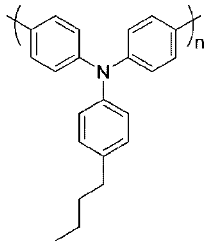

Electron Transport Layer:

The electron transport layer contains an electron transport material. In some embodiments, the electron transport layer is a single layer. In some embodiments, the electron transport layer has a plurality of layers.

In some embodiments, the electron transport material needs only to have a function of transporting electrons, which are injected from the cathode, to the light-emitting layer. In some embodiments, the electron transport material also functions as a hole barrier material. Examples of the electron transport layer that can be used in the present invention include, but are not limited to, a nitro-substituted fluorene derivative, a diphenylquinone derivative, a thiopyran dioxide derivative, carbodiimide, a fluorenylidene methane derivative, anthraquinodimethane, an anthrone derivative, an oxadiazole derivative, an azole derivative, an azine derivative, or a combination thereof, or a polymer thereof. In some embodiments, the electron transport material is a thiadiazole derivative, or a quinoxaline derivative. In some embodiments, the electron transport material is a polymer material. Preferred specific examples of compounds that can be used as the electron transport material are shown below.

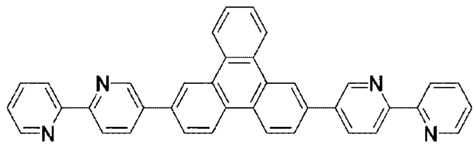
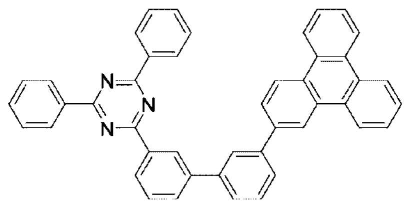
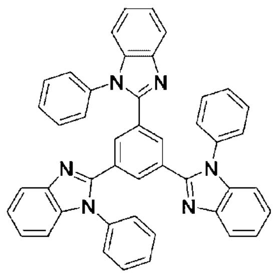
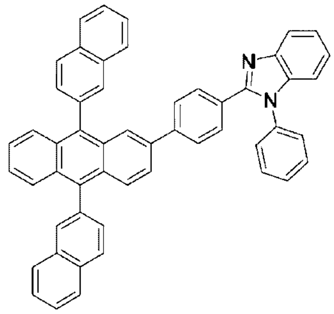
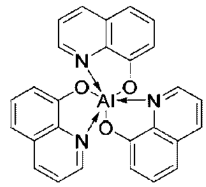
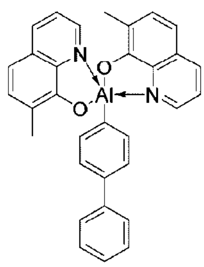
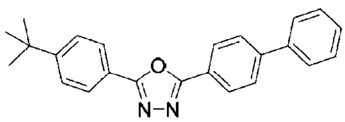
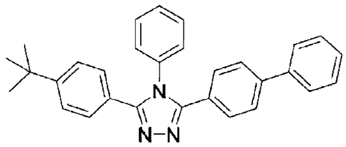

Examples of preferred compounds as the material that can be added to each organic layer are shown below. For example, the addition of a compound as a stabilizing material may be taken into consideration.

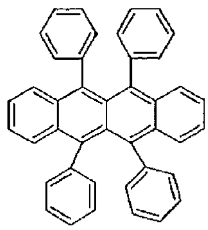
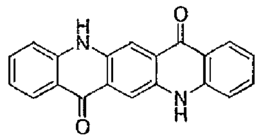
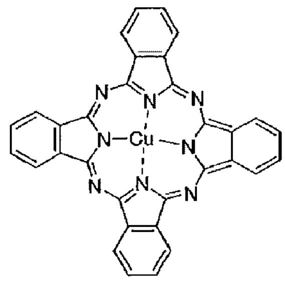

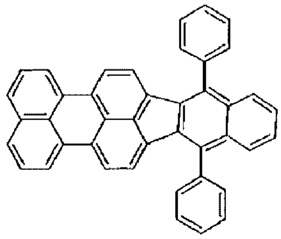
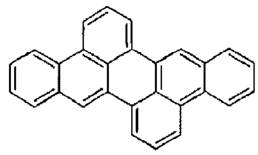

Preferred materials that can be used in the organic electroluminescent device have been specifically exemplified. However, the materials that can be used in the present invention should not be construed as being limited to the following exemplary compounds. In addition, even the compounds exemplified as the material having a specific function can be diverted as a material having another function.

Device:

In some embodiments, the light-emitting layer is incorporated into a device. For example, the device includes, but is not limited to, an OLED bulb, an OLED lamp, a display for a television, a computer monitor, a mobile phone, and a tablet.
In some embodiments, an electronic device includes an OLED having an anode, a cathode, and at least one organic layer containing a light-emitting layer between the anode and the cathode.
In some embodiments, compositions described herein can be incorporated into various light-sensitive or light-activated devices, such as an OLED or a photoelectronic device. In some embodiments, the composition can be useful in facilitating charge transfer or energy transfer within a device and/or as a hole transport material. Such devices include, for example, an organic light-emitting diode (OLED), an organic integrated circuit (OIC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC), or an organic laser diode (O-laser).

Bulb or Lamp:

In some embodiments, an electronic device includes an OLED including an anode, a cathode, and at least one organic layer including a light-emitting layer between the anode and the cathode.
In some embodiments, a device includes OLEDs that differ in color. In some embodiments, a device includes an array including a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.
In some embodiments, a device is an OLED light including:

a circuit board having a first surface with a mounting surface and an opposing second surface, and defining at least one opening,
at least one OLED on the mounting surface, the at least one OLED configured to emit light, the at least one OLED including an anode, a cathode, and at least one organic layer including a light-emitting layer between the anode and the cathode,
a housing for the circuit board, and
at least one connector arranged at an end of the housing, the housing and the at least one connector defining a package adapted for installation in a light fixture.

In some embodiments, the OLED light has a plurality of OLEDs mounted on a circuit board such that light is emitted in a plurality of directions. In some embodiments, a portion of the light emanated in a first direction is polarized and is emitted in a second direction. In some embodiments, a reflector is used to polarize the light emanated in a first direction.

Display or Screen:

In some embodiments, the light-emitting layer of the present invention can be used in a screen or a display. In some embodiments, the compounds according to the present invention are deposited onto a substrate using a process including, but not limited to, vacuum evaporation, deposition, vapor deposition, or chemical vapor deposition (CVD). In some embodiments, the substrate is a photoplate structure useful in a two-sided etching that provides a unique aspect ratio pixel. The screen (which may also be referred to as a mask) is used in a production process of an OLED display. The design of the corresponding artwork pattern allows for the placement of a very steep and narrow tie-bar between the pixels in the vertical direction as well as a large, sweeping bevel opening in the horizontal direction. This allows the fine patterning of pixels needed for high resolution displays while optimizing the chemical vapor deposition onto a TFT backplane.

The internal patterning of the pixel allows the construction of a three-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point, the entire pixel area is subjected to a similar etching rate but the depths are varying depending on the halftone pattern. Varying the size and spacing of the halftone pattern allows etching to be inhibited at different rates within the pixel and allow a localized deeper etching needed to create steep vertical bevels.

A preferred material for the deposition mask is invar. Invar is a metal alloy that is cold rolled into a long thin sheet in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etching. In further embodiments, a screen or display pattern is fabricated using plasma etching.

Method for Producing Device:

An OLED display is generally produced by forming a large mother panel and then cutting the mother panel in units of cell panels. In general, each of the cell panels on the mother panel is formed by forming a thin film transistor (TFT) having an active layer and a source/drain electrode on a base substrate, applying a planarization film to the TFT, sequentially forming a pixel electrode, a light-emitting layer, a counter electrode, and an encapsulation layer in order, and is cut from the mother panel.

An OLED display is generally produced by forming a large mother panel and then cutting the mother panel in units of cell panels. In general, each of the cell panels on the mother panel is formed by forming a thin film transistor (TFT) having an active layer and a source/drain electrode on a base substrate, applying a planarization film to the TFT, sequentially forming a pixel electrode, a light-emitting layer, a counter electrode, and an encapsulation layer in order, and is cut from the mother panel.

In another aspect of the present invention, provided herein is a method for producing an organic light-emitting diode (OLED) display, the method including:

a step of forming a barrier layer on a base substrate of a mother panel,
a step of forming a plurality of display units in units of cell panels on the barrier layer,
a step of forming an encapsulation layer on each of the display units of the cell panels, and
a step of applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of cell panels.

In some embodiments, the thin film transistor (TFT) layer has a light-emitting layer, a gate electrode, and a source/drain electrode. Each of the plurality of display units may have a thin film transistor (TFT) layer, a planarization film formed on the TFT layer, and a light-emitting unit formed on the planarization film, in which the organic film applied to the interface portion is formed of a same material as a material of the planarization film and is formed at a same time as the planarization film is formed. In some embodiments, a light-emitting unit is connected to the TFT layer with a passivation layer and a planarization film therebetween, and an encapsulation layer that covers and protects the light-emitting unit. In some embodiments of the production method, the organic film is connected to neither the display units nor the

encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, a step of attaching a carrier substrate formed of a glass material to another surface of the base substrate, and before the cutting along the interface portion, a step of separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is produced. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer has a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit having the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses a soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the production method further includes a step of cutting along the interface portion, in which a groove is formed in the barrier layer, at least a portion of the organic film is formed in the groove, and the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is completely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In one embodiment, the organic film covering the groove at the interface portion and the planarization film are spaced apart from each other. For example, when the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely produced, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks can be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

Examples

The features of the present invention will be described more specifically with reference to Synthesis Examples and Examples given below. The materials, processes, procedures and the like shown below can be appropriately modified unless they deviate from the substance of the present invention. Accordingly, the scope of the present invention is not construed as being limited to the specific examples shown below. Hereunder, the light emission characteristics were evaluated using a source meter (available from Keithley Instruments, Inc.: 2400 series), a semiconductor parameter analyzer (available from Agilent Technologies, Inc., E5273A), an optical power meter device (available from Newport Corporation, 1930C), an optical spectroscope (available from Ocean Optics, Inc., USB2000), a spectroradiometer (available from Topcon Corporation, SR-3), and a streak camera (available from Hamamatsu Photonics K.K., Model C4334).

(Synthesis Example 1) Synthesis of Compound C1

Compound c

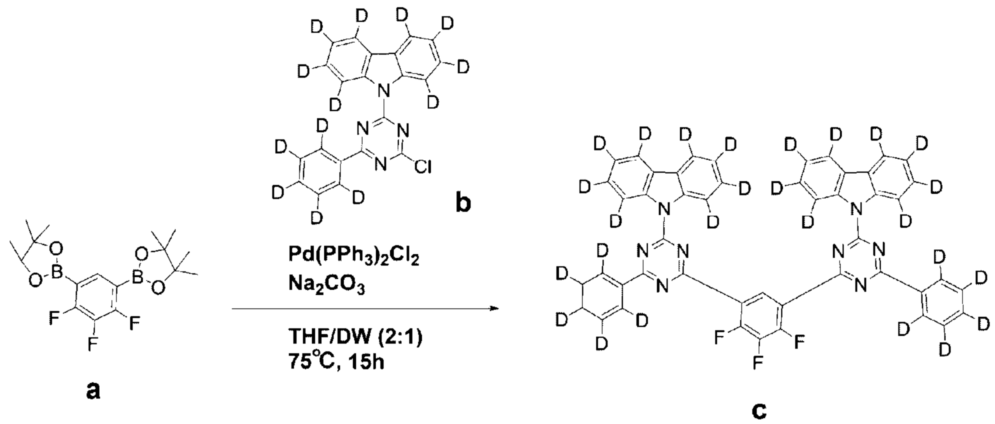


Compound a (5.29 g, 13.7 mmol) in a mixed solvent of tetrahydrofuran (THF, 140 mL) and water (70 mL) was subjected to nitrogen bubbling. To this solution, compound b (10.4 g, 28.1 mmol), bis(triphenylphosphine)palladium (II) dichloride (0.96 g, 1.37 mmol), and sodium carbonate (7.31 g, 69.0 mmol) were added under a nitrogen stream, and the mixture was heated and stirred at 75°C for 15 hours. The reaction vessel was cooled to room temperature and quenched by the addition of water. The solution was filtered and the obtained gray solid was separated by filtration, and washed with dichloromethane and ethyl acetate. The solid was dissolved in heated 1,2-dichlorobenzene (ODCB) and, when hot, passed through a funnel (Kiriyama funnel) packed with celite/silica gel/celite. The solution was concentrated under reduced pressure by an evaporator, and the obtained solid was washed with toluene and dried to obtain Compound c as a pale yellow solid (7.24 g, 9.06 mmol, yield 66%).
ASAP MS Spectral Analysis: $C_{48}HD_{26}F_3N_8$: theoretical value 798.39, observed value 799.46.

Compound C1

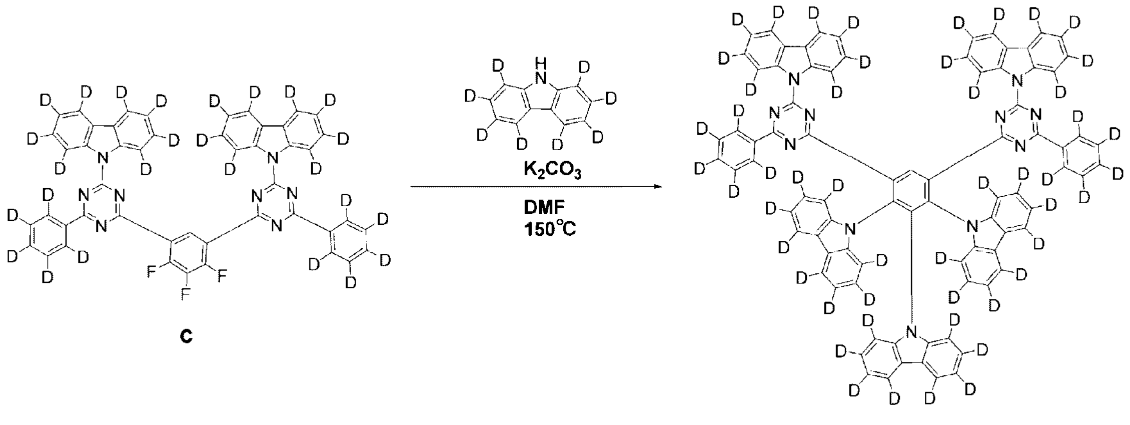


c

C1

Under a nitrogen stream, potassium carbonate (0.57 g, 4.15 mmol) was added to a solution of Compound c (0.83 g, 1.04 mmol) and carbazole-1,2,3,4,5,6,7,8-d8 (0.60 g, 3.42 mmol) in N,N-dimethylformamide (DMF, 40 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C1 (0.35 g, 0.276 mmol, yield 27%).

$^1$H-NMR (400 MHz, $CDCl_3$):$\delta$ 9.26 (s, 1H)
ASAP MS Spectral Analysis: $C_{84}HD_{50}N_{11}$: theoretical value 1263.7, observed value 1263.1.

(Synthesis Example 2) Synthesis of Compound C2

Compound C2

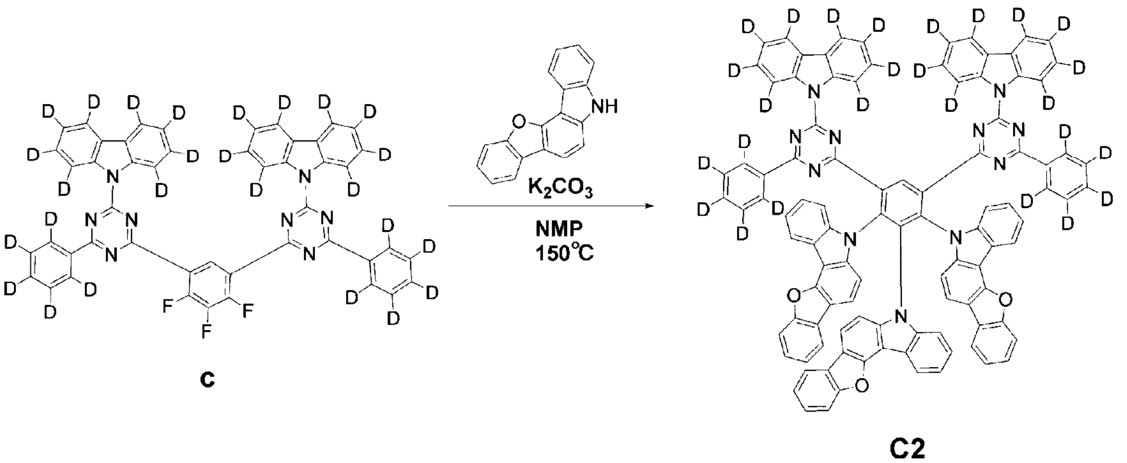


c

C2

Under a nitrogen stream, potassium carbonate (0.34 g, 2.50 mmol) was added to a solution of Compound c (0.50 g, 0.62 mmol) and 5H-benzofuro[3,2-c]carbazole (0.48 g, 1.87 mmol) in N-methyl-2-pyrrolidone (NMP, 30 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C2 (0.44 g, 0.29 mmol, yield 47%).
ASAP MS Spectral Analysis: $C_{102}H_{31}D_{26}N_{11}O_3$: theoretical value 1509.6, observed value 1510.7.

(Synthesis Example 3) Synthesis of Compound C3

Compound d

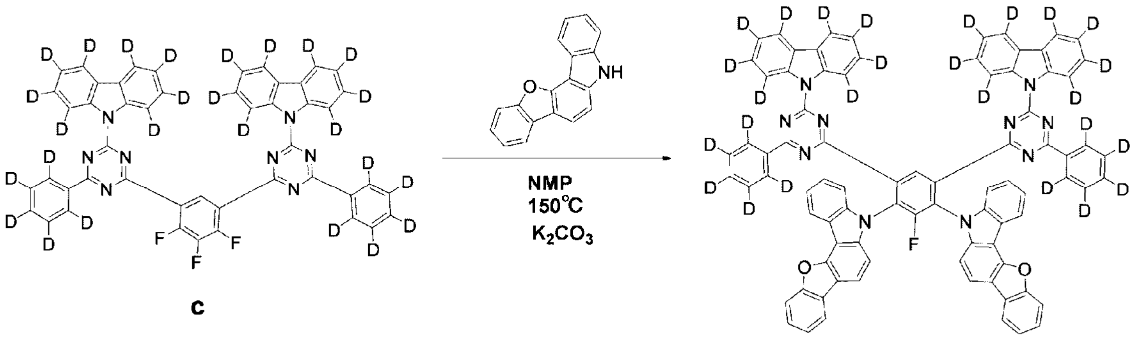


c

d

Under a nitrogen stream, potassium carbonate (0.86 g, 6.25 mmol) was added to a solution of Compound c (2.0 g, 2.50 mmol) and 5H-benzofuro[3,2-c]carbazole (1.28 g, 5.00 mmol) in NMP (90 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound d (1.93 g, 1.51 mmol, yield 61%).

$^1$H-NMR (400 MHz, $CDCl_3$): δ 9.40 (s, 1H), 8.40-8.30 (m,2H), 8.12-7.90 (m, 4H), 7.80-7.30 (m, 14H).
ASAP MS Spectral Analysis: $C_{84}H_{21}D_{26}FN_{10}O_2$: theoretical value 1272.5, observed value 1272.8.

Compound C3

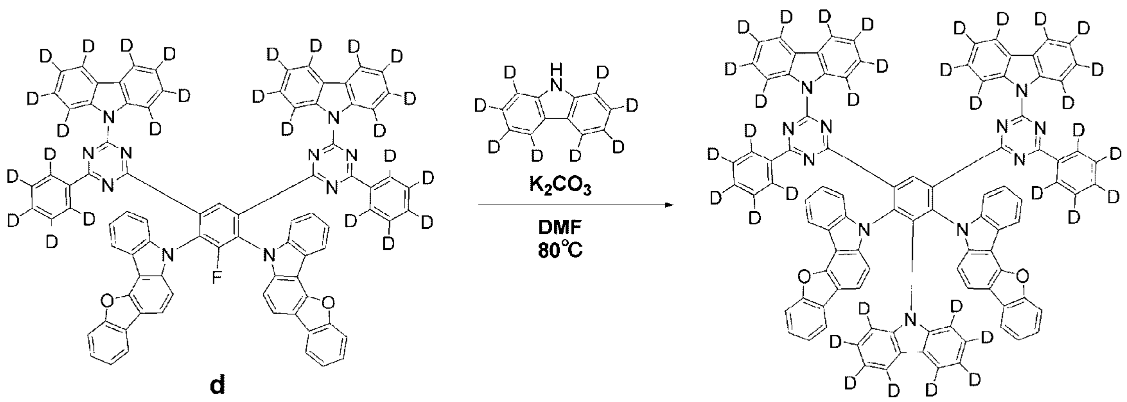


d

C3

Under a nitrogen stream, potassium carbonate (0.76 g, 5.52 mmol) was added to a solution of Compound d (1.76 g, 1.38 mmol) and carbazole-1,2,3,4,5,6,7,8-d8 (0.72 g, 4.14 mmol) in DMF (50 mL), and the mixture was stirred at 80°C for 72 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C3 (0.30 g, 0.21 mmol, yield 15%).

$^1$H-NMR (400 MHz, $CDCl_3$): δ 9.37 (s, 1H), 7.97 (dd,J=4.8Hz, 1.2 Hz, 2H), 7.80 (d, J=8.4Hz, 2H), 7.61-7.56 (m, 2H), 7.51 (d,J=8.4Hz, 2H), 7.45-7.35 (m, 4H),7.34-7.22 (m, 4H), 7.10-7.00 (m, 4H).
ASAP MS Spectral Analysis: $C_{96}H_{21}D_{34}N_{11}O_2$: theoretical value 1427.6, observed value 1427.8.

(Synthesis Example 4) Synthesis of Compound C4

Compound e

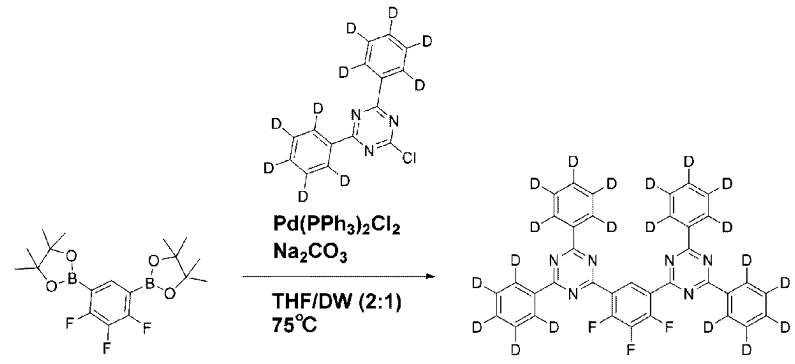


a

e

Compound a (2.30 g, 5.99 mmol) in a mixed solvent of THF (60 mL) and water (30 mL) was subjected to nitrogen bubbling. To this solution, 2-chloro-4,6-(diphenyl-2,3,4,5,6-d5)-1,3,5-triazine (3.39 g, 12.2 mmol), bis(triphenylphosphine) palladium (II) dichloride (0.42 g, 0.59 mmol), and sodium carbonate (3.17 g, 29.9 mmol) were added under a nitrogen stream, and the mixture was heated and stirred at 75°C for 18 hours. The reaction vessel was cooled to room temperature and quenched by the addition of water. The solution was filtered and the obtained gray solid was separated by filtration, and washed with dichloromethane and ethyl acetate. The solid was dissolved in heated ODCB and, when hot, passed through a funnel (Kiriyama funnel) packed with celite/silica gel/celite. The solution was concentrated under reduced pressure by an evaporator, and the obtained solid was washed with toluene and dried to obtain Compound e as a pale yellow solid (2.54 g, 4.13 mmol, yield 69%).
ASAP MS Spectral Analysis: $C_{36}HD_{20}F_3N_6$: theoretical value 614.30, observed value 615.40.

Compound f

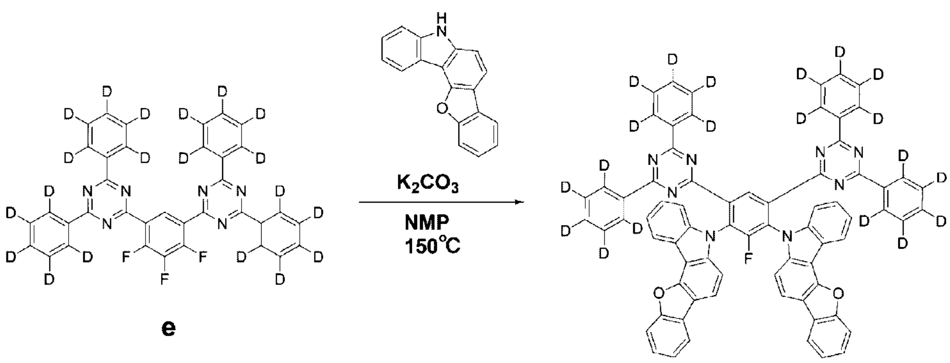


e

f

Under a nitrogen stream, potassium carbonate (1.12 g, 8.13 mmol) was added to a solution of Compound e (2.0 g, 3.25 mmol) and 5H-benzofuro[3,2-c]carbazole (1.67 g, 6.50 mmol) in NMP (120 mL), and the mixture was stirred at 150°C for 3 hours. The reaction solution was cooled to room temperature and quenched by the addition of water. The solution was filtered through a funnel packed with celite (Kiriyama funnel), and the solid remaining in the funnel was washed with water. This was dissolved in dichloromethane and dried over magnesium sulfate. The solution was filtered, and the obtained solution was concentrated under reduced pressure in an evaporator and then purified by column chromatography (toluene:hexane = 1:1), and the obtained solid was washed with methanol to obtain a yellow Compound f (2.61 g, 2.39 mmol, yield 73%).

$^1$H-NMR(400 MHz, $CDCl_3$): δ 9.61 (d, J=1.6 Hz, 1H), 8.46(d, J=7.6 Hz, 2H), 8.00-7.96 (m, 4H), 7.70 (d, J=8.4 Hz, 2H), 7.48-7.38 (m,12H).
ASAP MS Spectral Analysis: $C_{72}H_{21}D_{20}FN_8O_2$: theoretical value 1088.46, observed value 1089.66.

Compound C4

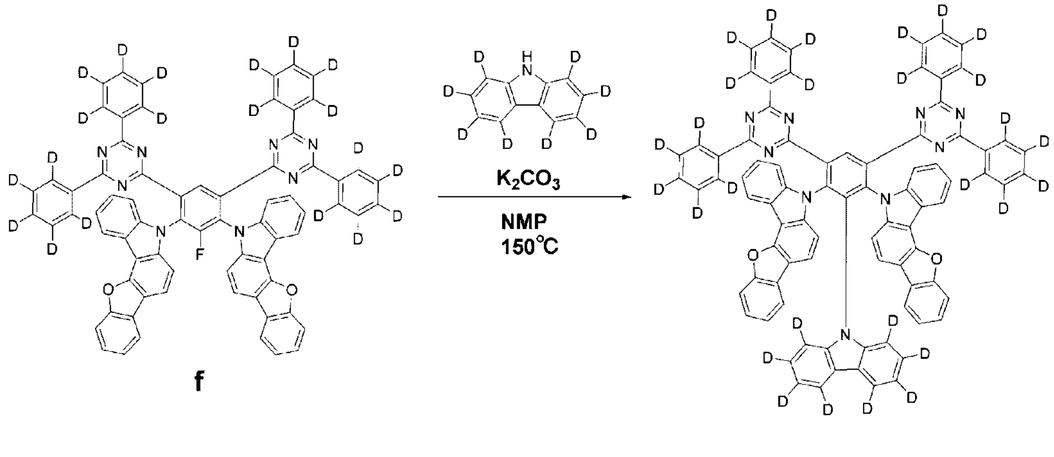


f

C4

Under a nitrogen stream, potassium carbonate (0.41 g, 2.96 mmol) was added to a solution of Compound f (1.61 g, 1.47 mmol) and carbazole-1,2,3,4,5,6,7,8-d8 (0.31 g, 1.77 mmol) in NMP (50 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C4 (1.59 g, 1.27 mmol, yield 86%).

> $^1$H-NMR (400 MHz, $CDCl_3$): δ 9.49 (s, 1H), 8.01 (d, J=7 Hz,2H), 7.83(d, J= 7Hz, 2H) 7.59-7.52(m, 4H), 7.38-7.28 (m, 8H), 7.10-7.12(m,4H).
> ASAP MS Spectral Analysis: $C_{84}H_{21}D_{28}N_9O_2$: theoretical value 1243.58, observed value 1244.73.

(Synthesis Example 5) Synthesis of Compound C5

Compound C5

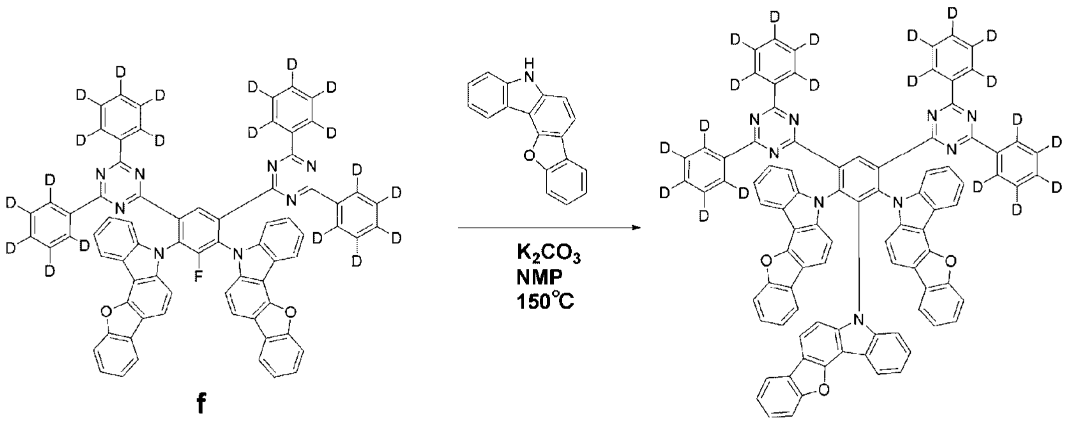


f

C5

Under a nitrogen stream, potassium carbonate (0.25 g, 1.83 mmol) was added to a solution of Compound f (1.0 g, 0.91 mmol) and 5H-benzofuro[3,2-c]carbazole (0.28 g, 1.10 mmol) in NMP (50 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator, then, purified by column chromatography (toluene: hexane = 1:1), washed with methanol, and recrystallized (toluene) to obtain a yellow Compound C5 (0.99 g, 0.746 mmol, yield 82%).

$^1$H-NMR (400 MHz, $CDCl_3$): δ 9.53 (s, 1H), 8.0-6.76(m, 30H)
ASAP MS Spectral Analysis: $C_{90}H_{31}D_{20}N_9O_3$: theoretical value 1325.54, observed value 1327.79.

(Synthesis Example 6) Synthesis of Compound C6

Compound h

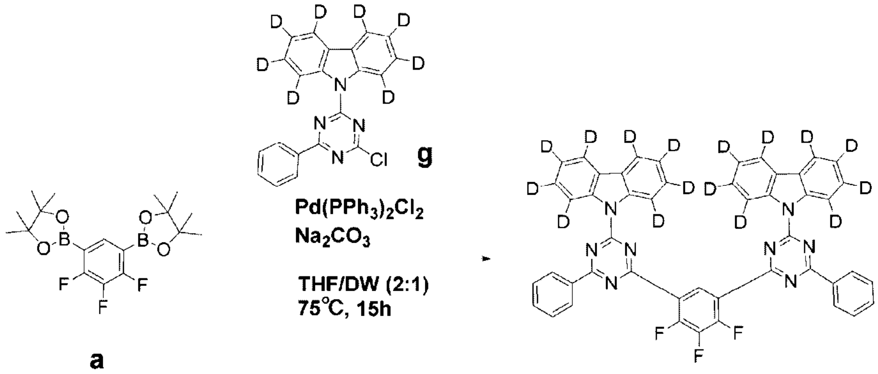


a g h

Compound a (4.84 g, 12.6 mmol) in a mixed solvent of THF (200 mL) and water (80 mL) was subjected to nitrogen bubbling. To this solution, compound g (9.40 g, 25.7 mmol), bis(triphenylphosphine)palladium (II) dichloride (0.88 g, 1.26 mmol), and sodium carbonate (6.69 g, 105.9 mmol) were added under a nitrogen stream, and the mixture was heated and stirred at 75°C for 15 hours. The reaction vessel was cooled to room temperature and quenched by the addition of water. The solution was filtered, and the obtained gray solid was separated by filtration and washed with water and toluene to obtain Compound h as a gray solid (9.2 g, 11.6 mmol, yield 92%).
ASAP MS Spectral Analysis: $C_{48}H_{11}D_{16}F_3N_8$: theoretical value 788.3, observed value 789.62.

Compound C6

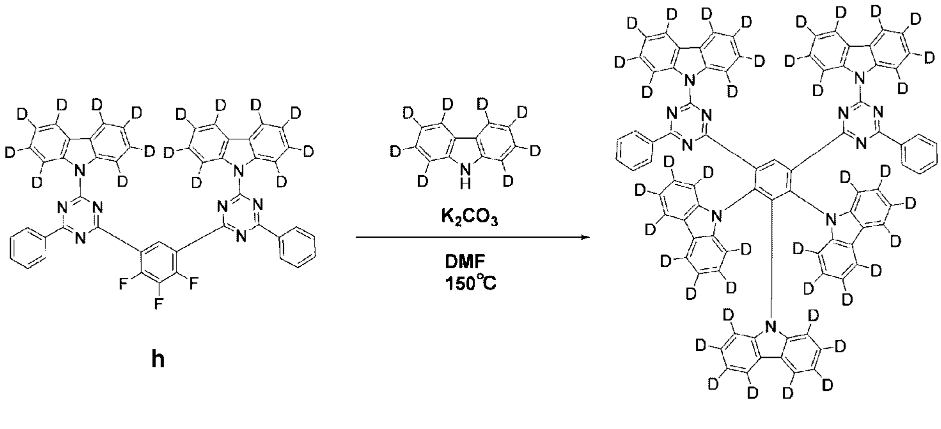


h C6

Under a nitrogen stream, potassium carbonate (1.05 g, 7.60 mmol) was added to a solution of Compound h (1.50 g, 1.90 mmol) and carbazole-1,2,3,4,5,6,7,8-d8 (1.33 g, 7.60 mmol) in DMF (70 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound C6 (0.66 g, 0.52 mmol, yield 28%).

$^1$H-NMR (400 MHz, $CDCl_3$): δ 9.25 (s, 1H), 7.52-7.48(m,4H), 7.42-7.38 (m, 2H), 7.26-7.16(m,4H)
ASAP MS Spectral Analysis: $C_{84}H_{11}D_{40}N_{11}$: theoretical value 1253.6, observed value 1254.0.

(Synthesis Example 7) Synthesis of Compound C7

Compound j

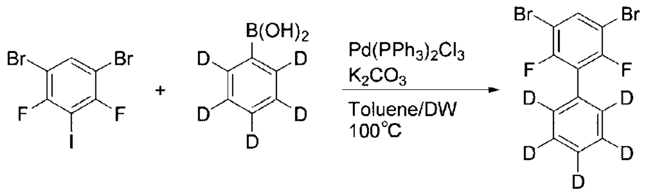


j

To a mixed solution of 1,5-dibromo-2,4-difluoro-3-iodobenzene (15.8 g, 39.9 mmol) in toluene (100 mL) and ion-exchanged water (30 mL), phenyl-d5-boronic acid (6.2 g, 48.8 mmol), bis(triphenylphosphine)palladium (II) dichloride (1.4 g, 2.0 mmol), and potassium carbonate (29.0 g, 210 mmol) were added, and the mixture was stirred at 100°C for 23 hours under a nitrogen atmosphere. The reaction solution was cooled to room temperature, and saturated saline water was added thereto to separate the solution into an organic phase and an aqueous phase. After the aqueous phase was extracted with toluene, the combined organic phase was dried over anhydrous magnesium sulfate and filtered, and then the filtrate was concentrated. The crude product was purified by silica gel chromatography (hexane) to obtain Compound j as a white solid (9.0 g, 25.5 mmol, yield 64%).

$^1$H-NMR (400 MHz, $CDCl_3$): δ 7.77 (t, J=6.8 Hz, 1H).
ASAP MS Spectral Analysis: $C_{12}HD_5Br_2F_2$: theoretical value 350.91, observed value 350.93 [M]

Compound k

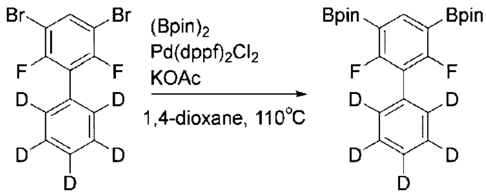


j k

A reaction mixture of Compound j (2.1 g, 5.98 mmol), [1,1'-bis(diphenylphosphino)ferrocene]dichloropalladium (II) (0.22 g, 0.30 mmol), potassium acetate (4.1 g, 42.0 mmol), bis(pinacolato)diboron (7.1 g, 27.9 mmol), and 1,4-dioxane (60 mL) was stirred at 110°C for 15 hours. The reaction mixture was cooled to room temperature, diluted with ethyl acetate, and filtered through silica. After the filtrate was concentrated, the obtained reaction mixture was dissolved in methylene chloride and subjected to silica filtration. The obtained solid was washed with hexane to obtain Compound k as a white solid (1.84 g, 4.11 mmol, yield 69%).
$^1$H-NMR (400 MHz, $CDCl_3$): δ 8.11 (t, J=7.2 Hz, 1H), 1.36(s, 24H).

Compound m

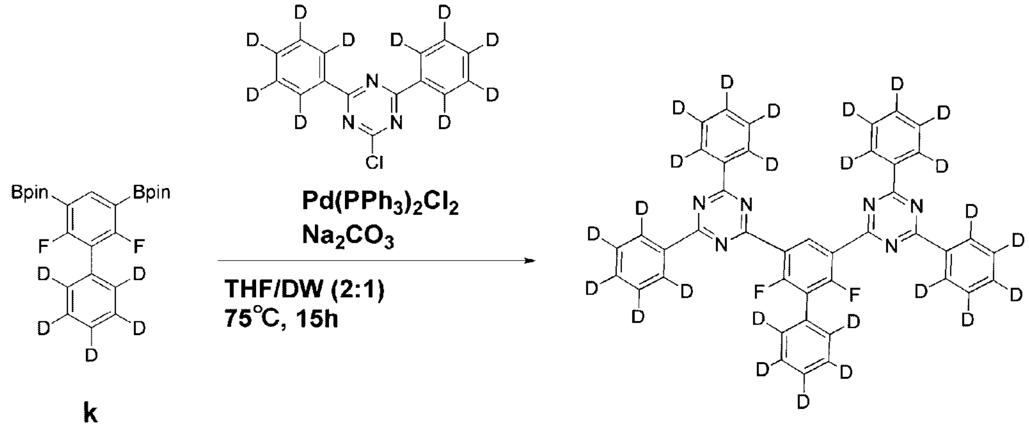


k

m

Compound k (8.00 g, 17.89 mmol) was dissolved in THF (138 mL) and ion-exchanged water (46 mL), and 2-chloro-4,6-bis(phenyl-d5)-1,3,5-triazine (4.35 g, 35.78 mmol), bis(triphenylphosphine)palladium (II) dichloride (0.63 g, 0.89 mmol) and sodium carbonate (9.48 g, 89.46 mmol) were added thereto, and the mixture was stirred at 75°C for 15 hours. The reaction vessel was cooled to room temperature, and the obtained gray solid was separated by filtration. The solid was washed with ion-exchanged water, methanol, and THF. The collected solid was stirred in hot toluene, and the solid was separated by filtration and washed with toluene to obtain Compound m as a black solid (4.1 g, 6.05 mmol, yield 34%).
ASAP MS Spectral Analysis: $C_{42}HD_{25}F_2N_6$: theoretical value 677, observed value 678 [M+H$^+$].

Compound C7

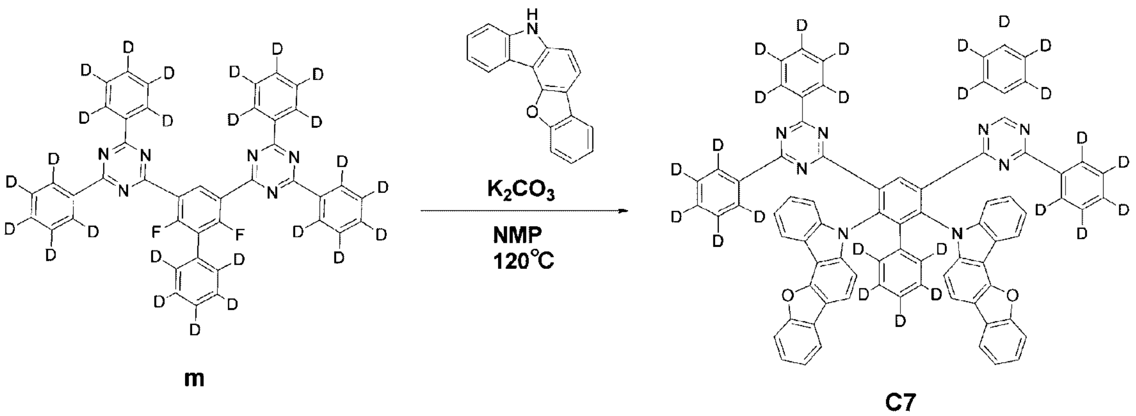


m

C7

Under a nitrogen stream, potassium carbonate (1.07 g, 7.74 mmol) was added to a solution of Compound m (1.75 g, 2.58 mmol) and 5H-benzofuro[3,2-c]carbazole (1.60 g, 6.22 mmol) in NMP (90 mL), and the mixture was stirred at 120°C for 18 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator, then, purified by column chromatography (toluene: hexane = 1:1), washed with methanol, and recrystallized (toluene) to obtain a yellow Compound C7 (0.80 g, 0.69 mmol, yield 27%).

$^1$H-NMR (400 MHz, $CDCl_3$): δ 9.51 (s, 1H), 7.57 (d,J=7.2Hz, 2H), 7.94-7.87 (m, 4H), 7.62 (d, J=7.2Hz, 2H), 7.38-7.24 (m, 12H).
ASAP MS Spectral Analysis: $C_{78}H_{21}D_{25}N_8O_2$: theoretical value 1151.5, observed value 1151.6.

(Synthesis Example 8) Synthesis of Compound C8

Compound n

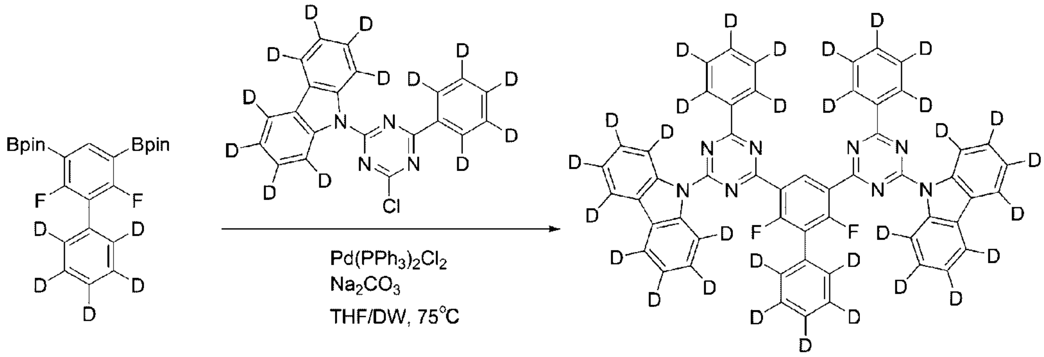


k

n

Compound k (1.81 g, 4.05 mmol) was dissolved in THF (30 mL) and ion-exchanged water (10 mL), and 9-(4-chloro-6-(phenyl-d5)-1,3,5-triazin-2-yl)-9H-carbazole-1,2,3,4,5,6,7,8-d8 (3.05 g, 8.25 mmol), bis(triphenylphosphine) palladium (II) dichloride (0.15 g, 0.21 mmol), and sodium carbonate (2.20 g, 20.8 mmol) were added thereto, and the mixture was stirred at 75°C for 16 hours. The reaction vessel was cooled to room temperature, and the obtained gray solid was separated by filtration. The solid was washed with ion-exchanged water, methanol, and THF. The collected solid was stirred in hot toluene, and the solid was separated by filtration and washed with toluene to obtain Compound n as a black solid (3.30 g, 3.82 mmol, yield 94%).
ASAP MS Spectral Analysis: $C_{54}HD_{31}F_2N_8$: theoretical value 861.47, observed value 862.72 [M+H$^+$].

Compound C8

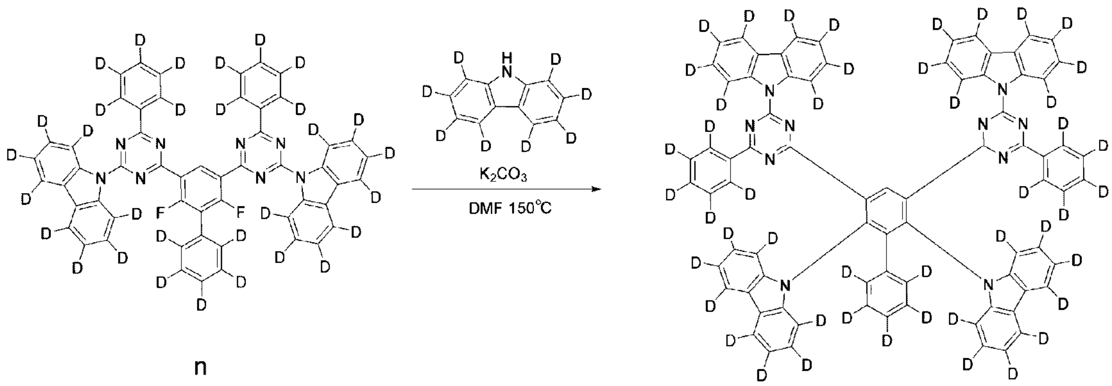


n

**C8**

To a mixture of Compound n (2.85 g, 3.30 mmol) and DMF (65 mL), carbazole-1,2,3,4,5,6,7,8-d8 (1.28 g, 7.30 mmol) and potassium carbonate (1.37 g, 9.91 mmol) were added, and the mixture was stirred at 150°C for 3 hours. The reaction solution was cooled to room temperature, and the solid was filtered and washed with ethyl acetate. The obtained filtrate was concentrated, methanol was added thereto, and the resulting solid was separated by filtration and washed with methanol. The obtained crude product was purified by column chromatography (toluene/hexane = 3:1), and the obtained solid was reprecipitated with ethyl acetate/hexane, thereby obtaining 2.13 g (1.81 mmol, yield 55%) of a pale green Compound C8.

$^1$H-NMR (400 MHz, DMSO-d6): δ9.20 (s, 1H).
ASAP MS Spectral Analysis: $C_{78}HD_{47}N_{10}$: theoretical value 1171.70, observed value 1172.15 [M+H$^+$].

(Synthesis Example 9) Synthesis of Compound C9

Compound p

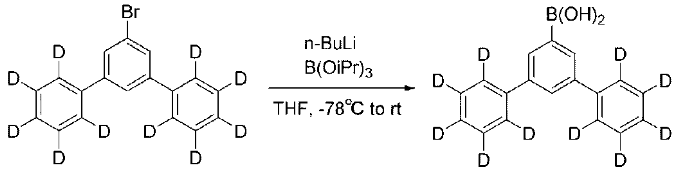


p

A 2.3M solution of n-butyllithium in cyclohexane (21 mL, 48.3 mmol) was slowly added dropwise to a THF (75 mL) solution of 5'-bromo-1,1':3',1"-terphenyl-2,2",3,3",4,4",5,5",6,6"-d10 (12.0 g, 37.6 mmol) under a nitrogen atmosphere at -78°C. After stirring for 80 minutes, triisopropyl borate (12.8 mL, 56.4 mmol) was added, and the temperature of the reactor was raised to room temperature. After stirring for 2 hours, the mixture was cooled to 0°C, and a 4N hydrochloric acid aqueous solution (70 mL) was added thereto. After stirring for 30 minutes at room temperature, the organic and aqueous phases were separated. The aqueous phase was extracted with diethyl ether, and the combined organic phase was washed with ion-exchanged water and saturated saline water. The organic phase was concentrated, and the obtained white solid was washed with hexane to obtain a white Compound p (9.30 g, 32.7 mmol, yield 87%).
$^1$H-NMR (400 MHz, DMSO-d6): $\delta$8.26 (brs, 2H), 8.08 (d, J=1.6 Hz, 2H),7.92-7.93 (m, 1H).

Compound q

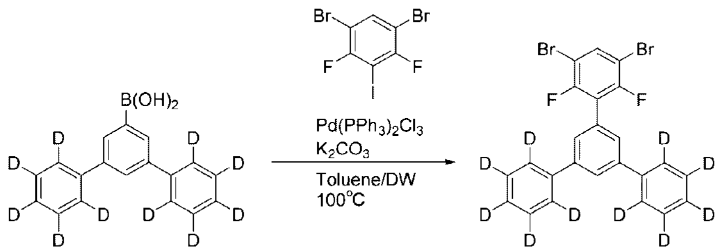


p q

To a mixed solution of 1,5-dibromo-2,4-difluoro-3-iodobenzene (5.1 g, 13.0 mmol) in toluene (30 mL) and ion-exchanged water (10 mL), Compound p (4.0 g, 14.0 mmol), bis(triphenylphosphine)palladium (II) dichloride (0.46 g, 0.64 mmol), and potassium carbonate (3.60 g, 26.0 mmol) were added, and the mixture was stirred at 100°C for 15 hours. The reaction solution was cooled to room temperature, and saturated saline water was added thereto to separate the solution into an organic phase and an aqueous phase. After the aqueous phase was extracted with ethyl acetate, the combined organic phase was dried over anhydrous magnesium sulfate and filtered, and then the filtrate was concentrated. The crude product was purified by silica gel chromatography (hexane/ethyl acetate = 40:1) and (hexane/methylene chloride = 10:1), and the obtained white solid was washed with hexane to obtain 4.84 g (9.48 mmol, yield 73%) of Compound q.

$^1$H-NMR (400 MHz, $CDCl_3$): $\delta$ 7.88 (t, J=2.0 Hz, 1H), 7.81(t, J=6.8 Hz, 1H), 7.62 (q, J=2.0 Hz, 2H).
ASAP MS Spectral Analysis: $C_{24}H_4D_{10}Br_2F_2$: theoretical value 508.00, observed value 509.03 [M+H$^+$].

Compound r

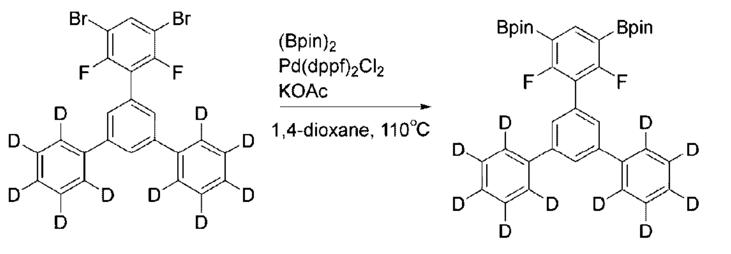


q r

A reaction mixture of Compound q (4.8 g, 9.40 mmol), [1,1'-bis(diphenylphosphino)ferrocene]dichloropalladium (II) (0.36 g, 0.50 mmol), potassium acetate (6.5 g, 66.2 mmol), bis(pinacolato)diboron (12.1 g, 47.6 mmol), and 1,4-dioxane

(100 mL) was stirred at 110°C for 15 hours. The reaction mixture was cooled to room temperature, diluted with ethyl acetate, and filtered through silica. After the filtrate was concentrated, the obtained reaction mixture was dissolved in methylene chloride and subjected to silica filtration. The obtained solid was washed with hexane to obtain Compound r as a yellow solid (5.00 g, 8.27 mmol, yield 88%).

$^1$H-NMR (400 MHz, $CDCl_3$): δ 8.15 (t, J=6.8 Hz, 1H),7.79-7.80 (m, 1H), 7.66 (brs, 2H), 1.26 (s, 24H).
ASAP MS Spectral Analysis: $C_{36}H_{28}D_{10}B_2F_2O_4$: theoretical value 604.37, observed value 605.47 [M+H$^+$].

Compound s

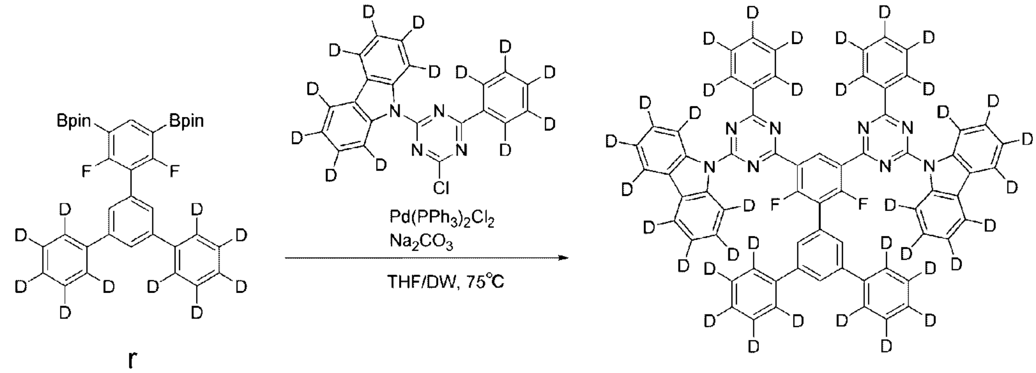


r

s

Compound r (4.98 g, 8.24 mmol) was dissolved in THF (60 mL) and ion-exchanged water (20 mL), and 9-(4-chloro-6-(phenyl-d5)-1,3,5-triazin-2-yl)-9H-carbazole-1,2,3,4,5,6,7,8-d8 (6.10 g, 16.5 mmol), bis(triphenylphosphine) palladium (II) dichloride (0.29 g, 0.41 mmol), and sodium carbonate (4.37 g, 41.2 mmol) were added thereto, and the mixture was stirred at 75°C for 20 hours. The reaction vessel was cooled to room temperature, and the obtained gray solid was separated by filtration. The solid was washed with THF and ion-exchanged water. The collected solid was stirred in hot toluene, and the solid was separated by filtration, washed with toluene, and dried to obtain Compound s as a gray solid (7.98 g, 7.83 mmol, yield 95%).
ASAP MS Spectral Analysis: $C_{66}H_4D_{36}F_2N_8$: theoretical value 1018.56, observed value 1019.83 [M+H$^+$].

Compound C9

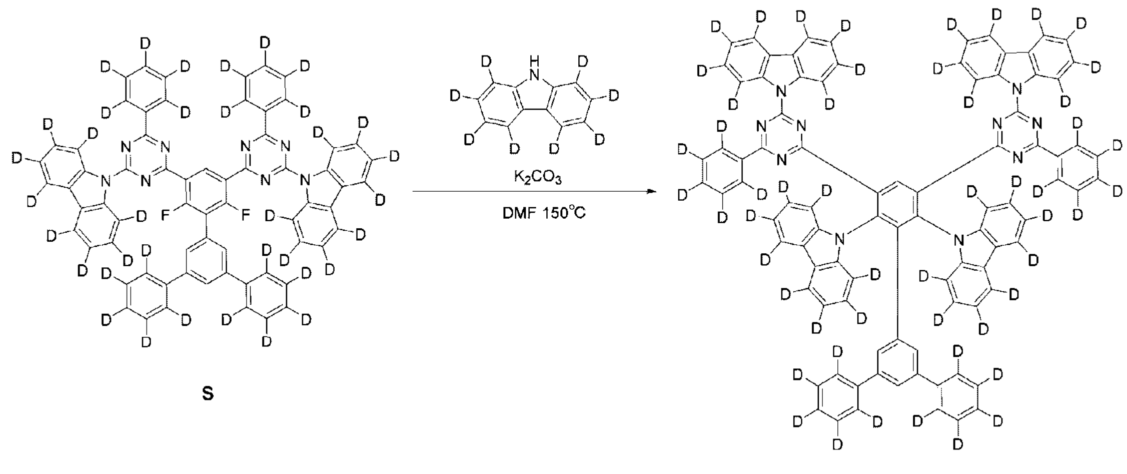


s

C9

To a mixture of Compound s (3.06 g, 3.00 mmol) and DMF (60 mL), carbazole-1,2,3,4,5,6,7,8-d8 (1.16 g, 6.61 mmol) and potassium carbonate (1.24 g, 8.97 mmol) were added, and the mixture was stirred at 150°C for 3 hours. The reaction solution was cooled to room temperature, and the solid was filtered and washed with ethyl acetate. The obtained filtrate was concentrated, methanol was added thereto, and the resulting solid was separated by filtration and washed with methanol. The obtained crude product was purified by column chromatography (toluene/hexane = 1:1 to 2:1), and the obtained solid was reprecipitated with ethyl acetate/hexane, thereby obtaining 1.47 g (1.10 mmol, yield 37%) of a green Compound C9.

$^1$H-NMR (400 MHz, DMSO-d6): δ9.40 (s, 1H), 7.14 (d, J=1.6 Hz, 2H), 7.07 (d, J=1.6 Hz, 1H).
ASAP MS Spectral Analysis: $C_{90}H_4D_{52}N_{10}$: theoretical value 1328.80, observed value 1329.84 [M+H$^+$].

(Synthesis Example 10) Synthesis of Compound C10

Compound t

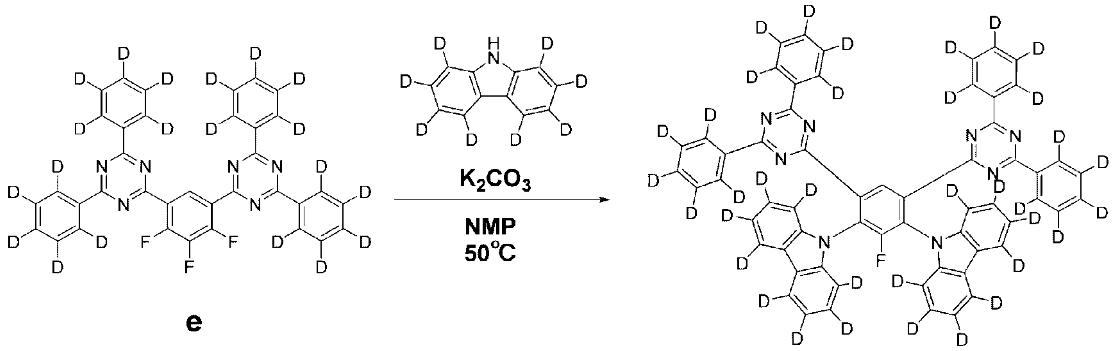


e

t

Under a nitrogen stream, potassium carbonate (3.37 g, 24.4 mmol) was added to a solution of Compound e (5.0 g, 8.13 mmol) and carbazole-1,2,3,4,5,6,7,8-d8 (2.85 g, 16.2 mmol) in NMP (30 mL), and the mixture was stirred at 50°C for 15 hours. The reaction solution was cooled to room temperature and quenched by the addition of water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound t (4.80 g, 5.18 mmol, yield 63%).

$^1$H-NMR (400 MHz, $CDCl_3$): δ 9.50 (dd, J=1.6 Hz, 1H).
ASAP MS Spectral Analysis: $C_{60}HD_{36}FN_8$: theoretical value 924.5, observed value 924.7.

Compound C10

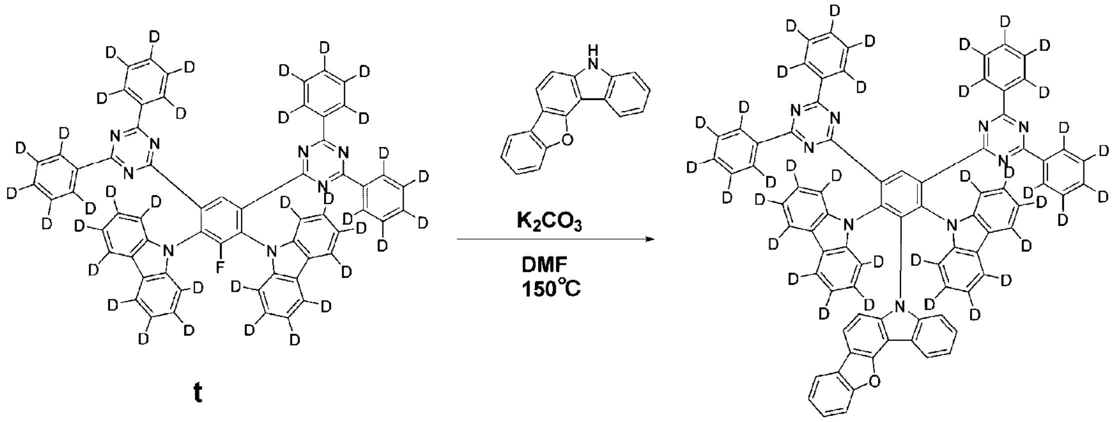


t

C10

Under a nitrogen stream, potassium carbonate (0.87 g, 6.27 mmol) was added to a solution of Compound t (1.45 g, 1.57 mmol) and 5H-benzofuro[3,2-c]carbazole (1.21 g, 4.70 mmol) in DMF (50 mL), and the mixture was stirred at 150°C for 3 hours. The reaction solution was cooled to room temperature and quenched by the addition of water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound C10 (1.47 g, 1.26 mmol, yield 81%).

$^1$H-NMR (400 MHz, $CDCl_3$): δ 9.44 (s, 1H), 7.87 (d, J=7.2Hz, 1H), 7.74(d, J= 7.2Hz, 1H) 7.54-7.49(m, 1H), 7.32-7.22 (m, 5H), 6.93 (t,J=7.2 Hz, 1H), 6.78 (t, J=7.2 Hz, 1H).
ASAP MS Spectral Analysis: $C_{78}H_{11}D_{36}N_9O$: theoretical value 1161.6, observed value 1162.1.

(Synthesis Example 11) Synthesis of Compound C11

Compound C11

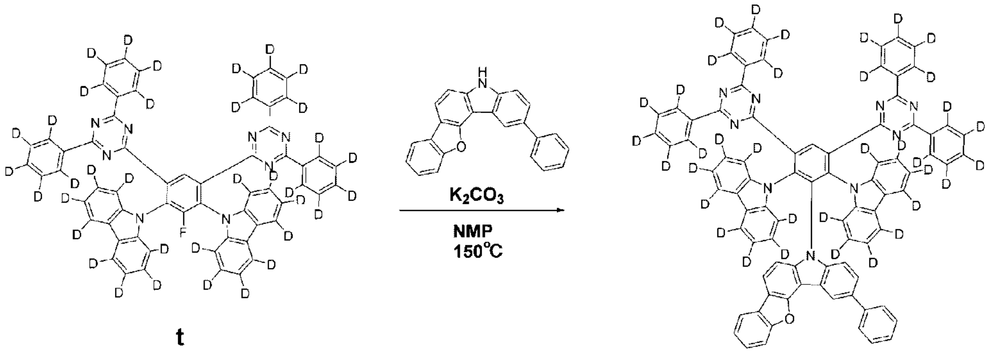


t

C11

Under a nitrogen stream, potassium carbonate (0.60 g, 4.32 mmol) was added to a solution of Compound t (1.0 g, 1.08 mmol) and 2-phenyl-5H-benzofuro[3,2-c]carbazole (1.08 g, 3.24 mmol) in DMF (40 mL), and the mixture was stirred at 150°C for 3 hours. The reaction solution was cooled to room temperature and quenched by the addition of water. The solution was filtered and the solid remaining in the funnel was washed with water. The solution was filtered and the solid remaining in the funnel was washed with water and methanol. The solid was dissolved in heated ODCB, and the solution was charged and purified by column chromatography (toluene:hexane = 1:1). The purified product was further recrystallized from toluene to obtain a yellow Compound C11 (0.98 g, 0.08 mmol, yield 73%).

$^1$H-NMR (400 MHz, $CDCl_3$): δ 9.45 (s, 1H), 8.08 (d, J=1.2Hz, 1H), 7.75 (d, J=6.8 Hz, 1H), 7.54-7.51(m, 3H), 7.40(t, J=7.2 Hz, 2H)7.34-7.24 (m, 6H), 7.04-7.00(m, 1H).
ASAP MS Spectral Analysis: $C_{84}H_{15}D_{36}N_9O$: theoretical value 1237.6, observed value 1239.2.

(Synthesis Example 12) Synthesis of Compound C12

Compound v

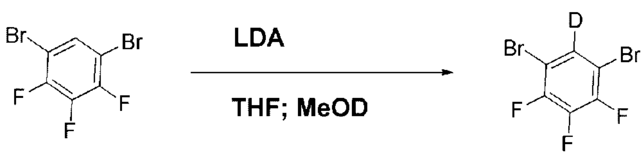


u

v

A solution of Compound u (25.7 g, 88.6 mmol) in THF (295 mL) was subjected to nitrogen bubbling, and the solution was cooled to -78°C. Under a nitrogen stream, 1M lithium diisopropylamide (LDA, 99.6 mL, 99.6 mmol) was added dropwise to this solution over 30 minutes. After the mixture was further stirred at -78°C for 1 hour, $CH_3OD$ (7.3 g, 221 mmol) was added thereto, and the mixture was stirred overnight. After the mixture was warmed to room temperature and a saturated aqueous ammonium chloride solution was added thereto, THF was distilled off by an evaporator. Dichloromethane and water were added to extract an organic layer, which was then washed with saturated saline water and dried over sodium sulfate. The solution was concentrated and purified by silica gel chromatography. The purified product was further purified by distillation under reduced pressure to obtain Compound v as a transparent liquid (10 g, 34.4 mmol, yield 36.6%).

Compound x

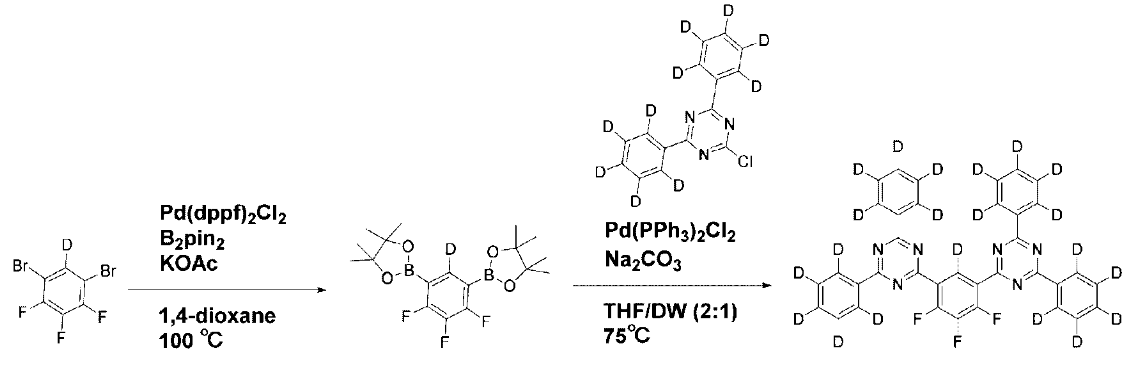


v w x

A reaction mixture of Compound v (9 g, 30.9 mmol), [1,1'-bis(diphenylphosphino)ferrocene]dichloropalladium (II) (2.3 g, 3.09 mmol), potassium acetate (21.1 g, 21.1 mmol), bis(pinacolato)diboron (39.1 g, 154 mmol), and 1,4-dioxane (310 mL) was stirred at 110°C for 15 hours. The reaction mixture was cooled to room temperature, diluted with ethyl acetate, and filtered through silica. The obtained solid was washed with hexane to obtain Compound w as a white solid (10.0 g, 25.9 mmol, yield 76%).

Compound w (10.0 g, 25.9 mmol) was dissolved in THF (260 mL) and ion-exchanged water (130 mL), and 2-chloro-4,6-bis(phenyl-d5)-1,3,5-triazine (17.9 g, 64.7 mmol), bis(triphenylphosphine)palladium (II) dichloride (1.81 g, 2.59 mmol) and sodium carbonate (8.22 g, 77.6 mmol) were added thereto, and the mixture was stirred at 75°C for 15 hours. The reaction vessel was cooled to room temperature, and the reaction solution was concentrated. The obtained gray solid was separated by filtration, and the solid was washed with ion-exchanged water, methanol, and THF. The collected solid was stirred in hot toluene, and the solid was separated by filtration and washed with toluene to obtain Compound x as a black solid (1.86 g, 3.02 mmol, yield 11.6%). ASAP MS Spectral Analysis: $C_{36}D_{21}F_3N_6$: theoretical value 615.31, observed value 616.41.

Compound y

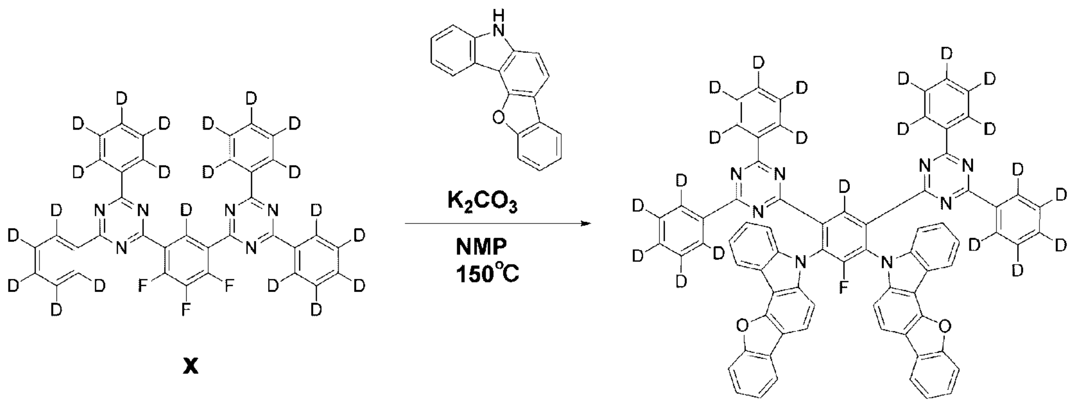


x

y

Under a nitrogen stream, a solution of Compound x (1.32 g, 2.14 mmol) and 5H-benzofuro[3,2-c]carbazole (1.37 g, 5.35 mmol) in THF (71 mL) was cooled to -25°C, and a solution of potassium tert-butoxide (0.72 g, 6.42 mmol) in THF (71 mL) was added dropwise. After the temperature was raised to room temperature, the mixture was stirred for 15 hours. After that, water was added, the suspension was filtered, and the solid remaining in the funnel was washed with water and methanol. This was dissolved in dichloromethane, purified by column chromatography (toluene:hexane = 1:1), and the obtained solid was washed with methanol to obtain a yellow Compound y (1.18 g, 1.08 mmol, yield 51%).

$^1$H-NMR(400 MHz, $CDCl_3$): 8.46 (d, J=7.6 Hz, 2H), 8.01-7.95(m, 4H), 7.71 (d, J=8.4 Hz, 2H), 7.52-7.35 (m, 12H). ASAP MS Spectral Analysis: $C_{72}H_{20}D_{21}FN_8O_2$: theoretical value 1089.47, observed value 1090.61.

Compound C12

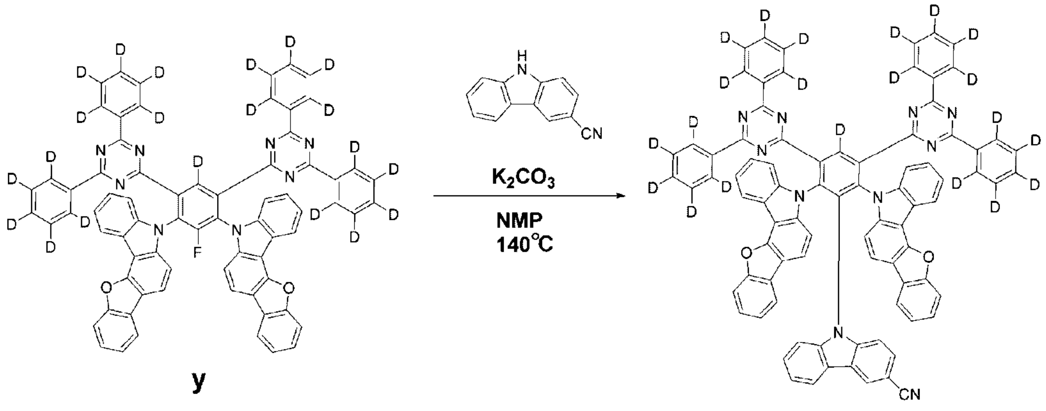


y

C12

Under a nitrogen stream, potassium carbonate (0.15 g, 1.09 mmol) was added to a solution of Compound y (0.60 g, 0.55 mmol) and 9H-carbazole-3-carbonitrile (0.16 g, 0.84 mmol) in NMP (5.5 mL), and the mixture was stirred at 140°C for 15 hours. The reaction solution was cooled to room temperature and quenched by the addition of water. The solution was filtered and the solid remaining in the funnel was washed with water and methanol. The solid was dissolved in dichloromethane and dried over magnesium sulfate. After filtration, the solution was concentrated under reduced pressure in an evaporator and then purified by column chromatography (toluene:hexane = 1:1) to obtain a yellow Compound C12 (0.51 g, 0.40 mmol, yield 74%).

$^1$H-NMR (400 MHz, $CDCl_3$): δ 8.07-8.00 (m, 2H), 7.84 (dd,J=7.2 Hz, 12.8 Hz, 2H), 7.69 (s, 1H), 7.63-7.50(m, 4H), 7.43-7.19 (m, 11H),7.16.-6.69(m, 7H).
ASAP MS Spectral Analysis: $C_{85}H_{27}D_{21}N_{10}O_2$: theoretical value 1261.53, observed value 1262.77.

(Synthesis Example 13) Synthesis of Compound C13

Compound C13

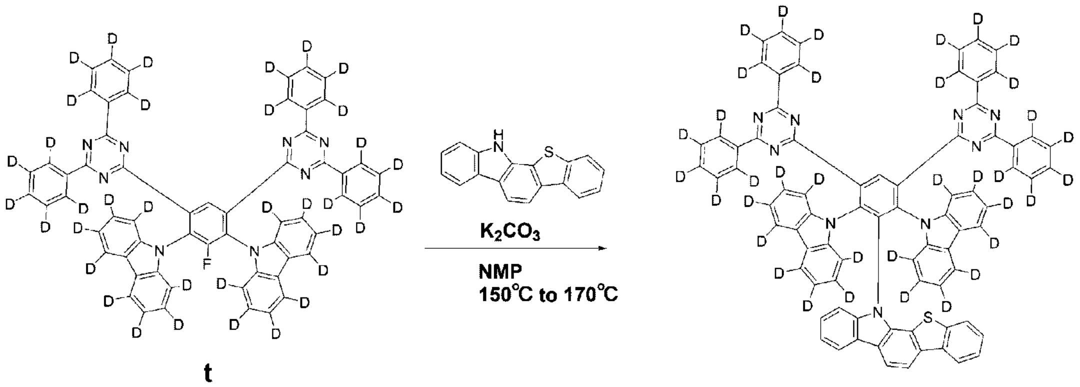


t

C13

Under a nitrogen stream, potassium carbonate (0.45 g, 3.23 mmol) was added to a solution of Compound t (1.0 g, 1.08 mmol) and 12H-[1]benzothieno[2,3-a]carbazole (0.59 g, 2.16 mmol) in NMP (11 mL), and the mixture was stirred at 150°C for 15 hours. Further, the temperature was raised to 170°C and stirred for 3 hours. The reaction solution was cooled to room temperature and quenched by the addition of water. The solution was filtered and the solid remaining in the funnel was washed with water and methanol. The solution was filtered and the solid remaining in the funnel was washed with water and methanol. The solid was dissolved in heated ODCB, and the solution was charged and purified by column chromatography (toluene:hexane = 1:1) to obtain a yellow solid Compound C13 (0.35 g, 0.29 mmol, yield 27%).

$^1H$-NMR (400 MHz, $CDCl_3$): δ 9.73 (s, 1H), 7.93-7.90 (m, 1H) 7.83-7.80 (m, 1H), 7.61 (m, 5H), 7.08 (d, J = 8 Hz, 1H), 6.90-6.84(m, 1H),6.81-6.75 (m, 1H).
ASAP MS Spectral Analysis: $C_{78}H_{11}D_{36}N_9S$: theoretical value 1177.59, observed value 1178.71.

(Synthesis Example 14) Synthesis of Compound C14

Compound C14

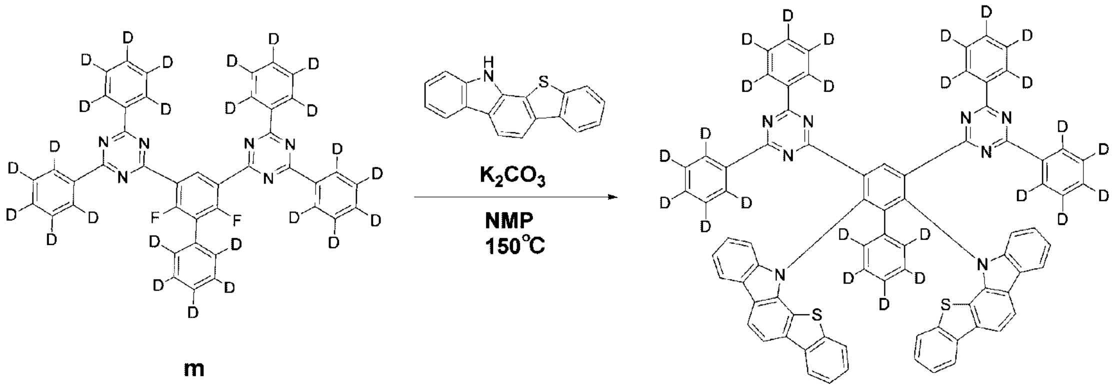


m

C14

Under a nitrogen stream, potassium carbonate (2.41 g, 8.85 mmol) was added to a solution of Compound m (2 g, 2.95 mmol) and 12H-[1]benzothieno[2,3-a]carbazole (1.60 g, 6.22 mmol) in NMP (90 mL), and the mixture was stirred at 150°C for 15 hours. The reaction solution was cooled to room temperature and quenched by the addition of water. The solid was dissolved in heated ODCB, and the solution was charged and purified by column chromatography (toluene:hexane = 1:1) to obtain a yellow Compound C14 (1.53 g, 1.57 mmol, yield 53%).

$^1H$-NMR (400 MHz, $CDCl_3$): δ10.18 (s, 1H, major rotamer),9.95 (s, 1H, minor rotamer), 8.21-8.15 (m, 2H), 7.97-7.91(m, 7H), 7.81-7.77 (m,1H), 7.54-7.29 (m, 8H), 7.22-7.17 (m, 2H).
ASAP MS Spectral Analysis: $C_{78}H_{21}D_{25}N_8S_2$: theoretical value 1183.49, observed value 1184.75.

(Synthesis Example 15) Synthesis of Comparative Compound A

Compound z

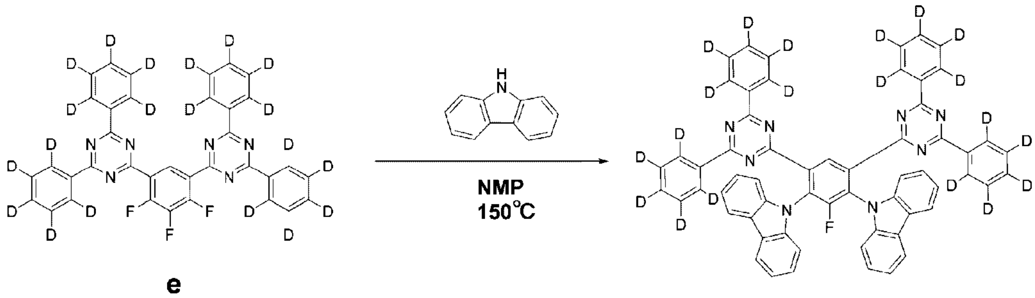


e

z

Under a nitrogen stream, potassium carbonate (0.84 g, 6.10 mmol) was added to a solution of Compound e (1.50 g, 2.44 mmol) and 9H-carbazole (0.81 g, 4.88 mmol) in NMP (90 mL), and the mixture was stirred at 150°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Compound z (1.27 g, 1.39 mmol, yield 57%).

$^1$H-NMR (400 MHz, $CDCl_3$): δ 9.51 (d, J=1.6 Hz, 1H), 8.03 (d, J=8.0 Hz, 4H), 7.40-7.37 (m, 8H), 7.28-7.23 (m, 4H). ASAP MS Spectral Analysis: $C_{60}H_{17}D_{20}FN_8$: theoretical value 908.4, observed value 909.75.

Comparative Compound A

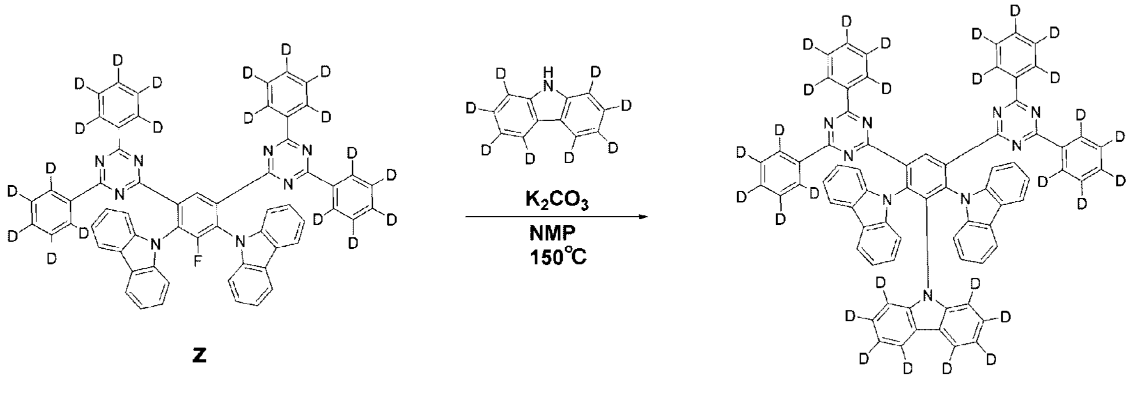


z

A

Under a nitrogen stream, potassium carbonate (0.31 g, 2.26 mmol) was added to a solution of Compound z (1.03 g, 1.13 mmol) and carbazole-1,2,3,4,5,6,7,8-d8 (0.29 g, 1.69 mmol) in NMP (50 mL), and the mixture was stirred at 130°C for 3 hours. The reaction solution was cooled to room temperature and quenched by the addition of water. The solution was filtered and the solid remaining in the funnel was washed with water. The solid was dissolved in dichloromethane and dried over magnesium sulfate. The solution was concentrated under reduced pressure in an evaporator and then purified by column chromatography (toluene:hexane = 1:1) to obtain a yellow Comparative compound A (0.78 g, 0.73 mmol, yield 65%).

$^1$H-NMR (400 MHz, $CDCl_3$): δ 9.40 (d, J=4.8 Hz, 1H), 7.57(dd, J=4.8Hz, 1.2 Hz, 4H), 7.28-7.24 (m, 4H), 6.98-6.94 (m, 8H).
ASAP MS Spectral Analysis: $C_{72}H_{17}D_{28}N_9$: theoretical value 1063.5, observed value 1063.7.

(Example 1) Production and Evaluation of Organic Electroluminescent Device

On a glass substrate on which an anode made of indium-tin oxide (ITO) having a film thickness of 50 nm was formed, each thin film was laminated by a vacuum deposition method at a vacuum degree of $5.0 \times 10^{-5}$ Pa. First, HAT-CN was formed to a thickness of 10 nm on the ITO, NPD was formed to a thickness of 30 nm thereon, further Tris-PCz was formed to a thickness of 10 nm, and further H1 was formed to a thickness of 5 nm. Next, H1, Compound C1 and a dopant EM1 were co-deposited from different vapor deposition sources to form a layer with a thickness of 40 nm as a light-emitting layer. In the light-emitting layer, the content of H1 was 44.2% by mass, the content of Compound C1 was 55.0% by mass, and the content of EM1 was 0.8% by mass. Next, after SF3-TRZ was formed at a thickness of 10 nm, Liq and SF3-TRZ were co-deposited from different vapor deposition sources to form a layer with a thickness of 30 nm. The contents of Liq and SF3-TRZ in this layer were 30% by mass and 70% by mass, respectively. Furthermore, Liq was formed to a thickness of 2 nm, and aluminum (Al) was vapor-deposited to a thickness of 100 nm to form a cathode, thereby obtaining an organic electroluminescent device.

When an organic electroluminescent device produced using Compound C1 was driven, green delayed fluorescence was observed. When driven at 6.3 mA/cm$^2$, the measured voltage was 3.82 V. In addition, when driven at 6.3 mA/cm$^2$, the measured external quantum efficiency (EQE) was as high as 24.7%. These results show that the organic light-emitting device using the compound represented by the general formula (1) has excellent light emission characteristics.

(Example 2) Production and Evaluation of Organic Electroluminescent Device

Instead of the light-emitting layer of the organic electroluminescent device of Test Example 1, H2, Compound C1, and the dopant EM1 were co-deposited from different vapor deposition sources to form a light-emitting layer with a thickness of 40 nm in which the content of H2 was 64.2% by mass, the content of Compound C1 was 35.0% by mass, and the content of EM1 was 0.8% by mass. Otherwise, an organic electroluminescent device was produced according to the same procedure as in Example 1.

Further, instead of forming the light-emitting layer of the organic electroluminescent device of Example 1, H2, Compound C3, and the dopant EM1 were co-deposited from different vapor deposition sources to form a light-emitting layer with a thickness of 40 nm in which the content of H2 was 74.2% by mass, the content of Compound C3 was 25.0% by mass, and the content of EM1 was 0.8% by mass. Otherwise, an organic electroluminescent device was produced according to the same procedure as in Example 1. Furthermore, organic electroluminescent devices were produced in the same manner as above except for using Compound C4, Compound C5, Compound C12, Compound C14, and Comparative Compound A in place of Compound C3.

Each of these devices was driven at 25.2 mA/cm$^2$, and the time (LT95) from the start of driving until the light emission intensity reached 95% was measured. The measurement results are shown in Table 9 as relative values when LT95 of the organic electroluminescent device using Comparative Compound A is set to 1. The results in Table 9 show that the organic light-emitting device using the compound represented by the general formula (1) has a long lifetime and is excellent.

[Table 9]

| Compound used | Lifetime (relative value) |
|---|---|
| Compound C1 | 12.7 |
| Compound C3 | 10.4 |
| Compound C4 | 11.6 |
| Compound C5 | 10.9 |
| Compound C12 | 14.5 |
| Compound C14 | 9.5 |
| Comparative Compound A | 1 |

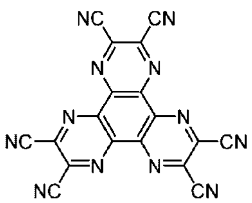
HAT-CN

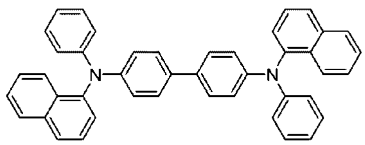
NPD

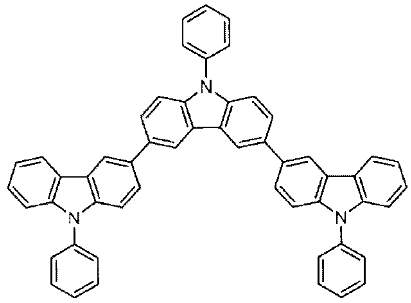
TrisPCz

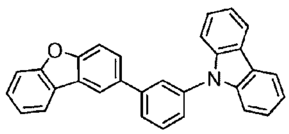
H1

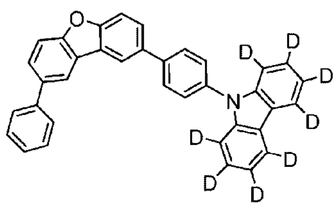
H2

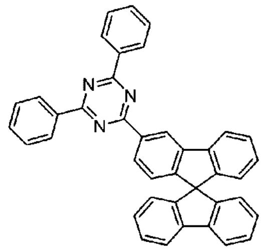
SF3-TRZ

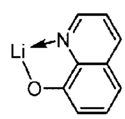
Liq

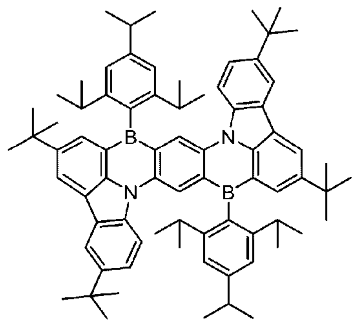

EM1

Industrial Applicability

The organic light-emitting device using the compound represented by the general formula (1) has good light emission characteristics. Therefore, the present invention has high industrial applicability.

## Claims

1. An organic light-emitting device comprising a compound represented by the following general formula (1) and a host material or a dopant material in the same layer:

General Formula (1)

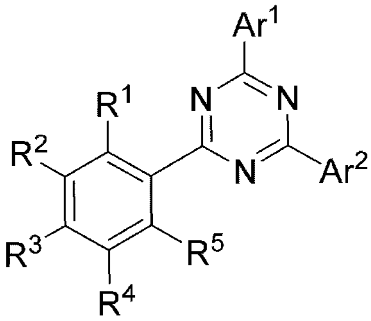

wherein $R^1$ to $R^5$ each independently represent a hydrogen atom, a deuterium atom, a substituted or unsubstituted alkyl group, a substituted or unsubstituted aryl group, a donor group, or a group represented by the following formula (2); $R^2$ or $R^3$ is a group represented by the following general formula (2), and two or more of $R^1$ to $R^5$ are donor groups; $Ar^1$ and $Ar^2$ each independently represent a substituted or unsubstituted aryl group, or a substituted or unsubstituted heteroaryl group;

General Formula (2)

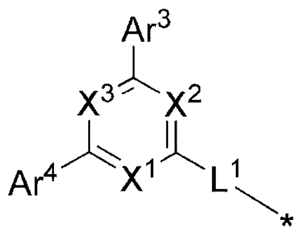

wherein $X^1$ to $X^3$ each independently represent N or C(R), and at least one of $X^1$ to $X^3$ is N; R represents a hydrogen atom, a deuterium atom, or a substituent; $Ar^3$ and $Ar^4$ each independently represent a substituted or unsubstituted aryl group, or a substituted or unsubstituted heteroaryl group; $L^1$ represents a single bond or a divalent linking group; * represents a bonding position;
provided that, in the compound represented by the general formula (1), at least one of $R^1$ to $R^5$ is a donor group

having a fused ring structure of four or more rings, at least one of $Ar^1$ to $Ar^4$ is a substituted or unsubstituted heteroaryl group (excluding a nitrogen-containing 6-membered ring group), or $R^1$ to $R^5$ and $Ar^1$ to $Ar^4$ satisfy both of these conditions.

**2.** The organic light-emitting device according to claim 1, wherein at least one of $R^1$ to $R^5$ in the general formula (1) is a donor group having a fused ring structure of four or more rings.

**3.** The organic light-emitting device according to claim 2, wherein at least one of $R^1$ to $R^5$ in the general formula (1) is a donor group represented by the following general formula (3):

General Formula (3)

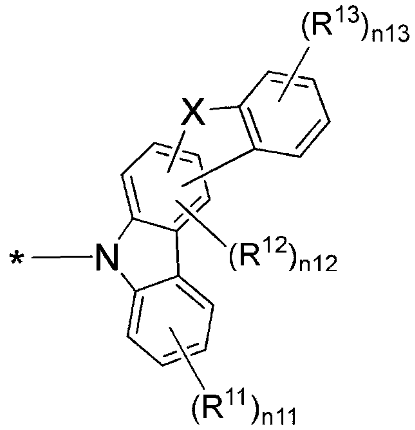

wherein X represents O, S or N-$R^{14}$; $R^{11}$ to $R^{13}$ each independently represent a deuterium atom, or a substituent; $R^{14}$ represents an aryl group optionally substituted with one or more selected from the group consisting of a deuterium atom, an alkyl group and an aryl group, or an alkyl group optionally substituted with one or more selected from the group consisting of a deuterium atom and an aryl group; $R^{11}$'s, $R^{12}$'s, and $R^{13}$'s each may be bonded to each other to form a cyclic structure; n11 and n13 each independently represent an integer of 0 to 4, and n12 represents an integer of 0 to 2.

**4.** The compound according to claim 2, wherein $Ar^1$ to $Ar^4$ in the general formulae (1) and (2) are each independently a substituted or unsubstituted aryl group.

**5.** The organic light-emitting device according to claim 1, wherein at least one of $Ar^1$ to $Ar^4$ in the general formulae (1) and (2) is a substituted or unsubstituted heteroaryl group (excluding a nitrogen-containing 6-membered ring group).

**6.** The organic light-emitting device according to claim 5, wherein at least one of $Ar^1$ to $Ar^4$ in the general formulae (1) and (2) is a heteroaryl group having a 5-membered ring bonded via a nitrogen atom.

**7.** The organic light-emitting device according to claim 1, wherein $R^2$ in the general formula (1) is a group represented by the general formula (2).

**8.** The organic light-emitting device according to claim 1, wherein $R^3$ in the general formula (1) is a group represented by the general formula (2).

**9.** The organic light-emitting device according to claim 1, wherein the donor group is a substituted or unsubstituted carbazol-9-yl group.

**10.** The organic light-emitting device according to claim 1, wherein three of $R^1$ to $R^5$ in the general formula (1) are donor groups.

**11.** The organic light-emitting device according to claim 1, wherein $X^1$ to $X^3$ in the general formula (2) are N.

**12.** The organic light-emitting device according to claim 1, wherein $L^1$ in the general formula (2) is a single bond.

**13.** The organic light-emitting device according to claim 1, wherein $R^1$ in the general formula (1) is a hydrogen atom.

**14.** The organic light-emitting device according to claim 1, wherein the compound has at least one deuterium atom.

**15.** The organic light-emitting device according to any one of claims 1 to 14, wherein the layer comprises the dopant material.

**16.** The organic light-emitting device according to claim 15, wherein an amount of light emitted from the dopant material is larger than an amount of light emitted from the compound.

**17.** The organic light-emitting device according to claim 15, wherein the dopant material is a compound represented by the following general formula (4):

General Formula (4)

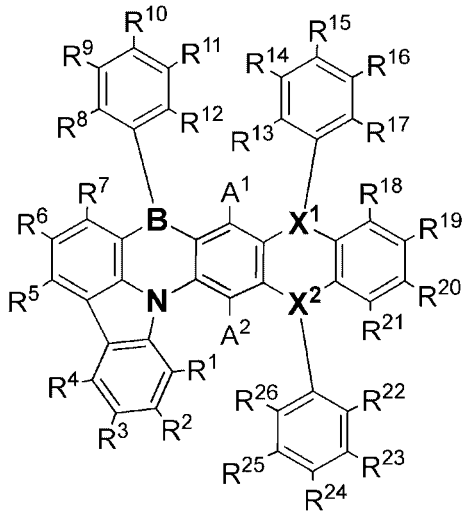

in which one of $X^1$ and $X^2$ is a nitrogen atom, and the other is a boron atom; $R^1$ to $R^{26}$, $A^1$ and $A^2$ each independently represent a hydrogen atom, a deuterium atom, or a substituent; $R^1$ and $R^2$, $R^2$ and $R^3$, $R^3$ and $R^4$, $R^4$ and $R^5$, $R^5$ and $R^6$, $R^6$ and $R^7$, $R^7$ and $R^8$ $R^8$ and $R^9$, $R^9$ and $R^{10}$, $R^{10}$ and $R^{11}$, $R^{11}$ and $R^{12}$, $R^{13}$ and $R^{14}$, $R^{14}$ and $R^{15}$, $R^{15}$ and $R^{16}$, $R^{16}$ and $R^{17}$, $R^{17}$ and $R^{18}$, $R^{18}$ and $R^{19}$, $R^{19}$ and $R^{20}$, $R^{20}$ and $R^{21}$, $R^{21}$ and $R^{12}$, $R^{12}$ and $R^{23}$, $R^{23}$ and $R^{24}$, $R^{24}$ and $R^{25}$, and $R^{25}$ and $R^{26}$ each may be bonded to each other to form a cyclic structure; provided that when $X^1$ is a nitrogen atom, $R^{17}$ and $R^{18}$ are bonded to each other to form a single bond and to form a pyrrole ring, and when $X^2$ is a nitrogen atom, $R^{21}$ and $R^{22}$ are bonded to each other to form a single bond and to form a pyrrole ring; provided that when $X^1$ is a nitrogen atom, $R^7$ and $R^8$, and $R^{21}$ and $R^{22}$ are each bonded via a nitrogen atom to form a 6-membered ring, and $R^{17}$ and $R^{18}$ are bonded to each other to form a single bond, at least one of $R^1$ to $R^6$ is a substituted or unsubstituted aryl group, or any one of $R^1$ and $R^2$, $R^2$ and $R^3$, $R^3$ and $R^4$, $R^4$ and $R^5$, or $R^5$ and $R^6$ is bonded to each other to form an aromatic ring or a heteroaromatic ring; when $X^1$ is a boron atom, $X^2$ is a nitrogen atom, and $R^7$ and $R^8$, and $R^{17}$ and $R^{18}$ are each bonded to each other to form a cyclic structure containing a boron atom, the cyclic structure is a 5- to 7-membered ring, and in the case of a 6-membered ring, $R^7$ and $R^8$, and $R^{17}$ and $R^{18}$ are each bonded to each other to form -B($R^{32}$)-, -CO-, -CS-, or -N($R^{27}$)-, and $R^{27}$ represents a hydrogen atom, a deuterium atom, or a substituent.

**18.** The organic light-emitting device according to any one of claims 1 to 14, wherein the layer comprises the host material.

**19.** The organic light-emitting device according to claim 18, wherein an amount of light emitted from the compound is largest among the materials contained in the layer.

**20.** The organic light-emitting device according to claim 18, wherein the host material is a compound represented by the following general formula (5):

General Formula (5)

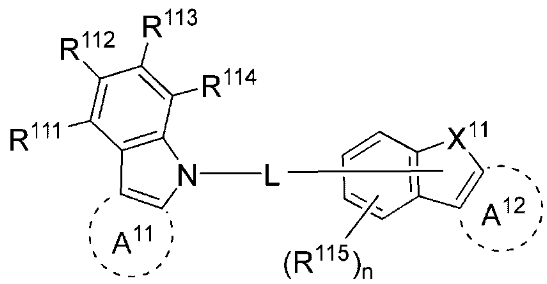

General formula (5) wherein $X^{11}$ represents O, S, N($R^A$), or C($R^B$)($R^C$); $A^{11}$ and $A^{12}$ each independently represent a benzene ring, a furan ring, a thiol ring, a pyrrole ring, or a cyclopentadiene ring, which may be further fused with another ring or substituted; $R^{111}$ to $R^{114}$, $R^B$, and $R^C$ each independently represent a hydrogen atom, a deuterium atom, a substituted or unsubstituted aryl group, a substituted or unsubstituted heteroaryl group, a substituted or unsubstituted alkyl group, or a cyano group; each of $R^{115}$'s independently represents a hydrogen atom, a deuterium atom, a substituted or unsubstituted aryl group, a substituted or unsubstituted heteroaryl group, a substituted or unsubstituted alkyl group, a cyano group, or a bond with L; $R^A$ represents a substituted or unsubstituted aryl group, a substituted or unsubstituted heteroaryl group, a substituted or unsubstituted alkyl group, or a bond with L; $R^{111}$ and $R^{112}$, $R^{112}$ and $R^{113}$, $R^{113}$ and $R^{114}$, two adjacent $R^{115}$'s, and $R^B$ and $R^C$ may be bonded to each other to form a cyclic structure; n represents an integer of 3 or 4; and L represents a single bond, a substituted or unsubstituted arylene group, a substituted or unsubstituted heteroarylene group, or a linking group in which two or more of these groups are bonded.

**21.** The organic light-emitting device according to any one of claims 1 to 14, which emits delayed fluorescence.

**INTERNATIONAL SEARCH REPORT**

International application No.
**PCT/JP2024/012428**

**A. CLASSIFICATION OF SUBJECT MATTER**

***H10K 50/12***(2023.01)i; ***C07D 403/14***(2006.01)i; ***C07D 491/048***(2006.01)i; ***C09K 11/06***(2006.01)i; ***H10K 85/60***(2023.01)i; *H10K 101/20*(2023.01)n; *H10K 101/40*(2023.01)n
FI: H10K50/12; C07D403/14; C07D491/048; C09K11/06 660; C09K11/06 690; H10K85/60; H10K101:20; H10K101:40

According to International Patent Classification (IPC) or to both national classification and IPC

**B. FIELDS SEARCHED**

Minimum documentation searched (classification system followed by classification symbols)

H10K50/00-H10K102/20; C07D403/14; C07D491/048; C09K11/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2018/237385 A1 (KYULUX, INC.) 27 December 2018 (2018-12-27)<br>claims, p. 29, line 13 to p. 69, line 1 | 1-21 |
| A | WO 2020/189117 A1 (KONICA MINOLTA, INC.) 24 September 2020 (2020-09-24)<br>paragraphs [0036]-[0038], [0128], [0135] | 1-21 |
| A | JP 2022-500481 A (LG CHEM, LTD.) 04 January 2022 (2022-01-04)<br>entire text | 1-21 |
| A | JP 2022-501333 A (LG CHEM, LTD.) 06 January 2022 (2022-01-06)<br>entire text | 1-21 |
| A | WO 2021/040467 A1 (LG CHEM, LTD.) 04 March 2021 (2021-03-04)<br>entire text | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 May 2024** | **04 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/012428**

**C. DOCUMENTS CONSIDERED TO BE RELEVANT**

| Category* | Citation of document, with indication, where appropriate, of the relevant passages | Relevant to claim No. |
|---|---|---|
| A | KR 10-2018-0047306 A (RESEARCH & BUSINESS FOUNDATION SUNGKYUNKWAN UNIVERSITY) 10 May 2018 (2018-05-10)<br>entire text | 1-21 |
| A | CN 105481845 A (SHANGHAI TIANMA ORGANIC LIGHT-EMITTING DISPLAY TECHNOLOGY CO., LTD.) 13 April 2016 (2016-04-13)<br>whole document | 1-21 |
| P, X | CN 115894457 A (BEIJING ETERNAL MATERIAL TECHNOLOGY CO., LTD.) 04 April 2023 (2023-04-04)<br>claims, paragraphs [0007]-[0113], [0133]-[0142], [0160]-[0173] | 1-2, 4, 7-13, 15, 18-21 |
| P, A | | 3, 5-6, 14, 16-17 |

Form PCT/ISA/210 (second sheet) (July 2022)

**INTERNATIONAL SEARCH REPORT**
**Information on patent family members**

International application No.
**PCT/JP2024/012428**

| Patent document cited in search report | Publication date (day/month/year) | Patent family member(s) | Publication date (day/month/year) |
|---|---|---|---|
| WO 2018/237385 A1 | 27 December 2018 | US 2020/0115364 A1 | |
| | | US 2020/0115376 A1 | |
| | | US 2020/0119287 A1 | |
| | | WO 2018/237389 A1 | |
| | | WO 2018/237393 A1 | |
| | | EP 3642303 A1 | |
| | | KR 10-2020-0019694 A | |
| | | CN 110914378 A | |
| | | KR 10-2020-0023371 A | |
| | | JP 2020-525438 A | |
| | | JP 2020-525437 A | |
| WO 2020/189117 A1 | 24 September 2020 | US 2022/0185824 A1 paragraphs [0051]-[0055], [0109], D-144, D-145, D-162, D-163 | |
| JP 2022-500481 A | 04 January 2022 | US 2021/0355128 A1 | |
| | | US 2021/0363132 A1 | |
| | | US 2021/0399232 A1 | |
| | | WO 2020/111673 A1 | |
| | | EP 3842428 A1 | |
| | | KR 10-2020-0063060 A | |
| | | CN 112673004 A | |
| | | TW 202026291 A | |
| JP 2022-501333 A | 06 January 2022 | US 2021/0355128 A1 | |
| | | US 2021/0363132 A1 | |
| | | WO 2020/111780 A1 | |
| | | EP 3842428 A1 | |
| | | EP 3854792 A1 | |
| | | KR 10-2020-0063076 A | |
| | | CN 112673004 A | |
| | | CN 112673005 A | |
| | | TW 202026291 A | |
| | | TW 202026401 A | |
| WO 2021/040467 A1 | 04 March 2021 | KR 10-2021-0027172 A | |
| | | CN 113454078 A | |
| KR 10-2018-0047306 A | 10 May 2018 | (Family: none) | |
| CN 105481845 A | 13 April 2016 | (Family: none) | |
| CN 115894457 A | 04 April 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019191665 A1
- WO 2022168956 A1
- WO 2015022974 A
- WO 2022270354 A1
- WO 2013154064 A
- WO 2013011954 A
- WO 2013011955 A
- WO 2013081088 A
- JP 2013256490 A
- JP 2013116975 A
- WO 2013133359 A
- WO 2013161437 A
- JP 2014009352 A
- JP 2014009224 A
- JP 2017119663 A
- JP 2017119664 A
- JP 2017222623 A
- JP 2017226838 A
- JP 2018100411 A
- WO 2018047853 A
- JP 2013253121 A
- WO 2014034535 A
- WO 2014115743 A
- WO 2014122895 A
- WO 2014126200 A
- WO 2014136758 A
- WO 2014133121 A
- WO 2014136860 A
- WO 2014196585 A
- WO 2014189122 A
- WO 2014168101 A
- WO 2015008580 A
- WO 2014203840 A
- WO 2015002213 A
- WO 2015016200 A
- WO 2015019725 A
- WO 2015072470 A
- WO 2015108049 A
- WO 2015080182 A
- WO 2015072537 A
- WO 2015080183 A
- JP 2015129240 A
- WO 2015129714 A
- WO 2015129715 A
- WO 2015133501 A
- WO 2015136880 A
- WO 2015137244 A
- WO 2015137202 A
- WO 2015137136 A
- WO 2015146541 A
- WO 2015159541 A


### Non-patent literature cited in the description


- **HANSCH, C.** *Chem. Rev.*, 1991, vol. 91, 165-195